# EUROPEAN PATENT SPECIFICATION

(11) **EP 0 527 851 B1**
(45) Date of publication and mention of the grant of the patent: **23.12.1998**
(21) Application number: 91909072.0
(22) Date of filing: 06.05.1991
(51) Int. Cl.: C07D 249/08, C07D 249/10, C07D 249/12, C07D 403/10, C07D 409/10, C07D 409/14, A61K 31/41

(54) **1H-SUBSTITUTED-1,2,4-TRIAZOLE COMPOUNDS FOR TREATMENT OF CARDIOVASCULAR DISORDERS**
1H-SUBSTITUIERTE-1,2,4-TRIAZOLVERBINDUNGEN ZUR BEHANDLUNG VON HERZKRANKHEITEN
COMPOSES DE 1,2,4-TRIAZOLE A SUBSTITUTION 1H UTILISES DANS LE TRAITEMENT DE TROUBLES CARDIOVASCULAIRES

(30) Priority: 04.05.1990 US 519380
(43) Date of publication of application: 24.02.1993
(73) Proprietor: G.D. SEARLE & CO., Chicago IL 60680-5110 (US)
(72) Inventor: REITZ, David, B., Chesterfield, MO 63017 (US)
(74) Representative: Beil, Hans Chr., Dr.
(86) International application number: PCT/US91/02926
(87) International publication number: WO 91/17148

(56) References cited:
- EP-A- 0 253 310
- EP-A- 0 289 919
- EP-A- 0 323 841
- EP-A- 0 409 332

## Description

### Field of the Invention

Non-peptidic 1H-substituted-1,2,4-triazole compounds are described for use in treatment of cardiovascular disorders such as hypertension and congestive heart failure. Of particular interest are angiotensin II antagonist compounds provided by 1,2,4-triazoles having a biphenylmethyl moiety attached to the nitrogen atom at the one-position of the 1,2,4-triazole.

### Background of the Invention

The renin-angiotensin system is one of the hormonal mechanisms involved in regulation of pressure/volume homeostasis and in expression of hypertension. Activation of the renin-angiotensin cascade begins with renin secretion from the juxtaglomerular apparatus of the kidney and culminates in the formation of angiotensin II, the primary active species of this system. This octapeptide, angiotensin II, is a potent vasoconstrictor agent and also produces other physiological effects such as promoting aldosterone secretion, promoting sodium and fluid retention, inhibiting renin secretion, increasing sympathetic nervous system activity, increasing vasopressin secretion, causing positive cardiac inotropic effect and modulating other hormonal systems.

Previous studies have shown that antagonizing angiotensin II at its receptors is a viable approach to inhibit the renin-angiotensin system, given the pivotal role of this octapeptide which mediates the actions of the renin-angiotensin system through interaction with various tissue receptors. There are several known angiotensin II antagonists, most of which are peptidic in nature. Such peptidic compounds are of limited use due to their lack of oral bioavailability or their short duration of action. Also, commercially-available peptidic angiotensin II antagonists (e.g., Saralasin) have a significant residual agonist activity which further limit their therapeutic application.

Non-peptidic compounds with angiotensin II antagonist properties are known. For example, the sodium salt of 2-n-butyl-4-chloro-1-(2-chlorobenzyl)imidazole-5-acetic acid has specific competitive angiotensin II antagonist activity as shown in a series of binding experiments, functional assays and in vivo tests [P. C. Wong et al, J. Pharmacol. Exp. Ther., 247 (1), 1-7 (1988)]. Also, the sodium salt of 2-butyl-4-chloro-1-(2-nitrobenzyl)imidazole-5-acetic acid has specific competitive angiotensin II antagonist activity as shown in a series of binding experiments, functional assays and in vivo tests [A. T. Chiu et al, European J. Pharmacol., 157, 3121 (1988)]. A family of 1-benzylimidazole-5-acetate derivatives has been shown to have competitive angiotensin II antagonist properties [A. T. Chiu et al, J. Pharmacol. Exp. Ther., 250 (3), 867-874 (1989)]. US-A-4,816,463 to Blankey et al describes a family of 4,5,6,7-tetrahydro-1H-imidazo (4, 5-c)-tetrahydro-pyridine derivatives useful as antihypertensives, some of which are reported to antagonize the binding of labelled angiotensin II to rat adrenal receptor preparation and thus cause a significant decrease in mean arterial blood pressure in conscious hypertensive rats. EP-A- 253,310, published 20 january 1988, describes a series of aralkyl imidazole compounds, including in particular a family of biphenylmethyl substituted imidazoles, as antagonists to the angiotensin II receptor. EP-A- 323,841 published 12 July 1989 describes four classes of angiotensin II antagonists, namely, biphenylmethylpyrroles, biphenylmethylpyrazoles, biphenylmethyl-1,2,3-triazoles and biphenylmethyl 4-substituted-4H-1,2,4-triazoles, including the compound 3,5-dibutyl-4-[(2'-carboxybiphenyl-4-yl)methyl]-4H-1,2,4-triazole. US-A- 4,880,804 to Carini et al describes a family of biphenylmethylbenzimidazole compounds as angiotensin II receptor blockers for use in treatment of hypertension and congestive heart failure.

There are several families of 1,2,4-triazole compounds having substituents attached to the nitrogen atom at the one-position of the 1H-triazole. For example, US-A- 4,118,487 to Regel et al describes a family azol-1-ylmethane compounds for use as antimycotic and antibacterial agents including, specifically, the compound (1-biphenyl-4-yl-1-phenyl)methyl-1H-1,2,4-triazole. US-A- 4,381,306 to Regel et al describes a family of hydroxypropyl-triazole compounds for use as antimycotic agents including, specifically, the compound (1,2,4-triazol-1-yl)methyl-4-chlorobenzyl-biphenyl-4-ylcarbinol. US-A- 4,480,114 to Regel describes a family of 2-(4-biphenyl)-2-(halophenyl)-oxirane compounds having antimycotic activity including, specifically, the compound (1,2,4-triazol-1-yl)methyl-4-chlorophenyl-4-chlorobiphenyl-4-yl carbinol.

EP-A-0 323 841 discloses pyrrol-1-yl, pyrazol-1-yl, 1,2,3-triazol-1-yl and 1,2,4-triazol-4-yl derivatives being useful as angiotensine II antagonists.

EP-A-0 289 919 describes azol-1-yl derivatives including 1,2,4-triazol-2-yl which is linked to phenoxy phenyl by a CH₂-O-group. EP-A-0 253 310 is directed to imidazol-1-yl derivatives which are useful as angiotensine II antagonists. EP-A-0 409 332 (published after the present priority) discloses 1,2,4-triazol-4-yl compounds.

### DESCRIPTION OF THE INVENTION

A class of 1H-substituted-1,2,4-triazole compounds useful in treating circulatory and cardiovascular disorders is defined by Formula I: 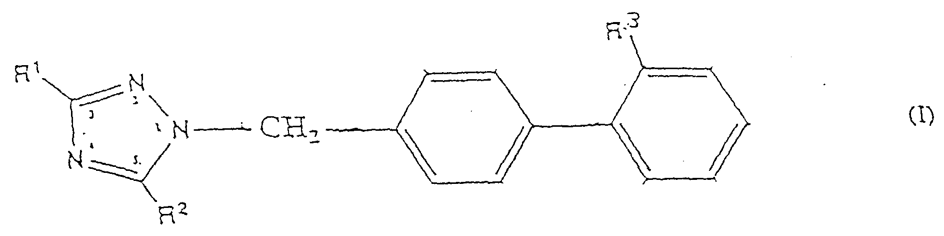 wherein each of R¹ and R² is independently selected from hydrogen, C₁-C₂₀ alkyl, phenyl, phenyl substituted by C₁-C₂₀ alkyl, C₃-C₁₀ cycloalkyl, C₃-C₁₀ cycloalkyl, attached to or substituted with C₁-C₂₀ alkyl, C₁-C₁₀ alkoxy, C₁-C₂₀-di-fluoroalkyl substituted by phenyl, C₂-C₂₀ alkenyl, C₂-C₂₀ alkynyl, C₁-C₂₀ alkylcarbonyl; difluoro-substituted C₁-C₂₀ alkyl; C₁-C₁₀-hydroxyalkyl, C₁-C₂₀ alkyl substituted by one or more alkoxy groups having alkyl portions of 1 to 10 carbon atoms; wherein R³ is an acidic moiety selected from CO₂H, CONHNHSO₂CF₃, 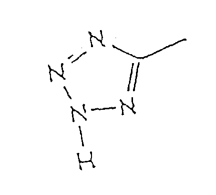 and the salts of said acidic moieties; or a tautomer thereof or a pharmaceutically-acceptable salt thereof.

Compounds of Formula I would be useful in treating a variety of circulatory disorders including cardiovascular disorders, such as hypertension, congestive heart failure and arteriosclerosis, and to treat other disorders such as glaucoma. These compounds would also be useful as adjunctive therapies. For example, compounds of Formula I may be used in combination with other drugs, such as a diuretic, to treat hypertension. Also, compounds of Formula I could be used in conjunction with certain surgical procedures. For example, these compounds could be used to prevent post-angioplasty re-stenosis. Compounds of Formula I are therapeutically effective in treatment of cardiovascular disorders by acting as antagonists to, or blockers of, the angiotensin II (AII) receptor. Compounds of Formula I would be therapeutically effective in treatment of the above-mentioned circulatory and cardiovascular disorders or would be precursors to, or prodrugs of, therapeutically-effective compounds.

Preferred compounds of Formula I are all characterized in having a substituent, other than hydrido, attached to one or both of the three- and five-positions of the triazole ring. Such substituents are selected from the aforementioned R¹ and R² groups. Compounds having C₁₋₂₀ alkyl groups, especially lower alkyl groups, at both of the R¹ and R² positions are particularly useful as angiotensin II antagonists. Also especially useful are compounds having one of the R¹ and R² substituents selected from C₁₋₂₀ alkylcarbonyl, mono C₁₋₁₀ alkoxy C₁₋₂₀ alkyl, di C₁₋₁₀ alkoxy C₁₋₂₀ alkyl and difluoro-substituted C₁₋₂₀ alkyl groups. When the selected substituent for R¹ and R² is difluoro C₁₋₂₀ alkyl, then it is particularly useful for both of the fluoro atoms of the difluoroalkyl group to be substituted on the difluoroalkyl group carbon atom attached at the R¹ or R² positions of the triazole ring. Such difluoroalkyl group may be characterized as an "alpha-carbon difluoro-substituted difluoroalkyl group", or as an "alpha,alpha-difluoro-substituted alkyl group". When the selected substituent for R¹ or R² is monoalkoxyalkyl or dialkoxyalkyl, then it is particularly useful for the single alkoxy group or the two alkoxy groups, respectively, to be substituted on the carbon atom of the selected substituent which is attached at the R¹ or R² positions of the triazole ring. Such alkoxyalkyl groups may be characterized as "alpha-carbon monoalkoxy- or dialkoxy-substituted alkoxyalkyl groups", respectively, or "alpha-monoalkoxy-substituted or alpha,alpha-dialkoxy-substituted alkyl groups", respectively. When the selected substituent is C₁₋₂₀ alkylcarbonyl, then it is particularly useful for the carbonyl group to be attached at the R¹ or R² positions of the triazole ring. Such alkylcarbonyl group may be characterized as an "alpha-oxo-substituted alkyl group", and may be exemplified by the substituents 1-oxoethyl, 1-oxopropyl and 1-oxobutyl. Where compounds of Formula I contain any of these above-mentioned particularly-useful alpha-carbon substituted R¹ or R² groups at the triazole ring three- or five-position, it is preferred that such particularly-useful group be attached at the three-position, that is, as an R¹ substituent.

Typically, the acidic group defined as R³ should has a proton-donor capability such that the product compound of Formula I has a pKₐ in a range from one to twelve. More typically, the Formula I compound would have a pKₐ in a range from two to seven.

The amide of compounds wherein R³ = COOH and the salts of said acidic moieties; or a tautomer thereof or a pharmaceutically-acceptable salt thereof, are useful in the preparation of pharmaceutical compositions as medicaments.

A group of preferred compounds are those wherein R³ is selected from COOH or tetrazolyl.

The term "hydrido" denotes a single hydrogen atom (H). This hydrido group may be attached, for example, to an oxygen atom to form a hydroxyl group; or, as another example, one hydrido group may be attached to a carbon atom to form a 〉CH- group; or, as another example, two hydrido groups may be attached to a carbon atom to form a -CH₂- group. Where the term "alkyl" is used, either alone or within other terms such as "haloalkyl" and "hydroxyalkyl", the term "alkyl" embraces linear or branched radicals and has one to twenty carbon atoms or, preferably, one to twelve carbon atoms. More preferred alkyl radicals are "lower alkyl" radicals having one to ten carbon atoms. Most preferred are lower alkyl radicals having one to five carbon atoms. The term "cycloalkyl" embraces mono-carbocyclic saturated radicals and has three to ten ring carbon atoms, preferably three to six carbon atoms, such as cyclopropyl, cyclobutyl, cyclopentyl and cyclohexyl. The term "cycloalkylalkyl" denotes a cycloalkyl radical attached to an alkyl radical which is attachable to a substitutable position of Formula I. Examples of "cycloalkylalkyl" radicals are cyclopentylmethyl and cyclohexylethyl. The term "difluoroalkyl" embraces alkyl groups having two fluoro atoms substituted on any one or two of the alkyl group carbon atoms. The terms "alkylol" and "hydroxyalkyl" embrace linear or branched alkyl group which have one to ten carbon atoms any one of which may be substituted with one or more hydroxyl groups. The term "alkenyl" embraces linear or branched radicals which have two to twenty carbon atoms, preferably three to ten carbon atoms, and containing at least one carbon-carbon double bond, which carbon-carbon double bond may have either cis or trans geometry within the alkenyl moiety. The term "alkynyl" embraces linear or branched radicals which has two to twenty carbon atoms, preferably two to ten carbon atoms, and containing at least one carbon-carbon triple bond. The terms "alkoxy" and "alkoxyalkyl" embrace linear or branched oxy-containing radicals each having alkyl portions of one to ten carbon atoms. An example of alkoxy is methoxy group. The term "alkoxyalkyl" also embraces alkyl radicals having two or more alkoxy groups attached to an alkyl radical. The term "dialkoxyalkyl" is exemplified by dimethoxymethyl and 2,2-diethoxyethyl.

For any of the foregoing defined radicals, preferred radicals are those containing from one to about ten carbon atoms.

Specific examples of alkyl groups are methyl, ethyl, n-propyl, isopropyl, n-butyl, sec-butyl, isobutyl, tert-butyl, n-pentyl, isopentyl, methylbutyl, dimethylbutyl and neopentyl. Typical alkenyl and alkynyl groups may have one unsaturated bond, such as an allyl group, or may have a plurality of unsaturated bonds, with such plurality of bonds either adjacent, such as allene-type structures, or in conjugation, or separated by several saturated carbons.

Compounds of Formula I have been found to inhibit the action of angiotensin II in mammals. Angiotensin II is a potent vasoconstrictor and participates in the formation of aldosterone which regulates sodium and water balance in mammals. Thus, compounds of Formula I are therapeutically useful in methods for treating hypertension by administering to a hypertensive subject or patient a therapeutically-effective amount of a compound of Formula I. The phrase "hypertensive subject or patient" means, in this context, a mammalian subject suffering from or afflicted by the effects of hypertension or susceptible to a hypertensive condition if not treated to prevent or control such hypertension.

Included within the family of compounds of Formula I are the tautomeric forms of the described compounds, as well as the stereoisomers and pharmaceutically-acceptable salts thereof. The term "pharmaceutically-acceptable salts" embraces salts commonly used to form alkali metal salts and to form addition salts of free acids or free bases. The nature of the salt is not critical, provided that it is pharmaceutically-acceptable. Suitable pharmaceutically-acceptable acid addition salts of compounds of Formula I may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, example of which are formic, acetic, propionic, succinic, glycolic, gluconic, lactic, malic, tartaric, citric, ascorbic, glucuronic, maleic, fumaric, pyruvic, aspartic, glutamic, benzoic, anthranilic, p-hydroxybenzoic, salicyclic, phenylacetic, mandelic, embonic (pamoic), methansulfonic, ethane sulfonic, 2-hydroxyethanesulfonic, pantothenic, benzenesulfonic, toluenesulfonic, sulfanilic, mesylic, cyclohexylaminosulfonic, stearic, algenic, β-hydroxy butyric, malonic, galactaric and galacturonic acid. Suitable pharmaceutically-acceptable base addition salts of compounds of Formula I include metallic salts made from aluminium, calcium, lithium, magnesium, potassium, sodium and zinc or organic salts made from N,N'-dibenzylethylenediamine, chloroprocaine, choline, diethanolamine, ethylenediamine, meglumine (N-methylglucamine) and procaine. All of these salts may be prepared by conventional means from the corresponding compound of Formula I by reacting, for example, the appropriate acid or base with the compound of Formula I.

Of particular interest are the following compounds:
4'-[(3,5-dibutyl-1H-1,2,4-triazol-1-yl) methyl][1,1'-biphenyl]-2-carboxylic acid, 2-[(trifluoromethyl)sulfonyl]hydrazide;
and the specific compounds within Formula I, wherein R³ is COOH acid:
4'-[(5-butyl-3-propyl-1H-1,2,4-triazol-1-yl)methyl][1,1'-biphenyl]-2-carboxylic acid;
4'-[(3,5-dibutyl-1H-1,2,4-triazol-1-yl)methyl][1,1'-biphenyl]-2-carboxylic acid;
4'-[(5-butyl-3-isopencyl-1H-1,2,4-triazol-1-yl)methyl][1,1'-biphenyl]-2-carboxylic acid;
4'-[[5-butyl-3-(1,1-dimethoxybutyl)-1H-1,2,4-triazol-1-yl]methyl][1,1'-biphenyl]-2-carboxylic acid;
4'-[[5-butyl-3-(1-oxobutyl)-1H-1,2,4-triazol-1-yl]methyl][1,1'-biphenyl]-2-carboxylic acid;
4'-[[5-butyl-3-(1-oxopentyl)-1H-1,2,4-triazol-1-yl]methyl][1,1'-biphenyl]-2-carboxylic acid;
4'-[[5-butyl-3-(1,1-difluoroethyl)-1H-1,2,4-triazol-1-yl]methyl][1,1'-biphenyl]-2-carboxylic acid;
4'-[[5-butyl-3-(1,1-difluoropropyl)-1H-1,2,4-triazol-1-yl]methyl][1,1'-biphenyl]-2-carboxylic acid;
4'-[[5-butyl-3-(1,1-difluorobutyl)-1H-1,2,4-triazol-1-yl]methyl][1,1'-biphenyl]-2-carboxylic acid;
4'-[[3-butyl-5-(1,1-difluorobutyl)-1H-1,2,4-triazol-1-yl]methyl][1,1'-biphenyl]-2-carboxylic acid;
4'-[[5-butyl-3-(1,1-difluoropentyl)-1H-1,2,4-triazol-1-yl]methyl][1,1'-biphenyl]-2-carboxylic acid;
4'-[(3,5-dipropyl-1H-1,2,4-triazol-1-yl)methyl][1,1'-biphenyl]-2-carboxylic acid;
4'-[(3,5-isopropyl-1H-1,2,4-triazol-1-yl)methyl][1,1'-biphenyl]-2-carboxylic acid;
4'-[(5-propyl-3-butyl-1H-1,2,4-triazol-1-yl)methyl][1,1'-biphenyl]-2-carboxylic acid;
4'-[[5-propyl-3-(1,1-difluoroethyl)-1H-1,2,4-triazol-1-yl]methyl][1,1'-biphenyl]-2-carboxylic acid;
4'-[[5-propyl-3-(1,1-difluoropropyl)-1H-1,2,4-triazol-1-yl]methyl][1,1'-biphenyl]-2-carboxylic acid;
4'-[[5-propyl-3-(1,1-difluorobutyl)-1H-1,2,4-triazol-1-yl]methyl][1,1'-biphenyl]-2-carboxylic acid;
4'-[[5-propyl-3-(1,1-difluoropentyl)-1H-1,2,4-triazol-1-yl]methyl][1,1'-bipheny]-2-carboxylic acid;
4'-[[5-ethyl-3-(1,1-difluorobutyl)-1H-1,2,4-triazol-1-yl]methyl][1,1'-biphenyl]-2-carboxylic acid;
4'-[(3,5-disecbutyl-1H-1,2,4-triazol-1-yl)methyl][1,1'-biphenyl]-2-carboxylic acid;
4'-[[5-secbutyl-3-(1,1-difluorobutyl)-1H-1,2,4-triazol-1-yl]methyl][1,1'-biphenyl]-2-carboxylic acid;
4'-[(3,5-diisobutyl-1H-1,2,4-triazol-1-yl)methyl][1,1'-biphenyl]-2-carboxylic acid;
4'-[[5-isobutyl-3-(1,1-difluorobutyl)-1H-1,2,4-triazol-1-yl]methyl][1,1'-biphenyl]-2-carboxylic acid;
4'-[(3,5-ditertbutyl-1H-1,2,4-triazol-1-yl)methyl][1,1'-biphenyl]-2-carboxylic acid;
4'-[[5-tertbutyl-3-(1,1-difluorobutyl)-1H-1,2,4-triazol-1-yl]methyl][1,1'-biphenyl]-2-carboxylic acid;
4'-[(3,5-dipentyl-1H-1,2,4-triazol-1-yl)methyl][1,1'-biphenyl]-2-carboxylic acid;
4'-[[5-pentyl-3-(1,1-difluorobutyl)-1H-1,2,4-triazol-1-yl]methyl][1,1'-biphenyl]-2-carboxylic acid;
4'-[(3,5-diisopentyl-1H-1,2,4-triazol-1-yl)methyl][1,1'-biphenyl]-2-carboxylic acid;
4'-[[5-isopentyl-3-(1,1-difluorobutyl]-1H-1,2,4-triazol-1-yl]methyl][1,1'-biphenyl]-2-carboxylic acid;
4'-[[5-(1-butenyl)-3-(1,1-difluorobutyl)-1H-1,2,4-triazol-1-yl]methyl][1,1'-biphenyl]-2-carboxylic acid;
4'-[[5-(2-butenyl)-3-(1,1-difluorobutyl)-1H-1,2,4-triazol-1-yl]methyl][1,1'-biphenyl]-2-carboxylic acid;
4'-[[5-(3-butenyl)-3-(1,1-difluorobutyl)-1H-1,2,4-triazol-1-yl]methyl][1,1'-biphenyl]-2-carboxylic acid;
4'-[[5-(1-butynyl)-3-(1,1-difluorobutyl)-1H-1,2,4-triazol-1-yl]methyl][1,1'-biphenyl]-2-carboxylic acid;
4'-[[5-(2-butynyl)-3-(1,1-difluorobutyl)-1H-1,2,4-triazol-1-yl]methyl][1,1'-biphenyl]-2-carboxylic acid;
4'-[[5-(3-butynyl)-3-(1,1-difluorobutyl)-1H-1,2,4-triazol-1-yl]methyl][1,1'-biphenyl]-2-carboxylic acid;
4'-[(3,5-diethoxy-1H-1,2,4-triazol-1-yl)methyl][1,1'-biphenyl]-2-carboxylic acid;
4'-[(3,5-dipropoxy-1H-1,2,4-triazol-1-yl)methyl][1,1'-biphenyl]-2-carboxylic acid;
4'-[(3,5-diisopropoxy-1H-1,2,4-triazol-1-yl)methyl][1,1'-biphenyl]-2-carboxylic acid;
4'-[(3,5-dibutoxy-1H-1,2,4-triazol-1-yl)methyl][1,1'-biphenyl]-2-carboxylic acid;
4'-[[3,5-di(1-butenyl)-1H-1,2,4-triazol-1-yl]methyl][1,1'-biphenyl]-2-carboxylic acid;
4'-[[3,5-di(2-butenyl)-1H-1,2,4-triazol-1-yl]methyl][1,1'-biphenyl]-2-carboxylic acid;
4'-[[3,5-di(3-butenyl)-1H-1,2,4-triazol-1-yl]methyl][1,1'-biphenyl]-2-carboxylic acid;
4'-[[3,5-di(1-butynyl)-1H-1,2,4-triazol-1-yl]methyl][1,1'-biphenyl]-2-carboxylic acid;
4'-[[3,5-di(2-butynyl)-1H-1,2,4-triazol-1-yl]methyl][1,1'-biphenyl]-2-carboxylic acid;
4'-[[3,5-di(3-butynyl)-1H-1,2,4-triazol-1-yl]methyl][1,1'-biphenyl]-2-carboxylic acid.

A further group of compounds of formula I and their pharmaceutically-acceptable salts, wherein R³ is tetrazolyl, is the following group:
5-[4'-[(5-butyl-3-propyl-1H-1,2,4-triazol-1-yl)methyl][1,1'-biphenyl]-2-yl]-1H-tetrazole;
5-[4'-[(3,5-dibutyl-1H-1,2,4-triazol-1-yl) methyl][1,1'-biphenyl]-2-yl]-1H-tetrazole;
5-[4'-[(5-butyl-3-isopentyl-1H-1,2,4-triazol-1-yl-)methyl][1,1'-biphenyl]-2-yl]-1H-tetrazole;
5-[4'-[[5-butyl-3-(1,1 dimethoxybutyl)-1H-1,2,4-triazol-1-yl]methyl][1,1'-biphenyl]-2-yl]-1H-tetrazole;
5-[4'-[[5-butyl-3-(1-oxobutyl)-1H-1,2,4-triazol-1-yl]methyl][1,1'-biphenyl]-2-yl]-1H-tetrazole;
5-[4'-[[5-butyl-3-(1,1-difluoroethyl)-1H-1,2,4-triazol-1-yl]methyl][1,1'-biphenyl]-2-yl]-1H-tetrazole;
5-[4'-[[5-butyl-3-(1,1-difluoropropyl)-1H-1,2,4-triazol-1-yl]methyl][1,1'-biphenyl]-2-yl]-1H-tetrazole;
5-[4'-[[5-butyl-3-(1,1-difluorobutyl)-1H-1,2,4-triazol-1-yl]methyl][1,1'-biphenyl]-2-yl]-1H-tetrazole;
5-[4'-[[3-butyl-5-(1,1-difluorobutyl)-1H-1,2,4-triazol-1-yl]methyl][1,1'-biphenyl]-2-yl]-1H-tetrazole;
5-[4-'-[[5-butyl-3-(1,1-difluoropentyl)-1H-1,2,4-triazol-1-yl]methyl][1,1'-biphenyl]-2-yl]-1H-tetrazole;
5-[4'-[(3,5-dipropyl-1H-1,2,4-triazol-1-yl]methyl][1,1'-biphenyl]-2-yl]-1H-tetrazole;
5-[4'-[(3-butyl-5-propyl-1H-1,2,4-triazol-1-yl)methyl]-[1,1'-biphenyl]-2-yl]-1H-tetrazole;
5-[4'-[(5-butyl-3-isopentyl-1H-1,2,4-triazol-1-yl]methyl)-[1,1'-biphenyl]-2-yl]-1H-tetrazole;
5-[4'-[(3-phenyl-5-butyl-1H-1,2,4-triazol-1-yl)methyl](1,1'-biphenyl)-2-yl]-1H-tetrazole;
5-[4'-[(5-butyl-3-phenylethyl-1H-1,2,4-triazol-1-yl)methyl]-(1,1'-biphenyl)-2-yl]-1H-tetrazole;
5-(4'-[(5-butyl-3-ethyl-1H-1,2,4-triazol-1-yl)methyl][1,1'-biphenyl]-2-yl]-1H-tetrazole;
5-[4'-[(3-butyl-5-ethyl-1H-1,2,4-triazol-1-yl)methyl][1,1'-biphenyl]-2-yl]-1H-tetrazole; glutamine, 1,1-dimethylethyl ester;
5-[4'-[[5-butyl-3-(2-cyclohexylethyl)-1H-1,2,4-triazol-1-yl]-methyl][1,1'-biphenyl]-2-yl]-1H-tetrazole;
5-[4'-[[5-butyl-3-(phenylmethyl)-1H-1,2,4-triazol-1-yl]-methyl][1,1'-biphenyl]-2-yl]-1H-tetrazole;
5-[4'-[(5-butyl-3-cyclohexyl-1H-1,2,4-triazol-1-yl)methyl]-[1,1'-biphenyl]-2-yl]-1H-tetrazole;
5-[4'-[(3-cyclohexylmethyl-5-butyl-1H-1,2,4-triazol-1-yl)-methyl][1,1'-biphenyl]-2-yl]-1H-tetrazole;
5-[4'-[(3-butyl-5-cyclohexyl-1H-1,2,4-triazol-1-yl)methyl]-[1,1'-biphenyl]-2-yl]-1H-tetrazole;
5-[4'-[(3,5-isopropyl-1H-1,2,4-triazol-1-yl)methyl][1,1'-biphenyl]-2-yl]-1H-tetrazole;
5-[4'-[(5-propyl-3-isopentyl-1H-1,2,4-triazol-1-yl)methyl][1,1'-biphenyl]-2-yl]-1H-tetrazole;
5-[4'-[[5-propyl-3-(1,1-dimethoxybutyl)-1H-1,2,4,-triazol-1-yl]methyl][1,1'-biphenyl]-2-yl]-1H-tetrazole;
5-[4'-[[5-propyl-3-(1-oxobutyl)-1H-1,2,4-triazol-1-yl]methyl][1,1'-biphenyl]-2-yl]-1H-tetrazole;
5-[4'-[[5-propyl-3-(1,1-difluoroethyl)-1H-1,2,4-triazol-1-yl]methyl][1,1'-biphenyl]-2-yl]-1H-tetrazole;
5-[4'-[[5-propyl-3-(1,1-difluoropropyl)-1H-1,2,4-triazol-1-yl]methyl][1,1'-biphenyl]-2-yl]-1H-tetrazole;
5-[4'-[[5-propyl-3-(1,1-difluorobutyl)-1H-1,2,4-triazol-1-yl]methyl][1,1'-biphenyl]-2-yl]-1H-tetrazole;
5-[4'-[[5-propyl-3-(1,1-difluoropentyl)-1H-1,2,4-triazol-1-yl]methyl][1,1'-biphenyl]-2-yl]-1H-tetrazole;
5-[4'-[[5-ethyl-3-(1,1-difluorobutyl)-1H-1,2,4-triazol-1-yl]methyl][1,1'-biphenyl]-2-yl]-1H-tetrazole;
5-[4'-[(5-secbutyl-3-propyl-1H-1,2,4-triazol-1-yl)methyl][1,1'-biphenyl]-2-yl]-1H-tetrazole;
5-[4'-[(5-secbutyl-3-butyl-1H-1,2,4-triazol-1-yl)methyl][1,1'-biphenyl]-2-yl]-1H-tetrazole;
5-[4'-[(3,5-disecbutyl-1H-1,2,4-triazol-1-yl)methyl][1,1'-biphenyl]-2-yl]-1H-tetrazole;
5-[4'-[(5-secbutyl-3-isopentyl-1H-1,2,4-triazol-1-yl)methyl][1,1'-biphenyl]-2-yl]-1H-tetrazole;
5-[4'-[[5-secbutyl-3-(1,1-difluoroethyl)-1H-1,2,4-triazol-1-yl]methyl][1,1'-biphenyl]-2-yl]-1H-tetrazole;
5-[4'-[[5-secbutyl-3-(1,1-difluoropropyl)-1H-1,2,4-triazol-1-yl]methyl][1,1'-biphenyl]-2-yl]-1H-tetrazole;
5-[4'-[[5-secbutyl-3-(1,1-difluorobutyl)-1H-1,2,4-triazol-1-yl]methyl][1,1'-biphenyl]-2-yl]-1H-tetrazole;
5-[4'-[[5-secbutyl-3-(1,1-difluoropentyl)-1H-1,2,4-triazol-1-yl]methyl][1,1'-biphenyl]-2-yl]-1H-tetrazole;
5-[4'-[(5-isobutyl-3-propyl-1H-1,2,4-triazol-1-yl)methyl][1,1'-biphenyl]-2-yl]-1H-tetrazole;
5-[4'-[(5-isobutyl-3-butyl-1H-1,2,4-triazol-1-yl)methyl][1,1'-biphenyl]-2-yl]-1H-tetrazole;
5-[4'-[(3,5-diisobutyl-1H-1,2,4-triazol-1-yl)methyl][1,1'-biphenyl]-2-yl]-1H-tetrazole;
5-[4'-[(5-isobutyl-3-isopentyl-1H-1,2,4-triazol-1-yl)methyl][1,1'-biphenyl]-2-yl]-1H-tetrazole;
5-[4'-[[5-isobutyl-3-(1,1-difluoroethyl)-1H-1,2,4-triazol-1-yl]methyl][1,1'-biphenyl]-2-yl]-1H-tetrazole;
5-[4'-[[5-isobutyl-3-(1,1-difluoropropyl)-1H-1,2,4-triazol-1-yl]methyl][1,1'-biphenyl]-2-yl]-1H-tetrazole;
5-[4'-[[5-isobutyl-3-(1,1-difluorobutyl)-1H-1,2,4-triazole-1-yl]methyl][1,1'-biphenyl]-2-yl]-1H-tetrazole;
5-[4'-[[5-isobutyl-3-(1,1-difluoropentyl)-1H-1,2,4-triazol-1-yl]methyl][1,1'-biphenyl]-2-yl]-1H-tetrazole;
5-[4'-[(5-tertbutyl-3-propyl-1H-1,2,4-triazol-1-yl)methyl][1,1'-biphenyl]-2-yl]-1H-tetrazole;
5-[4'-[(5-tertbutyl-3-butyl-1H-1,2,4-triazol-1-yl)methyl][1,1'-biphenyl]-2-yl]-1H-tetrazole;
5-[4'-[(3,5-ditertbutyl-1H-1,2,4-triazole-1-yl)methyl][1,1'-biphenyl]-2-yl]-1H-tetrazole;
5-[4'-[(5-tertbutyl-3-isopentyl-1H-1,2,4-triazol-1-yl)methyl][1,1'-biphenyl]-2-yl]-1H-tetrazole;
5-[4'-[[5-tertbutyl-3-(1,1-difluoroethyl)-1H-1,2,4-triazol-1-yl]methyl][1,1'-biphenyl]-2-yl]-1H-tetrazole;
5-[4'-[[5-tertbutyl-3-(1,1-difluoropropyl)-1H-1,2,4-triazol-1-yl]methyl][1,1'-biphenyl]-2-yl]-1H-tetrazole;
5-[4'-[[5-tertbutyl-3-(1,1-difluorobutyl)-1H-1,2,4-triazol-1-yl]methyl][1,1'-biphenyl]-2-yl]-1H-tetrazole;
5-[4'-[[5-tertbutyl-3-(1,1-difluoropentyl)-1H-1,2,4-triazol-1-yl]methyl][1,1'-biphenyl]-2-yl]-1H-tetrazole;
5-[4'-[(5-pentyl-3-propyl-1H-1,2,4-triazol-1-yl)methyl][1,1'-biphenyl]-2-yl]-1H-tetrazole;
5-[4'-[(5-pentyl-3-butyl-1H-1,2,4-triazol-1-yl)methyl][1,1'-biphenyl]-2-yl]-1H-tetrazole;
5-[4'-[(3,5-dipentyl-1H-1,2,4-triazol-1-yl)methyl][1,1'-biphenyl]-2-yl]-1H-tetrazole;
5-[4'-[(5-pentyl-3-isopentyl-1H-1,2,4-triazol-1-yl)methyl][1,1'-biphenyl]-2-yl]-1H-tetrazole;
5-[4'-[[5-pentyl-3-(1,1-difluoroethyl)-1H-1,2,4-triazol-1-yl]methyl][1,1'-biphenyl]-2-yl]-1H-tetrazole;
5-[4'-[[5-pentyl-3-(1,1-difluoropropyl)-1H-1,2,4-triazol-1-yl]methyl][1,1'-biphenyl]-2-yl]-1H-tetrazole;
5-[4'-[[5-pentyl-3-(1,1-difluorobutyl)-1H-1,2,4-triazol-1-yl]methyl][1,1'-biphenyl]-2-yl]-1H-tetrazole;
5-[4'-[[5-pentyl-3-(1,1-difluoropentyl)-1H-1,2,4-triazol-1-yl]methyl][1,1'-biphenyl]-2-yl]-1H-tetrazole;
5-[4'-[(5-isopentyl-3-ethyl-1H-1,2,4-triazol-1-yl)methyl][1,1'-biphenyl]-2-yl]-1H-tetrazole;
5-[4'-[(5-isopentyl-3-propyl-1H-1,2,4-triazol-1-yl)methyl][1,1'-biphenyl]-2-yl]-1H-tetrazole;
5-[4'-[(5-isopentyl-3-butyl-1H-1,2,4-triazol-1-yl)methyl][1,1'-biphenyl]-2-yl]-1H-tetrazole;
5-[4'-[(3,5-diisopentyl-1H-1,2,4-triazol-1-yl)methyl][1,1'-biphenyl]-2-yl]-1H-tetrazole;
5-[4'-[[5-isopentyl-3-(1,1-difluoroethyl)-1H-1,2,4-triazol-1-yl]methyl][1,1'-biphenyl]-2-yl]-1H-tetrazole;
5-[4'-[[5-isopentyl-3-(1,1-difluoropropyl)-1H-1,2,4-triazol-1-yl]methyl][1,1'-biphenyl]-2-yl]-1H-tetrazole;
5-[4'-[[5-isopentyl-3-(1,1-difluorobutyl)-1H-1,2,4-triazol-1-yl]methyl][1,1'-biphenyl]-2-yl]-1H-tetrazole;
5-[4'-[[5-isopentyl-3-(1,1-difluoropentyl)-1H-1,2,4-triazol-1-yl]methyl][1,1'-biphenyl]-2-yl]-1H-tetrazole;
5-[4'-[[5-(1-butenyl)-3-propyl-1H-1,2,4-triazol-1-yl]methyl][1,1'-biphenyl]-2-yl]-1H-tetrazole;
5-[4'-[[5-(1-butenyl)-3-butyl-1H-1,2,4-triazol-1-yl]methyl][1,1'-biphenyl]-2-yl]-1H-tetrazole;
5-[4'-[[5-(1-butenyl)-3-isopentyl-1H-1,2,4-triazol-1-yl]methyl][1,1'-biphenyl]-2-yl]-1H-tetrazole;
5-[4'-[[5-(1-butenyl)-3-(1,1-dimethoxybutyl)-1H-1,2,4-triazol-1-yl]methyl][1,1'-biphenyl]-2-yl]-1H-tetrazole;
5-[4'-[[5-(1-butenyl)-3-(1,1-difluorobutyl)-1H-1,2,4-triazol-1-yl]methyl][1,1'-biphenyl]]-2-yl]-1H-tetrazole;
5-[4'-[[5-(2-butenyl)-3-(1,1-difluorobutyl)-1H-1,2,4-triazol-1-yl]methyl][1,1'-biphenyl]-2-yl]-1H-tetrazole;
5-[4'-[[5-(3-butenyl)-3-(1,1-difluorobutyl)-1H-1,2,4-triazol-1-yl]methyl][1,1'-biphenyl]-2-yl]-1H-tetrazole;
5-[4'-[[5-(1-butynyl)-3-(1,1-difluorobutyl)-1H-1,2,4-triazol-1-yl]methyl][1,1'-biphenyl]-2-yl]-1H-tetrazole;
5-[4'-[[5-(2-butynyl)-3-(1,1-difluorobutyl)-1H-1,2,4-triazol-1-yl]methyl][1,1'-biphenyl]-2-yl]-1H-tetrazole;
5-[4'-[[5-(3-butynyl)-3-(1,1-difluorobutyl)-1H-1,2,4-triazol-1-yl]methyl][1,1'-biphenyl]-2-yl]-1H-tetrazole;
5-[4'-[[3,5-di(1-butenyl)-1H-1,2,4-triazol-1-yl]methyl][1,1'-biphenyl]-2-yl]-1H-tetrazole;
5-[4'-[[3,5-di(2-butenyl)-1H-1,2,4-triazol-1-yl]methyl][1,1'-biphenyl]-2-yl]-1H-tetrazole;
5-[4'-[[3,5-di(3-butenyl)-1H-1,2,4-triazol-1-yl]methyl][1,1'-biphenyl]-2-yl]-1H-tetrazole;
5-[4'-[[3,5-di(1-butynyl)-1H-1,2,4-triazol-1-yl]methyl][1,1'-biphenyl]-2-yl]-1H-tetrazole;
5-[4'-[[3,5-di(2-butynyl)-1H-1,2,4-triazol-1-yl]methyl][1,1'-biphenyl]-2-yl]-1H-tetrazole; and
5-[4'-[[3,5-di(3-butynyl)-1H-1,2,4-triazol-1-yl]methyl][1,1'-biphenyl]-2-yl]-1H-tetrazole.

Further preferred compounds of formula I, wherein R³ is COOH, and their pharmaceutically-acceptable salts, are of the group of compounds consisting of:
4'-[(3,5-dibutyl-1H-1,2,4-triazol-1-yl)methyl][1,1'-biphenyl]-2-carboxylic acid;
4'-[(3-ethyl-5-butyl-1H,1,2,4-triazol-1-yl)methyl][1,1'-biphenyl] -2-carboxylic acid;
4'-[(3-butyl-5-ethyl-1H-1,2,4-triazol-1-yl)methyl][1,1'-biphenyl]-2-carboxylic acid;
4'-[(5-butyl-1H-1,2,4-triazol-1-yl)methyl][1,1'-biphenyl]-2-carboxylic acid;
4'-[(3-butyl-1H-1,2,4-triazol-1-yl)methyl][1,1'-biphenyl]-2-carboxylic acid.

Especially preferred are the following compounds:
4'[(3,5-dibutyl-1H-1,2,4-triazol-1-yl)methyl][1,1'-biphenyl]-2-carboxylic acid;
5-[4'-[(3,5-dibutyl-1H-1,2,4-triazol-1-yl)methyl][1,1'-biphenyl]-2-yl]-1H-tetrazole;
5-[4'-[[5-butyl-3-(1,1-difluorobutyl)-1H-1,2,4-triazol-1-yl]methyl]-1,1'-biphenyl]-2-yl]-1H-tetrazole;
5-[4'-[(3,5-dipropyl-1H-1,2,4-triazol-1-yl)methyl][1,1'-biphenyl]-2-yl)-1H-tetrazole;
5-[4'-[(3,5-dipentyl-1H-1,2,4-triazol-1-yl)methyl][1,1'-bisphenyl]-2-yl] -1H-tetrazole;
5-[4'-[(5-butyl-3-propyl-1H-1,2,4-triazol-1-yl)methyl]-[1,1'-biphenyl]-2-yl]-1H-tetrazole;
5-[4'-[(3-butyl-5-propyl-1H-1,2,4-triazol-1-yl)methyl]-[1,1'-biphenyl]-2-yl]-1H-tetrazole;
5-[4'-[(3-butyl-5-isopentyl-1H-1,2,4-triazol-1-yl)methyl]-[1,1'-biphenyl-2-yl]-1H-tetrazole;
5-[4'-[(5-butyl-3-isopentyl-1H-1,2,4-triazol-1-yl)methyl]-[1,1'-biphenyl]-2-yl]-1H-tetrazole;
5-[4'-[(3-phenyl-5-butyl-1H-1,2,4-triazol-1-yl)methyl]-(1,1'-biphenyl)-2-yl]-1H-tetrazole;
5-[4'-[(5-butyl-3-ethyl-1H-1,2,4-triazol-1-yl)methyl]-[1,1'-biphenyl]-2-yl]-1H-tetrazole;
5-[4'-[(3-butyl-5-ethyl-1H-1,2,4-triazol-1-yl)methyl]-[1,1'-biphenyl]-2-yl]-1H-tetrazole;
5-[4'-[[5-butyl-3-(2-cyclohexylethyl)-1H-1,2,4-triazol-1-yl]methyl][1,1'-biphenyl]-2-yl]-1H-tetrazole;
5-[4'-[[5-butyl-3-(phenylmethyl)-1H-1,2,4-triazol-1-yl]-methyl][1,1'-biphenyl]-2-yl]-1H-tetrazole;
5-[4'-[(3-butyl-5-cyclohexyl-1H-1,2,4-triazol-1-yl)-methyl][1,1'-biphenyl]-2-yl]-1H-tetrazole;
5-[4'-[(5-butyl-3-cyclohexyl-1H-1,2,4-triazol-1-yl)-methyl][1,1'-biphenyl]-2-yl]-1H-tetrazole;
5-[4'-[(3-cyclohexylmethyl-5-butyl-1H-1,2,4-triazol-1-yl)-methyl][1,1'-biphenyl]-2-yl]-1H-tetrazole.

### General Synthetic Procedures

The compounds of the invention can be synthesized according to the following procedures of Schemes I-VI.

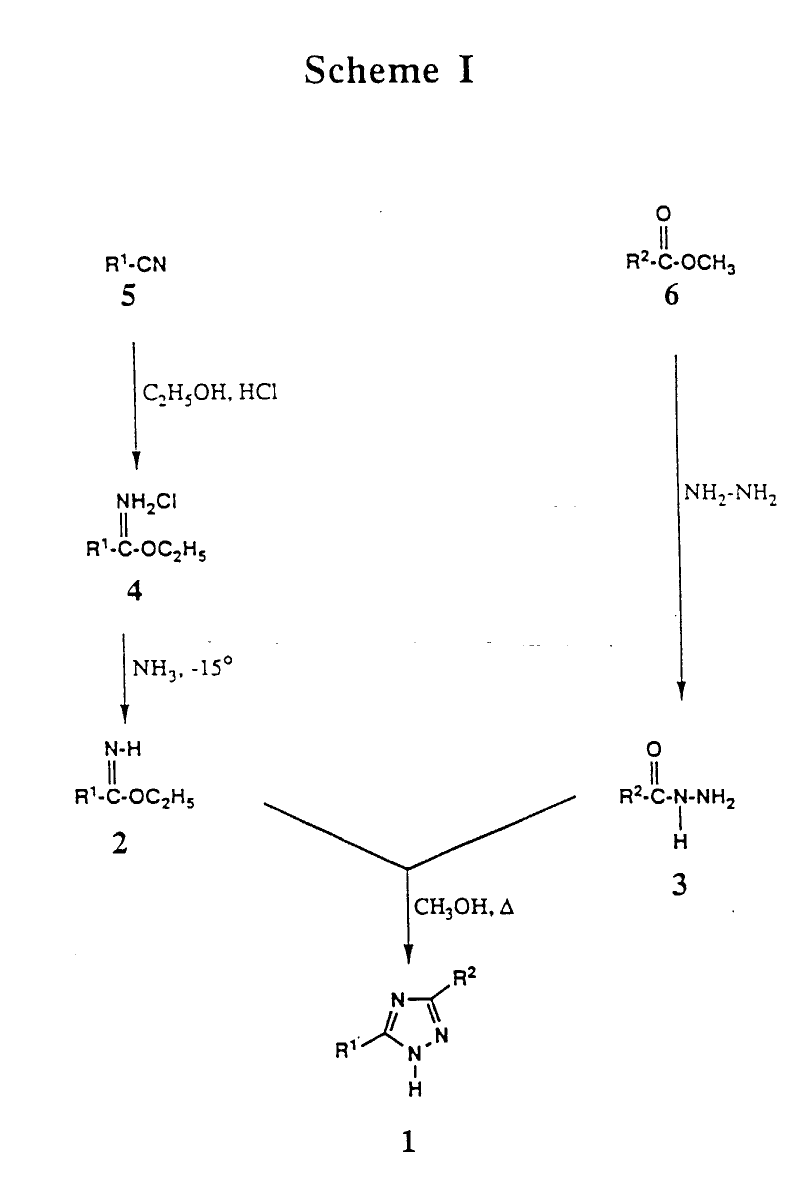

Synthetic Scheme I shows the preparation of 1H-1,2,4-triazoles 1 from ethyl iminoesters 2 and the corresponding hydrizide 3 via the general proceure outlined by H. Paul, G. Hilgetog, and G. Jahnchen, Chem. Ber., 101 , 2033 (1968). The free, iminoesters 2 can be prepared from the corresponding iminoester hydrochlorides 4, which in turn can be prepared from the corresponding nitrile 5; the procedures for the preparation of 2 and 4 from 5 are outlined by P. Reynaud and R. D. Moreau, Bull. Soc. Chim. France, 2997 (1964). The hydrazides 3 can be either purchased or prepared from the corresponding methyl esters 6 and hydrazine.

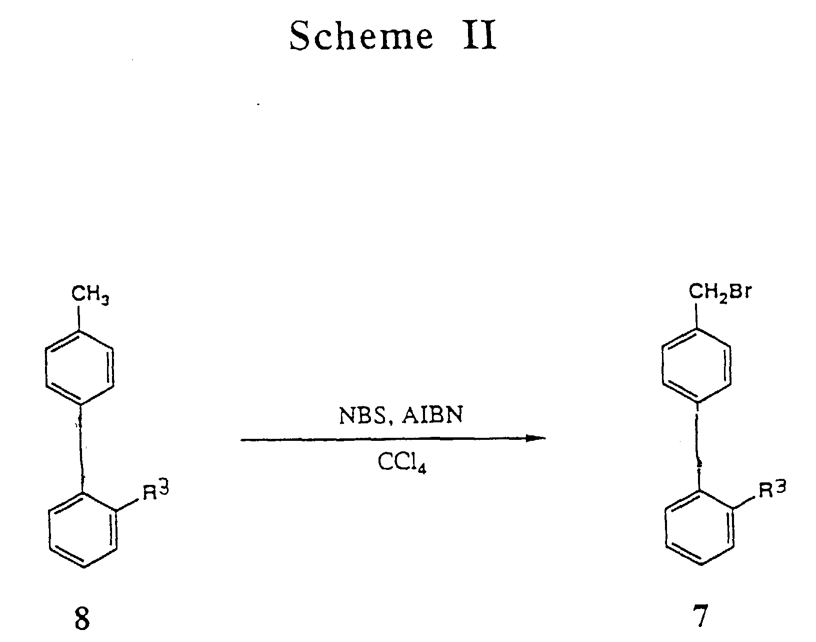

Synthetic Scheme II shows the preparation of the alkylating agent 7 from the corresponding precursor 8. When R³ equals CO₂CH₃ 8 was purchased from Chemo Dynamics Inc.

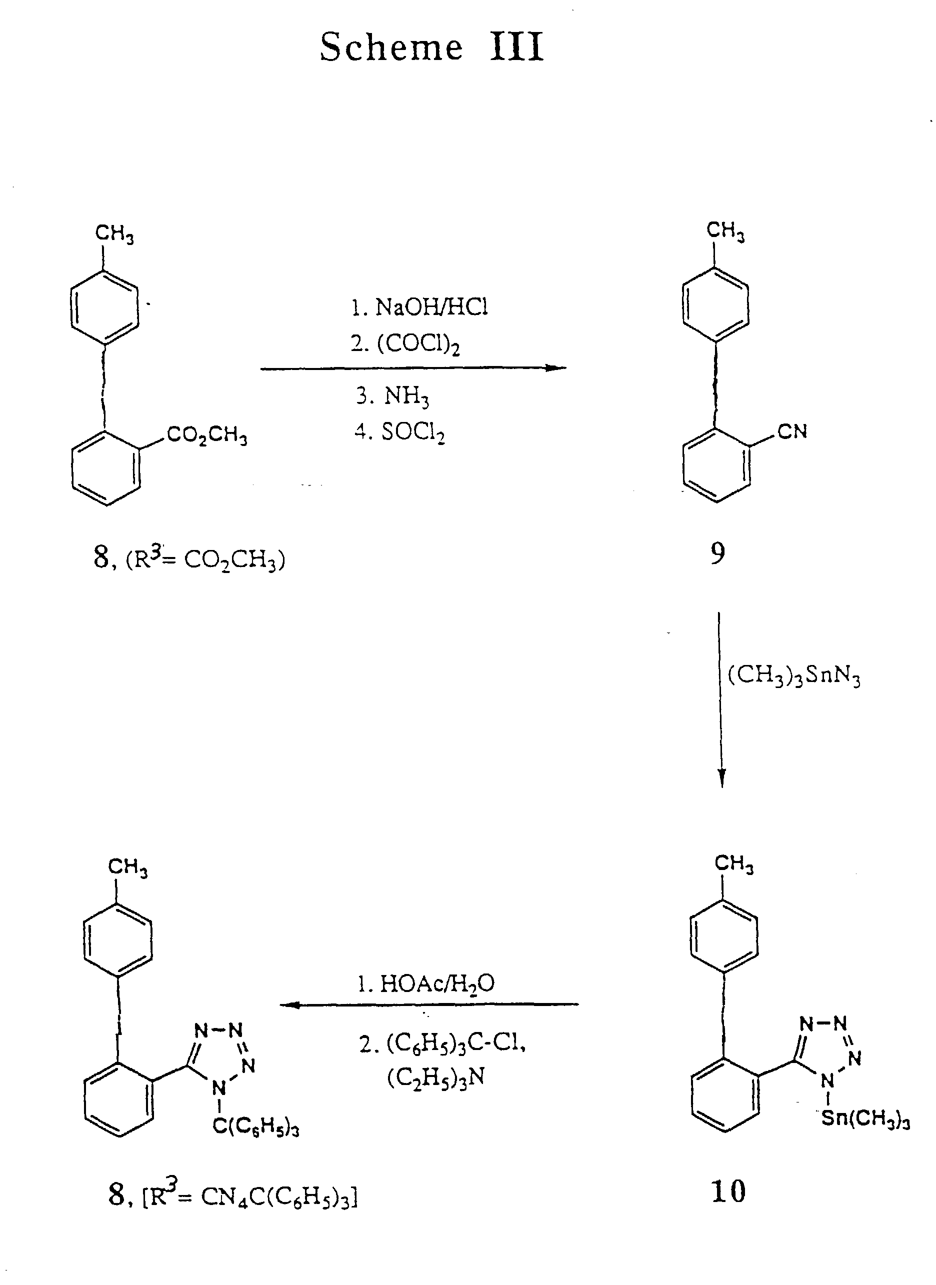

Synthetic Scheme III shows the preparation of the alkylating agent precursor 8 where R³ equal CN₄C(C₆H₅)₃ from the corresponding methyl ester 8 (R³=CO₂CH₃). In step 1, the methyl ester is converted to the corresponding acid (R³=CO₂H) by the action of sodium hydroxide/hydrochloric acid. In step 2, the acid is converted to the corresponding acid chloride (R³=COCl) by the action of oxalyl chloride. In step 3, the acid chloride is converted to the corresponding primary amide (R³=CONH₂) by the action of ammonium. In step 4, the amide is converted to the corresponding nitrile 9 by the action of thionyl chloride at reflux. The nitrile 9 is reacted with trimethyltinazide in toluene at reflux to give the corresponding trimethytin protected tetrazole 10; deprotection with acetic acid/water and reprotection with triphenylmethyl chloride/triethyl-amine gives the N-trityl tetrazole 8 (R³=CN₄C(C₆H₅)₃).

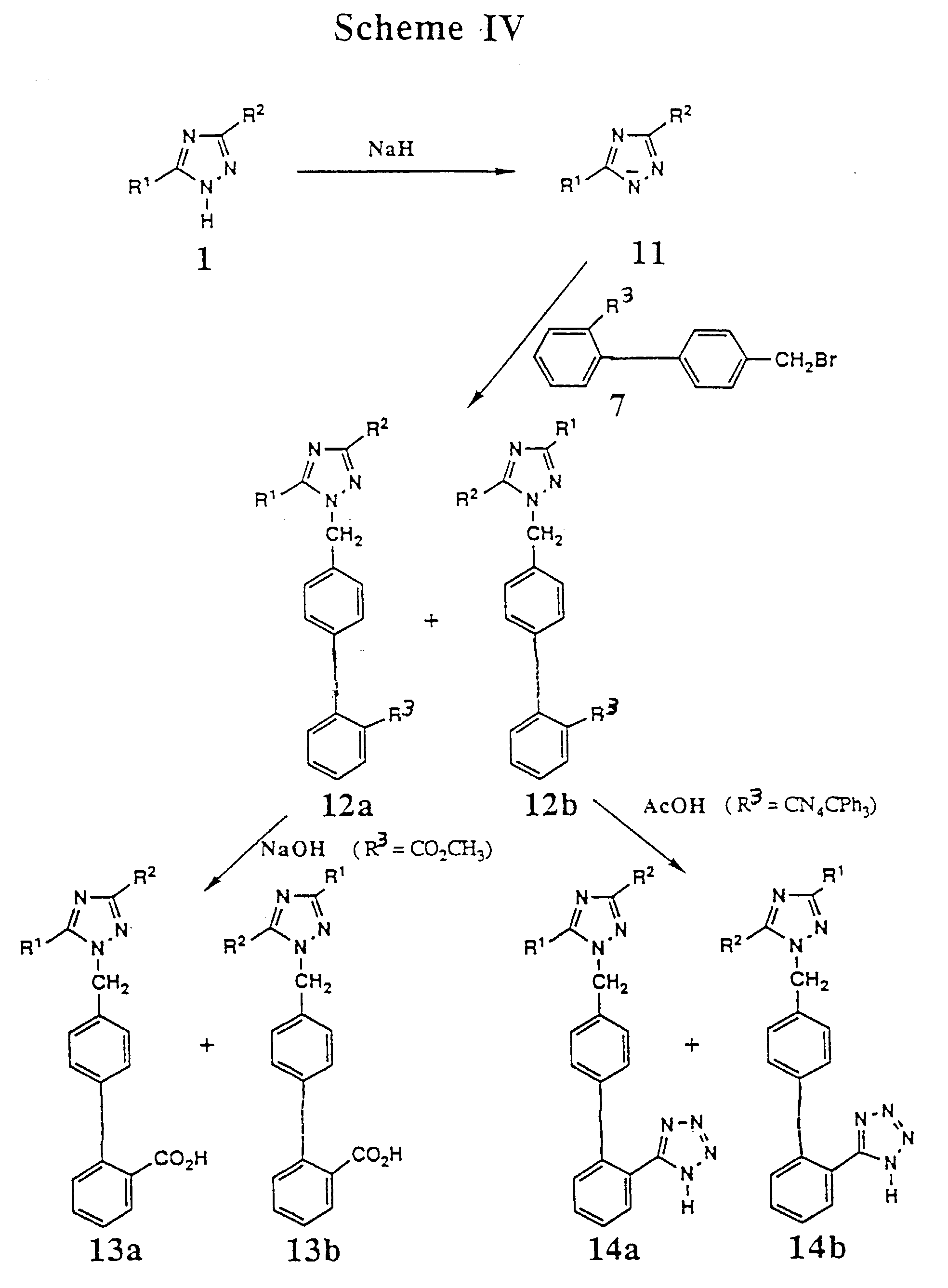

Synthetic Scheme IV shows the coupling reaction of the 1H-1,2,4-triazole 1 with the appropriate alkylating reagent 7. In the first step, 1 is treated with a base, such as sodium hydride, to generate the corresponding anion 11. Anion 11 is reacted with an alkylating agent 7 to give a mixture of regioisomers 12a and 12b. The isomer mixture may be converted to mixtures of the corresponding acids 13a and 13b or tetrazoles 14a and 14b by treatment with the appropriate reagent. Or, the isomers 12a and 12b may be separated by chromatographic methods, and each isomer may be reacted with the appropriate reagent to provide the acid- or tetrazole-substituted end product.

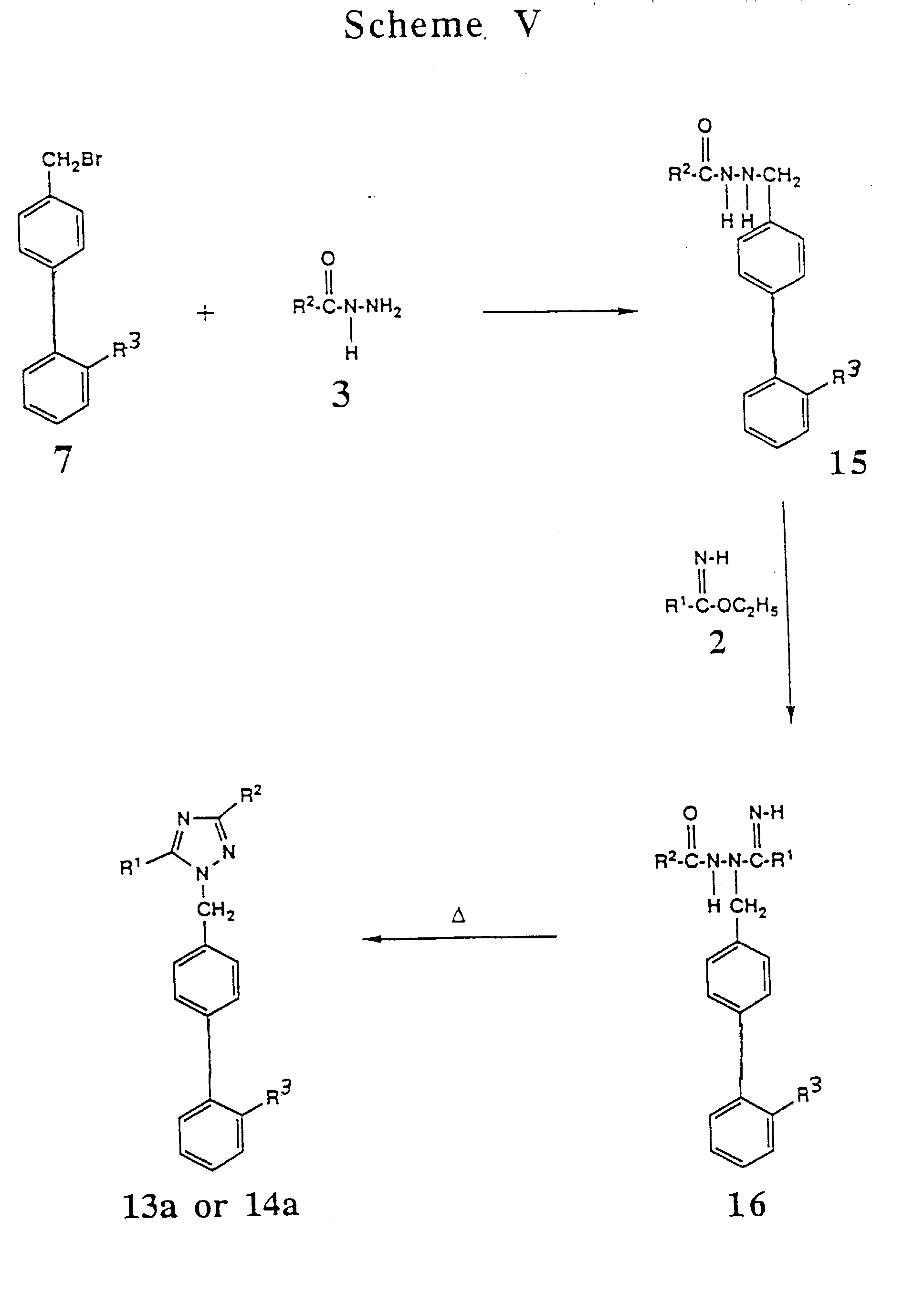

Synthetic Scheme V shows the regioselective synthesis of isomer 13a or 14a from Scheme IV. In the first step of the reaction, an alkylating agent 7 is reacted with an appropriate hydrazide 3 to provide substituted hydrazide 15. An imidate 2 is reacted with hydrazide 15 to provide intermediate 16 which cyclizes upon heating to provide the corresponding product compound 13a or 14a.

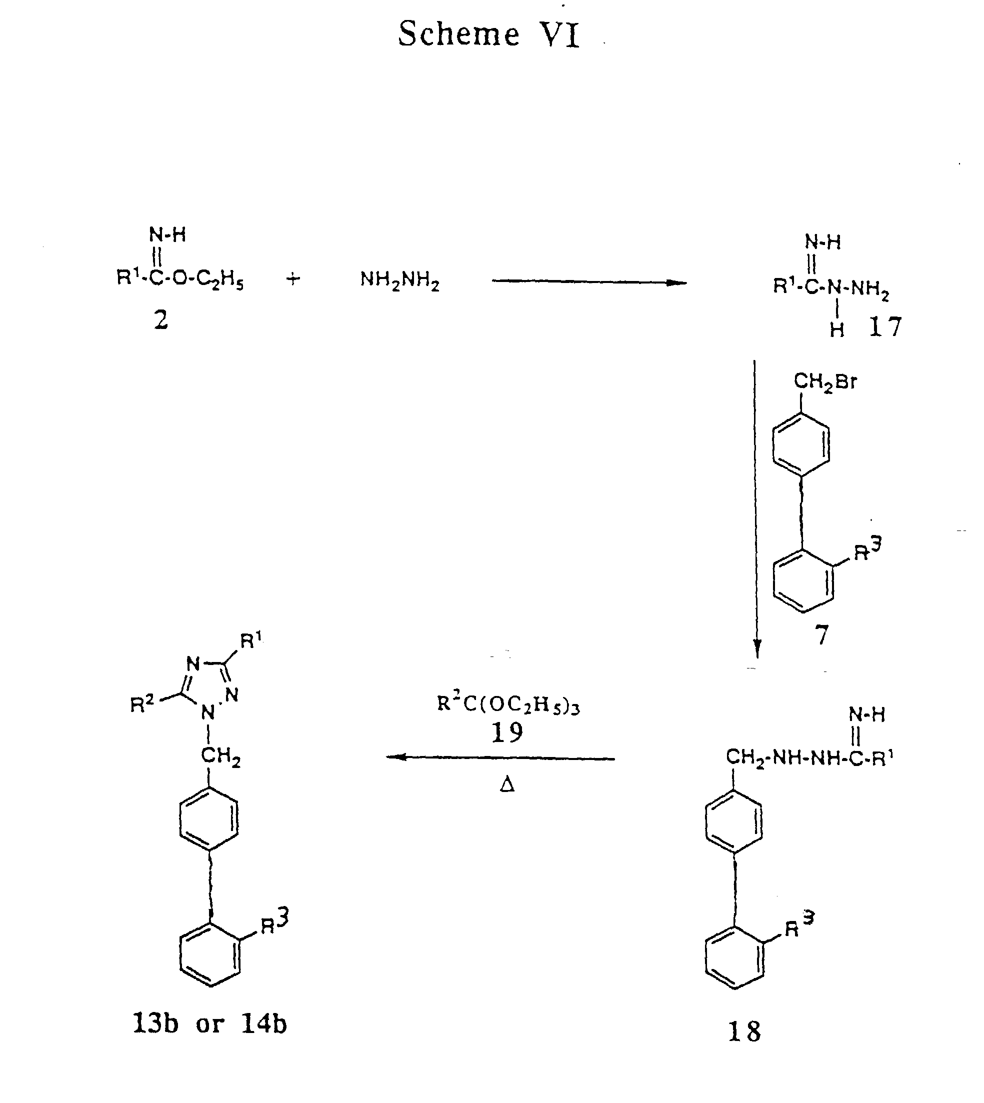

Synthetic Scheme VI shows the regioselective synthesis of isomer 13b or 14b from Scheme IV. In the first step of the reaction, imidate 2 is reacted with hydrazine to give amidazone 17. This intermediate is reacted with alkylating agent 7 to give intermediate 18 which is then cyclized in the presence of heat and an appropriate orthoester 19 to yield the corresponding product compound 13b or 14b.

The following Examples 1-47 are detailed descriptions of the methods of preparation of compounds of Formula I and, more specifically, within Formula II. These detailed preparations fall within the scope of, and serve to exemplify, the above described General Synthetic Procedures which form part of the invention. All parts are by weight unless otherwise indicated.

### Example 1 (intermediate)

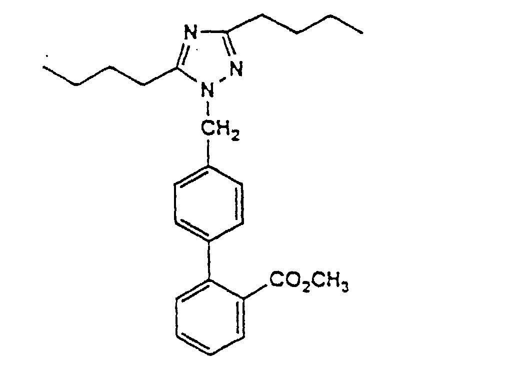

### methyl 4'-[(3,5-dibutyl-1H-1,2,4-triazol-1-yl)methyl][1,1'-biphenyl]-2-carboxylate

### Step 1: Preparation of 4-bromomethyl-2'-methoxycarbonylbiphenyl.

A 47.46 g (210 mmol) sample of methyl 2-(p-tolyl)benzoate (Chemo Dynamics Inc.) was dissolved in 3L of carbon tetrachloride and treated with 37.33 g (209 mmol) of N-bromosuccinimide (NBS) and 1.17 g (7.13 mmol) of azobisisobutyronitrile (AIBN) at reflux under nitrogen for 24 hours. The reaction mixture was treated again with 1.0 g (6.1 mmol) of AIBN and stirred at reflux for an additional 24 hours. The reaction was filtered and the solvent removed in in vacuo. Purification by silica gel chromatography (Waters Prep-500A) using ethyl acetate/hexane (5:95) as eluent provided 50.0 g (78%) of a colorless solid: mp 48-51°C; NMR (CDCl₃) δ 3.64 (s, 3H), 4.54 (s, 2H), 7.23-7.63 (m, 7H), 7.81-7.89 (m, 1H). NMR indicated that this material was only 91% pure; it contained 9% of the corresponding dibromocompound (δ 6.70); however, no further attempts at purification were made and this mixture was used in all subsequent alkylation reactions.

### Step 2: Preparation of 3,5-dibutyl-1H-1,2,4-triazole.

A solution of 64.5 g (0.50 mol) of ethyl iminovalerate [P. Reynaud and R. C. Moreau, Bull. Soc. Chim. France , 2997 (1964)] in 100 mL of methanol was added slowly to 58.0 g (0.50 mol) of valeric acid hydrazide (Lancaster Synthesis) in 400 mL of methanol at 0°C under a nitrogen atmosphere. After the addition was complete, the reaction was allowed to warm to ambient temperature and then stir at reflux for 2 days. The solvent was removed in vacuo; purification by silica gel chromatography (Waters Prep-500A) using ethyl acetate/hexane (80:20) gave 78.9 g (93%) of a colorless solid: mp 50.5-51.5°C; NMR (CDCl₃) δ 0.88 (t, J=7 Hz, 6H), 1.28-1.33 (m, 4H), 1.63-1.77 (m, 4H), 2.72 (t, J=7 Hz, 4H); MS (FAB) m/e (rel intensity) 183 (12%), 182 (100), 181 (3), 180 (6), 152 (8), 139 (4); HRMS. Calcd for M+H: 182.1657. Found: 182.1661.

### Step 3: Preparation of methyl 4'-[(3,5-dibutyl-1H-1,2,4-triazol-1-yl)methyl][1,1'-biphenyl]-2-carboxylate.

Under a static nitrogen atmosphere, 2.01 g (11.0 mmol) of solid 3,5-dibutyl-1H-1,2,4-triazole was added in small portions to 12 mmol of sodium hydride in 50 ml of dimethylformamide (DMF); stirring was continued until hydrogen evolution had ceased. The anion solution was cooled to -10°C (ice/methanol) and treated with a solution of 3.37 g (11.0 mmol) of 4-bromomethyl-2'-methoxycarbonyl-biphenyl in 20 ml of dry DMF. The reaction was allowed to warm to ambient temperature and stir overnight. Methanol (10 ml) was added to destroy any unreacted sodium hydride and the DMF was removed in vacuo. The residue was dissolved in ethyl acetate, washed with water, and dried (MgSO₄). Silica gel chromatography (Waters Prep-500A) using 40% ethyl acetate/hexane gave 2.0 g (41%) of compound as an oil: NMR (CDCl₃) δ 0.90 (t, J = 7 HZ, 3H), 0.94 (t, J = 7 Hz, 3H), 1.28-1.47 (m, 4H), 1.62-1.80 (m, 4H), 2.63-2.75 (m, 4H), 3.63 (s, 3H), 5.27 (s, 2H), 7.13-7.18 (m, 2H), 7.25-7.35 (m, 3H), 7.37-7.44 (m, 1H), 7.48-7.55 (m, 1H), 7.80-7.85 (m, 1H).

### Example 2

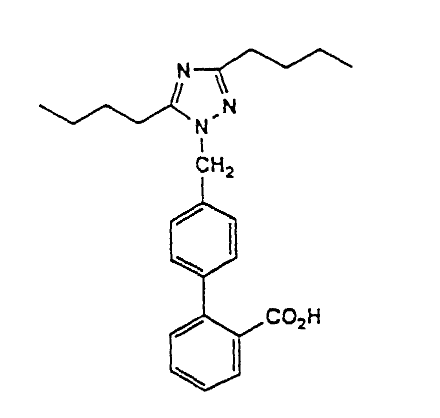

### 4'-[(3,5-dibutyl-1H-1,2,4-triazol-1-yl)methyl[1,1'-biphenyl]-2-carboxylic acid

A 2.0 g (4.9 mmol) sample of the methyl ester product compound from Example 1 was dissolved in 80 ml of ethanol and treated with 80 ml of 10% NaOH at ambient temperature for 3 days. The ethanol was removed in vacuo and the aqueous phase acidified to pH 1 with hydrochloric acid which caused the product to precipitate; filtration and drying in vacuo gave 1.65 g (86%) of colorless compound: mp 134-135°C; NMR (DMSO-d₆) δ 0.85 (t, J = 7 Hz, 3H), 0.90 (t, J = 7 Hz, 3H), 1.23-1.39 (m, 4H), 1.53-1.68 (m, 4H), 2.59 (t, J = 7 Hz, 2H), 2.78 (t, J = 7 Hz, 2H), 5.37 (s, 2H), 5.37 (s, 2H), 7.18-7.26 (m, 2H), 7.28-7.37 (m, 3H), 7.42-7.48 (m, 1H), 7.53-7.60 (m, 1H), 7.70-7.75 (m, 1H).

### Example 3

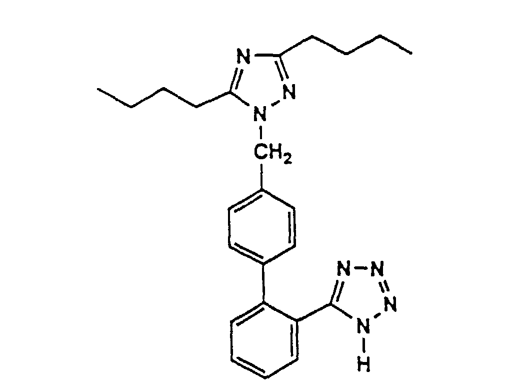

### 5'[4'-[(3,5-dibutyl-1H-1,2,4-triazol-1-yl)methyl][1,1'-biphenyl]-2-yl]-1H-tetrazole

### Step 1: Preparation of N-Triphenylmethyl-5-[2-(4'-bromomethylbiphen-2-yl]tetrazole.

A 542.5 g (2.4 mol) sample of methyl 2-(p-tolyl)benzoate (Chemo Dynamics Inc.) was dissolved in 5.5 L of ethanol and treated with 3 L (7.5 mol) of 2.5 N sodium hydroxide. The reaction was stirred overnight at ambient temperature and treated with an additional 480 ml (6.0 mol) of sodium hydroxide; stirring was continued for an additional 24 h and the ethanol removed in vacuo. The remaining solution was cooled in ice and acidified to pH 1 with hydrochloric acid which caused the product to precipitate; filtration and drying in vacuo gave 510 g (100%) of crude 2-(p-tolyl)benzoic acid: mp 145.0-147.5°C; NMR (CDCl₃) δ 2.40 (s, 3H), 7.17-7.28 (m, 4H), 7.35-7.45 (m, 2H), 7.51-7.59 (m, 1H), 7.90-7.97 (m, 1H). The crude acid was suspended in 1 L of toluene and slowly treated with 400 g (3.15 mol) of oxalyl chloride under nitrogen. The reaction was allowed to stir at ambient temperature for 4.5 h and concentrated in vacuo to remove excess oxalyl chloride. The residue was redissolved in 2L of toluene and treated with 92.8 g (5.46 mol) of anhydrous ammonia. The reaction was filtered and the filtrate concentrated in vacuo producing 424 g (84%) of crude 2-(p-tolyl)benzamide: mp 128-130°C; NMR (CDCl₃) δ 2.40 (s, 3H), 5.28 (br s, 1H), 5.77 (br s, lH), 7.21-7.53 (m, 7H), 7.76-7.83 (m, 1H). The crude amide was treated with 1420 ml (19.5 mol) of thionyl chloride at reflux for 3.5 h. The reaction was filtered and the thionyl chloride removed in vacuo. The residue was dissolved in 800 ml of toluene and reconcentrated in vacuo. On standing overnight, the residue crystallized. The crystals were collected and washed with hexane to give 296 g (64%) of 2-(p-tolyl)benzonitrile: mp 50.5-52.0°C; NMR (CDCl₃) δ 2.42 (s, 3H), 7.22-7.34 (m, 2H), 7.37-7.52 (m, 3H), 7.58-7.66 (m, 1H), 7.72-7.78 (m, 1H). A 286 g (1.48 mol) sample of the crude nitrile was dissolved in 1630 mL to toluene and treated with 377 g (1.8 mol) of trimethyltinazide at reflux for 24 h. The reaction was cooled; filtration gave 600 g of crude N-trimethylstannyl-5-[2-(4'-methylbiphen-2-yl]tetrazole: mp 271-272°C (dec.); NMR (DMSO-d₆) δ 0.36 (br t, J=34 Hz, 9H), 2.24 (s, 3H), 6.89-7.06 (m, 4H), 7.35-7.55 (m, 4H). The crude N-trimethylstannyl tetrazole was suspended in 4270 mL of toluene and 287 mL of anhydrous tetrahydrofuran (THF) and treated with 63.4 g (173 mol) of anhydrous hydrogen chloride at ambient temperature under nitrogen with stirring. The reaction was allowed to stand overnight and filtered; recrystallization from toluene gave 217 g (62%) of 5-[2-(4'methylbiphen-2-yl)]tetrazole as a solid: mp 149-152°C; NMR (DMSO-d₆) δ 2.28 (s, 3H), 6.94-7.02 (m, 2H), 7.08-7.15 (m, 2H), 7.50-7.59 (m, 2H), 7.62-7.72 (m, 2H). A 200 g (0.85 mol) sample of the tetrazole was suspended in 3.3 L of dichloromethane and treated with 262 g (0.91 mol) of triphenylmethyl chloride and 141 mL (1.0 mol) of anhydrous triethylamine. The reaction was stirred at reflux for 3 h under nitrogen, washed with water, dried (MgSO₄), and concentrated in vacuo. Recrystallization gave 338 g (83%) of N-triphenylmethyl-5-[2-(4'-methylbiphen-2-yl)]tetrazole as a colorless solid: mp 170-173°C; NMR (CDCl₃) δ 2.27 (s, 3H), 6.86-6.96 (m, 8H), 6.98-7.04 (m, 2H), 7.09-7.52 (m, 12H), 7.86-7.94 (m, 1H). The N-triphenylmethyl tetrazole was dissolved in 4260 mL of carbon tetrachloride and treated with 126.4 g (0.71 mol) of N-bromosuccinimide (NBS) and 11.9 g (49 mmol) of benzoyl peroxide at reflux for 3.5 h. The reaction was filtered and the solvent removed in vacuo. Recrystallization from toluene gave 277 g (59%) of N-triphenylmethyl-5-[2-(4'-bromomethylbiphen-2-yl)]tetrazole as a colorless solid: mp 140-142°C; NMR (CDCl₃) δ 4.39 (s, 2H), 6.85-6.95 (m, 7H), 7.06-7.15 (m, 4H), 7.22-7.43 (m, 9H), 7.45-7.55 (m, 2H), 7.94-8.01 (m, 1H). NMR indicated that this material was only 85% pure; it contained 7% of corresponding dibromo- compound (6 6.50) and 8% of starting material (δ 2.27); however, no further attempts at purification were made and this mixture was used in all subsequent alkylation reactions.

### Step 2: Preparation of 5-[4'-[(3,5-dibutyl-1H-1,2,4-triazol-1-yl)methyl][1,1'-biphenyl]-2-yl]-1H-tetrazole

Under a static nitrogen atmosphere, 2.77 g (15.0 mmol) of solid 3,5-dibutyl-1H-1,2,4-triazole (from Step 2 of Example 1) was added in small portions to 15 mmol of sodium hydride in 50 ml of dimethylformamide (DMF); stirring was continued until hydrogen evolution had ceased. The anion solution was cooled to -10°C (ice/methanol) and treated with a solution of 8.45 g (15.0 mmol) of N-triphenylmethyl-5-[2-(4'-bromomethylbiphen-2-yl)]tetrazole in 20 ml of dry DMF. The reaction was allowed to warm to ambient temperature and stir overnight. Methanol (10 ml) was added to destroy any unreacted sodium hydride and the DMF was removed in vacuo. The residue was dissolved in ethyl acetate, washed with water, anc dried (MgSO₄). The crude material (8.61 g ) was redissolved in 86 ml of toluene and treated with anhydrous HCl to remove the protecting group. Purification by reverse phase chromatography (Waters Delta Prep-3000) using isocratic 40% acetonitrile/water (0.05% TFA) provided 1.72 g (27%) of colorless compound: mp 150.5-152.0°C; NMR (DMSO-d₆) δ 0.84 (t, J = 7 Hz, 3H), 0.88 (t, J = 7 Hz, 3H), 1.22-1.38 (m, 4H), 1.49-1.66 (m, 4H), 2.66 (t, J = 7 Hz, 2H), 2.73 (t, J = 7 Hz, 2H), 5.32 (s, 2H), 7.05-7.15 (m, 4H), 7.50-7.72 (m, 4H); MS (FAB) m/e (rel intensity) 416 (30), 373 (10), 235 (5), 207 (100), 192 (30); HRMS. Calcd for M+H: 416.2563. Found: 416.2600.

### Example 4 (intermediate)

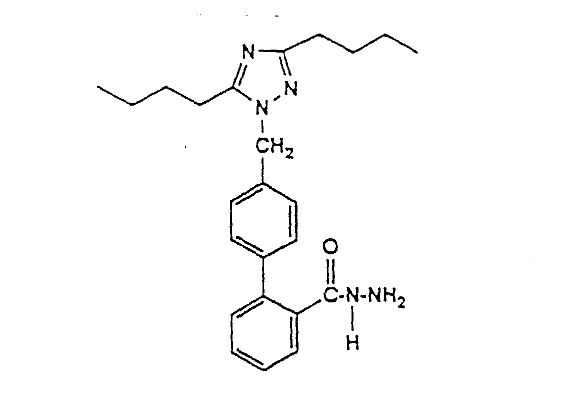

### 4'-[(3,5-dibutyl-1H-1,2,4-triazol-1-yl)methyl][1,1'-biphenyl]-2-carboxylic acid, hydrazide

A 7.10 g (17.5 mmol) sample of the methyl ester product compound of Example 1 was dissolved in 150 ml of methanol and treated with 22 ml (22.2 g 695 mmol) of anhydrous hydrazine under a static nitrogen atmosphere. The reaction was stirred at reflux for 2 days and concentrated in vacuo to give 7.03 g (99%) of compound which was a colorless glass: NMR (CDCl₃) δ 0.88 (t, J = 7 Hz, 3H), 0.94 (t, J = 7 Hz, 3H), 1.28-1.47 (m, 4H), 1.62-1.78 (m, 4H), 2.62-2.73 (m, 4H), 3.5-4.1 (br s, 2H), 5.26 (s, 2H), 6.53 (s, 1H), 7.13-7.63 (m, 8H).

### Example 5

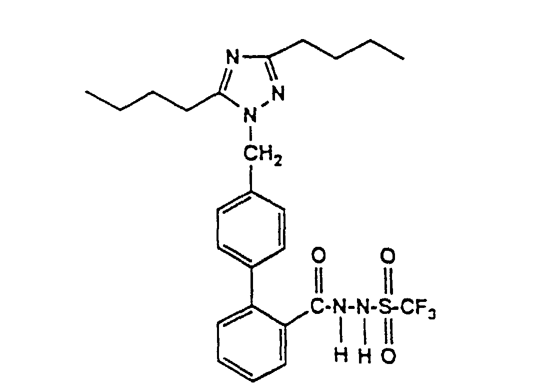

### 4'-[(3,5-dibutyl-1H-1,2,4-triazol-1-yl)methyl][1,1'-biphenyl]-2-carboxylic acid, 2-[(trifluoromethyl) sulfonyl]hydrazide

A 1.0 g (2.48 mmol) sample of the hydrazide product compound of Example 4 was dissolved in 20 ml of toluene and cooled to 0°C under a static nitrogen atmosphere. The solution was treated with 1.41 g (5.0 mmol) of trifluoromethylsulfonyl anhydride. Concentration in vacuo produced 3.91 g of crude material which was a dark oil. Purification by silica gel chromatography (Waters Prep-500A) using 50% ethyl acetate/hexane gave 1.0 g (75%) of pale yellow compound: mp 345°C (dec); NMR (DMSO-d₆) δ 0.86 (t, J = 7 HZ, 3H), 0.88 (t, J = 7 Hz, 3H), 1.25-1.38 (m, 4H), 1.55-1.65 (m, 4H), 2.50-2.59 (m, 2H), 2.67-2.73 (m, 2H), 5.32 (s, 2H), 7.15-7.50 (m, 8H); MS (FAB) m/e (rel intensity) 538, (100), 405 (15), 389 (10), 374 (25), 356 (15).

### Example 6

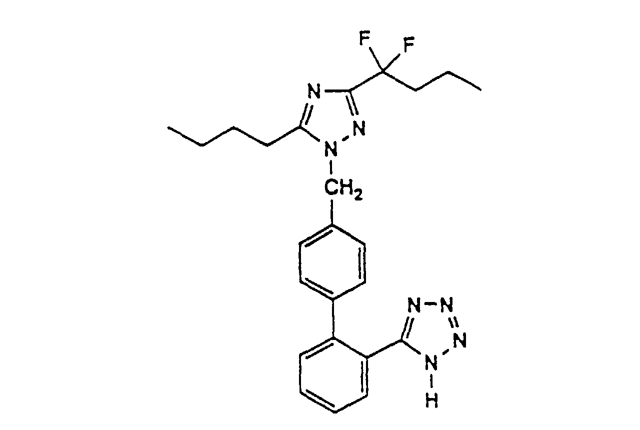

### 5-[4'-[[5-butyl-3-(1,1-difluorobutyl)-1H-1,2,4-triazol-1-yl]methyl][1,1'-biphenyl]-2-yl]-1H-tetrazole

### Step 1: Preparation of 3-butyl-5-(1,1-difluoro)butyl-1H-1,2,4-triazole.

Under nitrogen, a stirred solution of 56.9 g (0.65 mol) of N-tert-butyl-N-methylamine (Fluka) and 66.1 g (0.65 mol) of triethylamine in 1 L of methylene chloride was cooled to 0°C and treated with neat difluoroacetic anhydride [E. Sawicki, J. Org. Chem., 21, 376 (1956)] at such a rate as to maintain the reaction temperature below 10°C. The reaction was allowed to warm to ambient temperature and stir overnight. All volitiles were removed in vacuo (bath temperature < 35°C) and the residue redissolved in methylene chloride; the solution was washed with saturated sodium bicarbonate, dried (MgSO₄), and concentrated to give 94 g (89%) of a yellow liquid. Vacuum distillation gave 86 g (81%) of colorless N-tert-butyl-N-methyldifluoroacetamide: bp 87-88°C (22 mm); 'H NMR (CDCl₃) δ 1.37 (s, 9H), 2.93 (t, J=3 Hz, 3H), 5.97 (t, J=57 Hz, 1H); ¹⁹F NMR (CDCl₃) δ -122.20 (d, J=57 Hz, 2F). A 17.0 g (103 mmol) sample of the amide was dissolved in 50 mL of dry THF and added slowly to a solution of 145 mnol of lithium diisopropylamine (LDA) in 450 mL of dry THF at -78°C. The reaction was allowed to stir for 1 h at -78°C prior to the addition of 17 mL (175 mmol) of 1-iodopropane by syringe. Stirring at -78°C was continued for 1 hr and then the reaction was allowed to warm to ambient temperature overnight. Methanol (10 mL) was added and the reaction was concentrated in vacuo; the residue was dissolved in methylene chloride and washed with 1N hydrochloric acid, dried (MgSO₄) and reconcentrated to give 17.6 g (83%) of crude product. Purification by vacuum distillation gave 13.3 g (62%) of colorless N-tert-butyl-N-methyl-2,2-difluoro-valeramide: bp 125-130°C (84 mm); 'H NMR (CDCl₃) δ 0.94 (t, J=7 Hz, 3H), 1.37 (s, 9H), 1.40-1.52 (m, 2H), 1.95-2.15 (m, 2H); ¹⁹F NMR (CDCl₃) δ -100.29 (t, J = 20Hz, 2F). The difluorovaleramide was dissolved in 30 mL of trifluoroacetic acid (TFA) and stirred at reflux overnight under nitrogen. The solvent was removed in vacuo and the residue dissolved in methylene chloride; the solution was washed with water, dried (MgSO₄) and concentrated to give 9.7 g (100%) of crude N-methyl-2,2-difluorovaleramide: 'H NMR (CDCl₃) δ 0.92 (t, J = 7 Hz, 3H), 1.36-1.51 (m, 2H), 1.90-2.11 (m, 2H), 2.84 (d, J = 6 Hz, 3H), 6.60-6.85 (br s, 1H); ¹⁹F NMR (CDCl₃) δ -106.98 (t, J = 19 Hz, 2F). The crude N-methyl amide was dissolved in 40 mL of 6N hydrochloric acid and stirred at reflux for 24 hr. The reaction was cooled to ambient temperature and extracted with methylene chloride; the extracts were combined, dried (MgSO₄), and concentrated to give 8.0 g (56%) of crude 2,2-difluorovaleric acid: 'H NMR (CDCl₃) δ 1.00 (t, J = 7 Hz, 3H), 1.46-1.63 (m, 2H), 1.97-2.17 (m, 2H); ¹⁹F NMR (CDCl₃) δ -107.16 (t, J = 18 Hz, 2F). A 4.83 g (35 mmol) sample of the crude acid was dissolved in 25 mL (35.2 g, 174 mmol) of phthaloyl chloride in a flask equipped with a reflux condenser and stirred under nitrogen in a 110°C oil bath for 6 hrs. The condenser was replaced with a distillation head and 3.22 g (60%) of colorless 2,2-difluorovaleryl chloride was collected: bp 96°; 'H NMR (CDCl₃) δ 1.03 (t, J = 7 Hz, 3H), 1.48-1.65 (m, 2H), 2.03-2.23 (m, 2H); ¹⁹F NMR (CDCl₃) δ -102.41 (t, J = 18 Hz, 2F). The 2,2-difluorovaleryl chloride (20.6 mmol) was dissolved in 10 mL of methylene chloride and dropwise to a solution of 135 g (42 mmol) of anhydrous hydrazine in 20 mL of methylene chloride at 0°C. After the addition was complete, the reaction was stirred at ambient temperature for 1 h, washed with water, dried (MgSO₄), and concentrated to give 3.12 g (91%) of 2,2-difluorovaleric acid hydrazide: NMR (CDCl₃) δ 0.96 (t, J = 7 Hz, 3H), 1.40-1.56 (m, 2H), 1.96-2.17 (m, 2H), 3.93 (br s, 2H), 7.67 (br s, 1H). A 2.84 g (18.7 mmol) sample of the crude hydrazide was dissolved in 50 mL of methanol and treated with 2.41 g (18.7 mmol) of ethyl iminovalerate. Under nitrogen, the reaction was stirred at reflux for 3 days and concentrated in vacuo. Purification by silica gel chromatography (Waters Prep-500A) using ethyl acetate/hexane (60:40) gave 3.0 g (74%) of 3-butyl-5-(1,1-difluoro)butyl-1H-1,2,4-triazole as a colorless solid: mp 92-93°C; 'H NMR (CDCl₃) δ 0.97 (t, J = Hz, 3H), 0.92 (t, J = 7 Hz, 3H), 1.30-1.45 (m, 2H), 1.47-1.62 (m, 2H), 1.66-1.81 (m, 2H), 2.18-2.38 (m, 2H), 2.83 (t, J = 7 Hz, 2H); ¹⁹F NMR (CDCl₃) δ -97.27 (t, J = 18 Hz, 2F); MS (FAB) m/e (rel intensity) 218 (100), 198 (8), 188 (5), 178 (8), 170 (5); HRMS. Calcd for M+H: 218.1469. Found: 218.1461.

### Step 2: Preparation of 5-[4'-[5-butyl-3-(1,1-difluorobutyl]-1H-1,2,4-triazol-1-yl]methyl][1,1'-biphenyl]-2-yl]-1H-tetrazole.

Under a static nitrogen atmosphere, 2.0 g (9.2 mmol) of solid 3-butyl-5-(1,1-difluoro)butyl-1H-1,2,4-triazole was added in small portions to 10 mmol of sodium hydride in 50 ml of dimethylformamide (DMF); stirring was continued until hydrogen evolution had ceased. The anion solution was cooled to -10°C (ice/methanol) and treated with a solution of 5.1 g (9.2 mmol) of N-triphenylmethyl-5-[2-(4'-bromomethylbiphen-2-yl)]tetrazole (from Step 1 of Example 3) in 20 ml of dry DMF. The reaction was allowed to warm to ambient temperature and stir overnight. Methanol (10 ml) was added to destroy any unreacted sodium hydride and the DMF was removed in vacuo. the residue was dissolved in ethyl acetate, washed with water, and dried (MgSO₄). Silica gel chromatography using 2.5% ethyl acetate/toluene produced 680 mg (10.7%) of a faster moving isomer and 4.91 g (77.2%) of a slower moving isomer. The slower moving isomer was dissolved in 40 ml 10% water/acetic acid and stirred at ambient temperature overnight. The solvent was removed in vacuo; the residue was dissolved in water and the pH adjusted to 9. The water layer was washed with benzene, acidified to pH 4 with 3N HCl, and extracted with ethyl acetate. The ethyl acetate was dried (Mg SO₄) and remove in vacuo. The product was recrystallized from acetonitrile yielding 1.38 g (44%) of the 3-(1,1-difluoro)butyl isomer as colorless solid: mp 165.0-167.5°C; NMR (CDCl₃) δ d 0.86 (t, J=7 Hz, 3H), 0.94 (t, J=7 Hz, 3H), 1.24-1.38 (m, 2H), 1.40-1.55 (m, 2H), 1.55-1.68 (m, 2H), 2.10-2.29 (m, 2H), 2.63 (t, J=7 Hz, 2H), 5.27 (s, 2H), 7.00-7.05 (m, 2H), 7.10-7.17 (m, 2H), 7.38-7.43 (m, 1H), 7.48-7.64 (m, 2H), 7.92-7.97 (m, 1H); ¹⁹F NMR (CDCl₃) δ -97.38 (t, J = 18 Hz, 2F); MS (FAB) m/e (rel intensity) 452 (83), 432 (21), 235 (45), 207 (100), 192 (42), 178 (29); HRMS. Calcd for M+H: 452.2374. Found: 452.2398.

### Example 7

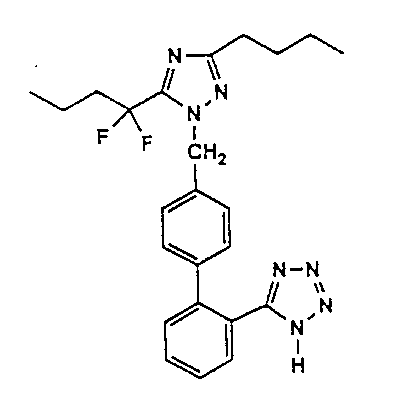

### 5-[4'-[[3-butyl-5-(1,1-difluorobutyl)-1H-1,2,4-triazol-1-yl]methyl][1,1'-biphenyl]-2-yl]-1H-tetrazole

The faster moving isomer isolated in Example 6 was hydrolyzed in an identical manner. Purification by reverse phase chromatography (Waters Delta Prep-3000) using 42% acetonitrile/water (0.05% TFA) for 20 minutes, 45% acetonitrile/water (0.05% TFA) for 20 minutes, 50% acetonitrile/water (0.05% TFA) for 20 minutes and then 60% acetonitrile/water (0.05% TFA). The acetonitrile was removed in vacuo, the pH was adjusted to pH4 with 1N NaOH, and the product extracted with ethyl acetate. The ethyl acetate was dried (MgSO₄) and removed in vacuo to give 150 mg (35%) of the 5-(1,1-difluoro)butyl isomer: NMR (CDCl₃) δ d 0.91 (t, J=7 Hz, 3H), 1.01 (t, J=7 Hz, 3H), 1.27-1.42 (m, 2H), 1.54-1.72 (m, 4H), 2.27-2.43 (m, 2H), 2.61 (t, J=7 Hz, 2H), 5.46 (s, 2H), 7.17-7.22 (m, 2H), 7.25-7.30 (m, 2H), 7.38-7.45 (m, 1H), 7.52-7.65 (m, 2H), 8.15-8.20 (m, 1H); MS (FAB) m/e (rel intensity) 452 (47), 235 (90), 207 (100), 178 (33); HRMS. Calcd for M+H: 452.2374. Found: 452.2418.

### Example 8

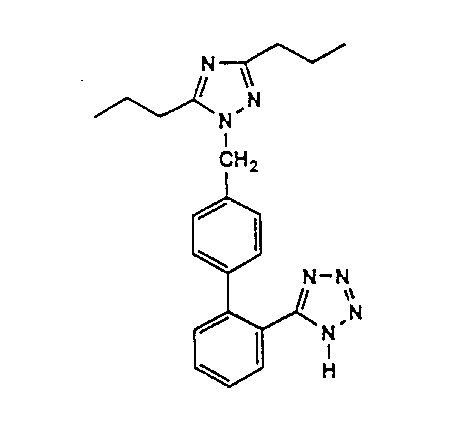

### 5-[4'-[(3,5-dipropyl-1H-1,2,4-triazol-1-yl)methyl][1,1'-biphenyl]-2-yl]-1H-tetrazole

### Step 1: Preparation of 3,5-dipropyl-1H-1,2,4-triazole.

Under nitrogen, a solution of 375 g (11.8 mol) of anhydrous hydrazine in 1600 mL of methanol was cooled to 0°C and treated with 1.0 kg (9.8 mol) of methyl butyrate. The reaction was allowed to warm to ambient temperature and stir overnight prior to stirring at reflux for 5 h. The reaction was concentrated in vacuo; recrystallization from toluene/ethyl acetate gave 825.5 g (83%) of butyric acid hydrazide as a colorless solid: mp 44.5-46.0°C; NMR (CDCl₃) δ 0.88 (t, J = 7 Hz, 3H), 1.53-1.68 (m, 2H), 2.09 (t, J = 7 Hz, 2H), 3.89 (br s, 2H), 7,62 (br s, 1H). A 5.0 g (43 mmol) sample of the hydrazide was dissolved in 40 mL of methanol and treated with 4.4 g (43 mmol) of ethyl iminobutyrate [P. Reynaud and R. C. Moreau, Bull. Soc. Chim. France, 2997 (1964)] under nitrogen. The reaction was stirred at reflux for 3 days and concentrated in vacuo. Purification by silica gel chromatography (Waters Prep-500A) using ethyl acetate/hexane (60:40) gave 5.37 g (81%) of 3,5-dipropyl-1H-1,2,4-triazole as a colorless solid: mp 69-70°C; NMR (CDCl₃) δ 0.92 (t, J = 7 Hz, 6H), 1.64-1.81 (m, 4H), 2.68 (t, J = 7 Hz, 4H); MS (FAB) m/e (rel intensity) 154 (100), 138 (5), 125 (5), 112 (3); HRMS. Calcd for M+H: 154.1344. Found: 154.1390.

### Step 2: Preparation of 5-[4'-[(3,5-dipropyl-1H-1,2,4-triazole-1-yl)methyl][1,1'-biphenyl]-2-yl]-1H-tetrazole.

Under a static nitrogen atmosphere, 2.00 g (13.1 mmol) of solid 3,5-dipropyl-1H-1,2,4-triazole was added in small portions to 14 mmol of sodium hydride in 50 ml of dimethylformamide (DMF); stirring was continued until hydrogen evolution had ceased. The anion solution was cooled to -10°C (ice/methanol) and treated with a solution of 7.3 g (13.1 mmol) of N-triphenylmethyl-5-[2-(4'-bromomethylbiphen-2-yl)]tetrazole (from Step 1 of Example 3) in 20 ml of dry DMF. The reaction was allowed to warm to ambient temperature and stir overnight. Methanol (10 ml) was added to destroy any unreacted sodium hydride and the DMF was removed in vacuo. The residue was dissolved in ethyl acetate, washed with water, and dried (MgSO₄). Silica gel chromatography using 33% ethyl acetate/toluene produced 6.5 g (78.6%) of the protected tetrazole which was dissolved in 50 ml of 10% water/acetic acid and stirred at ambient temperature overnight. The solvent was removed in vacuo and the crude product dissolved in water which was made basic with 2.5 N NaOH. The water was extracted with benzene, acidified, and extracted with ethyl acetate. The ethyl acetate was dried (MgSO₄) and removed in vacuo. Recrystallization from acetonitrile gave 2.67 g (67%) of a colorless solid: mp 164.5-167.0°C; NMR (CDCl₃) δ d 0.77 (t, J = 7 Hz, 3H), 0.83 (t, J = 7 Hz, 3H), 1.39-1.59 (m, 4H), 2.21 (t, J = 7 Hz, 2H), 2.38 (t, J = 7 Hz, 2H), 5.16 (s, 2H), 6.80-6.87 (m, 2H), 7.03-7.08 (m, 2H), 7.41-7.44 (m, 1H), 7.50-7.66 (m, 2H), 7.81-7.87 (m, 1H); MS (FAB) m/e (rel intensity) 388 (29), 207 (100), 192 (38), 178 (28), 165 (20), 154 (37); HRMS. Calcd for M+H: 388.2249. Found: 388.2284.

### Example 9

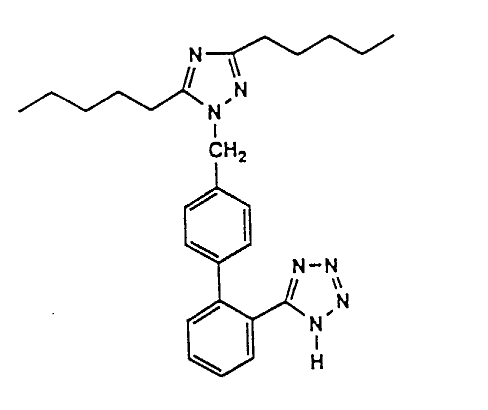

### 5-[4'-[(3,5-dipentyl-1H-1,2,4-triazol-1-yl)methyl][1,1'-biphenyl]-2-yl]-1H-tetrazole

### Step 1: Preparation of 3,5-dipentyl-1H-1,2,4-triazole.

Under nitrogen, a solution of 262 g (8.2 mol) of anhydrous hydrazine in 1300 mL of methanol was cooled to 0°C and treated with 885 g (6.8 mol) of methyl caproate. The reaction was allowed to warm to ambient temperature and stir overnight prior to stirring at reflux for 7 h. The reaction was concentrated in vacuo; recrystallization from toluene gave 707 g (80%) of caproic acid hydrazide as a colorless solid: mp 72.5-73.9°C; NMR (CDCl₃) δ 0.82 (t, J = 7 Hz, 3H), 1.15-1.33 (m, 4H), 1.51-1.64 (m, 2H), 2.10 (t, J = 7 Hz, 2H), 3.91 (br s, 2H), 7.56 (br s, 1H). A 5.0 g (39 mmol) sample of the hydrazide was dissolved in 40 mL of methanol and treated with 11.2 g (78 mmol) of ethyl iminocaproate [P. Reynaud and R. C. Moreau, Bull. Soc., Chim. France, 2997 (1964)-which was only about 50% pure by NMR] under nitrogen. The reaction was stirred at reflux for 2 days and concentrated in vacuo. Purification by silica gel chromatography (Waters Prep-500A) using ethyl acetate/hexane (48:52) gave 7.56 g (92%) of 3,5-dipentyl-1H-1,2,4-triazole as a colorless solid: mp 66-68°C; NMR (CDCl₃) δ 0.88 (t, J = 7 Hz, 6H), 1.24-1.41 (m, 8H), 1.66-1.81 (m, 4H), 2.72 (t, J = 7 Hz, 4H); MS (FAB) m/e (rel intensity) 210 (100), 166 (14); HRMS. Calcd for M+H: 210.1970. Found: 210.1964.

### Step 2: Preparation of 5-[4'[(3,5-dipentyl-1H-1,2,4-triazol-1-yl)methyl][1,1'-biphenyl]-2-yl]-1H-tetrazole.

Under a static nitrogen atmosphere, 2.0 g (9.6 mmol) of solid 3,5-dipentyl-1H-1,2,4-triazole was added in small portions to 10 mmol of sodium hydride in 50 ml of dimethylformamide (DMF); stirring was continued until hydrogen evolution had ceased. The anion solution was cooled to -10°C (ice/methanol) and treated with a solution of 5.35 g (9.6 mmol) of N-triphenylmethyl-5-[2-(4'-bromo-methylbiphen-2-yl)]-tetrazole (from Step 1 of Example 3) in 20 ml of dry DMF. The reaction was allowed to warm to ambient temperature and stir overnight. Methanol (10 ml) was added to destroy any unreacted sodium hydride and the DMF was removed in vacuo. The residue was dissolved in ethyl acetate, washed with water, and dried (MgSO₄). Silica gel chromatography using 33% ethyl acetate/hexane produced 5.1 g (78%) of the protected tetrazole which was taken up in 40 ml of 10% water in acetic acid and stirred at ambient temperature for 3 days. The solvent was removed in vacuo, the residue dissolved in water, and the pH adjusted to 9. The aqueous layer was washed in benzene, acidified and extracted with ethyl acetate. The solution was dried (MgSO₄) and concentrate in vacuo. Recrystallization from acetonitrile gave 2.1 g (62%) of a colorless solid: mp 116-120°C; NMR (CDCl₃) δ d 0.82 (t, J = 7 Hz, 6H), 1.07-1.31 (m, 8H), 1.40-1.60 (m, 4H), 2.22 (t, J = 7 Hz, 2H), 2.41 (t, J = 7 Hz, 2H), 5.15 (s, 2H), 6.80-6.87 (m, 2H), 7.03-7.10 (m, 2H), 7.41-7.48 (m, 1H), 7.52-7.66 (m, 2H), 7.87-7.93 (m, 1H); MS (FAB) m/e (rel intensity) 444 (100), 207 (72), 178 (13); HRMS. Calcd for M+H: 444.2876. Found: 444.2910.

### Example 10

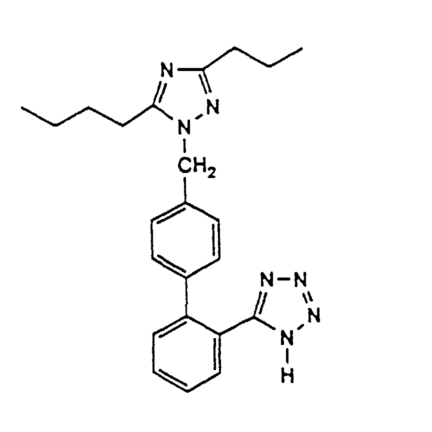

### 5-[4'-[(5-butyl-3-propyl-1H-1,2,4-triazol-1-yl)methyl]-[1,1'-biphenyl]-2-yl]-1H-tetrazole

### Step 1: Preparation of 5-butyl-3-propyl-1H-1,2,4-triazole.

A 3.95 g (38.7 mmol) sample of butyric acid hydrazide (from Step 1 of Example 8 was dissolved in 30 mL of methanol and treated with 5.0 g (38.8 mmol) of ethyl iminovalerate under nitrogen. The reaction was stirred at reflux for 3 days and concentrated in vacuo. Purification by silica gel chromatography (Waters Prep-500A) using ethyl acetate/hexane (80:20) gave 5.51 g (85%) of 5-butyl-3-propyl-1H-1,2,4-triazole as a colorless solid: mp 48.5-50.0°C; NMR (CDCl₃) δ 0.92 (t, J = 7 Hz, 3H), 0.97 (t, J = 7 Hz, 3H), 1.31-1.46 (m, 2H), 1.66-1.84 (m, 4H), 2.72 (t, J = 7 Hz, 2H), 2.75 (t, J = 7 Hz, 2H); MS (FAB) m/e (rel intensity) 168 (100), 166 (4), 152 (3), 138 (3), 125 (3); HRMS. Calcd for M+H: 168.1501. Found: 168.1534.

### Step 2: Preparation of 5-[4'-[(5-butyl-3-propyl-1H-1,2,4-triazol-1-yl)methyl][1,1'-biphenyl]-2-yl]-1H-tetrazole.

Under a static nitrogen atmosphere, 2.0 g (12 mmol) of solid 5-butyl-3-propyl-1H-1,2,4-triazole was added in small portions to 12 mmol of sodium hydride in 50 ml of dimethylformamide (DMF); stirring was continued until hydrogen evolution had ceased. The anion solution was cooled to -10°C (ice/methanol) and treated with a solution of 6.7 g (12 mmol) of N-triphenylmethyl-5-[2-(4'-bromo-methylbiphen-2-yl)]tetrazole (from Step 1 of Example 3) in 20 ml of dry DMF. The reaction was allowed to warm to ambient temperature and stir overnight. Methanol (10 ml) was added to destroy any unreacted sodium hydride and the DMF was removed in vacuo. The residue was dissolved in ethyl acetate, washed with water, and dried (MgSO₄). Silica gel chromatography using 15% ethyl acetate/toluene produced 5.96 g (77%) of a mixture of the two isomers which was dissolved in 40 ml of 10% water in acetic acid and stirred at ambient temperture ovenight. The solvent was removed in vacuo and the residue dissolved in 50% acetonitrile/water. Purification of 1 g sample of the isomeric product mixture by reverse phase chromatography (Waters Delta Prep-3000) using 25% acetonitrile/water (0.05% TFA) for 50 minutes followed by 28% acetonitrile/water (0.05% TFA) provided two isomers. The faster moving isomer compound was dissolved in the minimal amount of acetonitrile and diluted with water. The pH was adjusted to 8 with 1N NaOH, the acetonitrile removed in vacuo, the pH readjusted to 5 with 1N HC1, and the product extracted with ethyl acetate. The solvent was removed in vacuo and the product lyophylized from acetonitrile/water providing 175 mg (18%) of the 5-butyl-3-propyl isomer as a colorless solid: NMR (CDCl₃) δ d 0.78 (t, J = 7 Hz, 3H), 0.82 (t, J = 7 Hz, 3H), 1.20-1.33 (m, 2H), 1.41-1.57 (m, 4H), 2.21 (t, J = 7 Hz, 2H), 2.41 (t, J = 7 Hz, 2H), 5.16 (s, 2H), 6.82-6.89 (m, 2H), 7.05-7.11 (m, 2H), 7.42.7.48 (m, lH), 7.52-7.67 (m, 2H), 7.88-7.94 (m, 1H); MS (FAB) m/e (rel intensity) 402 (50), 207 (100), 192 (25), 168 (39), 152 (8), 125 (5); HRMS. Calcd for M+H: 402.2406. Found: 402.2424.

### Example 11

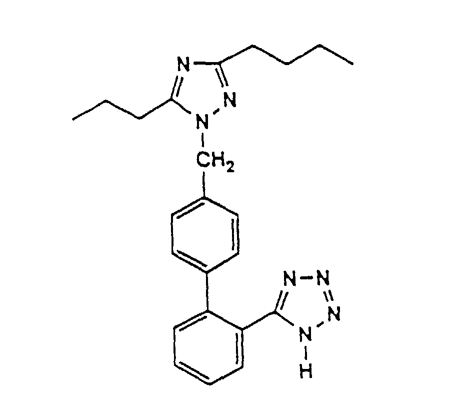

### 5-[4'-[(3-butyl-5-propyl-1H-1,2,4-triazol-1-yl)methyl]-[1,1'-biphenyl]-2-yl]-1H-tetrazole

The slower moving isomer for Example 10 was isolated in an identical manner and provided 108 mg (11%) of the 3-butyl-5-propyl isomer as a colorless solid: NMR (CDCl₃) δ d 0.86 (t, J = 7 Hz, 3H), 0.89 (t, J = 7 Hz, 3H), 1.19-1.32 (m, 2H), 1.48-1.69 (m, 4H), 2.41 (t, J = 7 Hz, 2H), 2.52 (t, J = 7 Hz, 2H), 5.22 (s, 2H), 6.95-7.02 (m, 2H), 7.11-7.16 (m, 2H), 7.42-7.48 (m, 1H), 7.53-7.68 (m, 2H), 7.97-8.02 (m, 1H); MS (FAB) m/e (rel intensity) 402 (50), 259 (10), 235 (5), 225 (10), 207 (100), 192 (38); HRMS. Calcd for M+H: 402.2406. Found: 402.2435.

### Example 12

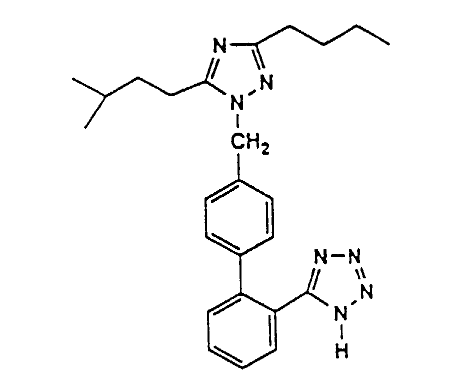

### 5-[4'-[(3-butyl-5-isopentyl-1H-1,2,4-triazol-1-yl)methyl]-[1,1'-biphenyl]-2-yl]-1H-tetrazole

### Step 1: Preparation of 3-butyl-5-isopentyl-1H-1,2,4-triazole.

Under nitrogen, a solution of 53.8 g (1.68 mol) of anhydrous hydrazine in 300 mL of methanol was cooled to 0°C and treated with 186 g (1.4 mol) of methyl 4-methylvalerate. The reaction was allowed to warm to ambient temperature and stir overnight prior to stirring at reflux for 4 h. The reaction was concentrated in vacuo giving 166 g (93%) of 4-methylvaleric acid hydrazide as a colorless solid: 49-51°C; NMR (CDCl₃) δ 0.89 (d, J = 7 Hz, 6H), 1.48-1.64 (m, 3H), 2.15 (t, J = 7 Hz, 2H), 3.91 (br s, 2H), 6.94 (br s, 1H). A 7.86 g (60 mmol) sample of the hydrazide was dissolved in 50 mL of methanol and treated with 7.8 g (60 mmol) of ethyl iminovalerate under nitrogen. The reaction was stirred at reflux overnight and concentrated in vacuo. Purification by silica gel chromatography (Waters Prep-500A) using ethyl acetate/hexane (40:60) gave 9.6 g (82%) of 3-butyl-5-isopentyl-1H-1,2,4-triazole as a colorless solid which melts close to ambient temperature: NMR (CDCl₃) δ 0.81-0.90 (m, 9H), 1.25-1.40 (m, 2H), 1.47-1.74 (m, 5H), 2.70 (t, J = 7 Hz, 4H); MS (FAB) m/e (rel intensity) 196 (100); HRMS. Calcd for M+H: 196.1814. Found: 196.1832.

### Step 2: Preparation of 5-[4'-[(3-butyl-5-isopentyl-1H-1,2,4-triazol-1-yl)methyl][1,1'-biphenyl]-2-yl]-1H-tetrazole.

Under a static nitrogen atmosphere, 2.0 g (10.3 mmol) of solid 3-butyl-5-isopentyl-1H-1,2,4-triazole was added in small portions to 11 mmol of sodium hydride in 50 ml of dimethylformamide (DMF); stirring was continued until hydrogen evolution had ceased. The anion solution was cooled to -10°C (ice/methanol) and treated with a solution of 5.7 g (10.2 mmol) of N-triphenylmethyl-5-[2-(4'-bromo-methylbiphen-2-yl)]tetrazole (from Step 1 of Example 3) in 20 ml of dry DMF. The reaction was allowed to warm to ambient temperature and stir overnight. Methanol (10 ml) was added to destroy any unreacted sodium hydride and the DMF was removed in vacuo. The residue was dissolved in ethyl acetate, washed with water, and dried (MgSO₄). The crude material (8.0 g) was deprotected in 50 ml of 10% water in acetic acid at ambient temperature overnight. Purification of 1.5 g sample by reverse phase chromatography (Waters Delta Prep-3000) using isocratic 30% acetonitrile/water (0.05% TFA) provided two isomers. The faster moving isomer compound was dissolved in the minimal amount of acetonitrile and diluted with water. The pH was adjusted to 9 with 1N NaOH, the acetonitrile removed in vacuo, the pH readjusted to 5 with 1N HCl, and the product extracted with ethyl acetate. The solvent was removed in vacuo and the product lyophylized from acetonitrile/water providing 33.1 mg (2.2%) of the 3-butyl-5-isopentyl isomer as a colorless solid: NMR (CDCl₃) δ d 0.85 (t, J = 7 Hz, 9H), 1.18-1.31 (m, 2H), 1.43-1.59 (m, 4H), 2,38 (t, J = 7 Hz, 2H), 2.54 (t, J = 7 Hz, 2H), 5.19 (s, 2H), 6.93-7.00 (m, 2H), 7.09-7.16 (m, 2H), 7.41-7.47 (m, 1H), 7.52-7.67 (m, 2H), 7.96-8.01 (m, 1H); MS (FAB) m/e (rel intensity) 430 (52), 387 (7), 207 (100), 196 (43), 178 (23), 165 (16), 152 (13); HRMS. Calcd for M+H: 430.2719. Found: 430.2772.

### Example 13

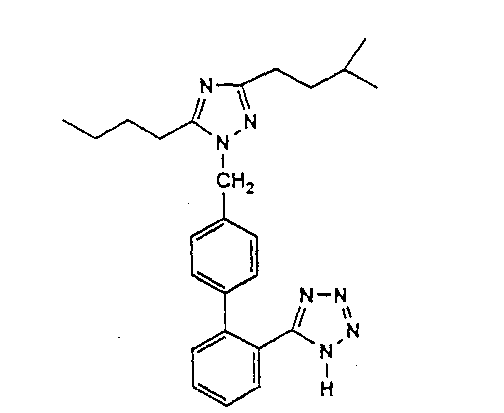

### 5-[4'-[(5-butyl-3-isopentyl-1H-1,2,4-triazol-1-yl]methyl)-[1,1'-biphenyl]-2-yl]-1H-tetrazole

The slower moving isomer from Example 12 was isolated in an identical manner and provided 36.5 mg (2.43%) of the 5-butyl-isopentyl isomer as a colorless solid: NMR (CDCl₃) δ d 0.79-0.88 (m, 9H), 1.22-1.36 (m, 2H), 1.38-1.61 (m, 5H), 5.18 (s, 2H), 6.88-6.96 (m, 2H), 7.08-7.14 (m, 2H), 7.41-7.47 (m, 1H), 7.53-7.67 (m, 2H) , 7.94-7.99 (m, 1H) : MS (FAB) m/e (rel intensity) 430 (38), 387 (8), 207 (100), 196 (37), 178 (27), 165 (15), 152 (12); HRMS. Calcd for M+H: 430.2719. Found: 430.2760.

### General Procedure A: The Reaction of Triazoles with Alkylating Reagents.

Under a static nitrogen atmosphere, 10-50 mmol of the 1H-1,2,4-triazole was added in small portions to 10-50 mmol of sodium hydride in 50 ml of dimethylformamide (DMF); stirring was continued until hydrogen evolution had ceased. The anion solution was cooled to -10 °C (ice/methanol) and treated with a solution of 10-50 mmol of the alkyling reagent from either step 1 of Example 1 or step 1 of Example 3 in 20 ml of dry DMF. The reaction was allowed to warm to ambient temperature and stir overnight. Methanol (10 ml) was added to destroy any unreacted sodium hydride and the DMF was removed in vacuo. The residue was dissolved in ethyl acetate, washed with water, and dried (MgSO₄). Silica gel chromatography (Waters Prep-500A) using ethyl acetate/hexane gave the pure coupled material as either a mixture of regioisomers or isomerically pure, depending upon the individual triazole coupled. A detailed procedure for the hydrolysis, purification, and isolation of the individual regioisomers when the alkylating reagent used was the reagent prepared in step 1 of Example 1 may be found in step 2 of Example 6. Likewise, a detailed procedure for the deprotection, purification, and isolation of the individual regioisomers, when the alkylating reagent used was the reagent prepared in step 1 of Example 3, may be found in Example 10.

### Example 14

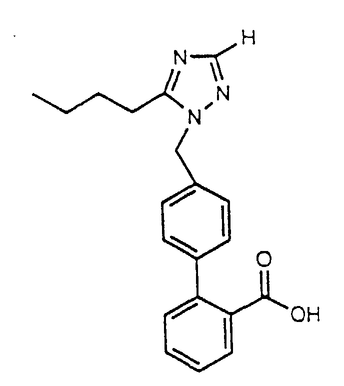

### 4'-[(5-butyl-1H-1,2,4-triazol-1-yl)methyl][1,1'-biphenyl]-2-carboxylic acid

Following General Procedure A, 5.0 g (39.9 mmol) of 5-butyl-1H-1,2,4-triazole was coupled with 12.2 g (39.9 mmol) of the alkylating reagent prepared in step 1 of Example 1 to give 3.1 g (22%) of a faster moving isomer: NMR (CDCl₃) δ 0.90 (t, J = 8 Hz, 3H), 1.29-1.45 (m, 2H), 1.63-1.76 (m, 2H), 2.71 (t, J = 8 Hz, 2H), 3.62 (s, 3H), 5.34 (s, 2H), 7.14-7.20 (m, 2H), 7.24-7.33 (m, 3H), 7.40 (dt, J = 8 and 2 Hz, 1H), 7.50 (dt, J = 8 and 2 Hz, 1H), 7.82 (dd, J = 8 and 2 Hz, 1H), 7.85 (s, 1H) and 3.7 g (26%) of a slower moving isomer: NMR (CDCl₃) δ 0.92 (t, J = 8 Hz, 3H), 1.31-1.46 (m, 2H), 1.66-1.79 (m, 2H), 2.73 (t, J = 8 Hz, 2H), 3.62 (s, 3H), 5.28 (s, 2H), 7.21-7.34 (m, 5H), 7.39 (dt, J = 8 and 2 Hz, 1H), 7.50 (dt, J = 8 and 2 Hz, 1H), 7.83 (dd, J = 8 and 2 Hz, 1H), 7.94 (s, 1H). The faster moving isomer was hydrolyzed to give 2.75 g (92%) of 4'-[(5-butyl-1H-1,2,4-triazol-1-yl)methyl]-[1,1'-biphenyl]-2-carboxylic acid as a colorless solid: NMR (DMSO-d₆) δ 0.85 (t, J = 8 Hz, 3H), 1.23-1.38 (m, 2H), 1.52-1.65 (m, 2H), 2.76 (t, J = 7 Hz, 2H), 5.41 (s, 2H), 7.16-7.24 (m, 2H), 7.27-7.37 (m, 3H), 7.44 (dt, J = 8 and 2 Hz, 1H), 7.56 (dt, J = 8 and 2 Hz, 1H), 7.72 (dd, J = 8 and 2 Hz, 1H), 7.86 (s, 1H); MS (FAB) m/e (rel intensity) 336 (100), 307 (7), 289 (7), 224 (8), 211 (100), 193 (9).

### Example 15

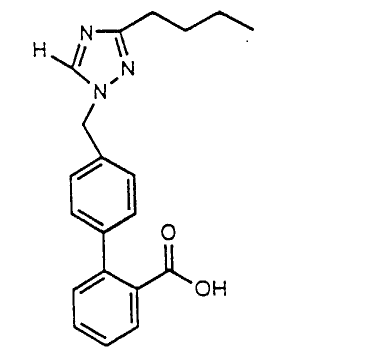

### 4'-[(3-butyl-1H-1,2,4-triazol-1-yl)methyl][1,1'-bipenyl]-2-carboxylic acid

The slower moving isomer from Example 14 was hydrolyzed to give 2.3 g (67%) of 4'-[(3-butyl-1H-1,2,4-triazol-1-yl)methyl][1,1'-biphenyl]-2-carboxylic acid as a colorless solid: NMR (DMSO-d₆) δ 0.87 (t, J = 8 Hz, 3H), 1.23-1.38 (m, 2H), 1.54-1.67 (m, 2H), 2.58 (t, J = 8 Hz, 2H), 5.36 (s, 2H), 7.23-7.38 (m, 5H), 7.44 (dt, J = 8 and 2 Hz, 1H), 7.56 (dt, J = 8 and 2 Hz, 1H), 7.72 (dd, J = 8 and 2 Hz, 1H), 8.51 (s, 1H); MS (FAB) m/e (rel intensity) 336 (85), 211 (100), 165 (24), 126 (52).

### Example 16

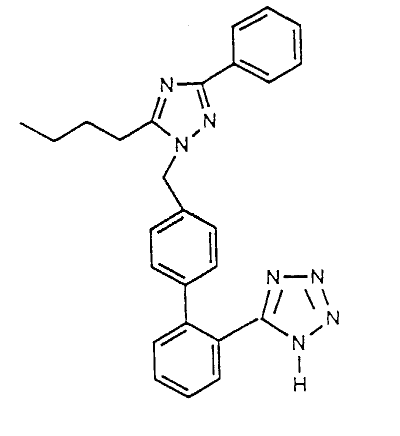

### 5-[4'-[(3-phenyl-5-butyl-1H-1,2,4-triazol-1-yl)methyl](1,1'-biphenyl)-2-yl]-1H-tetrazole

Following General Procedure A, 2.0 g (9.9 mmol) of 5-butyl-3-phenyl-1H-1,2,4 triazole was coupled with 5.5 g (9.9 mmol) of the alkylating reagent prepared in step 1 of Example 3 to give 5.2 g (77%) of a faster moving isomer: NMR (CDCl₃) 60.90 (t, J = 8 Hz), 1.30-1.44 (m, 2H), 1.63-1.77 (m, 2H), 2.67 (t, J = 8 Hz, 2H), 5.24 (s, 2H), 6.88-7.01 (m, 8H), 7.08-7.53 (m, 17H), 7.95 (dd, J = 8 and 2 Hz, 1H), 8.12 (dd, J = 8 and 2 Hz, 2H) and 420 mg (6.3%) of a slower moving isomer: NMR (CDCl₃) δ 0.96 (t, J = 8 Hz, 3H), 1.38-1.52 (m, 2H), 1.75-1.89 (m, 2H), 2.84 (t, J = 8 Hz, 2H), 5.27 (s, 2H), 6.88-6.97 (m, 8H), 7.13 (d, J = 8 Hz, 2H), 7,17-7.54 (m, 15H), 7.57 (d, J = 8 Hz, 2H), 7.93 (dd, J = 8 and 2 Hz, 1H). A 4.8 g (7.1 mmol) sample of the faster moving isomer was deportected to give 1.64 g (53%) of 5-[4'-[(3-phenyl-5-butyl-1H-1,2,4-triazol-1-yl)methyl](1,1'-biphenyl-2-yl]-1H-tetrazole as a colorless solid: mp 113 °C (dec); NMR (CDCl₃) δ 0.87 (t, J = 8 Hz, 3H), 1.24-1.39 (m, 2H), 1.55-1.69 (m, 2H), 2.60 (t, J = 8 2Hz, 5.19 (s, 2H), 7.04 (d, J = 8 Hz, 2H), 7.11 (d, J = 8 Hz, 2H), 7.27-7.39 (m, 4H), 7.47-7.60 (m, 2H), 7.87 (dd, J = 8 and 2 Hz, 2H), 7.95 (dd, J = 8 and 2 Hz, 1H); MS (FAB) m/e (rel intensity) 436 (100), 393 (8), 207 (60), 192 (20), 178 (13), 165 (8); HRMS. Calc'd for M+H: 436.2249. Found: 436.2240.

### Example 17

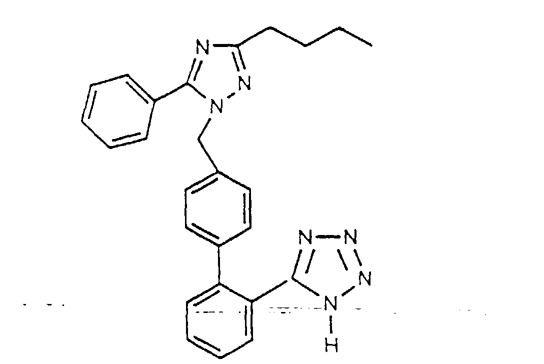

### 5-[4'-[(3-butyl-phenyl-1H-1,2,4-triazol-1-yl)methyl](1,1'-biphenyl)-2-yl]-1H-tetrazole

The slower moving isomer from Example 16 was deprotected to give 251 mg (60%) of 5-[4'-[(3-butyl-5-phenyl-1H-1,2,4-triazol-1-yl)methyl][1,1'-biphenyl]-2-yl]-1H-tetrazole as a colorless solid: mp 205 °C (dec); NMR (CDCl₃) δ 0.88 (t, J = 8 Hz, 3H), 1.23-1.38 (m, 2H), 1.55-1.68 (m, 2H), 2.52 (t, J = 8 Hz, 2H), 5.32 (s, 2H), 6.99 (d, J = 8 Hz, 2H), 7.08 (d, J = 8 Hz, 2H), 7.31-7.61 (m, 8H), 7.90 (dd, J = 8 and 2 Hz, 1H); MS (FAB) m/e (rel intensity) 436 (100), 393 (9), 374 (7), 277 (7), 247 (15), 207 (45); HRMS. Calc'd for M+H: 436.2249. Found: 436.2201.

### Example 18

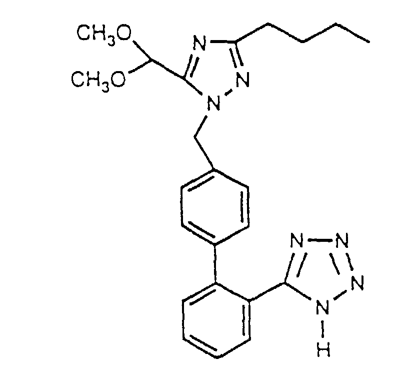

### 5-[4'-[[3-butyl-5-(dimethoxymethyl)-1H-1,2,4-triazol-1-yl]methyl][1,1'-biphenyl]-2-yl]-1H-tetrazole

Following General Procedure A, 2.0 g (10 mmol) bf 5-butyl-3-(dimethoxymethyl)-1H-1,2,4-triazole was coupled with 5.6 g (10 mmol) of the alkylating regagent prepared in step 1 of Example 3 to give 1.65 g (24%) of a faster moving isomer: NMR (CDCl₃) δ 0.92 (t, J = 8 Hz, 3H), 1.31-1.45 (m, 2H), 1.66-1.78 (m, 2H), 2.69 (t, J = 8 Hz, 2H), 3.33 (s, 6H), 5.30 (s, 2H), 5.42 (s, 1H), 6.90-6.96 (m, 5H), 7.02-7.12 (m, 4H), 7.21-7.38 (m, 11H), 7.41-7.52 (m, 2H), 7.86-7.92 (m, 1H) and 3.65 g (54.0%) of a slower moving isomer: NMR (CDCl₃) δ 0.85 (t, J = 8 Hz, 3H), 1.22-1.36 (m, 2H), 1.57-1.70 (m, 2H), 2.59 (t, J = 8 Hz, 2H), 3.45 (s, 6H), 5.20 (s, 2H), 5.55 (s, 1H), 6.89-6.97 (m, 5H), 7.08-7.19 (m, 4H), 7.21-7.37 (m, 11H), 7.41-7.52 (m, 2H), 7.90-7.95 (m, 1H). The faster moving isomer was deprotected to give 737 mg (70%) of 5-[4'-[[3-butyl-5-(dimethoxymethyl)-1H-1,2,4-triazol-1-yl]methyl][1,1'-biphenyl]-2-yl]-1H-tetrazole as a colorless solid: mp 152.5-153.5 °C; NMR (CDCl₃) δ 0.87 (t, J = 8 Hz, 3H), 1.20-1.30 (m, 2H), 1.53-1.65 (m, 2H, 2.46 (t, J = 8 Hz, 2H), 3.30 (s, 6H), 5.37 (s, 2H), 5.40 (s, 1H), 7.05-8.05 (m, 4H), 7.45 (dd, J = 8 and 2 Hz, 1H), 7.51-7.65 (m, 2H), 8.02 (d, J = 8 Hz, 1H); MS (FAB) m/e (rel intensity) 434 (65), 402 (40), 370 (11), 342 (46), 249 (85), 235 (18), 207 (100), 192 (89), 168 (70); HRMS. Calc'd for M+H: 434.2304. Found: 434.2332.

### Example 19

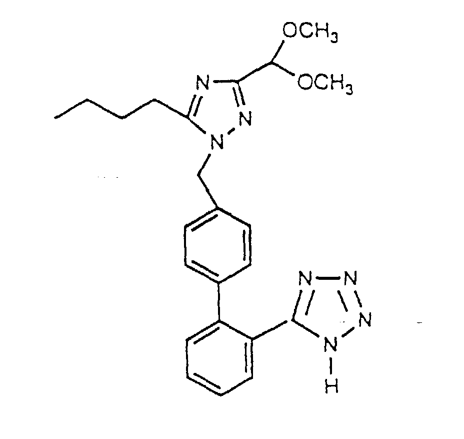

### 5-[4'-[(5-butyl-3-dimethoxymethyl-1H-1,2,4-triazol-1-yl)methyl](1,1'-biphenyl-2-yl]-1H-tetrazole

A 3.65 g (5.4 mmol) sample of the slower moving isomer from Example 18 was deprotected to give 108 mg of colorless 5-[4'-[[5-butyl-3-(dimethoxymethyl)-1H-1,2,4-triazol-1-yl]methyl][1,1'-biphenyl]-2-yl]-1H-tetrazole after lyophilization: NMR (CDCl₃) δ 0.86 (t, J = 8 Hz, 3H), 1.24-1.39 (m, 2H), 1.56-1.70 (m, 2H), 2.66 (t, J = 8 Hz, 2H), 3.30 (s, 6H), 5.23 (s, 2H), 5.35 (s, 1H), 7.02 (d, J = 8 Hz, 2H), 7.10 (d, J = 8 Hz, 2H), 7.40 (dd, J = 8 and 2 Hz, 1H), 7.51 (dt, J = 8 and 2 Hz, 1H), 7.59 (dt, J = 8 and 2 Hz, 1H), 7.89 (dd, J = 8 and 2 Hz, 1H); MS (FAB) m/e (rel intensity) 434 (10), 402 (40), 249 (100), 207 (36), 192 (54), 168 (12); HRMS. Calc'd for M+H: 434.2304. Found: 434.2271.

### Example 20

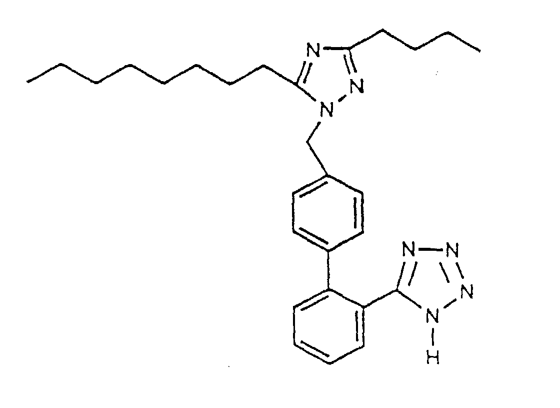

### 5-[4'-[(3-butyl-5-octyl-1H-1,2,4-triazol-1-yl)methyl][1,1'-biphenyl]-2-yl]-1H-tetrazole

Following General Procedure A, 2.0 g (8.4 mmol) of 3-butyl-5-octyl-1H-1,2,4-triazole was coupled with 5.5 g (8.4 mmol) of the alkylating reagent prepared in step 1 of Example 3. Silica gel chromatography (Waters Prep 500-A) using 25% ethyl acetate/hexane produced 4.5 g (75.4%) of a mixture of the two isomers which was dissolved in 40 ml of 10% water/acetic acid and stirred at ambient temperature overnight. The solvent was removed in vacuo. Purification of a sample of the isomeric product mix by reverse phase chromatography (Waters Delta Prep-3000) using 45% acetonitrile/water (0.05% TFA) for 30 minutes followed by 50% acetonitrile/water (0.05% TFA) provided two isomers. The faster moving isomer compound was dissolved in dilute base, the water was acidified (pH 4-5) with 1N HCl, and the product extracted with ethyl acetate. The ethyl acetate was dried (MgSO₄) and removed in vacuo yielding a solid which was recrystallized from acetonitrile providing 92 mg of 5-[4'-[(3-butyl-5-octyl-1H-1,2,4-triazol-1-yl)methyl][1,1'-biphenyl]-2-yl]-1H-tetrazole as a colorless solid: mp 136.5-138.0°C; NMR (CDCl₃) δ 0.82 (t, J = 8 Hz, 3H), 0.86 (t, J = 8 Hz, 3H), 1.11-1.33 (m, 12H), 1.40-1.60 (m, 4H), 2.27 (t, J = 8 Hz, 2H), 2.44 (t, J = 8 Hz, 2H), 5.16 (s, 2H), 6.87 (d, J = 8 Hz, 2H), 7.07 (d, J = 8 Hz, 2H), 7.44 (dd, J = 8 and 2 Hz, 1H), 7.50-7.66 (m, 2H), 7.90 (dd, J = 8 and 2 Hz, 1H); MS (FAB) m/e (rel intensity) 472 (5), 207 (7); HRMS. Calc'd for M+H: 472.3189. Found: 472.3180.

### Example 21

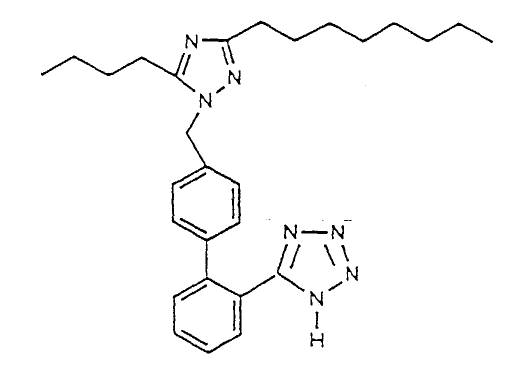

### 5-[4'-[(5-butyl-3-octyl-1H-1,2,4-triazol-1-yl)methyl][1,1'-biphenyl]-2-yl]-1H-tetrazole

The slower moving isomer from Example 20 was isolated in an identical manner and the product lyophilized from acetonitrile/water providing 53 mg of 5-[4'-[(5-butyl-3-octyl-1H-1,2,4-triazol-1-yl)methyl][1,1'-biphenyl]-2-yl]-1H-tetrazole as a colorless solid: NMR (CDCl₃) 6 0.83 (t, J = 8 Hz, 3H), 0.86 (t, J = 8 Hz, 3H), 1.12-1.37 (m, 12H), 1.46-1.63 (m, 4H), 2.35 (t, J = 8 Hz, 2H), 2.51 (t, J = 8 Hz, 2H), 5.19 (s, 2H), 6.92 (d, J = 8 Hz, 2H), 7.09 (d, J = 8 Hz, 2H), 7.40-7.48 (m, 1H), 7.51-7.66 (m, 2H), 7.91 (dd, J = 8 and 2 Hz, 1H); MS (FAB) m/e (rel intensity) 472 (6), 207 (8); HRMS. Calc'd for M+H: 472.3189. Found: 472.3230.

### Example 22

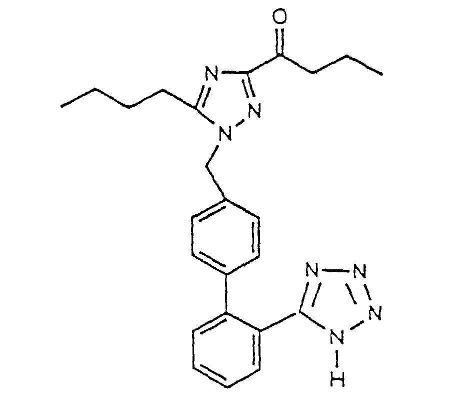

### 1-[[5-butyl-1-[2'-(1H-tetrazol-5-yl)[1,1'-biphenyl]-4-ylmethyl]-1H-1,2,4-triazol-3-yl]methyl]-1-butanone

Under a static nitrogen atmosphere, 30.0 g (150 mmol) 5-butyl-3-dimethoxy-1H-1,2,4-triazole (from Step 1 of Example 18 was added in small portions to 165 mmol of sodium hydride in 300 ml anhydrous THF; stirring was continued for 1 h. The anion solution was cooled to -10 °C (methanol/ice) and treated with Sem-Cl dropwise. The reaction was allowed to warm to ambient temperature and stir overnight. The solvent was removed in vacuo. The residue was dissolved in methylene chloride, washed with water, dried (MgSO₄), and the solvent removed again in vacuo. Silica gel chromatography using 20% ethyl acetate/hexane followed by ethyl acetate provided 13.6 g of the faster movfing isomer as an oil: NMR (CDCl₃) δ -0.07 (s, 9H), 0.82-0.92 (m, 5H), 1.26-1.40 (m, 2H), 1.62-1.74 (m, 2H), 2.67 (t, J = 8 Hz, 2H), 3.38 (s, 6H), 3.53-3.62 (m, 2H), 5.47 (s, 2H), 5.55 (s, 1H). Under a static nitrogen atmosphere, 12.6 g (38.2 mmol) of the faster moving Sem-protected triazole isomer from above was dissolved in 630 ml of anhydrous THF, cooled to -78 °C, and 45.8 mmol of sec-butyl lithium was added dropwise. The solution was stirred for 1 h and then the anion was quenched with 4.5 ml (45.8 mmol) of n-propyl iodide. The solution was stirred at -78 °C for 5 h and then allowed to warm to ambient temperature overnight. The solvent was removed in vacuo. The residue was dissolved in methylene chloride, washed with water, dried (MgSO₄) and the solvent removed providing 12.4 g of crude Sem-protected 5-butyl-3-(1,1-dimethoxybutane)-1H-1,2,4-triazole: NMR (CDCl₃) δ -0.04 (s, 9H), 0.75-0.93 (m, 8H), 0.98-1.11 (m, 2H), 1.25-1.41 (m, 2H), 1.62-1.74 (m, 2H), 1.99-2.08 (m, 2H), 2.68 (t, J = 8 Hz, 2H), 3.20 (s, 6H), 3.62 (t, J = 8 Hz, 2H), 5.56 (s, 2H). A 1.0 g (2.7 mmol) sample of the crude dimethoxybutyl compound was dissolved in 5 ml of ethanol and 5 ml of 3M HCl and stirred at reflux for 2.5 h. The solvent was removed in vacuo providing 560 mg of the crude HCl salt of 5-butyl-3-(1-butanone)-1H-1,2,4-triazole: NMR (DMSO-d₆) δ 0.83-0.94 (m, 6H), 1.21-1.36 (m, 2H), 1.55-1.75 (m, 4H), 2.83 (t, J = 8 Hz, 2H), 2.97 (t, J = 8 Hz, 2H). The crude HCl salt of the triazole was dissolved in 20 ml of fresh methanol, 3A molecular sieves were added, and the mixture stirred at reflux under nitrogen overnight. The solution was filtered through celite and the solvent removed in vacuo. The residue was dissolved in ethyl acetate and washed with saturated sodium bicarbonate solution. The solvent was dried (MgSO₄) and removed in vacuo providing 390 mg of crude 5-butyl-3-(1,1-dimethoxybutyl)-1H-1,2,4-triazole: NMR (CDCl₃) δ 0.90 (t, J = 8 Hz, 3H), 0.99 (t, J = 8 Hz, 3H), 1.29-1.46 (m, 2H), 1.66-1.84 (m, 4H), 2.80 (t, J = 8 Hz, 2H), 3.06 (t, J = 8 Hz, 2H), 3.18 (s, 6H). Under a static nitrogen atmosphere, 380 mg (1.6 mmol) of this material in 5 ml of anhydrous dimethylformamide (DMF) was added to 1.9 mmol sodium hydride in 5 ml of DMF; stirring was continued for 1 h. The anion solution was cooled to 0 °C and 890 mg (1.6 mmol) of the alkylating reagent prepared in from step 1 of Example 3 was added as a solid. The reaction was allowed to warm to ambient temperature and stir overnight. Methanol (1 ml) was added to destroy any unreacted sodium hydride and the DMF was removed in vacuo. The residue was dissolved in ethyl acetate, washed with water, and dried (MgSO₄). The solvent was removed in vacuo providing 1.2 g of crude material which was dissolved in 10 ml ethanol and 10 ml 3N HCl and stirred at reflux for 2 h. The solvent was removed in vacuo. Purification by reverse phase chromatography (Water Delta Prep 3000) using isocratic 37% acetonitrile/water (0.05% TFA) for 40 minutes followed by 50% acetonitrile/water (0.05% TFA) provided the TFA salt. The salt was dissolved in basic water (pH 9-10), the water was acidified to pH 4 with 3N HCl, and the product extracted with ethyl acetate. The ethyl acetate was dried (MgSO₄) and removed in vacuo yielding a solid which was recrystallized from acetonitrile providing 146 mg (21 %) of 1-[[5-butyl-1-[2'-(1H-tetrazol-5-yl)[1,1'-biphenyl]-4-ylmethyl]-1H-1,2,4-triazol-3-yl]methyl]-1-butanone mp 150.5-152.5°C; NMR (CDCl₃) δ 0.85 (t, J = 8 Hz, 3H), 0.96 (t, J = 8 Hz, 3H), 1.23-1.39 (m, 2H), 1.54-1.77 (m, 4H), 2.67 (t, J = 8 Hz, 2H), 2.97 (t, J = 8 Hz, 2H), 5.28 (s, 2H), 7.02 (d, J = 8 Hz, 2H), 7.10 (d, J = 8 Hz, 2H), 7.37 (d, J = 8 Hz, 1H), 7.43-7.62 (m, 2H), 7.86 (d, J = 8 Hz, 1H); MS (FAB) m/e (rel intensity) 430 (6), 207 (12); HRMS. Calc'd for M+H: 430.2355. Found: 430.2404.

### Example 23

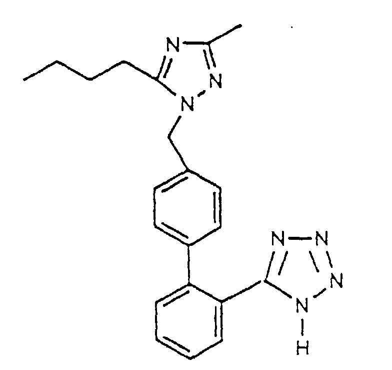

### 5-[4'-(5-butyl-3-methyl-1H-1,2,4-triazol-1-yl]methyl]-[1,1'-biphenyl]-2-yl]-1H-tetrazole

Following General Procedure A, 700 mg (5.0 mmol) of 5-butyl-3-methyl-1H-1,2,4-triazole was coupled with 2.8 g (5.0 mmol) of the aklylating reagent prepared in step 1 of Example 3. Silica gel chromatography using ethyl acetate/toluene (35:65) produced 2.75 g (90%) of a mixture of the two isomers which was dissolved in 30 ml 10% water/acetic acid and sitrred at ambient temperature for 3 days. The solvent was removed in vacuo and the residue dissolved in dilute base, washed with toleune, acidified to pH 4 with 1N HCl and the product extracted with ethyl acetate. Purification of the isomeric product mixture by reverse phase chromatography (Water Delta Prep-3000) using isocratic acetonitrile/water (23:77) (0.05% TFA) provided two isomers. The faster moving isomer compound was dissolved in dilute base, acidified to pH 3-4 and extracted with ethyl acetate. The solvent was removed in vacuo; recrystallization from acetonitrile gave 199 mg (12%) of 5-[4'-[(5-butyl-3-propyl-1H-1,2,4-triazol-1-yl)methyl]-[1,1'-biphenyl]-2-yl]-1H-tetrazole as a colorless solid: mp 170.0-170.5°C; NMR (CDCl₃) δ 0.79 (t, J = 8 Hz, 3H), 1.18-1.32 (m, 2H), 1.44-1.56 (m, 2H), 1.99 (s, 3H), 2.49 (t, J = 8 Hz, 2H), 5.15 (s, 2H), 6.87 (d, J = 8 Hz, 2H), 7.08 (d, J = 8 Hz, 2H), 7.44 (dd, J = 8 and 2 Hz, 1H), 7.51-7.65 (m, 2H), 7.90 (dd, J = 8 and 2 Hz, 1H); MS (FAB) m/e (rel intensity) 374 (100), 235 (13), 207 (100), 140 (29); HRMS. Calc'd for M+H: 374.2093. Found: 374.2062.

### Example 24

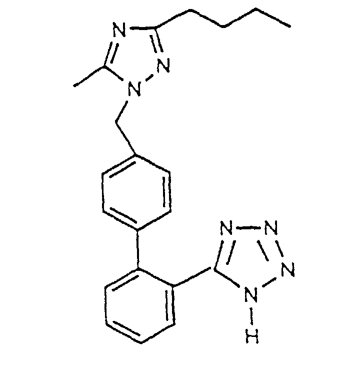

### 5-[4'-[(3-butyl-5-methyl-1H-1,2,4-triazol-1-yl]methyl][1,1'-biphenyl]-2-yl]-1H-tetrazole

The slower moving isomer from Example 23 was dissolved in dilute base, acidified to pH 3-4, and extraced with ethyl acetate. The solvent was removed in vacuo and the product lyophilized from acetonitrile/water which gave 536 mg (33 %) of 5-[4'-[(3-butyl-5-methyl-1H-1,2,4-triazol-1-yl)methyl]-[1,1'-biphenyl]-1-yl]-1H-tetrazole as a colorless solid: NMR (CDCl₃) δ 0.82 (t, J = 8 Hz, 3H), 1.21-1.35 (m, 2H), 1.53-1.66 (m, 2H), 2.35 (s, 3H), 2.56 (t, J = 8 Hz, 2H), 5.23 (s, 2H), 7.03 (d, J = 8 Hz, 2H), 7.11 (d, J = 8 Hz, 2H), 7 .42 (dd, J = 8 and 2 Hz, 1H), 7.47-7.63 (m, 2H), 7.88 (dd, J = 8 and 2 Hz, 1H); MS (FAB) m/e (rel intensity) 374 (65), 331 (12), 235 (13), 207 (100), 192 (38), 140 (56); HRMS. Calc'd for M+H: 374.2093. Found: 374.2071.

### Example 25

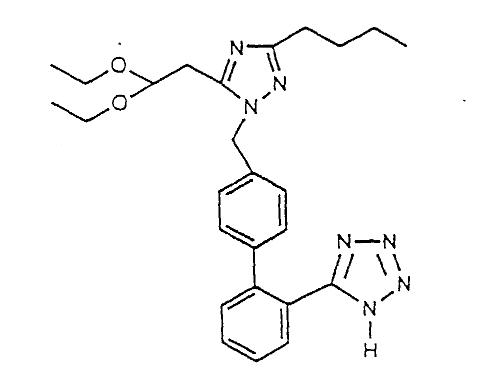

### 5-[4'-[[3-butyl-5-(2,2-diethyoxyethyl)-1H-1,2,4-triazol-1-yl]methyl][1,1'-biphenyl]-2-yl]-1H-tetrazole

Following General Procedure A, 4.7 g (19.5 mmol) of 5-butyl-3-(2,2-diethoxyethyl)-1H-1,2,4-triazole was coupled with 19.5 mmol of the alkylating reagent prepared in step 1 of Example 3. Silica gel chromatography (Waters Prep 500A) using ethyl acetate/hexane (2:3) gave 5.98 g (43%) of a faster moving isomer: NMR (CDCl₃) δ 0.94 (t, J = 8 Hz, 3H), 1.13 (t, J = 8 Hz, 6H), 1.33-1.48 (m, 2H), 1.67-1.79 (m, 2H), 2.70 (t, J = 8 Hz, 2H), 2.94 (d, J = 8 Hz, 2H), 3.39-3.51 (m, 2H), 3.63-3.75 (m, 2H), 4.81 (t, J = 7 Hz, 1H), 5.22 (s, 2H), 6.87-6.98 (m, 8H), 7.09 (d, J = 8 Hz, 2H), 7.21-7.29 (m, 6H), 7.30-7.37 (m, 4H), 7.41-7.52 (m, 2H), 7.91-7.96 (m, 1H) and 6.3 g (45%) of a slower moving isomer: NMR (CDCl₃) δ 0.88 (t, J = 8 Hz, 3H), 1.15 (t, J = 7 Hz, 6H), 1.24-1.37 (m, 2H), 1.56-1.68 (m, 2H), 2.57 (t, J = 8 Hz, 2H), 3.06 (d, J = 7 Hz, 2H), 3.46-3.60 (m, 2H), 3.66-3.78 (m, 2H), 5.05 (t, J =7 Hz, 1H), 5.14 (s, 2H), 6.87-6.95 (m, 8H), 7.10 (d, J = 8 Hz, 2H), 7.21-7.29 (m, 6H), 7.31-7.37 (m, 4H), 7.42-7.53 (m, 2H), 7.90-7.96 (m, 1H). The faster moving isomer was dissolved in 50 ml of acetic acid/water (4:1) and stirred at ambient temperature for 3 days. The solvent was removed in vacuo; the residue dissolve in dilute base and washed with toluene. The water layer was cooled to 0 °C, acidified to pH 4-5 and extracted with ethyl acetate. Lyophilization from acetonitrile/water gave 3.8 g (98%) of 5-[4'-[[3-butyl-5-(2,2-diethyoxyethyl)-1H-1,2,4-triazol-1-yl]methyl][1,1'-biphenyl]-2-yl]-1H-tetrazole as a colorless solid: NMR (CDCl₃) δ 0.80 (t, J = 8 Hz, 3H), 1.10 (t, J = 8 Hz, 6H), 1.10-124 (m, 2H), 1.37-1.49 (m, 2H), 2.31 (t, J = 8 Hz, 2H), 2.66 (d, J = 7 Hz, 2H), 3.34-3.47 (m, 2H), 3.59-3.71 (m, 2H), 4.75 (t, J = 7 Hz, 1H), 5.28 (s, 2H), 6.88 (d, J = 8 Hz, 2H), 7.06 (d, J = 8 Hz, 2H), 7.46 (dd, J = 8 and 2 Hz, 1H), 7.55 (dt, J = 8 and 2 Hz, 1H), 7.62 (dt, J = 8 and 2 Hz, 1H), 7.91 (dd, J = 8 and 2 Hz, 1H); MS (FAB) m/e (rel intensity) 476 (20), 430 (28), 356 (13), 235 (15), 207 (100), 192 (67); HRMS. Calc'd for M+H: 476.2773. Found: 476.2723.

### Example 26

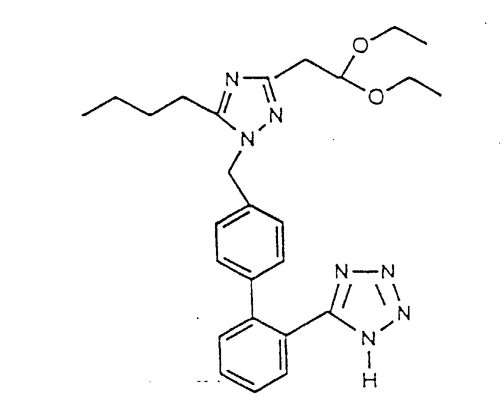

### 5-[4'-[[5-butyl-3-(2,2-diethyoxyethyl)-1H-1,2,4-triazol-1-yl]methyl][1,1'-biphenyl]-2-yl]-1H-tetrazole

The 6.2 g (8.6 mmol) of the slower moving isomer from Example 25 was dissolved in 50 ml of acetic acid/water (4:1) and stirred at ambient temperature for 3 days. The solvent was removed in vacuo; the residue dissolved in dilute base, and washed with toluene. The water layer was cooled to 0 °C, acidified with 1N HCl to pH 4-5, and extracted with ethyl acetate. Lyophilization from acetonitrile/water gave 3.7 g (92%) of 5-[4'-[[5-butyl-3-(2,2-diethyoxyethyl)-1H-1,2,4-triazol-1-yl]methyl][1,1'-biphenyl]-2-yl]-1H-tetrazole as colorless solid: NMR (CDCl₃) δ 0.85 (t, J = 8 Hz, 3H), 1.09 (t, J = 8 Hz, 6H), 1.24-1.38 (m, 2H, 1.51-1.63 (m, 2H), 2.55 (t, J = 8 Hz, 2H), 2.75 (d, J = 7 Hz, 2H), 3.38-3.50 (m, 2H), 3.54-3.66 (m, 2H), 4.87 (t, J = 7 Hz, 1H), 5.19 (s, 2H), 6.99 (d, J = 8 Hz, 2H), 7.12 (d, J = 8 Hz, 2H), 7.42 (dd, J = 8 and 2 Hz, 1H), 7.50-7.65 (m, 2H), 7.97 (dd, J = 8 and 2 Hz, 1H) ; MS (FAB) m/e (rel intensity) 476 (3), 430 (28), 235 (10), 207 (100), 192 (61); HRMS. Calc'd for M+H: 476.2773. Found: 476.2760.

### Example 27

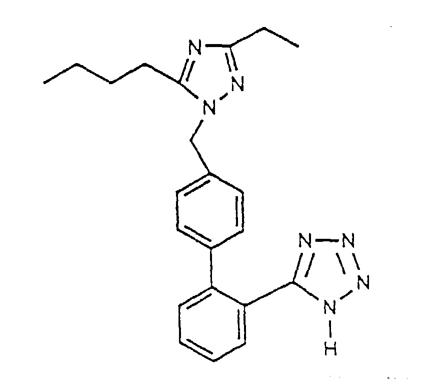

### 5-[4'-[(5-butyl-3-ethyl-1H-1,2,4-triazol-1-yl)methyl][1,1'-biphenyl]-2-yl]-1H-tetrazole

Following General Procedure A, 770 mg (5 mmol) of 5-butyl-3-ethyl-1H-1,2,4-triazole was coupled with 2.8 g (5 mmol) of the alkylating reagent prepared in step 1 of Example 3. Silica gel chromatography (Waters Prep-500A) using ethyl acetate/hexane (2:3) gave 2.24 g (72%) of a mixture of the two isomers which was dissolved in 30 ml of acetic acid/water (9:1) and stirred at ambient temperature for 4 days. The solvent was removed in vacuo; the residue dissolved in dilute base, and washed with toluene. The water layer was acidified to pH 4 and extracted with ethyl acetate. The extracts were combined, dried (MgSO₄) and concentrated in vacuo. Purification of a 600 mg sample of the isomeric product mixture by reverse phase chromatography (Waters Delta Prep-3000) using isocratic acetonitrile/water (25:75) (0.05% TFA) provided two isomers. The faster moving isomer was dissolved in dilute base, acidified, extracted with ethyl acetate, dried (MgSO₄) and the ethyl acetate removed in vacuo. Lyophilization from acetonitrile/water gave 209 mg of 5-[4'-[(5-butyl-3-ethyl-1H-1,2,4-triazol-1-yl)methyl][1,1'-biphenyl]-2-yl]-1H-tetrazole as a colorless solid: NMR (CDCl₃) δ 0.88 (t, J = 8 Hz, 3H), 1.33-1.40 (m, 2H), 1.38 (t, J = 8 Hz, 3H), 1.58-1.70 (m, 2H), 2.67-2.85 (m, 4H), 5.26 (s, 2H), 7.10 (s, 4H), 7.41 (dd, J = 8 and 2 Hz, 1H), 7.74-7.62 (m, 2H), 7.86 (dd, J = 8 and 2 Hz, 1H); MS (FAB) m/e (rel intensity) 388 (58), 207 (100), 192 (35); HRMS. Calc'd for M+H: 388.2249. Found: 388.2218.

### Example 28

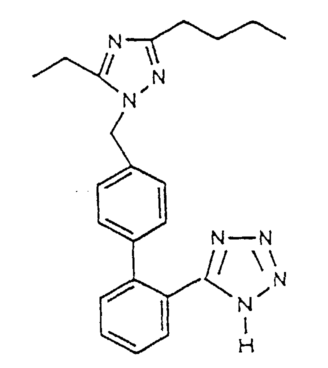

### 5-[4'-[(3-butyl-5-ethyl-1H-1,2,4-triazol-1-yl)methyl][1,1'-biphenyl]-2-yl]-1H-tetrazole

The slower moving isomer from Example 27 was dissolved in dilute base, acidified to pH 3-4, extracted with ethyl acetate, dried (MgSO₄), and the ethyl acetate removed in vacuo. Lyophilization from acetonitrile/water gave 188 mg of 5-[4'-[(3-butyl-5-ethyl-1H-1,2,4-triazol-1-yl)methyl][1,1'-biphenyl]-2-yl]-1H-tetrazole as a colorless solid: NMR (CDCl₃) δ 0.87 (t, J = 8 Hz, 3H), 1.16 (t, J = 8 Hz, 3H), 1.21-1.35 (m, 2H), 1.52-1.65 (m, 2H), 2.50 (t, J = 8 Hz, 2H), 2.67 (q, J = 8 Hz, 2H), 5.23 (s, 2H), 7.02 (d, J = 8 Hz, 2H), 7.12 (d, J = 8 Hz, 2H), 7.43 (dd, J = 8 and 2 Hz, 1H), 7.50-7.65 (m, 2H), 7.89 (dd, J = and 2 Hz, 1H); MS (FAB) m/e (rel intensity) 388 (35), 207 (100), 192 (47); HRMS. Calc'd for M+H: 388.2249. Found: 388.2222.

### Example 29

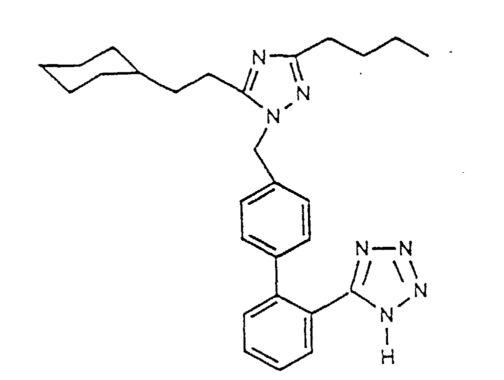

### 5-[4'-[(3-butyl-5-(2-cyclohexylethyl)-1H-1,2,4-triazol-1-yl]methyl][1,1'-biphenyl]-2-yl]-1H-tetrazole

Following General Procedure A, 1.2 g (5 mmol) of 5-butyl-3-(2-cyclohexylethyl)-1H-1,2,4-triazole was coupled with 5 mmol of the alkylating reagent prepared in step 1 of Example 3. Silica gel chromatography (Waters Prep 500A) using ethyl acetate/hexane (1:3) gave 3.6 g (100%) of a mixture of the two isomers which was dissolved in 50 ml of acetic acid/water (9:1) and stirred at ambient temperature overnight. The solvent was removed in vacuo; the residue dissolved in dilute base, acidified and washed with ethyl acetate. The ethyl acetate was removed in vacuo. Purification of a small sample of the isomer product mixture by reverse phase chromatography (Water Delta Prep-3000) using isocratic acetonitrile/water (41:59) (0.05% TFA) provided two isomers. The faster moving isomer was dissolved in dilute base, acidified to pH 3-4, extracted with ethyl acetate, dried (MgSO₄) and the ethyl acetate removed in vacuo. Lyophilization from acetonitrile/water gave 58 mg of 5-[4'-[[3-butyl-5-(2-cyclohexylethyl)-1H-1,2,4-triazol-1-yl]methyl][1,1'-biphenyl]-2-yl]-1H-tetrazole as a colorless solid: NMR (CDCl₃) δ 0.78-0.95 (m, 5H), 1.07-1.40 (m, 6H), 1.48-1.74 (m, 9H), 2.66 (t, J = 8 Hz, 2H), 2.78 (t, J = 8 Hz, 2H), 5.24 (s, 2H), 7.10 (q, J = 8 Hz, 4H), 7.41 (dd, J = 8 and 2 Hz, 1H), 7.48-7.63 (m, 2H), 7.89 (dd, J = 8 and 2 Hz, 1H); MS (FAB) m/e (rel intensity) 470 (45), 236 (35), 207 (100), 192 (33); HRMS. Calc'd for M+H: 470.3032. Found: 470.2990.

### Example 30

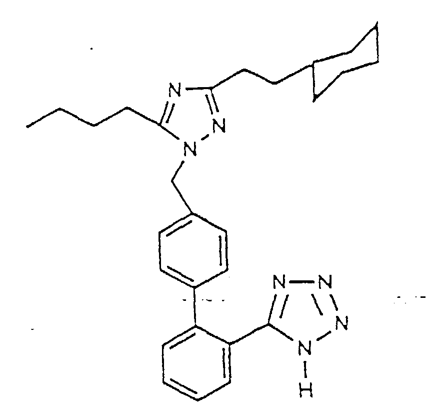

### 5-[4'-[[5-butyl-3-(2-cyclohexylethyl)-1H-1,2,4-triazol-1-yl]methyl][1,1'-biphenyl]-2-yl]-1H-tetrazole

The slower moving isomer from Example 29 was dissolved in dilute base, acidified to pH 3-4, extracted with ethyl acetate, dried (MgSO₄), and the ethyl acetate removed in vacuo. Lyophilization from acetonitrile/water gave 29 mg of 5-[4'-[[5-butyl-3-(2-cyclohexylethyl)-1H-1,2,4-triazol-1-yl]methyl][1,1'-biphenyl]-2-yl]-1H-tetrazole as a colorless solid: NMR (CDCl₃) δ 0.95-1.00 (m, 5H), 1.10-1.43 (m, 6H), 1.59-1.77 (m, 9H), 2.83 (t, J = 8 Hz, 2H), 2.92 (t, J = 8 Hz, 2H), 5.31 (s, 2H), 7.10-7.18 (m, 4H), 7.40 (dd, J = 8 and 2 Hz, 1H), 7.47-7.61 (m, 2H), 7.89 (dd, J = 8 and 2 Hz, 1H); MS (FAB) m/e (rel intensity) 470 (35), 236 (33), 207 (100), 192 (33); HRMS. Calc'd for M+H: 470.3032. Found: 470.2994.

### Example 31

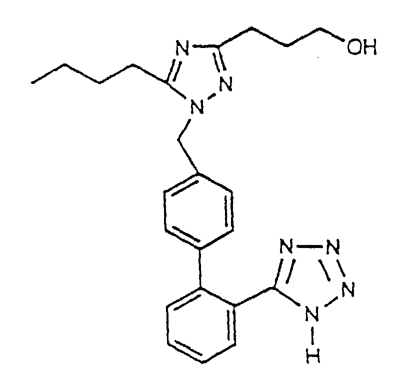

### 5-butyl-1-[2'-(1H-tetrazol-5-yl)[1,1'-biphenyl]-4-ylmethyl]-1H-1,2,4-triazole-3-propanol

Following General Procedure A, 920 mg (5.0 mmol) of 5-butyl-1H-1,2,4-triazole-3-propanol was coupled with 5.0 mmol of the alkylating reagent prepared in step 1 of Example 3. Silica gel chromatography (Waters Prep 500A) using isopropanol/chloroform (5:95) gave a mixture of the two pure isomers which was dissolved in 40 ml acetic acid/water (9:1) and stirred at ambient temperature for 3 days. The solvent was removed in vacuo. Purification of an aliquot by reverse phase chromatography (Waters Delta Prep 3000) using acetonitrile/water (26:74) (0.05% TFA) provided two isomers. Lyophilization of the faster moving isomer from acetonitrile/ water gave 124 mg of 5-butyl-1-[2'-(1H-tetrazol-5-yl)[1,1'-biphenyl]-4-ylmethyl]-1H-1,2,4-triazole-3-propanol as the colorless FTA salt: NMR (CDCl₃) δ 0.71 (t, J = 8 Hz, 3H), 1.10-1.24 (m, 2H), 1.42-1.54 (m, 2H), 1.70-1.81 (m, 2H), 2.49 (t, J = 8 Hz, 2H), 2.60 (t, J = 8 Hz, 2H), 3.46 (t, J = 6 Hz, 2H), 5.02 (s, 2H), 6.92 (q, J = 8 Hz, 4H), 7.24-7.34 (m, 2H), 7.40 (dt, J = 8 and 2 Hz, 1H), 7.52 (dd, J = 8 and 2 Hz, 1H); MS (FAB) m/e (rel intensity) 418 (100), 207 (48), 184 (10); HRMS. Calc'd for M+H: 418.2355. Found: 418.2370.

### Example 32

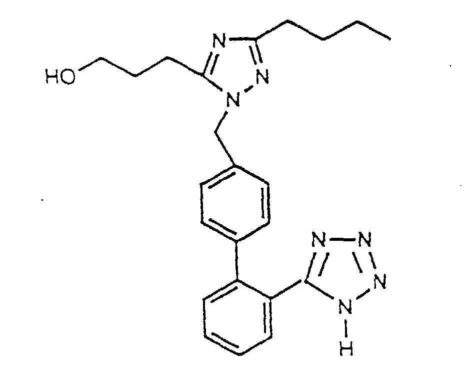

### 3-butyl-1-[2'-(1H-tetrazol-5-yl)[1,1'-biphenyl]-1ylmethyl]-1H-1,2,4-triazole-5-propanol

Lyophilization of the slower moving isomer from Example 31 gave 114 mg of 3-butyl-1-[2'-(1H-tetrazol-5-yl)[1,1'-biphenyl]-1ylmethyl]-1H-1,2,4-triazole-5-propanol as the colorless TFA salt: NMR (CDC13) δ 0.75 (t, J = 8 Hz, 3H), 1.13-1.27 (m, 2H), 1.48-1.60 (m, 2H), 1.64-1.75 (m, 2H), 2.52 (t, J = 8 Hz, 2H), 2.66 (t, J = 8 Hz, 2H), 3.39 (t, J = 6 Hz, 2H), 5.12 (s, 2H), 6.94 (s, 4H), 7.25-7.35 (m, 2H), 7.40 (dt, J = 8 and 2 Hz, 1H), 7.52 (d, J = 8 Hz, 1H); MS (FAB) m/e (rel intensity) 418 (100), 207 (30); HRMS. Calc'd for M+H: 418.2355. Found: 418.2344.

### Example 33

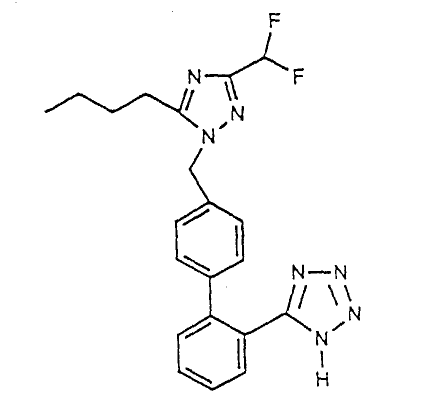

### 5-[4'-[[5-butyl-3-(difluoromethyl)-1H-1,2,4-triazol-1-yl]methyl][1,1'-biphenyl]-2-yl]-1H-tetrazole

Following General Procedure A, 470 mg (2.7 mmol) of 5-butyl-3-(difluoromethyl)-1H-1,2,4-triazole was coupled with 2.7 mmol of the alkylating reagent prepared in step 1 of Example 3. Purification by reverse phase chromatography (Waters Delta Prep 3000) using acetonitrile/water (36:64) (0.05% TFA) gave two isomers. The faster moving isomer was dissolved in dilute base, acidified to pH 3-4, extracted with ethyl acetate, dried (MgSO₄), and the ethyl acetate removed in vacuo. Lyophilization from acetonitrile/water gave 471 mg (27%) of 5-[4'-[[5-butyl-3-(difluoromethyl)-1H-1,2,4-triazol-1-yl]methyl][1,1'-biphenyl]-2-yl]-1H-tetrazole as a colorless solid: NMR (CDCl₃) δ 0.88 (t, J = 8 Hz, 3H), 1.28-1.43 (m, 2H), 1.60-1.73 (m, 2H), 2.72 (t, J = 8 Hz, 2H), 5.32 (s, 2H), 6.61 (t, J = 54 Hz, 1H), 7.12 (d, J = 8 Hz, 2H), 7.18 (d, J = 8 Hz, 2H), 7.42 (dd, J = 8 and 2 Hz, 1H), 7.51-7.65 (m, 2H), 8.00 (dd, J = 8 and 2 Hz, 1H); MS (FAB) m/e (rel intensity) 410 (45), 235 (55), 207 (100), 192 (57); HRMS. Calc'd for M+H: 410.1905. Found: 410.1903.

### Example 34

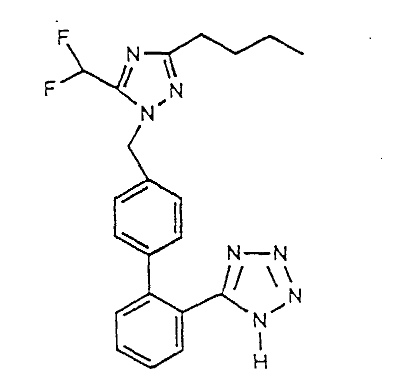

### 5-[4'-[[3-butyl-5-(difluoromethyl)-1H-1,2,4-triazol-1-yl]methyl][1,1'-biphenyl]-2-yl]-1H-tetrazole

The slower moving isomer from Example 33 was dissolved in dilute base, acidified, extracted with ethyl acetate, dried (MgSO₄), and the ethyl acetate removed in vacuo. Lyophilization from acetonitrile/water gave 104 mg (6%) of 5-[4'-[[3-butyl-5-(difluoromethyl)-1H-1,2,4-triazol-1-yl]methyl][1,1'-biphenyl]-2-yl]-1H-tetrazole as a colorless solid: NMR (CDCl₃) δ 0.91 (t, J = 8 Hz, 3H), 1.25-1.41 (m, 2H), 1.60-1.73 (m, 2H), 2.64 (t, J = 8 Hz, 2H), 5.43 (s, 2H), 6.81 (t, J = 52 Hz, 1H), 7.20 (d, J = 8 Hz, 2H), 7.28 (d, J = 8 Hz, 2H), 7.43 (dd, J = 8 and 2 Hz, 1H), 7.52-7.65 (m, 2H), 8.10 (dd, J = 8 and 2 Hz, 1H); MS (FAB) m/e (rel intensity) 410 (87), 235 (100), 207 (83); HRMS. Calc'd for M+H: 410.1905. Found: 410.1903.

### Example 35

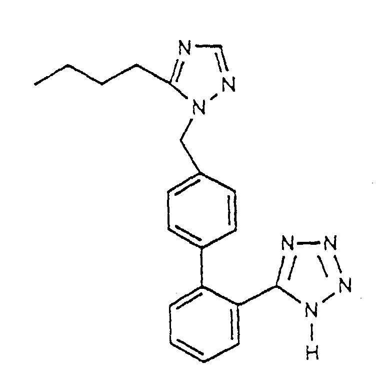

### 5-[4'-[(5-butyl-1H-1,2,4-triazol-1-yl)methyl][1,1'-biphenyl]-2-yl]-1H-tetrazole

Following general procedure A , 630 mg (5 mmol) of 3-butyl-1H-1,2,4-triazole was reacted with 5 mmol of the alkylating reagent prepared in step 1 of Example 3 to give 2.3 g (76%) of a mixture of the two isomers. Deprotection of the faster isomer provided 5-[4'-[(5-butyl-1H-1,2,4-triazol-1-yl)methyl][1,1'-biphenyl]-2-yl]1H-tetrazole: NMR (CDCl₃) δ 0.87 (t, J = 8 Hz, 3H), 1.35-1.41 (m, 2H), 1.59-1.72 (m, 2H), 2.72 (t, J = 8 Hz, 2H), 5.30 (s, 2H), 7.09 (d, J = 8 Hz, 2H), 7.17 (d, J = 8 Hz, 2H), 7.44 (dd, J = 8 and 2 Hz, 1H), 7.52-7.66 (m, 2H), 7.72 (s, 1H), 8.01 (dd, J = and 2 Hz, 1H); MS (FAB) m/e (rel intensity) 360 (88), 235 (20), 207 (100),192 (31), 178 (22); HRMS. .Calc'd for M+H: 360.1936. Found: 360.1938.

### Example 36

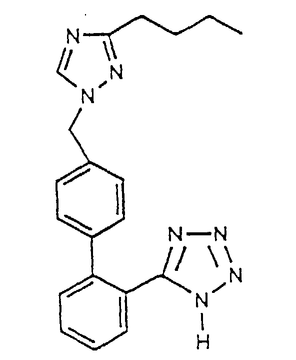

### 5-[4'[(3-butyl-1H-1,2,4-triazol-1-yl)methyl][1,1'-biphenyl]-2-yl]-1H-tetrazole

The slower moving isomer from Example 35 was deprotected to provide 5-[4'-[(3-butyl-1H-1,2,4-triazol-1-yl)methyl][1,1'-biphenyl]-2-yl]-1H-tetrazole: NMR (CDCl₃) δ 0.74 (t, J = 8 Hz, 3H), 1.19-1.33 (m, 2H), 1.51-1.63 (m, 2H), 2.55 (t, J = 8 Hz, 2H), 5.23 (s, 2H), 7.10 (q, J = 8 Hz, 4H), 7.44 (dd, J = 8 and 2 Hz, 1H), 7.50-7.64 (m, 2H), 7.92 (dd, J = 8 and 2 Hz, 1H), 8.02 (s, 1H); MS (FAB) m/e (rel intensity) 360 (84), 135 (18), 207 (100), 192 (26), 178 (16), 126 (22); HRMS. Calc'd for M+H: 360.1936. Found: 360.1971.

### Example 37

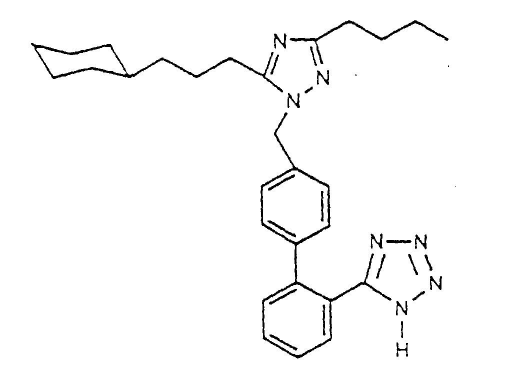

### 5-[4'-[[3-butyl-5-(3-cyclohexylpropyl)-1H-1,2,4-triazol-1-yl]methyl][1,1'-biphenyl]-2-yl]-1H-tetrazole

Following general procedure A, 1.25 g (5 mmol) of 5-butyl-3-(3-cyclohexylpropyl)-1H-1,2,4-triazole was reacted with 5 mmol of the alkylating reagent prepared in step 1 of Example 3 to give 3.1 g (85%) of a mixture of the two isomers. Deprotection of the faster moving isomer provided 5-[4'-[[3-butyl-5- (3-cyclohexylpropyl)-1H-1,2,4-triazol-1-yl]methyl][1,1'-biphenyl]-2-yl]-1H-tetrazole: NMR (CDCl₃) δ 0.73-0.92 (m, 2H), 0.87 (t, J = 8 Hz, 3H), 1.06-1.21 (m, 6H), 1.22-1.36 (m, 2H), 1.53-1.71 (m, 9H), 2.51 (t, J = 8 Hz, 2H), 2.61 (t, J = 8 Hz, 2H), 5.22 (s, 2H), 7.01 (d, J = 8 Hz, 2H), 7.12 (d, J = 8 Hz, 2H), 7.42 (dd, J = 8 and 2 Hz, 1H), 7.51-7.64 (m, 2H), 7.94 (dd, J = 8 and 2 Hz, 1H); MS (FAB) m/e (rel intensity) 484 (83), 250 (38), 207 (100), 178 (25); HRMS. Calc'd for M+H: 484.3189. Found: 484.3223.

### Example 38

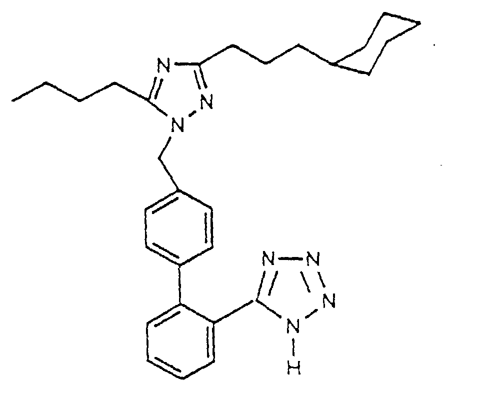

### 5-[4'-[[5-butyl-3-(3-cyclohexylpropyl)-1H-1,2,4-triazol-1-yl]methyl][1,1'-biphenyl]-2-yl]-1H-tetrazole

The slower moving isomer from Example 37 was deprotected to provide 5-[4'-[[5-butyl-3-(3-cyclohexylpropyl)-1H-1,2,4-triazol-1-yl]methyl][1,1'-biphenyl]-2-yl]-1H-tetrazole: NMR (CDCl₃) δ 0.74-0.90 (m, 2H), 0.83 (t, J = 8 Hz, 3H), 1.05-1.22 (m, 6H), 1.23-1.35 (m, 2H), 1.50-1.70 (m, 9H), 2.40 (t, J = 8 Hz, 2H), 2.57 (t, J = 8 Hz, 2H), 5.20 (s, 2H), 6.96 (d, J = 8 Hz, 2H), 7.10 (d, J = 8 Hz, 2H), 7.43 (dd, J = 8 and 2 Hz, 2H), 7.50-7.64 (m, 2H), 7.92 (dd, J = 8 and 2 Hz, 2H); MS (FAB) m/e (rel intensity) 484 (65), 250 (26), 207 (100), 178 (18); HRMS. Calc'd for M+H: 484.3189. Found: 484.3181.

### Example 39

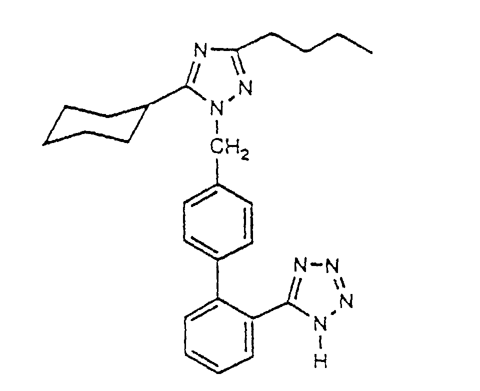

### 5-[4'-[(3-butyl-5-cyclohexyl-1H-1,2,4-triazol-1-yl)methyl][1,1'-biphenyl-2-yl]-1H-tetrazole

Following General Procedure A, 2.07 g (10 mmol) of 3-butyl-5-cyclohexyl-1H-1,2,4-triazole was reacted with 6.5 g (11.8 mmol) of the alkylating reagent prepared in step 1 of Example 3 to give 6.3 g (9.2 mmol) of a mixture of the two isomers. Deprotection of the raster moving isomer provided 33 mg of 5-[4'-[(3-butyl-5-cyclohexyl-1H-1,2,4-triazol-1-yl)methyl][1,1'-biphenyl]-2-yl]-1H-1,2,4-triazole as a colorless solid: mp 157.5-159.5 °C; NMR (CDCl₃) δ 0.87 (t, J = 7 Hz, 3H), 1.11-1.36 (m, 6H), 1,45-1.70 (m, 6H), 1.70-1.80 (m, 2H), 2.50 (t, J = 7 Hz, 2H), 2.64-2.78 (m, 1H), 5.23 (s, 2H), 6.98 (d, J = 8 Hz, 2H), 7.08 (d, J = 8 Hz, 2H), 7.41 (dd, J = 5 and 1 Hz, 1H, 7.49 (dt) J = 5 and 1 Hz, 1H), 7.57 (dt, J = 5 and 1Hz, 1H), 7.80 (dd, J = 5 and 1 Hz, 1H): MS (FAB) m/e (rel intensity), 442 (100), 414 (10), 399 (20), 235 (8), 207 (65), 192 (18).

### Example 40

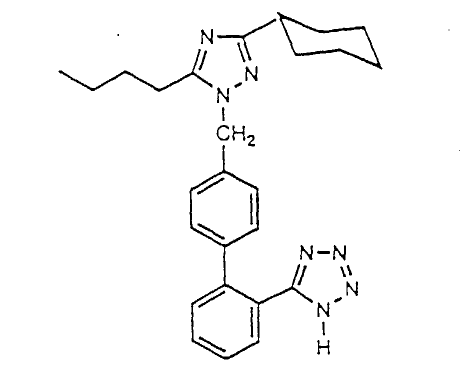

### 5-[4'-[(5-butyl-3-cyclohexyl-1H-1,2,4-triazol-1-yl)methyl][1,1'-biphenyl]-2-yl-1H-tetrazole

The slower moving isomer from Example 39 was deprotected to provide 135 mg of 5-[4'-[(5-butyl-3-cyclohexyl-1H-1,2,4-triazol-1-yl)methyl][1,1'-biphenyl]-2-yl]-1H-tetrazole as a colorless solid: mp 125-127 °C; NMR (CDCl₃) δ 0.85 (t, J = 7 Hz, 3H), 1.12-1.40 (m, J = 8 Hz, 8H), 1.52-1.75 (m, J = 8 Hz, 4H), 1.81 (d, J = 8 Hz, 2H), 2.50 (t, J = 8 Hz, 3H), 5.20 (s, 2H), 6.94 (d, J = 8 Hz, 2H), 7.09 (d, J = 8 Hz, 2H), 7.42 (dd, J = 8 and 2 Hz, 1H), 7.50-7.64 (m, J = 8 Hz, 2H), 7.88 (dd, J = 8 and 2 Hz, 1H); MS (FAB) m/e (rel intensity) 442 (100), 414 (10), 399 (20), 235 (10), 207 (75), 192 (17).

### Example 41

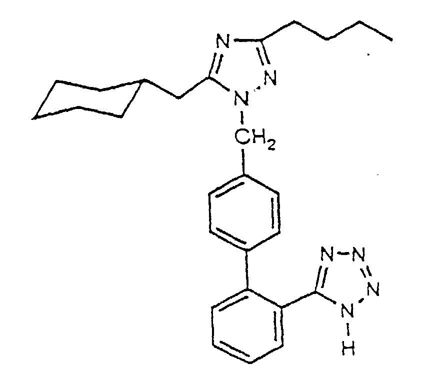

### 5-[4'-[(3-butyl)-5-cyclohexylmethyl-1H-1,2,4-triazol-1-yl)methyl][1,1'-biphenyl]-2-yl]-1H-tetrazole

Following General Procedure A, 2.21 g (10.0 mmol) of 3-butyl-5-cyclohexylmethyl-1H-1,2,4-triazole was reacted with 6.55 g (11.8 mmol) of the alkylating reagent prepared in step 1 of Example 3 to give 5.90 g (85%) of a mixture of the two isomers. The faster moving isomer was deprotected to provide 64 mg of 5-[4'-[(3-butyl-5-cyclohexylmethyl-1H-1,2,4-triazol-1-yl)methyl][1,1'-biphenyl]-2-yl]-1H-tetrazole as a colorless solid: NMR (CDCl₃) δ 0.92 (t, J = 7 Hz, 3H) 0.94-1.05 (m, 2H) 1.08-1.25 (m, 3H), 1.25-1.41 (m, 2H), 1.55-1.80 (m, 8H), 2.65 (d, J = 7 Hz, 2H), 2.68 (t, J = 7 Hz, 2H), 5.27 (s, 2H), 7.11 (q, J = 8 Hz, 4H), 7.42 (dd, J = 8 and 2 Hz, 1H), 7.49-7.63 (m, 2H), 7.94 (dd, J = 8 and 2 Hz, 1H); MS (FAB) m/e (rel intensity) 456 (100), 428 (10), 413 (20), 280 (10), 235 (10), 222 (25), 207 (80), 192-(30); HRMS, Calc'd. for M+H: 456.2876. Found: 456.2839.

### Example 42

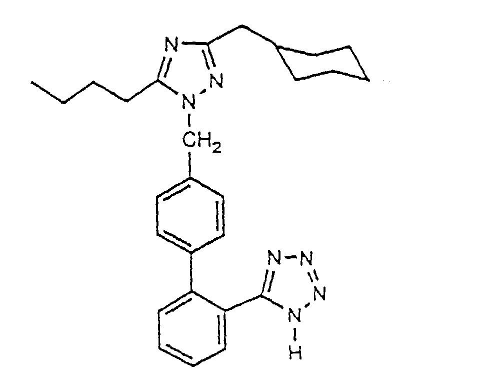

### 5-[4'-[(3-cyclohexylmethyl-5-butyl-1H-1,2,4-triazol-1-yl)methyl][1,1'-biphenyl]-2-yl]-1H-tetrazole

The slower moving isomer from Example 41 was deprotected to provide 51 mg of 5-[4'-[(3-cyclohexylmethyl-5-butyl-1H-1,2,4-triazol-1-yl)methyl][1,1'-biphenyl)-2-yl]-1H-tetrazole as a colorless solid: NMR (CDCl₃) δ 0.84 (t, J = 8 Hz, 3H), 0.92 (q, J = 8 Hz, 2H), 1.05-1.22 (m, 3H), 1.22-1.36 (m, 2H), 1.52-1.69 (m, 8H), 2.50 (d, J = 7 Hz, 2H), 2.63 (t, J = 8 Hz, 2H), 5.18 (s, 2H), 7.05 (q, J = 8 Hz, 4H), 7.35-7.55 (m, 3H), 7.68 (dd, J = 8 and 2 Hz, 1H); MS (FAB) m/e (rel intensity) 456 (90), 413 (10), 281 (10), 243 (10), 222 (35), 207 (100), 192 (35); HRMS. Calc'd for M+H: 456.2876. Found: 456.2849.

### Example 43

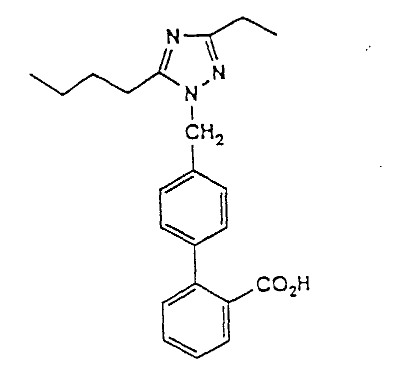

### 4'-[(3-ethyl-5-butyl-1H-1,2,4-triazol-1-yl)methyl][1,1'-biphenyl]-2-carboxylic acid

Following General Procedure A, 1.0 g (6.0 mmol) of 3-ethyl-5-butyl-1H-1,2,4-triazole was reacted with 2.41 g (7.9 mmol) of the alkylating reagent prepared in step 1 of Example 1. Hydrolysis of the faster moving isomer provided 220 mg of 4'-[(3-ethyl-5-butyl-1H-1,2,4-triazol-1-yl)methyl][1,1'-biphenyl]-2-carboxylic acid as a colorless solid: NMR (DMSO-d₆) δ 0.85 (t, J = 7 Hz, 3H), 1.19 (t, J = 7 Hz, 3H), 1.27-1.38 (m, 2H), 1.52-1.63 (m, 2H), 2.59 (q, J = 7 Hz, 2H), 2.74 (t, J = 7 Hz, 2H), 5.35 (s,. 2H), 7.21 (d, J = 8 Hz, 2H), 7.31 (d, J = 8 Hz, 2H), 7.35 (dd, J = 8 and 2 Hz, 1H), 7.48 (dt, J = 8 and 2 Hz, 1H), 7.57 (dt, J = 8 and 2 Hz, 1H), 7.72 (dd, J = 8 and 2 Hz, 1H).

### Example 44

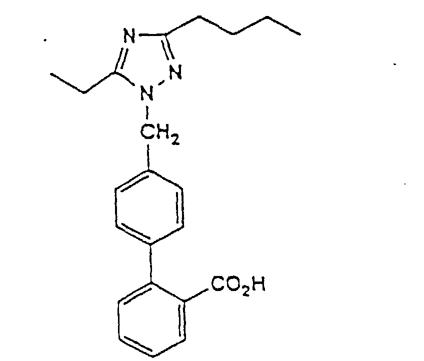

### 4'-[(3-butyl-5-ethyl-1H-1,2,4-triazol-1-yl)methyl][1,1'-biphenyl]-2-carboxylic acid

The slower moving isomer from Example 43 was hydrolyzed to provide 174 mg of 4'-[(3-butyl-5-ethyl-1H-1,2,4-triazol-1-yl)methyl][1,1'biphenyl]-2-carboxylic acid as a colorless solid: NMR (DMSO-d₆) δ 0.89 (t, J = 7 Hz, 3H), 1.17 (t, J = 7 Hz, 3H), 1.27-1.40 (m, 2H), 1.56-1.68 (m, 2H), 2.58 (t, J = 8 Hz, 2H), 2.77 (q, J = 8 Hz, 2H), 5.35 s, 2H), 7.21 (d, J = 8 Hz, 2H), 7.32 (d, J = 8 Hz, 2H), 7.36 (dd, J = 8 and 2 Hz, 1H), 7.46 (dt, J = 8 and 2 Hz, 1H), 7.57 (dt, J = 8 and 2 Hz, 1H), 7.73 (dd, J = 8 and 2 Hz, 1H).

### Example 45

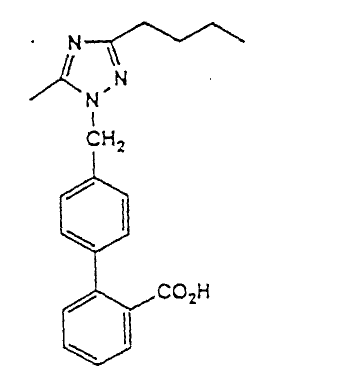

### 4'-[(3-butyl-5-methyl-1H-1,2,4-triazol-1-yl)methyl][1,1'-biphenyl]-2-carboxylic acid

Following General Procedure A, 1.0 g (7.2 mmol) of 3-butyl-5-methyl-1H-1,2,4-triazole was reacted with 2.89 g (9.5 mmol) of the alkylating reagent prepared in step 1 of Example 1. Hydrolysis of the slower moving isomer provided 220 mg of 4'-[(3-butyl-5-methyl-1H-1,2,4-triazol-1-yl)methyl][1,1'-biphenyl]-2-carboxylic acid as a colorless solid: NMR (DMSO-d₆) δ 0.88 (t, J = 7 Hz, 3H), 1.25-1.38 (m, 2H), 1.54-1.67 (m, 2H), 2.39 (s, 3H), 2.54 (t, J = 8 Hz, 2H), 5.32 (s, 2H), 7.21 (d, J = 8 Hz, 2H), 7.32 (d, J = 8 Hz, 2H), 7.36 (dd, J = 8 and 2 Hz, 1H), 7.46 (dt, J = 8 and 2 Hz, 1H), 7.56 (dt, J = 8 and 2 Hz, 1H), 7.72 (dd, J = 8 and 2 Hz, 1H).

### Example 46

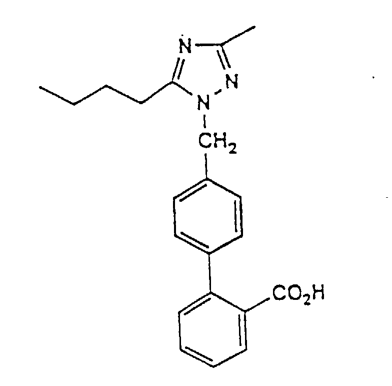

### 4'-[(3-methyl-5-butyl-1H-1,2,4-triazol-1-yl)methyl][1,1'-biphenyl]-2-carboxylic acid

The faster moving isomer from Example 45 was hydrolyzed to provide 19 mg of 4'-[(3-methyl-5-butyl-1H-1,2,4-triazol-1-yl)methyl][1,1'-biphenyl]-2-carboxylic acid as a colorless solid: NMR (DMSO-d₆) δ 0.86 (t, J = 7 Hz, 3H), 1.25-1.38 (m, 2H), 1.52-1.64 (m, 2H), 2.21 (s, 3H), 2.72 (t, J = 7 Hz, 2H), 5.32 (s, 2H), 7.21 (d, J = 8 Hz, 2H), 7.32 (d, J = 8 Hz, 2H), 7.35 (dd, J = 8 and 2 Hz, 1H), 7.45 (dt, J = 8 and 2 Hz, 1H), 7.57 (dt, J = 8 and 2 Hz, 1H), 7.72 (dd, J = 8 and 2 Hz, 1H).

### Example 47

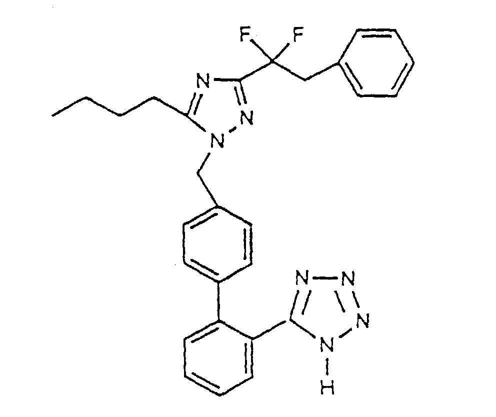

### 5-[4'-[[5-butyl-3-(1,1-difluoro-2-phenylethyl)-1H-1,2,4-triazol-1-yl]methyl][1,1'-biphenyl]-1-yl]-1H-tetrazole

Under nitrogen, 8.5 g (48 mmol) of 5-butyl-3-(difluoromethyl)-1H-1,2,4-triazole was added in portions to 53 mmol of sodium hydride in 85 ml of anhydrous THF; stirring was continued for 1 h. The anion solution was cooled to 0 °C and treated dropwise with 8.9 ml (50 mmol) of Sem-Cl. The reaction was allowed to warm to ambient temperature and stir overnight. The THF was removed in vacuo; the residue dissolved in methylene chloride, washed with water, and dried (MgSO₄). Purification by silica gel chromatography (Waters Prep 500A) using ethyl acetate/hexane (1:9) gave 4.9 g 34% of the Sem-protected triazole as an oil : NMR (CDCl₃) δ -0.04 (s, 9H), 0.86-0.95 (m, 5H), 1.39-1.43 (m, 2H), 1.65-1.76 (m, 2H), 2.71 (t, J = 8 Hz, 2H), 3.62 (t, J = 8 Hz, 2H), 5.53 (s, 2H), 6.82 (t, J = 52 Hz, 1H). Under nitrogen, 14.2 mmol of sec-butyllithium was added dropwise to 3.6 g (11.8 mmol) of the Sem-protected triazole in 200 ml of anhydrous THF at -78 °C. The reaction was allowed to stir for 1 h prior to the addition of 1.7 ml (14.2 mmol) of benzyl bromide. After stirring at -78 °C for 2 h, the reaction was allowed to warm to ambient temperature and stir overnight. The solvent was removed in vacuo; the residue dissolved in methylene chloride, washed with water, and dried (MgSO₄). Silica gel chromatography (Waters Prep 500A) using ethyl acetate/hexane (5:95) gave 600 mg (10%) of the Sem protected 5-butyl-3-(1,1-difluoro-2-phenylethyl)-1H-1,2,4-triazole: NMR (CDCl₃) δ -0.04 (s, 9H), 0.85 (t, J = 8Hz, 2H), 0.94 (t, J = 8 Hz, 3H), 1.30-1.44 (m, 2H), 1.67-1.79 (m, 2H), 2.73 (t, J = 8 Hz, 2H), 3.54 (t, J = 8 Hz, 2H), 3.70 (t, J = 16 Hz, 2H), 5.34 (s, 2H), 7.21-7.31 (m, 5H). The Sem protected triazole was dissolved in 5 ml of ethanol and 5 ml of 3M HCl and allowed to stir at reflux for 3 h. The ethanol was removed in vacuo and the pH adjusted to nine with dilute sodium hydroxide. The resulting solution was extracted with methylene chloride; the extracts were combined, dried (MgSO₄), and concentrated in vacuo to provide 5-butyl-3-(1,1-difluoro-2-phenethyl-1H-1,2,4-triazole. Following General Procedure A, 1.4 mmol of this material was coupled with 1.4 mmol of the alkylating reagent prepared in step 1 of Example 3. The crude product was dissolved in 10 mL of acetic acid/water (9:1) and stirred at ambient temperature for 3 days. The solvent was removed in vacuo; the residue dissolved in dilute base and washed with toluene. The water was acidified to pH 3-4, extracted with ethyl acetate, and dried (MgSO₄). Purification by reverse phase chromatography (Waters Delta Prep 3000) using acetonitrile/water (47:53) (0.05% TFA) gave the TFA salt. The salt was dissolved in dilute base, acidified to pH 3-4, extracted with ethyl acetate, and dried (MgSO₄). Lyophilization from acetonitrile/water gave 290 mg (42%) of 5-[4'-[[5-butyl-3-(1,1-difluoro-2-phenylethyl)-1H-1,2,4-triazol-1-yl]methyl][1,1'-biphenyl]-1-yl]-1H-tetrazole: NMR (CDCl₃) δ 0.86 (t, J = 8 Hz, 3H), 1.21-1.36 (m, 2H), 1.54-1.68 (m, 2H), 2.64 (t, J = 8 Hz, 2H), 3.57 (t, J = 16 Hz, 2H), 5.23 (s, 2H), 6.92 (d, J = 8 Hz, 2H), 7.10 (d, J = 8 Hz, 2H), 7.16-7.25 (m, 5H), 7.39 (dd, J = 8 and 2 Hz, 1H), 7.50-7.65 (m, 2H), 7.99 (dd, J = 8 and 2 Hz, 1H); MS (FAB) m/e (rel intensity) 500(100), 266 (6), 235 (8), 207 (25), 192 (8); HRMS. Calc'd for M+H: 500.2374. Found: 500.2358.

### BIOLOGICAL EVALUATION

### Assay A: Angiotensin II Binding Activity

Compounds of the invention were tested for ability to bind to the smooth muscle angiotensin II receptor using a rat uterine membrane preparation. Angiotensin II (AII) was purchased from Peninsula Labs. ¹²⁵I-angiotensin II (specific activity of 2200 Ci/mmol) was purchased from Du Pont-New England Nuclear. Other chemicals were obtained from Sigma Chemical Co. This assay was carried out according to the method of Douglas et al [Endocrinology, 106, 120-124 (1980)]. Rat uterine membranes were prepared from fresh tissue. All procedures were carried out at 4°C. Uteri were stripped of fat and homogenized in phosphate-buffered saline at pH 7.4 containing 5 mM EDTA. The homogenate was centrifuged at 1500 x g for 20 min., and the supernatant was recentrifuged at 100,000 x g for 60 min. The pellet was resuspended in buffer consisting of 2 mM EGTA and 50 mM Tris-HCl (pH 7.5) to a final protein concentration of 4 mg/ml. Assay tubes were charged with 0.25 ml of a solution containing 5 mM MgCl₂, 2 mM EDTA, 0.5% bovine serum albumin, 50 mM Tris-HCl, pH 7.5 and ¹²⁵I-AII (approximately 10⁵ cpm) in the absence or in the presence of unlabelled ligand. The reaction was initiated by the addition of membrane protein and the mixture was incubated at 25°C for 60 min. The incubation was terminated with ice-cold 50 mM Tris-HCl (pH 7.5) and the mixture was filtered to separate membrane-bound labelled peptide from the free ligand. The incubation tube and filter were washed with ice-cold buffer. Filters were assayed for radioactivity in a Micromedic gamma counter. Nonspecific binding was defined as biding in the presence of 10 µM of unlabelled AII. Specific binding was calculated as total binding minus nonspecific binding. The receptor binding affinity of an AII antagonist compound was indicated by the concentration (IC₅₀) of the tested AII antagonist which gives 50% displacement of the total specifically bound ¹²⁵I-AII from the high affinity ("Type 1") AII receptor. Binding data were analyzed by a nonlinear least-squares curve fitting program. Results are reported in Table I.

### Assay B: In Vitro Vascular Smooth Muscle-Response for AII

The compounds of the invention were tested for antagonist activity in rabbit aortic rings. Male New Zealand white rabbits (2-2.5 kg) were sacrificed using an overdose of pentobarbital and exsanguinated via the carotid arteries. The thoracic aorta was removed, cleaned of adherent fat and connective tissue and then cut into 3-mm ring segments. The endothelium was removed from the rings by gently sliding a rolled-up piece of filter paper into the vessel lumen. The rings were then mounted in a water-jacketed tissue bath, maintained at 37°C, between moveable and fixed ends of a stainless steel wire with the moveable end attached to an FTO3 Grass transducer coupled to a Model 8 Grass Polygraph for recording isometric force responses. The bath was filled with 20 ml of oxygenated (95% oxygen/5% carbon dioxide) Krebs solution of the following composition (mM): 130 NaCl, 15 NaHCO₃, 15 KCl, 1.2 NaH₂PO₄, 1.2 MgSO₄, 2.5 CaCl₂, and 11.4 glucose. The preparations were equilibrated for one hour before approximately one gram of passive tension was placed on the rings. Angiotensin II concentration-response curves were then recorded (3 X 10⁻¹⁰ to 1 X 10⁻⁵ M). Each concentration of All was allowed to elicit its maximal contraction, and then All was washed out repeatedly for 30 minutes before rechallenging with a higher concentration of AII. Aorta rings were exposed to the test antagonist at 10⁻⁵ M for 5 minutes before challenging with AII. Adjacent segments of the same aorta ring were used for all concentration-response curves in the presence or absence of the test antagonist. The effectiveness of the test compound was expressed in terms of pA₂ values and were calculated according to H.O. Schild [Br. J. Pharmacol., Chemother., 2,189-206 (1947)]. The pA₂ value is the concentration of the antagonist which increases the EC₅₀ value for All by a factor of 2. Each test antagonist was evaluated in aorta rings from two rabbits. Results are reported in Table I.

### Assay C: In Vivo Intraduodenal Pressor Assay Response for AII Antagonists

Male Sprague-Dawley rats weighing 225-300 grams were anesthetized with Inactin (100 mg/kg, i.p.) and catheters were implanted into the trachea, femoral artery, femoral vein and duodenum. Arterial pressure was recorded from the femoral artery catheter on a Gould chart recorder (Gould, Cleveland, OH). The femoral vein catheter was used for injections of angiotensin II, mecamylamine and atropine. The tracheal catheter allow for airway patency, and the duodenal catheter was used for intraduodenal (i.d.) administration of test compounds. After surgery, the rats were allowed to equilibrate for 30 minutes. Mecamylamine (3 mg/kg, 0.3 ml/kg) and atropine (400 ug/kg, 0.3 ml/kg) were then given i.v. to produce ganglion blockade. These compounds were administered every 90 minutes throughout the test procedure. Angiotensin II was given in bolus does i.v. (30 ng/kg in saline with 0.5% bovine serum albumin, 0.1 ml/kg) every 10 minutes three times or until the increase in arterial pressure produced was within 3 mmHg for two consecutive AII injections. The last two AII injections were averaged and were taken as the control All pressor response. Ten minutes after the final control AII injection, the test compound (dissolved in sodium bicarbonate) was administered i.d. at a dose of 3, 10, 30 or 100 mg/kg in a volume of 0.2 ml. Angiotensin II injections were then given 5, 10, 20, 30, 45, 60, 75, 90, and 120 minutes after administration of the test compound and response of arterial pressure was monitored. The response to AII was calculated as percent of the control response and then the percent inhibition is calculated as 100 minus the percent control response. Duration of action of a test compound was defined as the time from peak percent inhibition to 50% of peak. One compound at one dose was tested in each rat. Each test compound was tested in two rats and the values for the two rats were averaged. Results are reported in Table I.

### Assay D: In Vivo Intragastric Pressor Assay Response for All Antagonists

Male Sprague-Dawley rats weighing 225-300 grams were anesthetized with methohexital (30 mg/kg, i.p.) and catheters were implanted into the femoral artery and vein. The catheters were tunneled subcutaneously to exit dorsally, posterior to the head and between the scapulae. The catheters were filled with heparin (1000 units/ml of saline). The rats were returned to their cage and allowed regular rat chow and water ad libitum. After full recovery from surgery (3-4 days), rats were placed in Lucite holders and the arterial line was connected to a pressure transducer. Arterial pressure was recorded on a Gould polygraph (mmHg). After 1-2 hours of stable baseline recording, the intravenous infusion of angiotensin II (50 ng/kg/min) was given at a rate of 0.0096 ml/min. After allowing one hour for pressure to stabilize, the test compound (suspended in 0.5% methylcellulose in water) was administered by gavage. The volume administered was 2 ml/kg body weight. Arterial pressure was monitored for 5 hours post-dosing. The angiotensin II infusion was then discontinued and pressure was allowed to reach a stable recovery level. Percent inhibition (%I) of the angiotensin II pressor response was calculated from the difference in pressure at a given timepoint post-dosing with the test compound and the angiotensin II-infused pressure, divided by the difference in pressure with and without the angiotensin II infusion; this value was multiplied by 100. Duration of action of a test compound was defined as the time taken for pressure to return to angiotensin II-infused baseline levels after compound administration. A compound at one dose was tested in two rats. Results are reported in Table I.

**TABLE I**

| In Vivo and In Vitro Angiotensin II Activity of Compounds of the Invention | | | | | |
|---|---|---|---|---|---|
| Test Compound Example # | ¹Assay A IC₅₀ (nM) | ²Assay B pA₂ | Dose (mg/kg) | ³Assay C Inhibition (%) | Duration (min.) |
| 1 | NT | NT | NT | NT | NT |
| 2 | 95 | 7.37/7.59 | 10 | 95 | 60 |
| | | | 30 | 98 | 90-120 |
| 3 | 5.4 | 8.70 ± 0.2 | 10 | 50 | >180 |
| | | | 30 | 100 | 200⁺ |
| 4 | NT | NT | NT | NT | NT |
| 5 | 200 | 7.48/6.91 | 30 | 38 | 20-30 |
| | | | | | |
| 6 | 4.9 | 8.19/7.59 | 3 | 86 | 100 |
| | | | 30 | 100 | 240 |
| 7 | 160 | 6.45/6.77 | NT | NT | NT |
| 8 | 6.0 | 8.66/8.59 | NT | NT | NT |
| 9 | 17 | 8.70/8.85 | NT | NT | NT |
| 10 | 7.2 | 8.84/8.71 | NT | NT | NT |
| 11 | 16 | 8.31/8.30 | NT | NT | NT |
| 12 | 6.4 | 8.95/9.24 | NT | NT | NT |
| 13 | 4.0 | 8.64/8.40 | NT | NT | NT |
| 14 | 970 | 6.14/6.09 | NT | NT | NT |
| 15 | 12,000 | 5.18/5.35 | NT | NT | NT |
| | | | | | |
| 16 | 87 | 7.71.7.21 | NT | NT | NT |
| 17 | 460 | 6.60/6.46 | NT | NT | NT |
| 18 | 430 | 6.48/7.15 | NT | NT | NT |
| 19 | 10 | 7.56/7.73 | NT | NT | NT |
| | | | | | |
| 20 | 570 | 5.57/6.00 | NT | NT | NT |
| 21 | 160 | NT | NT | NT | NT |
| 22 | 22 | 7.73/7.88 | 30 | 50 | >180 |
| | | | | | |
| 23 | 16 | 7.68/7.29 | NT | NT | NT |
| 24 | 630 | 6.73/6.36 | NT | NT | NT |
| | | | | | |
| 25 | 340 | 6.90/6.85 | NT | NT | NT |
| 26 | 10 | 7.82/8.36 | NT | NT | NT |
| 27 | 10 | 7.88/7.84 | NT | NT | NT |
| 28 | 83 | 7.94/7.61 | NT | NT | NT |
| | | | | | |
| 29 | 370 | 6.56/6.26 | NT | NT | NT |
| 30 | 19 | 8.97/8.61 | NT | NT | NT |
| | | | | | |
| 31 | 4.4 | 8.41/8.24 | NT | NT | NT |
| | | | | | |
| 32 | 110 | 6.80/6.64 | NT | NT | NT |
| 33 | 21 | 7.85/7.58 | NT | NT | NT |
| 34 | 680 | 6.27/6.75 | NT | NT | NT |
| | | | | | |
| 35 | 65 | 7.56/7.83 | NT | NT | NT |
| 36 | 3100 | 6.02 | NT | NT | NT |
| 37 | 2300 | 6.00 | NT | NT | NT |
| 38 | 140 | 6.45/6.57 | NT | NT | NT |
| | | | | | |
| 39 | 23 | 7.85/7.75 | NT | NT | NT |
| 40 | 12 | 9.34/8.58 | NT | NT | NT |
| 41 | 790 | 7.65/7.46 | NT | NT | NT |
| 42 | 6.5 | 8.56/8.39 | NT | NT | NT |
| 43 | 570 | 6.00/5.45 | NT | NT | NT |
| 44 | 5400 | 5.52/5.78 | NT | NT | NT |
| 45 | 15,000 | 5.77 | NT | NT | NT |
| 46 | 480 | 6.41/6.35 | NT | NT | NT |
| 47 | 8.7 | 8.39/8.51 | NT | NT | NT |

| | | | | | |
|---|---|---|---|---|---|
| NT = NOT TESTED *Antagonist activity not observed up to 10 µM of test compound. ¹Assay A: Angiotensin II Binding Activity | | | | | |
| ²Assay B: In Vitro Vascular Smooth Muscle Response | | | | | |
| ³Assays C/D: In Vivo Pressor Response (test compounds administered intraduodenally, except for compounds of Examples #3, #21 and #24 which were given intragastrically). | | | | | |

Also embraced within this invention is a class of pharmaceutical compositions comprising one or more compounds of Formula I in association with one or more non-toxic, pharmaceutically acceptable carriers and/or diluents and/or adjuvants (collectively referred to herein as "carrier" materials) and, if desired, other active ingredients. The compounds of the present invention may be administered by any suitable route, preferably in the form of a pharmaceutical composition adapted to such a route, and in a dose effective for the treatment intended. Therapeutically effective doses of the compounds of the present invention required to prevent or arrest the progress of the medical condition are readily ascertained by one of ordinary skill in the art. The compounds and composition may, for example, be administered intravascularly, intraperitoneally, subcutaneously, intramuscularly or topically.

For oral administration, the pharmaceutical composition may be in the form of, for example, a tablet, capsule, suspension or licuid. The pharmaceutical composition is preferably made in the form of a dosage unit containing a particular amount of the active ingredient. Examples of such dosage units are tablets or capsules. These may with advantage contain an amount of active ingredient from about 1-to 250 mg, preferably from about 25 to 150 mg. A suitable daily dose for a mammal may vary widely depending on the condition of the patient and other factors. However, a dose of f=om about 0.1 to 3000 mg/kg body weight, particularly from about 1 to 100 mg/kg body weight, may be appropriate.

The active ingredient may also be administered by injection as a composition wherein, for example, saline, dextrose or water may be used as a suitable carrier. A suitable daily dose is from about 0.1 to 100 mg/kg body weight injected per day in multiple doses depending on the disease being treated. A preferred daily dose would be from about 1 to 30 mg/kg body weight. Compounds indicated for prophylactic therapy will preferably be administered in a daily dose generally in a range from about 0.1 mg to about 100 mg per kilogram of body weight per day. A more preferred dosage will be a range from about 1 mg to about 100 mg per kilogram of body weight. Most preferred is a dosage in a range from about 1 to about 50 mg per kilogram of body weight per day. A suitable dose can be administered, in multiple sub-doses per day. These sub-doses may be administered in unit dosage forms. Typically, a dose or sub-dose may contain from about 1 mg to about 100 mg of active compound per unit dosage form. A more preferred dosage will contain from about 2 mg to about 50 mg of active compound per unit dosage form. Most preferred is a dosage form containing from about 3 mg to about 25 mg of active compound per unit dose.

The dosage regimen for treating a disease condition with the compounds and/or compositions of this invention is selected in accordance with a variety of factors, including the type, age, weight, sex and medical condition of the patient, the severity of the disease, the route of administration, and the particular compound employed, and thus may vary widely.

For therapeutic purposes, the compounds of this invention are ordinarily combined with one or more adjuvants appropriate to the indicated route of administration. If administered per os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

## Claims (Claims for the following Contracting State(s): AT, BE, CH, LI, DE, DK, FR, GB, IT, LU, NL, SE)

1. Compound of Formula I 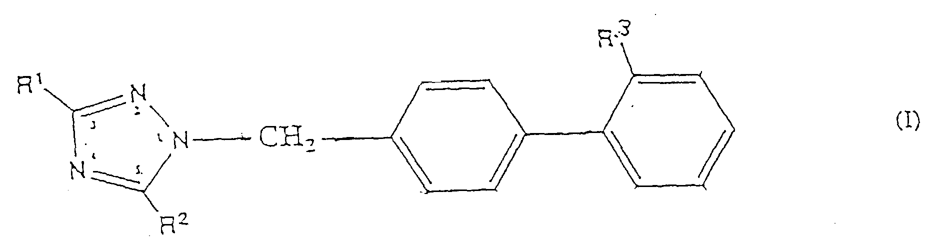 wherein each of R¹ and R² is independently selected from hydrogen, C₁-C₂₀ alkyl, phenyl, phenyl substituted by C₁-C₂₀ alkyl, C₃-C₁₀ cycloalkyl, C₃-C₁₀ cycloalkyl, attached to or substituted with C₁-C₂₀ alkyl, C₁-C₁₀ alkoxy, C₁-C₁₀-di-fluoroalkyl substituted by phenyl, C₂-C₂₀ alkenyl, C₂-C₂₀ alkynyl, C₁-C₂₀ alkylcarbonyl; difluoro-substituted C₁-C₂₀ alkyl;
C₁-C₁₀-hydroxyalkyl, C₁-C₂₀ alkyl substituted by one or more alkoxy groups having alkyl portions of 1 to 10 carbon atoms; wherein R³ is an acidic moiety selected from CO₂H, CONHNHSO₂CF₃, 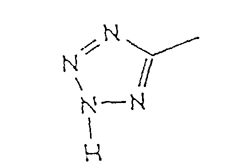 and the salts of said acidic moieties; or a tautomer thereof or a pharmaceutically-acceptable salt thereof.

2. Compound of Claim 1 wherein R³ is selected from COOH or tetrazolyl or a tautomer or a pharmaceutically-acceptable salt thereof.

3. Compound of Claim 2 selected from compounds, and their pharmaceutically-acceptable salts, of the group of compounds consisting of
4'-[(3,5-dibutyl-1H-1,2,4-triazol-1-yl)methyl][1,1'-biphenyl]-2-carboxylic acid, 2-[(trifluoromethyl)sulfonyl]hydrazide;
4'-[(5-butyl-3-propyl-1H-1,2,4-triazol-1-yl)methyl][1,1'-biphenyl]-2-carboxylic acid;
4'-[(3,5-dibutyl-1H-1,2,4-triazol-1-yl)methyl][1,1'-biphenyl]-2-carboxylic acid;
4'-[(5-butyl-3-isopentyl-1H-1,2,4-triazol-1-yl)methyl][1,1'-biphenyl]-2-carboxylic acid;
4'-[[5-butyl-3-(1,1-dimethoxybutyl)-1H-1,2,4-triazol-1-yl]methyl][1,1'-biphenyl]-2-carboxylic acid;
4'-[[5-butyl-3-(1-oxobutyl)-1H-1,2,4-triazol-1-yl]methyl][[1,1'-biphenyl]-2-carboxylic acid;
4'-[[5-butyl-3-(1-oxopentyl)-1H-1,2,4-triazol-1-yl]methyl][1,1'-biphenyl]-2-carboxylic acid;
4'-[[5-butyl-3-(1,1-difluoroethyl)-1H-1,2,4-triazol-1-yl]methyl][1,1'-biphenyl]-2-carboxylic acid;
4'-[[5-butyl-3-(1,1-difluoropropyl)-1H-1,2,4-triazol-1-yl]methyl][1,1'-biphenyl]-2-carboxylic acid;
4'-[[5-butyl-3-(1,1-difluorobutyl)-1H-1,2,4-triazol-1-yl]methyl][1,1'-biphenyl]-2-carboxylic acid;
4'-[[3-butyl-5-(1,1-difluorobutyl)-1H-1,2,4-triazol-1-yl]methyl][1,1'-biphenyl]-2-carboxylic acid;
4'-[[5-butyl-3-(1,1-difluoropentyl)-1H-1,2,4-triazol-1-yl]methyl][1,1'-biphenyl]-2-carboxylic acid;
4'-[(3,5-dipropyl-1H-1,2,4-triazol-1-yl)methyl][1,1'-biphenyl]-2-carboxylic acid;
4'-[(3,5-isopropyl-1H-1,2,4-triazol-1-yl)methyl][1,1'-biphenyl]-2-carboxylic acid;
4'-[(5-propyl-3-butyl-1H-1,2,4-triazol-1-yl)methyl][1,1'-biphenyl]-2-carboxylic acid;
4'-[[5-propyl-3-(1,1-difluoroethyl)-1H-1,2,4-triazol-1-yl]methyl][1,1'-biphenyl]-2-carboxylic acid;
4'-[[5-propyl-3-(1,1-difluoropropyl)-1H-1,2,4-triazol-1-yl]methyl][1,1'-biphenyl]-2-carboxylic acid;
4'-[[5-propyl-3-(1,1-difluorobutyl)-1H-1,2,4-triazol-1-yl]methyl][1,1'-biphenyl]-2-carboxylic acid;
4'-[[5-propyl-3-(1,1-difluoropentyl)-1H-1,2,4-triazol-1-yl]methyl][1,1'-biphenyl]-2-carboxylic acid;
4'-[[5-ethyl-3-(1,1-difluorobutyl)-1H-1,2,4-triazol-1-yl]methyl][1,1'-biphenyl]-2-carboxylic acid;
4'-[(3,5-disecbutyl-1H-1,2,4-triazol-1-yl)methyl][1,1'-biphenyl]-2-carboxylic acid;
4'-[[5-secbutyl-3-(1,1-difluorobutyl)-1H-1,2,4-triazol-1-yl]methyl][1,1'-biphenyl]-2-carboxylic acid;
4'-[(3,5-diisobutyl-1H-1,2,4-triazol-1-yl)methyl][1,1'-biphenyl]-2-carboxylic acid;
4'-[[5-isobutyl-3-(1,1-difluorobutyl)-1H-1,2,4-triazol-1-yl]methyl][1,1'-biphenyl]-2-carboxylic acid;
4'-[(3,5-ditertbutyl-1H-1,2,4-triazol-1-yl)methyl][1,1'-biphenyl]-2-carboxylic acid;
4'-[[5-tertbutyl-3-(1,1-difluorobutyl)-1H-1,2,4-triazol-1-yl]methyl][1,1'-biphenyl]-2-carboxylic acid;
4'-[(3,5-dipentyl-1H-1,2,4-triazol-1-yl)methyl][1,1'-biphenyl]-2-carboxylic acid;
4'-[[5-pentyl-3-(1,1-difluorobutyl)-1H-1,2,4-triazol-1-yl]methyl][1,1'-biphenyl]-2-carboxylic acid;
4'-[(3,5-diisopentyl-1H-1,2,4-triazol-1-yl)methyl][1,1'-biphenyl]-2-carboxylic acid;
4'-[[5-isopentyl-3-(1,1-difluorobutyl)-1H-1,2,4-triazol-1-yl]methyl][1,1'-biphenyl]-2-carboxylic acid;
4'-[[5-(1-butenyl)-3-(1,1-difluorobutyl)-1H-1,2,4-triazol-1-yl]methyl][1,1'-biphenyl]-2-carboxylic acid;
4'-[[5-(2-butenyl)-3(1,1-difluorobutyl)-1H-1,2,4-triazol-1-yl]methyl][1,1'-biphenyl]-2-carboxylic acid;
4'-[[5-(3-butenyl)-3-(1,1-difluorobutyl)-1H-1,2,4-triazol-1-yl]methyl][1,1'-biphenyl]-2-carboxylic acid;
4'-[[5-(1-butynyl)-3-(1,1-difluorobutyl)-1H-1,2,4-triazol-1-yl]methyl][1,1'-biphenyl]-2-carboxylic acid;
4'-[[5-(2-butynyl)-3-(1,1-difluorobutyl)-1H-1,2,4-triazol-1-yl]methyl][1,1'-biphenyl]-2-carboxylic acid;
4'-[[5-(3-butynyl)-3-(1,1-difluorobutyl)-1H-1,2,4-triazol-1-yl]methyl][1,1'-biphenyl]-2-carboxylic acid;
4'-[(3,5-diethoxy-1H-1,2,4-triazol-1-yl)methyl][1,1'-biphenyl]-2-carboxylic acid;
4'-[(3,5-dipropoxy-1H-1,2,4-triazol-1-yl)methyl][1,1-biphenyl]-2-carboxylic acid;
4'-[(3,5-diisopropoxy-1H-1,2,4-triazol-1-yl)methyl][1,1'-biphenyl]-2-carboxylic acid;
4'-[(3,5-dibutoxy-1H-1,2,4-triazol-1-yl)methyl][1,1'-biphenyl]-2-carboxylic acid;
4'-[[3,5-di(1-butenyl)-1H-1,2,4-triazol-1-yl]mathyl][1,1'-biphenyl]-2-carboxylic acid;
4'-[[3,5-di(2-butenyl)-1H-1,2,4-triazol-1-yl]methyl][1,1'-biphenyl]-2-carboxylic acid;
4'-[[3,5-di(3-butenyl)-1H-1,2,4-triazol-1-yl]methyl][1,1'-biphenyl]-2-carboxylic acid;
4'-[[3,5-di(1-butynyl)-1H-1,2,4-triazol-1-yl]methyl][1,1'-biphenyl]-2-carboxylic acid;
4'-[[3,5-di(2-butynyl)-1H-1,2,4-triazol-1-yl]methyl][1,1'-biphenyl]-2-carboxylic acid;
4'-[[3,5-di(3-butynyl)-1H-1,2,4-triazol-1-yl]methyl][1,1'-biphenyl]-2-carboxylic acid.

4. A compound of Claim 1 selected from compounds, and their pharmaceutically-acceptable salts, of the group of compounds consisting of
5-[4'-[(5-butyl-3-propyl-1H-1,2,4-triazol-1-yl)methyl][1,1'-biphenyl]-2-yl]-1H-tetrazole;
5-[4'-[(3,5-dibutyl-1H-1,2,4-triazol-1-yl)methyl][1,1'-biphenyl]-2-yl]-1H-tetrazole;
5-[4'-[(5-butyl-3-isopentyl-1H-1,2,4-triazol-1-yl)methyl][1,1'-biphenyl]-2-yl]-1H-tetrazole;
5-[4'-[(5-butyl-3-(1,1 dimethoxybutyl)-1H-1,2,4-triazol-1-yl]methyl][1,1'-biphenyl]-2-yl]-1H-tetrazole;
5-[4'-[[5-butyl-3-(1-oxobutyl)-1H-1,2,4-triazol-1-yl]methyl][1,1'-biphenyl]-2-yl]-1H-tetrazole;
5-[4'-[[5-butyl-3-(1,1-difluoroethyl)-1H-1,2,4-triazol-1-yl]methyl][1,1'-biphenyl]-2-yl]-1H-tetrazole;
5-[4'-[[5-butyl-3-(1,1-difluoropropyl)-1H-1,2,4-triazol-1-yl]methyl][1,1'-biphenyl]-2-yl]-1H-tetrazole;
5-[4'-[[5-butyl-3-(1,1-difluorobutyl)-1H-1,2,4-triazol-1-yl]methyl][1,1'-biphenyl]-2-yl]-1H-tetrazole;
5-[4'-[[3-butyl-5-(1,1-difluorobutyl)-1H-1,2,4-triazol-1-yl]methyl][1,1'-biphenyl]-2-yl]-1H-tetrazole;
5-[4'-[[5-butyl-3-(1,1-difluoropentyl)-1H-1,2,4-triazol-1-yl]methyl][1,1'-biphenyl]-2-yl]-1H-tetrazole;
5-[4'-[(3,5-dipropyl-1H-1,2,4-triazol-1-yl)methyl][1,1'-biphenyl]-2-yl]-1H-tetrazole;
5-[4'-[(3-butyl-5-propyl-1H-1,2,4-triazol-1-yl)methyl]-[1,1'-biphenyl]-2-yl]-1H-tetrazole;
5-[4'-[(5-butyl-3-isopentyl-1H-1,2,4-triazol-1-yl]methyl)-[1,1'-biphenyl]-2-yl]-1H-tetrazole;
5-[4'-[(3-phenyl-5-butyl-1H-1,2,4-triazol-1-yl)methyl](1,1'-biphenyl)-2-yl]-1H-tetrazole;
5-[4'[(5-butyl-3-phenylethyl-1H-1,2,4-triazol-1-yl)methyl](1,1'-biphenyl-2-yl]-1H-tetrazole;
5-[4'-[(5-butyl-3-ethyl-1H-1,2,4-triazol-1-yl)methyl][1,1'-biphenyl]-2-yl]-1H-tetrazole;
5-[4'-[(3-butyl-5-ethyl-1H-1,2,4-triazol-1-yl)methyl][1,1'-biphenyl]-2-yl]-1H-tetrazole; glutamine, 1,1-dimethylethyl ester;
5-[4'-[[5-butyl-3-(2-cyclohexylethyl)-1H-1,2,4-triazol-1-yl]methyl][1,1'-biphenyl]-2-yl]-1H-tetrazole;
5-[4'-[(5-butyl-3-(phenylmethyl)-1H-1,2,4-triazol-1-yl]methyl][1,1'-biphenyl]-2-yl]-1H-tetrazole;
5-[4'-[(5-butyl-3-cyclohexyl-1H-1,2,4-triazol-1-yl)methyl][1,1'-biphenyl]-2-yl]-1H-tetrazole;
5-[4'-[(3-cyclohexylmethyl-5-butyl-1H-1,2,4-triazol-1-yl)methyl][1,1'-biphenyl]-2-yl]-1H-tetrazole
5-[4'-[(3-butyl-5-cyclohexyl-1H-1,2,4-triazol-1-yl)methyl]-[1,1'-biphenyl]-2-yl]-1H-tetrazole.
5-[4'-[(3,5-isopropyl-1H-1,2,4-triazol-1-yl)methyl][1,1'-biphenyl]-2-yl]-1H-tetrazole;
5-[4'-[(5-propyl-3-isopentyl-1H-1,2,4-triazol-1-yl)methyl][1,1'-biphenyl]-2-yl]-1H-tetrazole;
5-[4'-[(5-propyl-3-(1,1-dimethoxybutyl)-1H-1,2,4-triazol-1-yl]methyl][1,1'-biphenyl]-2-yl]-1H-tetrazole;
5-[4'-[[5-propyl-3-(1-oxobutyl)-1H-1,2,4-triazol-1-yl]methyl][1,1'-biphenyl]-2-yl]-1H-tetrazole;
5-[4'-[[5-propyl-3-(1,1-difluoroethyl)-1H-1,2,4-triazol-1-yl]methyl][1,1'-biphenyl]-2-yl]-1H-tetrazole;
5-[4'-[[5-propyl-3-(1,1-difluoropropyl)-1H-1,2,4-triazol-1-yl]methyl][1,1'-biphenyl]-2-yl]-1H-tetrazole;
5-[4'-[[5-propyl-3-(1,1-difluorobutyl)-1H-1,2,4-triazol-1-yl]methyl][1,1'-biphenyl]-2-yl]-1H-tetrazole;
5-[4'-[[5-propyl-3-(1,1-difluropentyl)-1H-1,2,4-triazol-1-yl]methyl][1,1'-biphenyl]-2-yl]-1H-tetrazole;
5-[4'-[[5-ethyl-3-(1,1-difluorobutyl)-1H-1,2,4-triazol-1-yl]methyl][1,1'-biphenyl]-2-yl]-1H-tetrazole;
5-[4'-[(5-secbutyl-3-propyl-1H-1,2,4-trizol-1-yl)methyl][1,1'-biphenyl]-2-yl]-1H-tetrazole;
5-[4'-[(5-secbutyl-3-butyl-1H-1,2,4-triazol-1-yl)methyl][1,1'-biphenyl]-2-yl]-1H-tetrazole;
5-[4'-[(3,5-disecbutyl-1H-1,2,4-triazol-1-yl)methyl][1,1'-biphenyl]-2-yl]-1H-tetrazole;
5-[4'-[(5-secbutyl-3-isopentyl-1H-1,2,4-triazol-1-yl)methyl][1,1'-biphenyl]-2-yl]]-1H-tetrazole;
5-[4'-[[5-secbutyl-3-(1,1-difluoroethyl)-1H-1,2,4-triazol-1-yl]methyl][1,1'-biphenyl]-2-yl]-1H-tetrazole;
5-[4'-[[5-secbutyl-3-(1,1-difluoropropyl)-1H-1,2,4-triazol-1-yl]methyl][1,1'-biphenyl]-2-yl]-1H-tetrazole;
5-[4'-[[5-secbutyl-3-(1,1-difluorobutyl)-1H-1,2,4-triazol-1-yl]methyl][1,1'-biphenyl]-2-yl]-1H-tetrazole;
5-[4'-[[5-secbutyl-3-(1,1-difluoropentyl)-1H-1,2,4-triazol-1-yl]methyl][1,1'-biphenyl]-2-yl]-1H-tetrazole;
5-[4'-[(5-isobutyl-3-propyl-1H-1,2,4-triazol-1-yl)methyl][1,1'-biphenyl]-2-yl]-1H-tetrazole;
5-[4'-[(5-isobutyl-3-butyl-1H-1,2,4-triazol-1-yl)methyl][1,1'-biphenyl]2-yl]-1H-tetrazole;
5-[4'-[(3,5-diisobutyl-1H-1,2,4-triazol-1-yl)methyl][1,1'-biphenyl]-2-yl]-1H-tetrazole;
5-[4'-[(5-isobutyl-3-isopentyl-1H-1,2,4-triazol-1-yl)methyl][1,1'-biphenyl]-2-yl]-1H-tetrazole;
5-[4'-[[5-isobutyl-3-(1,1-difluoroethyl)-1H-1,2,4-triazol-1-yl]methyl][1,1'-biphenyl]-2-yl]-1H-tetrazole;
5-[4'-[[5-isobutyl-3-(1,1-difluoropropyl)-1H-1,2,4-triazol-1-yl]methyl][1,1'-biphenyl]-2-yl]-1H-tetrazole;
5-[4'-[[5-isobutyl-3-(1,1-difluorobutyl)-1H-1,2,4-triazol-1-yl]methyl][1,1'-biphenyl]-2-yl]-1H-tetrazole;
5-[4'-[[5-isobutyl-3-(1,1-difluoropentyl)-1H-1,2,4-triazol-1-yl]metyl][1,1'-biphenyl]-2-yl]-1H-tetrazole;
5-[4'-[(5-tertbutyl-3-propyl-1H-1,2,4-triazol-1-yl)methyl][1,1'-biphenyl]-2-yl]-1H-tetrazole;
5-[4'-[(5-tertbutyl-3-butyl-1H-1,2,4-triazol-1-yl)methyl][1,1'-biphenyl]-2-yl]-1H-tetrazole;
5-[4'-[(3,5-ditertbutyl-1H-1,2,4-triazol-1-yl)methyl][1,1'-biphenyl]-2-yl]-1H-tetrazole;
5-[4'-[(5-tertbutyl-3-isopentyl-1H-1,2,4-triazol-1-yl)methyl][1,1'-biphenyl]-2-yl]-1H-tetrazole;
5-[4'-[[5-tertbutyl-3-(1,1-difluoroethyl)-1H-1,2,4-triazol-1-yl]methyl][1,1'-biphenyl]-2-yl]-1H-tetrazole;
5-[4'-[[5-tertbutyl-3-(1,1-difluoropropyl)-1H-1,2,4-triazol-1-yl]methyl][1,1'-biphenyl]-2-yl]-1H-tetrazole;
5-[4'-[[5-tertbutyl-3-(1,1-difluorobutyl)-1H-1,2,4-triazol-1-yl]methyl][1,1'-biphenyl]-2-yl]-1H-tetrazole;
5-[4'-[[5-tertbutyl-3-(1,1-difluoropentyl)-1H-1,2,4-triazol-1-yl]methyl][1,1'-biphenyl]-2-yl]-1H-tetrazole;
5-[4'-[(5-pentyl-3-propyl-1H-1,2,4-triazol-1-yl)methyl][1,1'-biphenyl]-2-yl]-1H-tetrazole;
5-[4'-[(5-pentyl-3-butyl-1H-1,2,4-triazol-1-yl)methyl][1,1'-biphenyl]-2-yl]-1H-tetrazole;
5-[4'-[(3,5-dipentyl-1H-1,2,4-triazol-1-yl)methyl][1,1'-biphenyl]-2-yl]-1H-tetrazole;
5-[4'-[(5-pentyl-3-isopentyl-1H-1,2,4-triazol-1-yl)methyl][1,1'-biphenyl]-2-yl]-1H-tetrazole;
5-[4'-[[5-pentyl-3-(1,1-difluoroethyl)-1H-1,2,4-triazol-1-yl]methyl][1,1'-biphenyl]-2-yl]-1H-tetrazole;
5-[4'-[[5-pentyl-3-(1,1-difluoropropyl)-1H-1,2,4-triazol-1-yl]methyl][1,1'-biphenyl]-2-yl]-1H-tetrazole;
5-[4'-[[5-pentyl-3-(1,1-difluorobutyl)-1H-1,2,4-triazol-1-yl]methyl][1,1'-biphenyl]-2-yl]-1H-tetrazole;
5-[4'-[[5-pentyl-3-(1,1-difluoropentyl)-1H-1,2,4-triazol-1-yl]methyl][1,1'-biphenyl]-2-yl]-1H-tetrazole;
5-[4'-[(5-isopentyl-3-ethyl-1H-1,2,4-triazol-1-yl)methyl][1,1'-biphenyl]-2-yl]-1H-tetrazole;
5-[4'-[(5-isopentyl-3-propyl-1h-1,2,4-triazol-1-yl)methyl][1,1'-biphenyl]-2-yl]-1H-tetrazole;
5-[4'-[(5-isopentyl-3-butyl-1H-1,2,4-triazol-1-yl)methyl][1,1'-biphenyl]-2-yl]-1H-tetrazole;
5-[4'-[(3,5-diisopentyl-1H-1,2,4-triazol-1-yl)methyl][1,1'-biphenyl]-2-yl]-1H-tetrazole;
5-[4'-[[5-isopentyl-3-(1,1-difluoroethyl)-1H-1,2,4-triazol-1-yl]methyl][1,1'-biphenyl]-2-yl]-1H-tetrazole;
5-[4'-[[5-isopentyl-3-(1,1-difluoropropyl)-1H-1,2,4-triazol-1-yl]methyl][1,1'-bipheny]-2-yl]-1H-tetrazole;
5-[4'-[[5-isopentyl-3-(1,1-difluorobutyl)-1H-1,2,4-triazol-1-yl]methyl][1,1'-biphenyl]-2-yl]-1H-tetrazole;
5-[4'-[[5-isopentyl-3-(1,1-difluoropentyl)-1H-1,2,4-triazol-1-yl]methyl][1,1'-biphenyl-2-yl]-1H-tetrazole;
5-[4'-[[5-(1-butenyl)-3-propyl-1H-1,2,4-triazol-1-yl]methyl][1,1'-biphenyl]-2-yl]-1H-tetrazole;
5-[4'-[[5-(1-butenyl)-3-butyl-1H-1,2,4-triazol-1-yl]methyl][1,1'-biphenyl]-2-yl]-1H-tetrazole;
5-[4'-[[5-(1-butenyl)-3-isopentyl-1H-1,2,4-triazol-1-yl]methyl][1,1'-biphenyl]-2-yl]-1H-tetrazole;
5-[4'-[[5-(1-butenyl)-3-(1,1-dimethoxybutyl)-1H-1,2,4-triazol-1-yl]methyl][1,1'-biphenyl]-2-yl]-1H-tetrazole;
5-[4'-[[5-(1-butenyl)-3-(1,1-difluorobutyl)-1H-1,2,4-triazol-1-yl]methyl][1,1'-biphenyl]-2-yl]-1H-tetrazole;
5-[4'-[[5-(2-butenyl)-3-(1,1-difluorobutyl)-1H-1,2,4-triazol-1-yl]methyl][1,1'-biphenyl]-2-yl]-1H-tetrazole;
5-[4'-[[5-(3-butenyl)-3-(1,1-difluorobutyl)-1H-1,2,4-triazol-1-yl]methyl][1,1'-biphenyl]-2-yl]-1H-tetrazole;
5-[4'-[[5-(1-butynyl)-3-(1,1-difluorobutyl)-1H-1,2,4-triazol-1-yl]methyl][1,1'-biphenyl]-2-yl]-1H-tetrazole;
5-[4'-[[5-(2-butynyl)-3-(1,1-difluorobutyl)-1H-1,2,4-triazol-1-yl]methyl][1,1'-biphenyl]-2-yl]-1H-tetrazole;
5-[4'-[[5-(3-butynyl)-3-(1,1-difluorobutyl)-1H-1,2,4-triazol-1-yl]methyl][1,1'-biphenyl]-2-yl]-1H-tetrazole;
5-[4'-[[3,5-di(1-butenyl)-1H-1,2,4-triazol-1-yl]methyl][1,1'-biphenyl]-2-yl-]-1H-tetrazole;
5-[4'-[[3,5-di(2-butenyl)-1H-1,2,4-triazol-1-yl]methyl][1,1'-biphenyl]-2-yl]-1H-tetrazole;
5-[4'-[[3,5-di(3-butenyl)-1H-1,2,4-triazol-1-yl]methyl][1,1'-biphenyl]-2-yl]-1H-tetrazole;
5-[4'-[[3,5-di(1-butynyl)-1H-1,2,4-triazol-1-yl]methyl][1,1'-biphenyl]-2-yl]-1H-tetrazole;
5-[4'-[[3,5-di(2-butynyl)-1H-1,2,4-triazol-1-yl]methyl][1,1'-biphenyl]-2-yl]-1H-tetrazole; and
5-[4'-[[3,5-di(3-butynyl)-1H-1,2,4-triazol-1-yl]methyl][1,1'-biphenyl]-2-yl]-1H-tetrazole.

5. Compound of claim 1 selected from compounds, and their pharmaceutically-acceptable salts, of the group of compounds consisting of
4'-[(3,5-dibutyl-1H-1,2,4-triazol-1-yl)methyl][1,1'-biphenyl]-2-carboxylic acid;
4'-[(3-ethyl-5-butyl-1H,1,2,4-triazol-1-yl)methyl][1,1'-biphenyl]-2-carboxylic acid;
4'-[(3-butyl-5-ethyl-1H-1,2,4-triazol-1-yl)methyl][1,1'-biphenyl]-2-carboxylic acid;
4'-[(5-butyl-1H-1,2,4-triazol-1-yl)methyl][1,1'-biphenyl]-2-carboxylic acid;
4'-[(3-butyl-1H-1,2,4-triazol-1-yl)methyl][1,1'-biphenyl]-2-carboxylic acid.

6. A pharmaceutical composition comprising a therapeuti-cally-effective amount of an angiotensin II antagonist compound and a pharmaceutically-acceptable carrier or diluent said antagonist compound selected from a family of compounds according to any of Claims 1-5 or the amide of compounds wherein R³ = COOH, or their salts.

7. Use of a compound according to any of Claims 1-5 or the amide of compounds wherein R³ = COOH, or their salts for preparing a medicament for treating a circulatory disorder.

8. Use according to Claim 7 wherein said disorder is a cardiovascular disorder.

9. Use according to Claim 8 wherein said cardiovascular disorder is hypertension.

10. Use according to Claim 8 wherein said cardiovascular disorder is congestive heart failure.

11. Use of a compound, according to any of Claims 1-5 or the amide of compounds wherein R³ = COOH, or their salts for preparing a medicament for treating glaucoma.

12. A Compound which is methyl-4'-[(3,5-dibutyl-1H-1,2,4-triazol-1-yl)methyl][1,1'-biphenyl-2-carboxylate or 4'-[(3,5-dibutyl-1H-1,2,4-triazol-1-yl)methyl][1,1'-biphenyl]-2-carbxylic acid, hydrazide.

## Claims (Claims for the following Contracting State(s): ES)

1. A process for preparing a ompound of Formula I 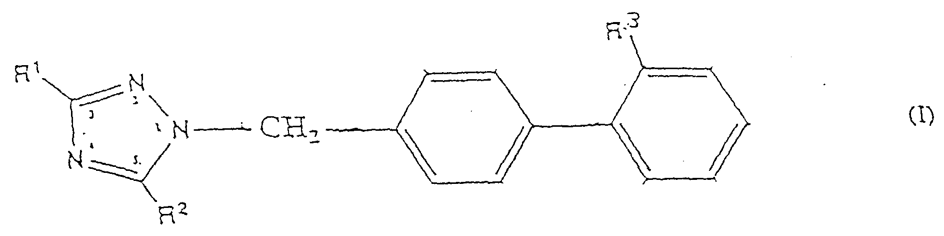 wherein each of R¹ and R² is independently selected from hydrogen, C₁-C₂₀ alkyl, phenyl, phenyl substituted by C₁-C₂₀ alkyl, C₃-C₁₀ cycloalkyl, C₃-C₁₀ cycloalkyl, attached to or substituted with C₁-C₂₀ alkyl, C₁-C₁₀-alkoxy, C₁-C₁₀-di-fluoroalkyl substituted by phenyl, C₂-C₂₀ alkenyl, C₂-C₂₀ alkynyl, C₁-C₂₀ alkylcarbonyl; difluoro-substituted C₁-C₂₀ alkyl; C₁-C₁₀-hydroxyalkyl, C₁-C₂₀ alkyl substituted by one or more alkoxy groups having alkyl portions of 1 to 10 carbon atoms; wherein R³ is an acidic moiety selected from CO₂H, CONHNHSO₂CF₃, 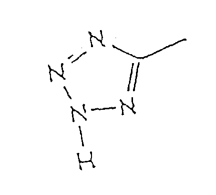 and the salts of said acidic moieties; or a tautomer thereof or a pharmaceutically-acceptable salt thereof,
characterized in that
A) a compound of the formula 7 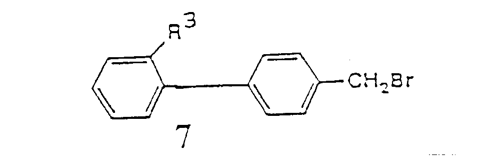 wherein R³ is as defined above in a protected form is reacted with a compound 1 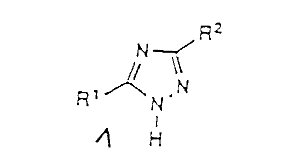 wherein R¹, R² are defined as indicated above under basic conditions to give the respective coupled regioisomers which may be converted either by acid or basic treatment to give the compounds wherein R³ is tetrazole or carboxylic acid or a derivative thereof;
or
B) that a compound of the formula 15 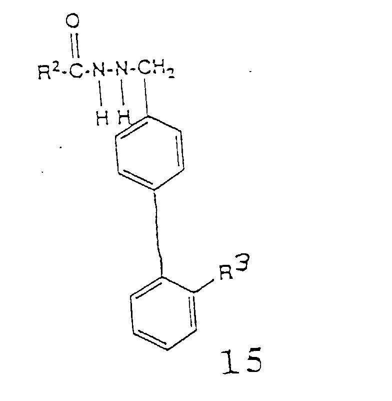 wherein R² and R³ are defined as indicated above is reacted with an imidate 2 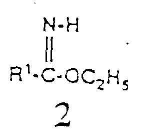 with R¹ as defined above, under appropriate conditiions to give upon heat-cyclizing the corresponding final products or
C) a suitably substituted imidazone 17 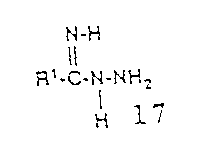 with R¹ as defined above
is reacted under appropriate conditions with an alkylating reagent 7 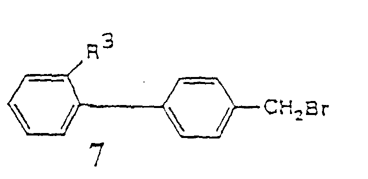 with R³ as defined above,
to give an intermediate which is then cyclized in the presence of heat and an appropriate orthoester to give the desired final compounds
which optionally may be transformed in a known manner into their respective amides, esters, salts or pharmaceutically acceptable salts thereof.

2. A process according to Claim 1 wherein R³ of the compound prepared is selected from COOH or tetrazolyl or a tautomer or a pharmaceutically-acceptable salt thereof.

3. A process according to Claim 2 wherein the compound prepared is selected from compounds, and their pharmaceutically-acceptable salts, of the group of compounds consisting of
4'-[(3,5-dibutyl-1H-1,2,4-triazol-1-yl)methyl][1,1'-biphenyl]-2-carboxylic acid, 2-(trifluoromethyl)sulfonyl]hydrazide;
4'-[(5-butyl-3-propyl-1H-1,2,4-triazol-1-yl)methyl][1,1'-biphenyl]-2-carboxylic acid;
4'-[(3,5-dibutyl-1H-1,2,4-triazol-1-yl)methyl][1,1'-biphenyl]-2-carboxylic acid;
4'-[(5-butyl-3-isopentyl-1H-1,2,4-triazol-1-yl)methyl][1,1'-biphenyl]-2-carboxylic acid;
4'-[[5-butyl-3-(1,1-dimethoxybutyl)-1H-1,2,4-triazol-1-yl]methyl][1,1'-biphenyl]-2-carboxylic acid;
4'-[[5-butyl-3-(1-oxobutyl)-1H-1,2,4-triazol-1-yl]methyl][1,1'-biphenyl]-2-carboxylic acid;
4'-[[5-butyl-3-(1-oxopentyl)-1H-1,2,4-triazol-1-yl]methyl][1,1'-biphenyl]-2-carboxylic acid;
4'-[[5-butyl-3-(1,1-difluoroethyl)-1H-1,2,4-triazol-1-yl]methyl][1,1'-biphenyl]-2-carboxylic acid;
4'-[[5-butyl-3-(1,1-difluoropropyl)-1H-1,2,4-triazol-1-yl]methyl][1,1'-biphenyl]-2-carboxylic acid;
4'-[[5-butyl-3-(1,1-difluorobutyl)-1H-1,2,4-triazol-1-yl]methyl][1,1'-biphenyl]-2-carboxylic acid;
4'-[[3-butyl-5-(1,1-difluorobutyl)-1H-1,2,4-triazol-1-yl]methyl][1,1'-biphenyl]-2-carboxylic acid;
4'-[[5-butyl-3-(1,1-difluoropentyl)-1H-1,2,4-triazol-1-yl]methyl][1,1,'-biphenyl]-2-carboxylic acid;
4'-[(3,5-dipropyl-1H-1,2,4-triazol-1-yl)methyl][1,1'-biphenyl]-2-carboxylic acid;
4'-[(3,5-isopropyl-1H-1,2,4-triazol-1-yl)methyl][1,1'-biphenyl]-2-carboxylic acid;
4'-[(5-propyl-3-butyl-1H-1,2,4-triazol-1-yl)methyl][1,1'-bipheny]-2-carboxylic acid;
4'-[[5-propyl-3-(1,1-difluoroethyl)-1H-1,2,4-triazol-1-yl]methyl][1,1'-biphenyl]-2-carboxylic acid;
4'-[[5-propyl-3-(1,1-difluoropropyl)-1H-1,2,4-triazol-1-yl]methyl][1,1'-biphenyl]-2-carboxylic acid;
4'-[[5-propyl-3-(1,1-difluorobutyl)-1H-1,2,4-triazol-1-yl]methyl][1,1'-biphenyl]-2-carboxylic acid;
4'-[[5-propyl-3-(1,1-difluoropentyl)-1H-1,2,4-triazol-1-yl]methyl][1,1'-biphenyl]-2-carboxylic acid;
4'-[[5-ethyl-3-(1,1-difluorobutyl)-1H-1,2,4-triazol-1-yl]methyl][1,1'-biphenyl]-2-carboxylic acid;
4'-[(3,5-disecbutyl-1H-1,2,4-triazol-1-yl)methyl][1,1'-biphenyl]-2-carboxylic acid;
4'-[[5-secbutyl-3-(1,1-difluorobutyl)-1H-1,2,4-triazol-1-yl]methyl][1,1'-biphenyl]-2-carboxylic acid;
4'-[(3,5-diisobutyl-1H-1,2,4-triazol-1-yl)methyl][1,1'-biphenyl]-2-carboxylic acid;
4'-[[5-isobutyl-3-(1,1-difluorobutyl)-1H-1,2,4-triazol-1-yl]methyl][1,1'-biphenyl]-2-carboxylic acid;
4'-[(3,5-ditertbutyl-1H-1,2,4-triazol-1-yl)methyl][1,1'-biphenyl]-2-carboxylic acid;
4'-[[5-tertbutyl-3-(1,1-difluorobutyl)-1H-1,2,4-triazol-1-yl]methyl][1,1'-biphenyl]-2-carboxylic acid;
4'-[(3,5-dipentyl-1H-1,2,4-triazol-1-yl)methyl][1,1'-biphenyl]-2-carboxylic acid;
4'-[[5-pentyl-3-(1,1-difluorobutyl)-1H-1,2,4-triazol-1-yl]methyl][1,1'-biphenyl]-2-carboxylic acid;
4'-[(3,5-diisopentyl-1H-1,2,4-triazol-1-yl)methyl][1,1'-biphenyl]-2-carboxylic acid;
4'-[[5-isopentyl-3-(1,1-difluorobutyl)-1H-1,2,4-triazol-1-yl]methyl][1,1'-biphenyl]-2-carboxylic acid;
4'-[[5-(1-butenyl)-3-(1,1-difluorobutyl)-1H-1,2,4-triazol-1-yl]methyl][1,1'-biphenyl]-2-carboxylic acid;
4'-[[5-(2-butenyl)-3-(1,1-difluorobutyl)-1H-1,2,4-triazol-1-yl]methyl][1,1'-biphenyl]-2-carboxylic acid;
4'-[[5-(3-butenyl)-3-(1,1-difluorobutyl)-1H-1,2,4-triazol-1-yl]methyl][1,1'-biphenyl]-2-carboxylic acid;
4'-[[5-(1-butynyl)-3-(1,1-difluorobutyl)-1H-1,2,4-triazol-1-yl]methyl][1,1'-biphenyl]-2-carboxylic acid;
4'-[[5-(2-butynyl)-3-(1,1-difluorobutyl)-1H-1,2,4-triazol-1-yl]methyl][1,1'-biphenyl]-2-carboxylic acid;
4'-[[5-(3-butynyl)-3-(1,1-difluorobutyl)-1H-1,2,4-triazol-1-yl]methyl][1,1'-biphenyl]-2-carboxylic acid;
4'-[(3,5-diethoxy-1H-1,2,4-triazol-1-yl)methyl][1,1'-biphenyl]-2-carboxylic acid;
4'-[(3,5-dipropoxy-1H-1,2,4-triazol-1-yl)methyl][1,1'-biphenyl]-2-carboxylic acid;
4'-[(3,5-diisopropoxy-1H-1,2,4-triazol-1-yl)methyl][1,1'-biphenyl]-2-carboxylic acid;
4'-[(3,5-dibutoxy-1H-1,2,4-triazol-1-yl)methyl][1,1'-biphenyl]-2-carboxylic acid;
4'-[[3,5-di(1-butenyl)-1H-1,2,4-triazol-1-yl]methyl][1,1'-biphenyl]-2-carboxylic acid;
4'-[[3,5-di(2-butenyl)-1H-1,2,4-triazol-1-yl]methyl][1,1'-biphenyl]-2-carboxylic acid;
4'-[[3,5-di(3-butenyl)-1H-1,2,4-triazol-1-yl]methyl][1,1'-biphenyl]-2-carboxylic acid;
4'-[[3,5-di(1-butynyl)-1H-1,2,4-triazol-1-yl]methyl][1,1'-biphenyl]-2-carboxylic acid;
4'-[[3,5-di(2-butynyl)-1H-1,2,4-triazol-1-yl]methyl][1,1'-biphenyl]-2-carboxylic acid;
4'-[[3,5-di(3-butynyl)-1H-1,2,4-triazol-1-yl]methyl][1,1'-biphenyl]-2-carboxylic acid.

4. A process according to Claim 1 wherein the compound prepared is selected from compounds, and their pharmaceutically-acceptable salts, of the group of compounds consisting of
5-[4'-[(5-butyl-3-propyl-1H-1,2,4-triazol-1-yl)methyl][1,1'-biphenyl]-2-yl]-1H-tetrazole;
5-[4'-[(3,5-dibutyl-1H-1,2,4-triazol-1-yl)methyl][1,1'-biphenyl]-2-yl]-1H-tetrazole;
5-[4'-[(5-butyl-3-isopentyl-1H-1,2,4-triazol-1-yl)methyl][1,1'-biphenyl]-2-yl]-1H-tetrazole;
5-[4'-[[5-butyl-3-(1,1 dimethoxybutyl)-1H-1,2,4-triazol-1-yl]methyl][1,1'-biphenyl]-2-yl]-1H-tetrazole;
5-[4'-[[5-butyl-3-(1-oxobutyl)-1H-1,2,4-triazol-1-yl]methyl][1,1'-biphenyl]-2-yl]-1H-tetrazole;
5-[4'-[[5-butyl-3-(1,1-difluoroethyl)-1H-1,2,4-triazol-1-yl]methyl][1,1'-biphenyl]-2-yl]-1H-tetrazole;
5-[4'-[[5-butyl-3-(1,1-difluoropropyl)-1H-1,2,4-triazol-1-yl]methyl][1,1'-biphenyl]-2-yl]-1H-tetrazole;
5-[4'-[[5-butyl-3-(1,1-difluorobutyl)-1H-1,2,4-triazol-1-yl]methyl][1,1'-biphenyl]-2-yl]-1H-tetrazole;
5-[4'-[[3-butyl-5-(1,1-difluorobutyl)-1H-1,2,4-triazol-1-yl]methyl][1,1'-biphenyl]-2-yl]-1H-tetrazole;
5-[4'-[[5-butyl-3-(1,1-difluoropentyl)-1H-1,2,4-triazol-1-yl]methyl][1,1'-biphenyl]-2-yl]-1H-tetrazole;
5-[4'-[(3,5-dipropyl-1H-1,2,4-triazol-1-yl)methyl][1,1'-biphenyl]-2-yl]-1H-tetrazole;
5-[4'-[(3-butyl-5-propyl-1H-1,2,4-triazol-1-yl)methyl]-[1,1'-biphenyl]-2-yl]-1H-tetrazole;
5-[4'-[(5-butyl-3-isopentyl-1H-1,2,4-triazol-1-yl]methyl)-[1,1'-biphenyl]-2-yl]-1H-tetrazole;
5-[4'-[(3-phenyl-5-butyl-1H-1,2,4-triazol-1-yl)methyl][1,1'-biphenyl)-2-yl]-1H-tetrazole;
5-[4'[(5-butyl-3-phenylethyl-1H-1,2,4-triazol-1-yl)methyl][1,1'-biphenyl-2-yl]-1H-tetrazole;
5-[4'-[(5-butyl-3-ethyl-1H-1,2,4-triazol-1-yl)methyl][1,1'-biphenyl]-2-yl]-1H-tetrazole;
5-[4'-[(3-butyl-5-ethyl-1H-1,2,4-triazol-1-yl)methyl][1,1'-biphenyl]-2-yl]-1H-tetrazole; glutamine, 1,1-dimethylethyl ester;
5-[4'-[[5-butyl-3-(2-cyclohexylethyl)-1H-1,2,4-triazol-1-yl]methyl][1,1'-biphenyl]-2-yl]-1H-tetrazole;
5-[4'-[[5-butyl-3-(phenylmethyl)-1H-1,2,4-triazol-1-yl]methyl][1,1'-biphenyl]-2-yl]-1H-tetrazole;
5-[4'-[(5-butyl-3-cyclohexyl-1H-1,2,4-triazol-1-yl)methyl][1,1'-biphenyl]-2-yl]-1H-tetrazole;
5-[4'-[(3-cyclohexylmethyl-5-butyl-1H-1,2,4-triazol-1-yl)methyl][1,1'-biphenyl]-2-yl]-1H-tetrazole
5-[4'-[(3-butyl-5-cyclohexyl-1H-1,2,4-triazol-1-yl)methyl]-[1,1'-biphenyl]-2-yl]-1H-tetrazole.
5-[4'[(3,5-isopropyl-1H-1,2,4-triazol-1-yl)methyl][1,1'-biphenyl]]-2-yl]-1H-tetrazole;
5-[4'-[(5-propyl-3-isopentyl-1H-1,2,4-triazol-1-yl)methyl][1,1'-biphenyl]-2-yl]-1H-tetrazole;
5-[4'-[(5-propyl-3-(1,1-dimethoxybutyl)-1H-1,2,4-triazol-1-yl]methyl][1,1'-biphenyl]-2-yl]-1H-tetrazole;
5-[4'-[[5-propyl-3-(1-oxobutyl)-1H-1,2,4-triazol-1-yl]methyl][1,1'-biphenyl]-2-yl]-1H-tetrazole;
5-[4'-[[5-propyl-3-(1,1-difluoroethyl)-1H-1,2,4-triazol-1-yl]methyl][1,1'-biphenyl]-2-yl]-1H-tetrazole;
5-[4'-[[5-propyl-3-(1,1-difluoropropyl)-1H-1,2,4-triazol-1-yl]methyl][1,1'-biphenyl]-2-yl]-1H-tetrazole;
5-[4'-[[5-propyl-3-(1,1-difluorobutyl)-1H-1,2,4-triazol-1-yl]methyl][1,1'-biphenyl]-2-yl]-1H-tetrazole;
5-[4'-[[5-propyl-3-(1,1-difluoropentyl)-1H-1,2,4-triazol-1-yl]methyl][1,1'-biphenyl]-2-yl]-1H-tetrazole;
5-[4'-[[5-ethyl-3-(1,1-difluorobutyl)-1H-1,2,4-triazol-1-yl]methyl][1,1'-biphenyl]-2-yl]-1H-tetrazole;
5-[4'-[(5-secbutyl-3-propyl-1H-1,2,4-triazol-1-yl)methyl](1,1'-biphenyl]-2-yl]-1H-tetrazole;
5-[4'-[(5-secbutyl-3-butyl-1H-1,2,4-triazol-1-yl)methyl][1,1'-biphenyl]-2-yl]-1H-tetrazole;
5-[4'-[(3,5-disecbutyl-1H-1,2,4-triazol-1-yl)methyl][1,1'-biphenyl]-2-yl]-1H-tetrazole;
5-[4'-[(5-secbutyl-3-isopentyl-1H-1,2,4-triazol-1-yl)methyl][1,1'-biphenyl]-2-yl]-1H-tetrazole;
5-[4'-[[5-secbutyl-3-(1,1-difluoroethyl)-1H-1,2,4-triazol-1-yl]methyl][1,1'-biphenyl]-2-yl]-1H-tetrazole;
5-[4'-[[5-secbutyl-3-(1,1-difluoropropyl)-1H-1,2,4-triazol-1-yl)methyl][1,1'-biphenyl]-2-yl]-1H-tetrazole;
5-[4'-[[5-secbutyl-3-(1,1-difluorobutyl)-1H-1,2,4-triazol-1-yl]methyl][1,1'-biphenyl]-2-yl]-1H-tetrazole;
5-[4'-[[5-secbutyl-3-(1,1-difluoropentyl)-1H-1,2,4-triazol-1-yl]methyl][1,1'-biphenyl]-2-yl]-1H-tetrazole;
5-[4'-[(5-isobutyl-3-propyl-1H-1,2,4-triazol-1-yl)methyl][1,1'-biphenyl]-2-yl]-1H-tetrazole;
5-[4'-[(5-isobutyl-3-butyl-1H-1,2,4-triazol-1-yl)methyl][1,1'-biphenyl]-2-yl]-1H-tetrazole;
5-[4'-[(3,5-diisobutyl-1H-1,2,4-triazol-1-yl)methyl][1,1'-biphenyl]-2-yl]-1H-tetrazole;
5-[4'-[(5-isobutyl-3-isopentyl-1H-1,2,4-triazol-1-yl)methyl][1,1'-biphenyl]-2-yl]-1H-tetrazole;
5-[4'-[[5-isobutyl-3-(1,1-difluoroethyl)-1H-1,2,4-triazol-1-yl]methyl][1,1'-biphenyl]-2-yl]-1H-tetrazole;
5-[4'-[[5-isobutyl-3-(1,1-difluoropropyl)-1H-1,2,4-triazol-1-yl]methyl][1,1'-biphenyl]-2-yl]-1H-tetrazole;
5-[4'-[[5-isobutyl-3-(1,1-difluorobutyl)-1H-1,2,4-triazol-1-yl]methyl][1,1'-biphenyl]-2-yl]-1H-tetrazole;
5-[4'-[[5-isobutyl-3-(1,1-difluoropentyl)-1H-1,2,4-triazol-1-yl]methyl][1,1'-biphenyl]-2-yl]-1H-tetrazole;
5-[4'-[(5-tertbutyl-3-propyl-1H-1,2,4-triazol-1-yl)methyl][1,1'-biphenyl]-2-yl]-1H-tetrazole;
5-[4'-[(5-tertbutyl-3-butyl-1H-1,2,4-triazol-1-yl)methyl][1,1'-biphenyl]-2-yl]-1H-tetrazole;
5-[4'-[(3,5-ditertbutyl-1H-1,2,4-triazol-1-yl)methyl][1,1'-biphenyl]-2-yl]-1H-tetrazole;
5-[4'-[(5-tertbutyl-3-isopentyl-1H-1,2,4-triazol-1-yl)methyl][1,1'-biphenyl]-2-yl]-1H-tetrazole;
5-[4'-[[5-tertbutyl-3-(1,1-difluoroethyl)-1H-1,2,4-triazol-1-yl]methyl][1,1'-biphenyl]-2-yl]-1H-tetrazole;
5-[4'-[[5-tertbutyl-3-(1,1-difluoropropyl)-1H-1,2,4-triazol-1-yl]methyl][1,1'-biphenyl]-2-yl]-1H-tetrazole;
5-[4'-[[5-tertbutyl-3-(1,1-difluorobutyl)-1H-1,2,4-triazol-1-yl]methyl][1,1'-biphenyl]-2-yl]-1H-tetrazole;
5-[4'-[[5-tertbutyl-3-(1,1-difluoropentyl)-1H-1,2,4-triazol-1-yl]methyl][1,1'-biphenyl]-2-yl]-1H-tetrazole;
5-[4'-[(5-pentyl-3-propyl-1H-1,2,4-triazol-1-yl)methyl][1,1'-biphenyl]-2-yl]-1H-tetrazole;
5-[4'-[(5-pentyl-3-butyl-1H-1,2,4-triazol-1-yl)methyl][1,1'-biphenyl]-2-yl]-1H-tetrazole;
5-[4'-[(3,5-dipentyl-1H-1,2,4-triazol-1-yl-1-yl)methyl][1,1'-biphenyl]-2-yl]-1H-tetrazole;
5-[4'-[(5-pentyl-3-isopentyl-1H-1,2,4-triazol-1-yl)methyl][1,1'-biphenyl]-2-yl]-1H-tetrazole;
5-[4'-[[5-pentyl-3-(1,1-difluoroethyl)-1H-1,2,4-triazol-1-yl]methyl][1,1'-biphenyl]-2-yl]-1H-tetrazole;
5-[4'-[[5-pentyl-3-(1,1-difluoropropyl)-1H-1,2,4-triazol-1-yl]methyl][1,1'-biphenyl]-2-yl]-1H-tetrazole;
5-[4'-[[5-pentyl-3-(1,1-difluorobutyl)-1H-1,2,4-triazol-1-yl]methyl][1,1'-biphenyl]-2-yl]-1H-tetrazole;
5-[4'-[[5-pentyl-3-(1,1-difluoropentyl)-1H-1,2,4-triazol-1-yl]methyl][1,1'-biphenyl]-2-yl]-1H-tetrazole;
5-[4'-[(5-isopentyl-3-ethyl-1H-1,2,4-triazol-1-yl)methyl][1,1'-biphenyl]-2-yl]-1H-tetrazole;
5-[4'-[(5-isopentyl-3-propyl-1H-1,2,4-triazol-1-yl)methyl][1,1'-biphenyl]-2-yl]-1H-tetrazole;
5-[4'-[(5-isopentyl-3-butyl-1H-1,2,4-triazol-1-yl)methyl][1,1'-biphenyl]-2-yl]-1H-tetrazole;
5-[4'-[(3,5-diisopentyl-1H-1,2,4-triazol-1-yl)methyl][1,1'-biphenyl]-2-yl]-1H-tetrazole;
5-[4'-[[5-isopentyl-3-(1,1-difluoroethyl)-1H-1,2,4-triazol-1-yl]methyl][1,1'-biphenyl]-2-yl]-1H-tetrazole;
5-[4'-[[5-isopentyl-3-(1,1-difluoropropyl)-1H-1,2,4-triazol-1-yl]methyl][1,1'-bipheny]-2-yl]-1H-tetrazole;
5-[4'-[[5-isopentyl-3-(1,1-difluorobutyl)-1H-1,2,4-triazol-1-yl]methyl][1,1'-biphenyl]-2-yl]-1H-tetrazole;
5-[4'-[[5-isopentyl-3-(1,1-difluoropentyl)-1H-1,2,4-triazol-1-yl]methyl][1,1'-biphenyl-2-yl]-1H-tetrazole;
5-[4'-[[5-(1-butenyl)-3-propyl-1H-1,2,4-triazol-1-yl]methyl][1,1'-biphenyl]-2-yl]-1H-tetrazole;
5-[4'-[[5-(1-butenyl)-3-butyl-1H-1,2,4-triazol-1-yl]methyl][1,1'-biphenyl]-2-yl]-1H-tetrazole;
5-[4'-[[5-(1-butenyl)-3-isopentyl-1H-1,2,4-triazol-1-yl]methyl][1,1'-biphenyl]-2-yl]-1H-tetrazole;
5-[4'-[[5-(1-butenyl)-3-(1,1-dimethoxybutyl)-1H-1,2,4-triazol-1-yl]methyl][1,1'-biphenyl]-2-yl]-1H-tetrazole;
5-[4'-[[5-(1-butenyl)-3-(1,1-difluorobutyl)-1H-1,2,4-triazol-1-yl]methyl][1,1'-biphenyl]-2-yl]-1H-tetrazole;
5-[4'-[[5-(2-butenyl)-3-(1,1-difluorobutyl)-1H-1,2,4-triazol-1-yl]methyl][1,1'-biphenyl]-2-yl]-1H-tetrazole;
5-[4'-[[5-(3-butenyl)-3-(1,1-difluorobutyl)-1H-1,2,4-triazol-1-yl]methyl][1,1'-biphenyl]-2-yl]-1H-tetrazole;
5-[4'-[[5-(1-butynyl)-3-(1,1-difluorobutyl)-1H-1,2,4-triazol-1-yl]methyl][1,1'-biphenyl]-2-yl]-1H-tetrazole;
5-[4'-[[5-(2-butynyl)-3-(1,1-difluorobutyl)-1H-1,2,4-triazol-1-yl]methyl][1,1'-biphenyl]-2-yl]-1H-tetrazole;
5-[4'-[[5-(3-butynyl)-3-(1,1-difluorobutyl)-1H-1,2,4-triazol-1-yl]methyl][1,1'-biphenyl]-2-yl]-1H-tetrazole;
5-[4'-[[3,5-di(1-butenyl)-1H-1,2,4-triazol-1-yl]methyl][1,1'-biphenyl]-2-yl-]-1H-tetrazole;
5-[4'-[[3,5-di(2-butenyl)-1H-1,2,4-triazol-1-yl]methyl][1,1'-biphenyl]-2-yl]-1H-tetrazole;
5-[4'-[[3,5-di(3-butenyl)-1H-1,2,4-triazol-1-yl]methyl][1,1'-biphenyl]-2-yl]-1H-tetrazole;
5-[4'-[[3,5-di(1-butynyl)-1H-1,2,4-triazol-1-yl]methyl][1,1'-biphenyl]-2-yl]-1H-tetrazole;
5-[4'-[[3,5-di(2-butynyl)-1H-1,2,4-triazol-1-yl]methyl][1,1'-biphenyl]-2-yl]-1H-tetrazole; and
5-[4'-[[3,5-di(3-butynyl)-1H-1,2,4-triazol-1-yl]methyl][1,1'-biphenyl]-2-yl]-1H-tetrazole.

5. A process according to Claim 1 wherein the compound prepared is selected from compounds, and their pharmaceutically-acceptable salts, of the group of compounds consisting of
4'-[(3,5-dibutyl-1H-1,2,4-triazol-1-yl)methyl][1,1'-biphenyl]-2-carboxylic acid;
4'-[(3-ethyl-5-butyl-1H,1,2,4-triazol-1-yl)methyl][1,1'-biphenyl]-2-carboxylic acid;
4'-[(3-butyl-5-ethyl-1H-1,2,4-triazol-1-yl)methyl][1,1'-biphenyl]-2-carboxylic acid;
4'-[(5-butyl-1H-1,2,4-triazol-1-yl)methyl][1,1'-biphenyl]-2-carboxylic acid;
4'-[(3-butyl-1H-1,2,4-triazol-1-yl)methyl][1,1'-biphenyl]-2-carboxylic acid.

6. A process for preparing a pharmaceutical composition characterized by admixing a therapeutically-effective amount of an angiotensin II antagonist compound and a pharmaceutically-acceptable carrier or diluent, said antagonist compound selected from a family of compounds according to any of Claims 1-5 or the amide of compounds wherein R³ = COOH, or their salts.

7. Use of a compound according to any of Claims 1-5 or the amide of compounds wherein R³ = COOH, or their salts for preparing a medicament for treating a circulatory disorder.

8. Use according to Claim 7 wherein said disorder is a cardiovascular disorder.

9. Use according to Claim 8 wherein said cardiovascular disorder is hypertension.

10. Use according to Claim 8 wherein said cardiovascular disorder is congestive heart failure.

11. Use of a compound according to any of Claims 1-5 or amide of compounds wherein R³ = COOH, or their salts for preparing a medicament for treating glaucoma.

12. A process for preparing a compound of the formula 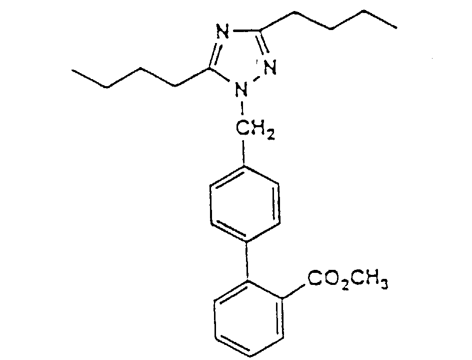 characterized in that 3,5-dibutyl-1H-1,2,4-triazole is added to 4-bromo-methyl-2'-methoxycarbonyl-biphenyl in the presence of sodium hydride.

13. A process for preparing a compound of the formula 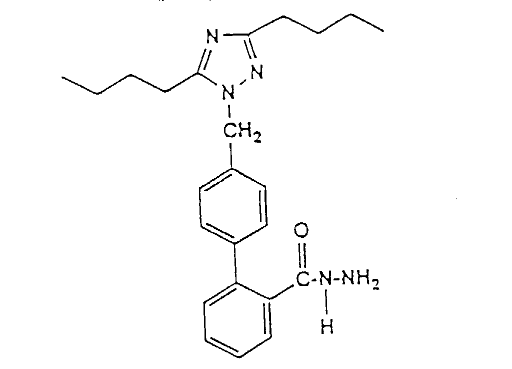 characterized in that the compound as prepared according to Claim 12 is reacted with anhydrous hydrazine.

## Claims (Claims for the following Contracting State(s): GR)

1. Compound of Formula I 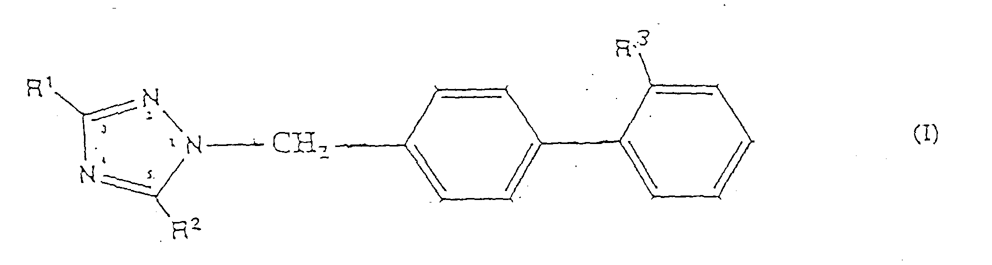 wherein each of R¹ and R² is independently selected from hydrogen, C₁-C₂₀ alkyl, phenyl, phenyl substituted by C₁-C₂₀ alkyl, C₃-C₁₀ cycloalkyl, C₃-C₁₀ cycloalkyl, attached to or substituted with C₁-C₂₀ alkyl, C₁-C₁₀ alkoxy, C₁-C₁₀-difluoroalkyl substituted by phenyl, C₂-C₂₀ alkenyl, C₂-C₂₀ alkynyl, C₁-C₂₀ alkylcarbonyl; difluoro-substituted C₁-C₂₀ alkyl;
C₁-C₁₀-hydroxyalkyl, C₁-C₂₀ alkyl substituted by one or more alkoxy groups having alkyl portions of 1 to 10 carbon atoms; wherein R³ is an acidic moiety selected from CO₂H, CONHNHSO₂CF₃, 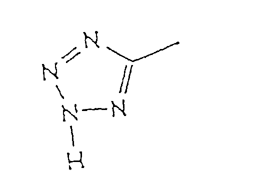 and the salts of said acidic moieties; or a tautomer thereof or a pharmaceutically-acceptable salt thereof.

2. Compound of Claim 1 wherein R³ is selected from COOH or tetrazolyl or a tautomer or a pharmaceutically-acceptable salt thereof.

3. Compound of Claim 2 selected from compounds, and their pharmaceutically-acceptable salts, of the group of compounds consisting of
4'-[(3,5-dibutyl-1H-1,2,4-triazol-1-yl)methyl][1,1'-biphenyl]-2-carboxylic acid, 2-[(trifluoromethyl)sulfonyl]hydrazide;
4'-[(5-butyl-3-propyl-1H-1,2,4-triazol-1-yl)methyl][1,1'-biphenyl]-2-carboxylic acid;
4'-[(3,5-dibutyl-1H-1,2,4-triazol-1-yl)methyl][1,1'-biphenyl]-2-carboxylic acid;
4'-[(5-butyl-3-isopentyl-1H-1,2,4-triazol-1-yl)methyl][1,1'-biphenyl]-2-carboxylic acid;
4'-[[5-butyl-3-(1,1-dimethoxybutyl)-1H-1,2,4-triazol-1-yl]methyl][1,1'-biphenyl]-2-carboxylic acid;
4'-[[5-butyl-3-(1-oxobutyl)-1H-1,2,4-triazol-1-yl]methyl][[1,1'-biphenyl]-2-carboxylic acid;
4'-[[5-butyl-3-(1-oxopentyl)-1H-1,2,4-triazol-1-yl]methyl][1,1'-biphenyl]-2-carboxylic acid;
4'-[[5-butyl-3-(1,1-difluoroethyl)-1H-1,2,4-triazol-1-yl]methyl][1,1'-biphenyl]-2-carboxylic acid;
4'-[[5-butyl-3-(1,1-difluoropropyl)-1H-1,2,4-triazol-1-yl]methyl][1,1'-biphenyl]-2-carboxylic acid;
4'-[[5-butyl-3-(1,1-difluorobutyl)-1H-1,2,4-triazol-1-yl]methyl][1,1'-biphenyl]-2-carboxylic acid;
4'-[[3-butyl-5-(1,1-difluorobutyl)-1H-1,2,4-triazol-1-yl]methyl][1,1'-biphenyl]-2-carboxylic acid;
4'-[[5-butyl-3-(1,1-difluoropentyl)-1H-1,2,4-triazol-1-yl]methyl][1,1,'-biphenyl]-2-carboxylic acid;
4'-[(3,5-dipropyl-1H-1,2,4-triazol-1-yl)methyl][1,1'-biphenyl]-2-carboxylic acid;
4'-[(3,5-isopropyl-1H-1,2,4-triazol-1-yl)methyl][1,1'-biphenyl]-2-carboxylic acid;
4'-[(5-propyl-3-butyl-1H-1,2,4-triazol-1-yl)methyl][1,1'-bipheny]-2-carboxylic acid;
4'-[[5-propyl-3-(1,1-difluoroethyl)-1H-1,2,4-triazol-1-yl]methyl][1,1'-biphenyl]-2-carboxylic acid;
4'-[[5-propyl-3-(1,1-difluoropropyl)-1H-1,2,4-triazol-1-yl]methyl][1,1'-biphenyl]-2-carboxylic acid;
4'-[[5-propyl-3-(1,1-difluorobutyl)-1H-1,2,4-triazol-1-yl]methyl][1,1'-biphenyl]-2-carboxylic acid;
4'-[[5-propyl-3-(1,1-difluoropentyl)-1H-1,2,4-triazol-1-yl]methyl][1,1'-biphenyl]-2-carboxylic acid;
4'-[[5-ethyl-3-(1,1-difluorobutyl)-1H-1,2,4-triazol-1-yl]methyl][1,1'-biphenyl]-2-carboxylic acid;
4'-[(3,5-disecbutyl-1H-1,2,4-triazol-1-yl)methyl][1,1'-biphenyl]-2-carboxylic acid;
4'-[[5-secbutyl-3-(1,1-difluorobutyl)-1H-1,2,4-triazol-1-yl]methyl][1,1'-biphenyl]-2-carboxylic acid;
4'-[(3,5-diisobutyl-1H-1,2,4-triazol-1-yl)methyl][1,1'-biphenyl]-2-carboxylic acid;
4'-[[5-isobutyl-3-(1,1-difluorobutyl)-1H-1,2,4-triazol-1-yl]methyl][1,1'-biphenyl]-2-carboxylic acid;
4'-[(3,5-ditertbutyl-1H-1,2,4-triazol-1-yl)methyl][1,1'-biphenyl]-2-carboxylic acid;
4'-[[5-tertbutyl-3-(1,1-difluorobutyl)-1H-1,2,4-triazol-1-yl]methyl][1,1'-biphenyl]-2-carboxylic acid;
4'-[(3,5-dipentyl-1H-1,2,4-triazol-1-yl)methyl][1,1'-biphenyl]-2-carboxylic acid;
4'-[[5-pentyl-3-(1,1-difluorobutyl)-1H-1,2,4-triazol-1-yl]methyl][1,1'-biphenyl]-2-carboxylic acid;
4'-[(3,5-diisopentyl-1H-1,2,4-triazol-1-yl)methyl][1,1'-biphenyl]-2-carboxylic acid;
4'-[[5-isopentyl-3-(1,1-difluorobutyl)-1H-1,2,4-triazol-1-yl]methyl][1,1'-biphenyl]-2-carboxylic acid;
4'-[[5-(1-butenyl)-3-(1,1-difluorobutyl)-1H-1,2,4-triazol-1-yl]methyl][1,1'-biphenyl]-2-carboxylic acid;
4'-[[5-(2-butenyl)-3-(1,1-difluorobutyl)-1H-1,2,4-triazol-1-yl]methyl][1,1'-biphenyl]-2-carboxylic acid;
4'-[[5-(3-butenyl)-3-(1,1-difluorobutyl)-1H-1,2,4-triazol-1-yl]methyl][1,1'-biphenyl]-2-carboxylic acid;
4'-[[5-(1-butynyl)-3-(1,1-difluorobutyl)-1H-1,2,4-triazol-1-yl]methyl][1,1'-biphenyl]-2-carboxylic acid;
4'-[[5-(2-butynyl)-3-(1,1-difluorobutyl)-1H-1,2,4-triazol-1-yl]methyl][1,1'-biphenyl]-2-carboxylic acid;
4'-[[5-(3-butynyl)-3-(1,1-difluorobutyl)-1H-1,2,4-triazol-1-yl]methyl][1,1'-biphenyl]-2-carboxylic acid;
4'-[(3,5-diethoxy-1H-1,2,4-triazol-1-yl)methyl][1,1'-biphenyl]-2-carboxylic acid;
4'-[(3,5-dipropoxy-1H-1,2,4-triazol-1-yl)methyl][1,1'-biphenyl]-2-carboxylic acid;
4'-[(3,5-diisopropoxy-1H-1,2,4-triazol-1-yl)methyl][1,1'-biphenyl]-2-carboxylic acid;
4'-[(3,5-dibutoxy-1H-1,2,4-triazol-1-yl)methyl][1,1'-biphenyl]-2-carboxylic acid;
4'-[[3,5-di(1-butenyl)-1H-1,2,4-triazol-1-yl]methyl][1,1'-biphenyl]-2-carboxylic acid;
4'-[[3,5-di(2-butenyl)-1H-1,2,4-triazol-1-yl]methyl][1,1'-biphenyl]-2-carboxylic acid;
4'-[[3,5-di(3-butenyl)-1H-1,2,4-triazol-1-yl]methyl][1,1'-biphenyl]-2-carboxylic acid;
4'-[[3,5-di(1-butynyl)-1H-1,2,4-triazol-1-yl]methyl][1,1'-biphenyl]-2-carboxylic acid;
4'-[[3,5-di(2-butynyl)-1H-1,2,4-triazol-1-yl]methyl][1,1'-biphenyl]-2-carboxylic acid;
4'-[[3,5-di(3-butynyl)-1H-1,2,4-triazol-1-yl]methyl][1,1'-biphenyl]-2-carboxylic acid.

4. A compound of Claim 1 selected from compounds, and their pharmaceutically-acceptable salts, of the group of compounds consisting of
5-[4'-[(5-butyl-3-propyl-1H-1,2,4-triazol-1-yl)methyl][1,1'-biphenyl]-2-yl]-1H-tetrazole;
5-[4'-[(3,5-dibutyl-1H-1,2,4-triazol-1-yl)methyl][1,1'-biphenyl]-2-yl]-1H-tetrazole;
5-[4'-[(5-butyl-3-isopentyl-1H-1,2,4-triazol-1-yl)methyl][1,1'-biphenyl]-2-yl]-1H-tetrazole;
5-[4'-[[5-butyl-3-(1,1 dimethoxybutyl)-1H-1,2,4-triazol-1-yl]methyl][1,1'-biphenyl]-2-yl]-1H-tetrazole;
5-[4'-[[5-butyl-3-(1-oxobutyl)-1H-1,2,4-triazol-1-yl]methyl][1,1'-biphenyl]-2-yl]-1H-tetrazole;
5-[4'-[[5-butyl-3-(1,1-difluoroethyl)-1H-1,2,4-triazol-1-yl]methyl][1,1'-biphenyl]-2-yl]-1H-tetrazole;
5-[4'-[[5-butyl-3-(1,1-difluoropropyl)-1H-1,2,4-triazol-1-yl]methyl][1,1'-biphenyl]-2-yl]-1H-tetrazole;
5-[4'-[[5-butyl-3-(1,1-difluorobutyl)-1H-1,2,4-triazol-1-yl]methyl][1,1'-biphenyl]-2-yl]-1H-tetrazole;
5-[4'-[[3-butyl-5-(1,1-difluorobutyl)-1H-1,2,4-triazol-1-yl]methyl][1,1'-biphenyl]-2-yl]-1H-tetrazole;
5-[4'-[[5-butyl-3-(1,1-difluoropentyl)-1H-1,2,4-triazol-1-yl]methyl][1,1'-biphenyl]-2-yl]-1H-tetrazole;
5-[4'-[(3,5-dipropyl-1H-1,2,4-triazol-1-yl)methyl][1,1'-biphenyl]-2-yl]-1H-tetrazole;
5-[4'-[(3-butyl-5-propyl-1H-1,2,4-triazol-1-yl)methyl]-[1,1'-biphenyl]-2-yl]-1H-tetrazole;
5-[4'-[(5-butyl-3-isopentyl-1H-1,2,4-triazol-1-yl]methyl)-[1,1'-biphenyl]-2-yl]-1H-tetrazole;
5-[4'-[(3-phenyl-5-butyl-1H-1,2,4-triazol-1-yl)methyl][1,1'-biphenyl)-2-yl]-1H-tetrazole;
5-[4'[(5-butyl-3-phenylethyl-1H-1,2,4-triazol-1-yl)methyl][1,1'-biphenyl-2-yl]-1H-tetrazole;
5-[4'-[(5-butyl-3-ethyl-1H-1,2,4-triazol-1-yl)methyl][1,1'-biphenyl]-2-yl]-1H-tetrazole;
5-[4'-[(3-butyl-5-ethyl-1H-1,2,4-triazol-1-yl)methyl][1,1'-biphenyl]-2-yl]-1H-tetrazole; glutamine, 1,1-dimethylethyl ester;
5-[4'-[[5-butyl-3-(2-cyclohexylethyl)-1H-1,2,4-triazol-1-yl]methyl][1,1'-biphenyl]-2-yl]-1H-tetrazole;
5-[4'-[[5-butyl-3-(phenylmethyl)-1H-1,2,4-triazol-1-yl]methyl][1,1'-biphenyl]-2-yl]-1H-tetrazole;
5-[4'-[(5-butyl-3-cyclohexyl-1H-1,2,4-triazol-1-yl)methyl][1,1'-biphenyl]-2-yl]-1H-tetrazole;
5-[4'-[(3-cyclohexylmethyl-5-butyl-1H-1,2,4-triazol-1-yl)methyl][1,1'-biphenyl]-2-yl]-1H-tetrazole
5-[4'-[(3-butyl-5-cyclohexyl-1H-1,2,4-triazol-1-yl)methyl]-[1,1'-biphenyl]-2-yl]-1H-tetrazole.
5-[4'-[(3,5-isopropyl-1H-1,2,4-triazol-1-yl)methyl][1,1'-biphenyl]]-2-yl]-1H-tetrazole;
5-[4'-[(5-propyl-3-isopentyl-1H-1,2,4-triazol-1-yl)methyl][1,1'-biphenyl]-2-yl]-1H-tetrazole;
5-[4'-[(5-propyl-3-(1,1-dimethoxybutyl)-1H-1,2,4-triazol-1-yl]methyl][1,1'-biphenyl]-2-yl]-1H-tetrazole;
5-[4'-[[5-propyl-3-(1-oxobutyl)-1H-1,2,4-triazol-1-yl]methyl][1,1'-biphenyl]-2-yl]-1H-tetrazole;
5-[4'-[[5-propyl-3-(1,1-difluoroethyl)-1H-1,2,4-triazol-1-yl]methyl][1,1'-biphenyl]-2-yl]-1H-tetrazole;
5-[4'-[[5-propyl-3-(1,1-difluoropropyl)-1H-1,2,4-triazol-1-yl]methyl][1,1'-biphenyl]-2-yl]-1H-tetrazole;
5-[4'-[[5-propyl-3-(1,1-difluorobutyl)-1H-1,2,4-triazol-1-yl]methyl][1,1'-biphenyl]-2-yl]-1H-tetrazole;
5-[4'-[[5-propyl-3-(1,1-difluoropentyl)-1H-1,2,4-triazol-1-yl]methyl][1,1'-biphenyl]-2-yl]-1H-tetrazole;
5-[4'-[[5-ethyl-3-(1,1-difluorobutyl)-1H-1,2,4-triazol-1-yl]methyl][1,1'-biphenyl]-2-yl]-1H-tetrazole;
5-[4'-[(5-secbutyl-3-propyl-1H-1,2,4-triazol-1-yl)methyl](1,1'-biphenyl]-2-yl]-1H-tetrazole;
5-[4'-[(5-secbutyl-3-butyl-1H-1,2,4-triazol-1-yl)methyl][1,1'-biphenyl]-2-yl]-1H-tetrazole;
5-[4'-[(3,5-disecbutyl-1H-1,2,4-triazol-1-yl)methyl][1,1'-biphenyl]-2-yl]-1H-tetrazole;
5-[4'-[(5-secbutyl-3-isopentyl-1H-1,2,4-triazol-1-yl)methyl][1,1'-biphenyl]-2-yl]-1H-tetrazole;
5-[4'-[[5-secbutyl-3-(1,1-difluoroethyl)-1H-1,2,4-triazol-1-yl]methyl][1,1'-biphenyl]-2-yl]-1H-tetrazole;
5-[4'-[[5-secbutyl-3-(1,1-difluoropropyl)-1H-1,2,4-triazol-1-yl)methyl][1,1'-biphenyl]-2-yl]-1H-tetrazole;
5-[4'-[[5-secbutyl-3-(1,1-difluorobutyl)-1H-1,2,4-triazol-1-yl]methyl][1,1'-biphenyl]-2-yl]-1H-tetrazole;
5-[4'-[[5-secbutyl-3-(1,1-difluoropentyl)-1H-1,2,4-triazol-1-yl]methyl][1,1'-biphenyl]-2-yl]-1H-tetrazole;
5-[4'[(3,5-isopropyl-1H-1,2,4-triazol-1-yl)methyl][1,1'-biphenyl]]-2-yl]-1H-tetrazole;
5-[4'-[(5-propyl-3-isopentyl-1H-1,2,4-triazol-1-yl)methyl][1,1'-biphenyl]-2-yl]-1H-tetrazole;
5-[4'-[(5-propyl-3-(1,1-dimethoxybutyl)-1H-1,2,4-triazol-1-yl]methyl][1,1'-biphenyl]-2-yl]-1H-tetrazole;
5-[4'-[[5-propyl-3-(1-oxobutyl)-1H-1,2,4-triazol-1-yl]methyl][1,1'-biphenyl]-2-yl]-1H-tetrazole;
5-[4'-[[5-propyl-3-(1,1-difluoroethyl)-1H-1,2,4-triazol-1-yl]methyl][1,1'-biphenyl]-2-yl]-1H-tetrazole;
5-[4'-[[5-propyl-3-(1,1-difluoropropyl)-1H-1,2,4-triazol-1-yl]methyl][1,1'-biphenyl]-2-yl]-1H-tetrazole;
5-[4'-[[5-propyl-3-(1,1-difluorobutyl)-1H-1,2,4-triazol-1-yl]methyl][1,1'-biphenyl]-2-yl]-1H-tetrazole;
5-[4'-[[5-propyl-3-(1,1-difluoropentyl)-1H-1,2,4-triazol-1-yl]methyl][1,1'-biphenyl]-2-yl]-1H-tetrazole;
5-[4'-[[5-ethyl-3-(1,1-difluorobutyl)-1H-1,2,4-triazol-1-yl]methyl][1,1'-biphenyl]-2-yl]-1H-tetrazole;
5-[4'-[(5-secbutyl-3-propyl-1H-1,2,4-triazol-1-yl)methyl](1,1'-biphenyl]-2-yl]-1H-tetrazole;
5-[4'-[(5-secbutyl-3-butyl-1H-1,2,4-triazol-1-yl)methyl][1,1'-biphenyl]-2-yl]-1H-tetrazole;
5-[4'-[(3,5-disecbutyl-1H-1,2,4-triazol-1-yl)methyl][1,1'-biphenyl]-2-yl]-1H-tetrazole;
5-[4'-[(5-secbutyl-3-isopentyl-1H-1,2,4-triazol-1-yl)methyl][1,1'-biphenyl]-2-yl]-1H-tetrazole;
5-[4'-[[5-secbutyl-3-(1,1-difluoroethyl)-1H-1,2,4-triazol-1-yl]methyl][1,1'-biphenyl]-2-yl]-1H-tetrazole;
5-[4'-[[5-secbutyl-3-(1,1-difluoropropyl)-1H-1,2,4-triazol-1-yl)methyl][1,1'-biphenyl]-2-yl]-1H-tetrazole;
5-[4'-[[5-secbutyl-3-(1,1-difluorobutyl)-1H-1,2,4-triazol-1-yl]methyl][1,1'-biphenyl]-2-yl]-1H-tetrazole;
5-[4'-[[5-secbutyl-3-(1,1-difluoropentyl)-1H-1,2,4-triazol-1-yl]methyl][1,1'-biphenyl]-2-yl]-1H-tetrazole;
5-[4'-[(5-pentyl-3-butyl-1H-1,2,4-triazol-1-yl)methyl][1,1'-biphenyl]-2-yl]-1H-tetrazole;
5-[4'-[(3,5-dipentyl-1H-1,2,4-triazol-1-yl)methyl][1,1'-biphenyl]-2-yl]-1H-tetrazole;
5-[4'-[(5-pentyl-3-isopentyl-1H-1,2,4-triazol-1-yl)methyl][1,1'-biphenyl]-2-yl]-1H-tetrazole;
5-[4'-[[5-pentyl-3-(1,1-difluoroethyl)-1H-1,2,4-triazol-1-yl]methyl][1,1'-biphenyl]-2-yl]-1H-tetrazole;
5-[4'-[[5-pentyl-3-(1,1-difluoropropyl)-1H-1,2,4-triazol-1-yl]methyl][1,1'-biphenyl]-2-yl]-1H-tetrazole;
5-[4'-[[5-pentyl-3-(1,1-difluorobutyl)-1H-1,2,4-triazol-1-yl]methyl][1,1'-biphenyl]-2-yl]-1H-tetrazole;
5-[4'-[[5-pentyl-3-(1,1-difluoropentyl)-1H-1,2,4-triazol-1-yl]methyl][1,1'-biphenyl]-2-yl]-1H-tetrazole;
5-[4'-[(5-isopentyl-3-ethyl-1H-1,2,4-triazol-1-yl)methyl][1,1'-biphenyl]-2-yl]-1H-tetrazole;
5-[4'-[(5-isopentyl-3-propyl-1h-1,2,4-triazol-1-yl)methyl][1,1'-biphenyl]-2-yl]-1H-tetrazole;
5-[4'-[(5-isopentyl-3-butyl-1H-1,2,4-triazol-1-yl)methyl][1,1'-biphenyl]-2-yl]-1H-tetrazole;
5-[4'-[(3,5-diisopentyl-1H-1,2,4-triazol-1-yl)methyl][1,1'-biphenyl]-2-yl]-1H-tetrazole;
5-[4'-[[5-isopentyl-3-(1,1-difluoroethyl)-1H-1,2,4-triazol-1-yl]methyl][1,1'-biphenyl]-2-yl]-1H-tetrazole;
5-[4'-[[5-isopentyl-3-(1,1-difluoropropyl)-1H-1,2,4-triazol-1-yl]methyl][1,1'-bipheny]-2-yl]-1H-tetrazole;
5-[4'-[[5-isopentyl-3-(1,1-difluorobutyl)-1H-1,2,4-triazol-1-yl]methyl][1,1'-biphenyl]-2-yl]-1H-tetrazole;
5-[4'-[[5-isopentyl-3-(1,1-difluoropentyl)-1H-1,2,4-triazol-1-yl]methyl][1,1'-biphenyl-2-yl]-1H-tetrazole;
5-[4'-[[5-(1-butenyl)-3-propyl-1H-1,2,4-triazol-1-yl]methyl][1,1'-biphenyl]-2-yl]-1H-tetrazole;
5-[4'-[[5-(1-butenyl)-3-butyl-1H-1,2,4-triazol-1-yl]methyl][1,1'-biphenyl]-2-yl]-1H-tetrazole;
5-[4'-[[5-(1-butenyl)-3-isopentyl-1H-1,2,4-triazol-1-yl]methyl][1,1'-biphenyl]-2-yl]-1H-tetrazole;
5-[4'-[[5-(1-butenyl)-3-(1,1-dimethoxybutyl)-1H-1,2,4-triazol-1-yl]methyl][1,1'-biphenyl]-2-yl]-1H-tetrazole;
5-[4'-[[5-(1-butenyl)-3-(1,1-difluorobutyl)-1H-1,2,4-triazol-1-yl]methyl][1,1'-biphenyl]-2-yl]-1H-tetrazole;
5-[4'-[[5-(2-butenyl)-3-(1,1-difluorobutyl)-1H-1,2,4-triazol-1-yl]methyl][1,1'-biphenyl]-2-yl]-1H-tetrazole;
5-[4'-[[5-(3-butenyl)-3-(1,1-difluorobutyl)-1H-1,2,4-triazol-1-yl]methyl][1,1'-biphenyl]-2-yl]-1H-tetrazole;
5-[4'-[[5-(1-butynyl)-3-(1,1-difluorobutyl)-1H-1,2,4-triazol-1-yl]methyl][1,1'-biphenyl]-2-yl]-1H-tetrazole;
5-[4'-[[5-(2-butynyl)-3-(1,1-difluorobutyl)-1H-1,2,4-triazol-1-yl]methyl][1,1'-biphenyl]-2-yl]-1H-tetrazole;
5-[4'-[[5-(3-butynyl)-3-(1,1-difluorobutyl)-1H-1,2,4-triazol-1-yl]methyl][1,1'-biphenyl]-2-yl]-1H-tetrazole;
5-[4'-[[3,5-di(1-butenyl)-1H-1,2,4-triazol-1-yl]methyl][1,1'-biphenyl]-2-yl-]-1H-tetrazole;
5-[4'-[[3,5-di(2-butenyl)-1H-1,2,4-triazol-1-yl]methyl][1,1'-biphenyl]-2-yl]-1H-tetrazole;
5-[4'-[[3,5-di(3-butenyl)-1H-1,2,4-triazol-1-yl]methyl][1,1'-biphenyl]-2-yl]-1H-tetrazole;
5-[4'-[[3,5-di(1-butynyl)-1H-1,2,4-triazol-1-yl]methyl][1,1'-biphenyl]-2-yl]-1H-tetrazole;
5-[4'-[[3,5-di(2-butynyl)-1H-1,2,4-triazol-1-yl]methyl][1,1'-biphenyl]-2-yl]-1H-tetrazole; and
5-[4'-[[3,5-di(3-butynyl)-1H-1,2,4-triazol-1-yl]methyl][1,1'-biphenyl]-2-yl]-1H-tetrazole.

5. Compound of claim 1 selected from compounds, and their pharmaceutically-acceptable salts, of the group of compounds consisting of
4'-[(3,5-dibutyl-1H-1,2,4-triazol-1-yl)methyl][1,1'-biphenyl]-2-carboxylic acid;
4'-[(3-ethyl-5-butyl-1H,1,2,4-triazol-1-yl)methyl][1,1'-biphenyl]-2-carboxylic acid;
4'-[(3-butyl-5-ethyl-1H-1,2,4-triazol-1-yl)methyl][1,1'-biphenyl]-2-carboxylic acid;
4'-[(5-butyl-1H-1,2,4-triazol-1-yl)methyl][1,1'-biphenyl]-2-carboxylic acid;
4'-[(3-butyl-1H-1,2,4-triazol-1-yl)methyl][1,1'-biphenyl]-2-carboxylic acid.

6. A process for preparing a pharmaceutical composition characterized by admixing a therapeutically-effective amount of an angiotensin II antagonist compound and a pharmaceutically-acceptable carrier or diluent, said antagonist compound selected from a family of compounds according to any of Claims 1-5 or the amide of compounds wherein R³ = COOH, or their salts.

7. Use of a compound according to any of Claims 1-5 or the amide of compounds wherein R³ = COOH, or their salts for preparing a medicament for treating a circulatory disorder.

8. Use according to Claim 7 wherein said disorder is a cardiovascular disorder.

9. Use according to Claim 8 wherein said cardiovascular disorder is hypertension.

10. Use according to Claim 8 wherein said cardiovascular disorder is congestive heart failure.

11. Use of a compound according to any of Claims 1-5 or the amide of compounds wherein R³ = COOH, or their salts for preparing a medicament for treating glaucoma.

12. A Compound which is methyl-4'-[(3,5-dibutyl-1H-1,2,4-triazol-1-yl)methyl][1,1'-biphenyl-2-carboxylate or 4'-[(3,5-dibutyl-1H-1,2,4-triazol-1-yl)methyl][1,1'-biphenyl]-2-carbxylic acid, hydrazide.

## Patentansprüche (Patentansprüche für folgende(n) Vertragsstaat(en): AT, BE, CH, LI, DE, DK, FR, GB, IT, LU, NL, SE)

1. Verbindung der Formel I 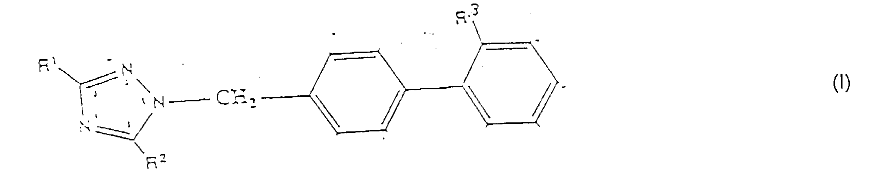 wobei R¹ und R² unabhängig voneinander ausgewählt werden aus Wasserstoff, C₁-C₂₀ Alkyl, Phenyl, durch C₁-C₂₀ Alkyl substituiertes Phenyl, C₃-C₁₀ Cycloalkyl, an C₁-C₂₀ Alkyl gebundenes oder durch dieses substituiertes C₃-C₁₀ Cycloalkyl, C₁-C₁₀ Alkoxy, durch Phenyl substituiertes C₁-C₁₀-Difluoroalkyl, C₂-C₂₀ Alkenyl, C₂-C₂₀ Alkynyl, C₁-C₂₀ Alkylcarbonyl; difluorosubstituiertes C₁-C₂₀ Alkyl;
C₁-C₁₀-Hydroxyalkyl, durch eine oder mehrere Alkoxygruppen mit Alkylanteilen von 1 bis 10 Kohlenstoffatomen substituiertes C₁-C₂₀ Alkyl; wobei R³ eine Säurekomponente ist, ausgewählt aus CO₂H, CONHNHSO₂CF₃, 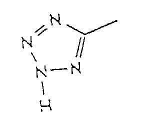 und den Salzen der Säurekomponenten; oder einem Tautomer hiervon oder einem pharmazeutisch akzeptablen Salz hiervon.

2. Verbindung von Anspruch 1, wobei R³ ausgewählt ist aus COOH oder Tetrazolyl oder einem Tautomer oder einem pharmazeutisch akzeptablem Salz hiervon.

3. Verbindung von Anspruch 2, ausgewählt aus Verbindungen und deren pharmazeutisch akzeptablen Salzen der Gruppe von Verbindungen, bestehend aus
4'-[(3,5-Dibutyl-1H-1,2,4-Triazol-1-yl)methyl][1,1'- Biphenyl]-2-Karbonsäure, 2-[(Trifluoromethyl)sulfonyl]hydrazid;
4'-[(5-Butyl-3-Propyl-1H-1,2,4-Triazol-1-yl)methyl][1,1'-Biphenyl]-2-Karbonsäure;
4'-[(3,5-Dibutyl-1H-1,2,4-Triazol-1-yl)methyl][1,1'-Biphenyl]-2-Karbonsäure;
4'-[(5-Butyl-3-Isopentyl-1H-1,2,4-Triazol-1-yl)methyl][1,1' - Biphenyl]-2-Karbonsäure;
4'-[[5-Butyl-3-(1,1-Dimethoxybutyl)-1H-1,2,4-Triazol-1-yl]methyl][1,1'-Biphenyl]-2-Karbonsäure;
4'-[[5-Butyl-3-(1-Oxobutyl)-1H-1,2,4-Triazol-1-yl]methyl][1,1'-Biphenyl)-2-Karbonsäure;
4'-[[5-Butyl-3-(1-Oxopentyl)-1H-1,2,4-Triazol-1-yl]methyl][1,1'-Biphenyl]-2-Karbonsäure;
4'-[[5-Butyl-3-(1,1-Difluoroethyl)-1H-1,2,4-Triazol-1-yl]methyl][1,1'-Biphenyl]-2-Karbonsäure;
4'-[[5-Butyl-3-(1,1-Difluoropropyl)-1H-1,2,4-Triazol-1-yl]methyl] [1,1'-Biphenyl]-2-Karbonsäure;
4'-[[5-Butyl-3-(1,1-Difluorobutyl)-1H-1,2,4-Triazol-1-yl]methyl] [1,1'-Biphenyl]-2-Karbonsäure;
4'-[[3-Butyl-5-(1,1-Difluorobutyl)-1H-1,2,4-Triazol-1-yl]methyl][1,1'-Biphenyl]-2-Karbonsäure;
4'-[[5-Butyl-3-(1,1-Difluoropentyl)-1H-1,2,4-Triazol-1-yl]methyl][1,1'-Biphenyl]-2-Karbonsäure;
4'-[(3,5-Dipropyl-1H-1,2,4-Triazol-1-yl)methyl] [1,1' - Biphenyl]-2-Karbonsäure;
4'-[(3'5-Isopropyl-1H-1,2,4-Triazol-1-yl)methyl][1,1' - Biphenyl]-2-Karbonsäure;
4'-[(5-Propyl-3-Butyl-1H-1,2,4-Triazol-1-yl)methyl] [1,1'- Biphenyl]-2-Karbonsäure;
4'-[[5-Propyl-3-(1,1-Difluoroethyl)-1H-1,2,4-Triazol-1-yl]methyl] [1,1'-Biphenyl]-2-Karbonsäure;
4'-[[5-Propyl-3-(1,1-Difluoropropyl)-1H-1,2,4-Triazol-1-yl]methyl] [1,1'-Biphenyl]-2-Karbonsäure;
4'-[[5-Propyl-3-(1,1-Difluorobutyl)-1H-1,2,4-Triazol-1-yl]methyl] [1,1'-Biphenyl]-2-Karbonsäure;
4'-[[5-Propyl-3-(1,1-Difluoropentyl)-1H-1,2,4-Triazol-1 -yl]methyl][1,1' - Biphenyl]-2-Karbonsäure;
4'-[[5-Ethyl-3-(1,1-Difluorobutyl)-1H-1,2,4-Triazol-1-yl]methyl] [1,1' - Biphenyl]-2-Karbonsäure;
4'-[(3,5-Disecbutyl-1H-1,2,4-Triazol-1-yl)methyl][1,1'- Biphenyl]-2-Karbonsäure;
4'-[[5-Secbutyl-3-(1,1-Difluorobutyl)-1H-1,2,4-Triazol-1-yl]methyl][1,1' - Biphenyl]-2-Karbonsäure;
4'-[(3,5-Diisobutyl-1H-1,2,4-Triazol-1-yl)methyl] [1,1' - Biphenyl]-2-Karbonsäure;
4'-[[5-Isobutyl-3-(1,1-Difluorobutyl)-1H-1,2,4-Triazol-1-yl]methyl][1,1' - Biphenyl]-2-Karbonsäure;
4'-[(3,5-Ditertbutyl-1H-1,2,4-Triazol-1-yl)methyl] [1,1' - Biphenyl]-2-Karbonsäure;
4'-[[5-Tertbutyl-3-(1,1-Difluorobutyl)-1H-1,2,4-Triazol-1-yl]methyl] [1,1' - Biphenyl]-2-Karbonsäure;
4'-[(3'5-Dipentyl-1H-1,2,4-Triazol-1-yl)methyl][1,1' - Biphenyl]-2-Karbonsäure;
4'-[[5-Pentyl-3-(1,1-Difluorobutyl)-1H-1,2,4-Triazol-1-yl]methyl] [1,1' - Biphenyl]-2-Karbonsäure;
4'-[(3,5-Diisopentyl-1H-1,2,4-Triazol-1-yl)methyl] [1,1' - Biphenyl]-2-Karbonsäure;
4'-[[5-Isopentyl-3-(1,1-Difluorobutyl)-1H-1,2,4-Triazol-1-yl]methyl] [1,1' - Biphenyl]-2-Karbonsäure;
4'-[[5-(1-Butenyl)-3-(1,1-Difluorobutyl)-1H-1,2,4-Triazol-1-yl]methyl] [1,1' - Biphenyl]-2-Karbonsäure;
4'-[[5-(2-Butenyl)-3-(1,1-Difluorobutyl)-1H-1,2,4-Triazol-1-yl]methyl][1-1' - Biphenyl]-2-Karbonsäure;
4'-[[5-(3-Butenyl)-3-(1,1-Difluorobutyl)-1H-1,2,4-Triazol-1-yl]methyl][1,1' - Biphenyl]-2-Karbonsäure;
4'-[[5-(1-Butynyl)-3-(1,1-Difluorobutyl)-1H-1,2,4-Triazol-1-yl]methyl] [1,1' - Biphenyl]-2-Karbonsäure;
4'-[[5-(2-Butynyl)-3-(1,1-Difluorobutyl)-1H-1,2,4-Triazol-1-yl]methyl][1,1' - Biphenyl]-2-Karbonsäure;
4'-[[5-(3-Butynyl)-3-(1,1-Difluorobutyl)-1H-1,2,4-Triazol-1-yl]methyl][1,1' - Biphenyl]-2-Karbonsäure;
4'-[(3,5-Diethoxy-1H-1,2,4-Triazol-1-yl)methyl] [1,1' - Biphenyl]-2-Karbonsäure;
4'-[(3,5-Dipropoxy-1H-1,2,4-Triazol-1-yl)methyl][1,1' - Biphenyl]-2-Karbonsäure;
4'-[(3,5-Diisopropoxy-1H-1,2,4-Triazol-1-yl)methyl] [1,1' - Biphenyl]-2-Karbonsäure;
4'-[(3,5-Dibutoxy-1H-1,2,4-Triazol-1-yl)methyl] [1,1' - Biphenyl]-2-Karbonsäure;
4'-[[3,5-di (1-Butenyl)-1H-1,2,4-Triazol-1-yl]methyl][1,1' - Biphenyl]-2-Karbonsäure;
4'-[[3,5-di (2-Butenyl)-1H-1,2,4-Triazol-1-yl]methyl] [1,1' - Biphenyl]-2-Karbonsäure;
4'-[[3,5-di (3-Butenyl)-1H-1,2,4-Triazol-1-yl]methyl] [1,1' - Biphenyl]-2-Karbonsäure;
4'-[[3,5-di (1-Butynyl)-1H-1,2,4-Triazol-1-yl]methyl][1,1' - Biphenyl]-2-Karbonsäure;
4'-[[3,5-di (2-Butynyl)-1H-1,2,4-Triazol-1-yl]methyl] [1,1' - Biphenyl]-2-Karbonsäure;
4'-[[3,5-di (3-Butynyl)-1H-1,2,4-Triazol-1-yl]methyl][1,1' - Biphenyl]-2-Karbonsäure;

4. Verbindung von Anspruch 1, ausgewählt aus Verbindungen und deren pharmazeutisch akzeptablen Salzen der Gruppe von Verbindungen, bestehend aus
5-[4'[(5-Butyl-3-Propyl-1H-1,2,4-Triazol-1-yl)methyl][1,1' - Biphenyl]-2-yl]-1H-Tetrazol;
5-[4'-[(3,5-Dibutyl-1H-1,2,4-Triazol-1-yl)methyl][1,1' - Biphenyl]-2-yl]-1H-Tetrazol;
5-[4'-[(5-Butyl-3-Isopentyl-1H-1,2,4-Triazol-1-yl)methyl][1,1' - Biphenyl]-2-yl]-1H-Tetrazol;
5-[4'-[[5-Butyl-3-(1,1-Dimethoxybutyl)-1H-1,2,4-Triazol-1-yl]methyl][1,1' - Biphenyl]-2-yl]-1H-Tetrazol;
5-[4'-[[5-Butyl-3-(1-Oxobutyl)-1H-1,2,4-Triazol-1-yl]methyl][1,1' - Biphenyl]-2-yl]-1H-Tetrazol;
5-[4'-[[5-Butyl-3-(1,1-Difluoroethyl)-1H-1,2,4-Triazol-1-yl]methyl][1,1' - Biphenyl]-2-yl]-1H-Tetrazol;
5-[4'-[[5-Butyl-3-(1,1-Difluoropropyl)-1H-1,2,4-Triazol-1-yl]methyl][1,1' - Biphenyl]-2-yl]-1H-Tetrazol;
5-[4'-[[5-Butyl-3-(1,1-Difluorobutyl)-1H-1,2,4-Triazol-1-yl]methyl][1,1' - Biphenyl]-2-yl]-1H-Tetrazol;
5-[4'-[[3-Butyl-5-(1,1-Difluorobutyl)-1H-1,2,4-Triazol-1-yl]methyl][1,1' - Biphenyl]-2-yl]-1H-Tetrazol;
5-[4'-[[5-Butyl-3-(1,1-Difluoropentyl)-1H-1,2,4-Triazol-1-yl]methyl][1,1' - Biphenyl]-2-yl]-1H-Tetrazol;
5-[4'-[(3,5-Dipropyl-1H-1,2,4-Triazol-1-yl)methyl][1,1' - Biphenyl]-2-yl]-1H-Tetrazol;
5-[4'-[(3-Butyl-5-Propyl-1H-1,2,4-Triazol-1-yl)methyl]-(1,1'-Biphenyl]-2-yl]-1H-Tetrazol;
5-[4'-[(5-Butyl-3-Isopentyl-1H-1,2,4-Triazol-1-yl)methyl]-(1,1'-Biphenyl]-2-yl]-1H-Tetrazol;
5-[4'-[(3-Phenyl-5-Butyl-1H-1,2,4-Triazol-1yl)methyl]-(1,1'-Biphenyl]-2-yl]-1H-Tetrazol;
5-[4'-[(5-Butyl-3-Phenylethyl-1H-1,2,4-Triazol-1-yl)methyl]-(1,1'-Biphenyl]-2-yl]-1H-Tetrazol;
5-[4'-[(5-Butyl-3-Ethyl-1H-1,2,4-Triazol-1-yl)methyl]-(1,1'-Biphenyl]-2-yl]-1H-Tetrazol;
5-[4'-[(3-Butyl-5-Ethyl-1H-1,2,4-Triazol-1-yl)methyl]-(1,1'-Biphenyl]-2-yl]-1H-Tetrazol;
Glutamin, 1,1-Dimethylethylester;
5-[4'-[[5-Butyl-3-(2-Cyklohexylethyl)-1H-1,2,4-Triazol-1-yl)methyl][1,1'-Biphenyl]-2-yl]-1H-Tetrazol;
5-[4'-[[5-Butyl-3-(Phenylmethyl)-1H-1,2,4-Triazol-1-yl)methyl][1,1'-Biphenyl]-2-yl]-1H-Tetrazol;
5-[4'-[(5-Butyl-3-Cydohexyl-1H-1,2,4-Triazol-1-yl)methyl][1,1'-Biphenyl]-2-yl]-1H-Tetrazol;
5-[4'-[(3-Cyclohexylmethyl-5-Butyl-1H-1,2,4-Triazol-1-yl)methyl][1,1'-Biphenyl]-2-yl]-1H-Tetrazol;
5-[4'-[(3-Butyl-5-Cyclohexyl-1H-1,2,4-Triazol-1-yl)methyl][1,1'-Biphenyl]-2-yl]-1H-Tetrazol;
5-[4'-[(3,5-Isopropyl-1H-1,2,4-Triazol-1-yl)methyl][1,1'-Biphenyl]-2-yl]-1H-Tetrazol;
5-[4'-[(5-Propyl-3-Isopentyl-1H-1,2,4-Triazol-1-yl)methyl][1,1'-Biphenyl]-2-yl]-1H-Tetrazol;
5-[4'-[[5-Propyl-3-(1,1'-Dimethoxybutyl)-1H-1,2,4-Triazol-1-yl]methyl][1,1'-Biphenyl]-2-yl]-1H-Tetrazol;
5-[4'-[[5-Propyl-3-(1-Oxobutyl)-1H-1,2,4-Triazol-1-yl]methyl][1,1'-Biphenyl]-2-yl]-1H-Tetrazol;
5-[4'-[[5-Propyl-3-(1,1-Difluoroethyl)-1H-1,2,4-Triazol-1-yl]methyl][1,1'-Biphenyl]-2-yl]-1H-Tetrazol;
5-[4'-[[5-Propyl-3-(1,1-Difluoropropyl)-1H-1,2,4-Triazol-1-yl]methyl][1,1'-Biphenyl]-2-yl]-1H-Tetrazol;
5-[4'-[[5-Propyl-3-(1,1-Difluorobutyl)-1H-1,2,4-Triazol-1-yl]methyl][1,1'-Biphenyl]-2-yl]-1H-Tetrazol;
5-[4'-[[5-Propyl-3-(1,1-Difluoropentyl)-1H-1,2,4-Triazol-1-yl]methyl][1,1'-Biphenyl]-2-yl]-1H-Tetrazol;
5-[4'-[[5-Ethyl-3-(1,1-Difluorobutyl)-1H-1,2,4-Triazol-1-yl]methyl][1,1'-Biphenyl]-2-yl]-1H-Tetrazol;
5-[4'-[(5-Secbutyl-3-Propyl-1H-1,2,4-Triazol-1-yl)methyl] [1,1'-Biphenyl]-2-yl]-1H-Tetrazol;
5-[4'-[(5-Secbutyl-3-Butyl-1H-1,2,4-Triazol-1-yl)methyl][1,1'-Biphenyl]-2-yl]-1H-Tetrazol;
5-[4'-[(3,5-Disecbutyl-1H-1,2,4-Triazol-1-yl)methyl][1,1'-Biphenyl]-2-yl]-1H-Tetrazol;
5-[4'-[(5-Secbutyl-3-Isopentyl-1H-1,2,4-Triazol-1-yl)methyl][1,1'-Biphenyl]-2-yl]-1H-Tetrazol;
5-[4'-[[5-Secbutyl-3-(1,1-Difluoroethyl)-1H-1,2,4-Triazol-1-yl]methyl][1,1'-Biphenyl]-2-yl]-1H-Tetrazol;
5-[4'-[[5-Secbutyl-3-(1,1-Difluoropropyl)-1H-1,2,4-Triazol-1-yl]methyl][1,1'-Biphenyl]-2-yl]-1H-Tetrazol;
5-[4'-[[5-Secbutyl-3-(1,1-Difluorobutyl)-1H-1,2,4-Triazol-1-yl]methyl][1,1'-Biphenyl]-2-yl]-1H-Tetrazol;
5-[4'-[[5-Secbutyl-3-(1,1-Difluoropentyl)-1H-1,2,4-Triazol-1-yl]methyl][1,1'-Biphenyl]-2-yl]-1H-Tetrazol;
5-[4'-[(5-Isobutyl-3-Propyl-1H-1,2,4-Triazol-1-yl)methyl][1,1'-Biphenyl]-2-yl]-1H-Tetrazol;
5-[4'-[(5-Isobutyl-3-Butyl-1H-1,2,4-Triazol-1-yl)methyl][1,1'-Biphenyl]-2-yl]-1H-Tetrazol;
5-[4'-[3,5-Diisobutyl-1H-1,2,4-Triazol-1-yl)methyl][1,1'-Biphenyl]-2-yl]-1H-Tetrazol;
5-[4'-[(5-Isobutyl-3-Isopentyl-1H-1,2,4-Triazol-1-yl)methyl][1,1'-Biphenyl]-2-yl]-1 H-Tetrazol;
5-[4'-[[5-Isobutyl-3-(1,1-Difluoroethyl)-1H-1,2,4-Triazol-1-yl]methyl][1,1'-Biphenyl]-2-yl]-1H-Tetrazol;
5-[4'-[[5-Isobutyl-3-(1,1-Difluoropropyl)-1H-1,2,4-Triazol-1-yl]methyl][1,1'-Biphenyl]-2-yl]-1H-Tetrazol;
5-[4'-[[5-Isobutyl-3-(1,1-Difluorobutyl)-1H-1,2,4-Triazol-1-yl]methyl][1,1'-Biphenyl]-2-yl]-1H-Tetrazol;
5-[4'-[[5-Isobutyl-3-(1,1-Difluoropentyl)-1H-1,2,4-Triazol-1-y]methyl][1,1'-Biphenyl]-2-yl]-1H-Tetrazol;
5-[4'-[(5-Tertbutyl-3-Propyl-1H-1,2,4-Triazol-1-yl)methyl][1,1'-Biphenyl]-2-yl]-1H-Tetrazol;
5-[4'-[(5-Tertbutyl-3-Butyl-1H-1,2,4-Triazol-1-yl)methyl][1,1'-Biphenyl]-2-yl]-1H-Tetrazol;
5-[4'-[(3,5-Ditertbutyl-1H-1,2,4-Triazol-1-yl)methyl][1,1'-Biphenyl]-2-yl]-1H-Tetrazol;
5-[4'-[(5-Tertbutyl-3-Isopentyl-1H-1,2,4-Triazol-1-yl)methyl][1,1'-Biphenyl]-2-yl]-1H-Tetrazol;
5-[4'-[[5-Tertbutyl-3-(1,1-Difluoroethyl)-1H-1,2,4-Triazol-1-yl]methyl][1,1'-Biphenyl]-2-yl]-1H-Tetrazol;
5-[4'-[[5-Tertbutyl-3-(1,1-Difluoropropyl)-1H-1,2,4-Triazol-1-yl]methyl][1,1'-Biphenyl]-2-yl]-1H-Tetrazol;
5-[4'-[[5-Tertbutyl-3-(1,1-Difluorobutyl)-1H-1,2,4-Triazol-1-yl]methyl][1,1'-Biphenyl]-2-yl]-1H-Tetrazol;
5-[4'-[[5-Tertbutyl-3-(1,1-Difluoropentyl)-1H-1,2,4-Triazol-1-yl]methyl][1,1'-Biphenyl]-2-yl]-1H-Tetrazol;
5-[4'-[(5-Pentyl-3-Propyl-1H-1,2,4-Triazol-1-yl)methyl][1,1'-Biphenyl]-2-yl]-1H-Tetrazol;
5-[4'-[(5-Pentyl-3-Butyl-1H-1,2,4-Triazol-1-yl)methyl][1,1'-Biphenyl]-2-yl]-1H-Tetrazol;
5-[4'-[(3,5-Dipentyl-1H-1,2,4-Triazol-1-yl)methyl][1,1'-Biphenyl]-2-yl]-1H-Tetrazol;
5-[4'-[(5-Pentyl-3-Isopentyl-1H-1,2,4-Triazol-1-yl)methyl][1,1'-Biphenyl]-2-yl]-1H-Tetrazol;
5-[4'-[[5-Pentyl-3-(1,1-Difluoroethyl)-1H-1,2,4-Triazol-1-yl]methyl][1,1'-Biphenyl]-2-yl]-1H-Tetrazol;
5-[4'-[[5-Pentyl-3-(1,1-Difluoropropyl)-1H-1,2,4-Triazol-1-yl]methyl][1,1'-Biphenyl]-2-yl]-1H-Tetrazol;
5-[4'-[[5-Pentyl-3-(1,1-Difluorobutyl)-1H-1,2,4-Triazol-1-yl]methyl][1,1'-Biphenyl]-2-yl]-1H-Tetrazol;
5-[4'-[[5-Pentyl-3-(1,1-Difluoropentyl)-1H-1,2,4-Triazol-1-yl]methyl][1,1'-Biphenyl]-2-yl]-1H-Tetrazol;
5-[4'-[(5-Isopentyl-3-Ethyl-1H-1,2,4-Triazol-1-yl)methyl][1,1'-Biphenyl]-2-yl]-1H-Tetrazol;
5-[4'-[(5-Isopentyl-3-Propyl-1H-1,2,4-Triazol-1-yl)methyl][1,1'-Biphenyl]-2-yl]-1H-Tetrazol;
5-[4'-[(5-Isopentyl-3-Butyl-1H-1,2,4-Triazol-1-yl)methyl][1,1'-Biphenyl]-2-yl]-1H-Tetrazol;
5-[4'-[(3,5-Diisopentyl-1H-1,2,4-Triazol-1-yl)methyl][1,1'-Biphenyl]-2-yl]-1H-Tetrazol;
5-[4'-[[5-Isopentyl-3-(1,1-Difluoroethyl)-1H-1,2,4-Triazol-1-yl]methyl][1,1'-Biphenyl]-2-yl]-1H-Tetrazol;
5-[4'-[[5-Isopentyl-3-(1,1-Difluoropropyl)-1H-1,2,4-Triazol-1-yl]methyl][1,1'-Biphenyl]-2-yl]-1H-Tetrazol;
5-[4'-[[5-Isopentyl-3-(1,1-Difluorobutyl)-1H-1,2,4-Triazol-1-yl]methyl][1,1'-Biphenyl]-2-yl]-1H-Tetrazol;
5-[4'-[[5-Isopentyl-3-(1,1-Difluoropentyl)-1H-1,2,4-Triazol-1-yl]methyl][1,1'-Biphenyl]-2-yl]-1H-Tetrazol;
5-[4'-[[5-(1-Butenyl)-3-Propyl-1H-1,2,4-Triazol-1-yl]methyl][1,1'-Biphenyl]-2-yl]-1H-Tetrazol;
5-[4'-[[5-(1-Butenyl)-3-Butyl-1H-1,2,4-Triazol-1-yl]methyl][1,1'-Biphenyl]-2-yl]-1H-Tetrazol;
5-[4'-[[5-(1-Butenyl)-3-Isopentyl-1H-1,2,4-Triazol-1-yl)methyl][1,1'-Biphenyl]-2-yl]-1H-Tetrazol;
5-[4'-[[5-(1-Butenyl)-3-(1,1-Dimethoxybutyl)-1H-1,2,4-Triazol-1-yl]methyl][1,1'-Biphenyl]-2-yl]-1H-Tetrazol;
5-[4'-[[5-(1-Butenyl)-3-(1,1-Difluorobutyl)-1H-1,2,4-Triazol-1-yl]methyl][1,1'-Biphenyl]-2-yl]-1H-Tetrazol;
5-[4'-[[5-(2-Butenyl)-3-(1,1-Difluorobutyl)-1H-1,2,4-Triazol-1-yl]methyl][1,1'-Biphenyl]-2-yl]-1H-Tetrazol;
5-[4'-[[5-(3-Butenyl)-3-(1,1-Difluorobutyl)-1H-1,2,4-Triazol-1-yl]methyl][1,1'-Biphenyl]-2-yl]-1H-Tetrazol;
5-[4'-[[5-(1-Butynyl)-3-(1,1-Difluorobutyl)-1H-1,2,4-Triazol-1-yl]methyl][1,1'-Biphenyl]-2-yl]-1H-Tetrazol;
5-[4'-[[5-(2-Butynyl)-3-(1,1-Difluorobutyl)-1H-1,2,4-Triazol-1-yl]methyl][1,1'-Biphenyl]-2-yl]-1H-Tetrazol;
5-[4'-[[5-(3-Butynyl)-3-(1,1-Difluorobutyl)-1H-1,2,4-Triazol-1-yl]methyl][1,1'-Biphenyl]-2-yl]-1H-Tetrazol;
5-[4'-[[3,5-di(1-Butenyl)-1H-1,2,4-Triazol-1-yl]methyl][1,1'-Biphenyl]-2-yl]-1H-Tetrazol;
5-[4'-[[3,5-di(2-Butenyl)-1H-1,2,4-Triazol-1-yl]methyl][1,1'-Biphenyl]-2-yl]-1H-Tetrazol;
5-[4'-[[3,5-di(3-Butenyl)-1 H-1,2,4-Triazol-1-yl]methyl][1,1'-Biphenyl]-2-yl]-1H-Tetrazol;
5-[4'-[[3,5-di(1-Butynyl)-1H-1,2,4-Triazol-1-yl)methyl][1,1'-Biphenyl]-2-yl]-1H-Tetrazol;
5-[4'-[[3,5-di(2-Butynyl)-1H-1,2,4-Triazol-1-yl]methyl][1,1'-Biphenyl]-2-yl]-1H-Tetrazol;
5-[4'-[[3,5-di(3-Butynyl)-1H-1,2,4-Triazol-1-yl]methyl][1,1'-Biphenyl]-2-yl]-1H-Tetrazol;

5. Verbindung von Anspruch 1, ausgewählt aus Verbindungen und deren pharmazeutisch akzeptablen Salzen aus der Gruppe von Verbindungen, bestehend aus
4'-[(3,5-Dibutyl-1H-1,2,4-Triazol-1-yl)methyl][1,1'-bi-Phenyl]-2-Karbonsäure;
4'-[(3-Ethyl-5-Butyl-1H-1,2,4-Triazol-1-yl)methyl][1,1'-Biphenyl]-2-Karbonsäure;
4'-[(3-Butyl-5-Ethyl-1H-1,2,4-Triazol-1-yl)methyl][1,1'-Biphenyl]-2-Karbonsäure;
4'-[(5-Butyl-1H-1,2,4-Triazol-1-yl)methyl][1,1'-Biphenyl]-2-Karbonsäure;
4'-[(3-Butyl-1H-1,2,4-Triazol-1-yl)methyl][1,1'-Biphenyl]-2-Karbonsäure;

6. Pharmazeutische Zusammensetzung, die eine therapeutisch wirksame Menge einer Angiotensin-II-antagonistischen Verbindung und einen pharmazeutisch akzeptablen Träger oder ein solches Verdünnungsmittel umfaßt, wobei die antagonistische Verbindung ausgewählt ist aus einer Familie von Verbindungen gemäß einem der Ansprüche 1 - 5 oder dem Amid der Verbindungen, worin R³ ist COOH, oder deren Salzen.

7. Benutzung einer Verbindung gemäß einem der Ansprüche 1 - 5 oder dem Amid der Verbindungen, worin R³ ist COOH, oder deren Salzen zur Herstellung eines Medikaments zur Behandlung einer Kreislauferkrankung.

8. Benutzung gemäß Anspruch 7, wobei die Erkrankung eine kardiovaskuläre Erkrankung ist.

9. Benutzung gemäß Anspruch 8, wobei die kardiovaskuläre Erkrankung Hypertonie ist.

10. Benutzung gemäß Anspruch 8, wobei die kardiovaskuläre Erkrankung kongestive Herzinsuffizienz ist.

11. Benutzung einer Verbindung gemäß einem der Ansprüche 1 - 5 oder des Amids der Verbindungen, wobei R³ ist COOH, oder deren Salzes zur Bereitung eines Medikaments zur Behandlung von Glaukoma.

12. Verbindung, die Methyl-4'-[(3,5-Dibutyl-1H-1,2,4-Triazol-1-yl)methyl][1,1'-Biphenyl-2-Karboxylat oder 4'-[(3,5-Dibutyl-1H-1,2,4-Triazol-1-yl)methyl][1,1'-Biphenyl]-2-Karbonsäure, Hydrazid ist

## Patentansprüche (Patentansprüche für folgende(n) Vertragsstaat(en): ES)

1. Verfahren zur Herstellung einer Verbindung der Formel I 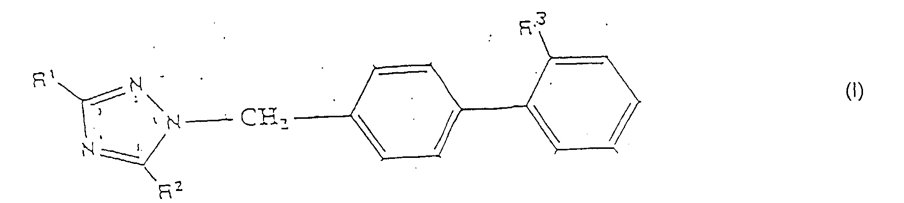 wobei R¹ und R² unabhängig voneinander ausgewählt werden aus Wasserstoff, C₁-C₂₀ Alkyl, Phenyl, durch C₁-C₂₀ Alkyl substituiertes Phenyl, C₃-C₁₀ Cycloalkyl, an C₁-C₂₀ Alkyl gebundenes oder durch dieses substituiertes C₃-C₁₀ Cycloalkyl, C₁-C₁₀ Alkoxy, durch Phenyl substituiertes C₁-C₁₀-Difluoroalkyl, C₂-C₂₀ Alkenyl, C₂-C₂₀ Alkynyl, C₁-C₂₀ Alkylcarbonyl; difluorosubstituiertes C₁-C₂₀ Alkyl; C₁-C₁₀-Hydroxyalkyl, durch eine oder mehrere Alkoxygruppen mit Alkylanteilen von 1 bis 10 Kohlenstoffatomen substituiertes C₁-C₂₀ Alkyl; wobei R³ eine Säurekomponente ist, ausgewählt aus CO₂H, CONHNHSO₂CF₃, 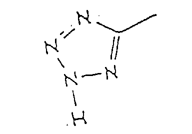 und den Salzen der Säurekomponenten; oder einem Tautomer hiervon oder einem pharmazeutisch akzeptablen Salz hiervon, dadurch gekennzeichnet, daß
A) eine Verbindung der Formel 7, 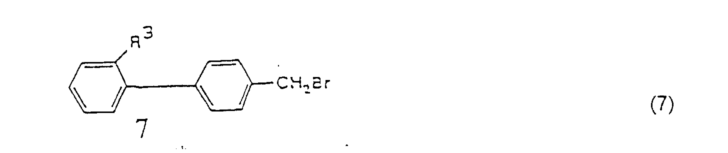 wobei R³ wie oben definiert in geschützter Form ist, mit einer Verbindung 1 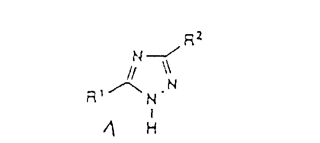 reagiert wird, wobei R¹, R² wie oben angezeigt unter basischen Bedingungen definiert sind, um die jeweiligen gekoppelten Regioisomere zu ergeben, die entweder durch Säure- oder Basenbehandlung konvertiert werden können, um die Verbindungen zu ergeben, wobei R³ ist Tetrazol oder Karbonsäure oder ein Derivativ hiervon;
B) eine Verbindung der Formel 15 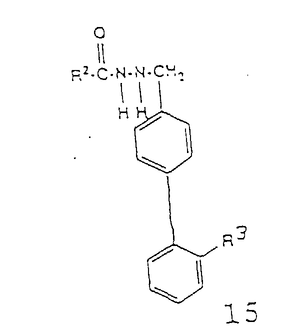 wobei R² und R³ so wie oben angezeigt definiert sind, mit einem Imidat 2 reagiert wird 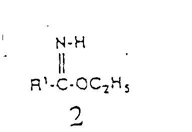 wobei R¹ wie oben definiert ist, um unter geeigneten Bedingungen nach Hitzezyklisierung die entsprechenden Endprodukte zu ergeben, oder
C) ein auf geeignete Weise substituiertes Imidazon 17 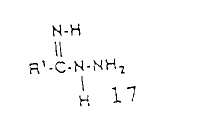 wobei R¹ wie oben definiert ist, unter geeigneten Bedingungen mit einem alkylierenden Reagens 7 reagiert wird, 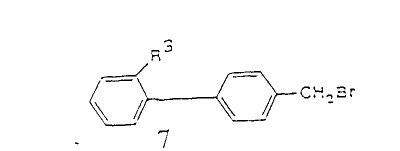 wobei R³ wie oben definiert ist,
um ein Zwischenprodukt zu ergeben, welches in der Folge unter Hitze und mit einem geeigneten Orthoester zyklisiert wird, um die gewünschten Endproduktverbindungen zu ergeben
die optional auf bekannte Art und Weise in ihre jeweiligen Amide, Ester, Salze oder deren pharmazeutisch akzeptablen Salze transformiert werden können.

2. Verfahren gemäß Anspruch 1, wobei R³ der bereiteten Verbindung ausgewählt ist aus COOH oder Tetrazolyl oder einem Tautomer oder einem pharmazeutisch akzeptablen Salz hiervon.

3. Verfahren gemäß Anspruch 2, wobei die bereitete Verbindung ausgewählt ist aus Verbindungen und deren pharmazeutisch akzeptablen Salzen der Gruppe von Verbindungen bestehend aus
4'-[(3,5-Dibutyl-1H-1,2,4-Triazol-1-yl)methyl][1,1' - Biphenyl]-2-Karbonsäure, 2-[(Trifluoromethyl)sulfonyl]hydrazid;
4'-[(5-Butyl-3-Propyl-1H-1,2,4-Triazol-1-yl)methyl][1,1' - Biphenyl]-2-Karbonsäure;
4'-[(3,5-Dibutyl-1H-1,2,4-Triazol-1-yl)methyl][1,1' - Biphenyl]-2-Karbonsäure;
4'-[(5-Butyl-3-Isopentyl-1H-1,2,4-Triazol-1-yl)methyl][1,1'- Biphenyl]-2-Karbonsäure;
4'-[[5-Butyl-3-(1,1-Dimethoxybutyl)-1H-1,2,4-Triazol-1-yl]methyl][1,1' - Biphenyl]-2-Karbonsäure;
4'-[[5-Butyl-3-(1-Oxobutyl)-1H-1,2,4-Triazol-1-yl]methyl][1,1' - Biphenyl]-2-Karbonsäure;
4'-[[5-Butyl-3-(1-Oxopentyl)-1H-1,2,4-Triazol-1-yl]methyl][1,1' - Biphenyl]-2-Karbonsäure;
4'-[[5-Butyl-3-(1,1-Difluoroethyl)-1H-1,2,4-Triazol-1-yl]methyl][1,1' - Biphenyl]-2-Karbonsäure;
4'-[[5-Butyl-3-(1,1-Difluoropropyl)-1H-1,2,4-Triazol-1-yl]methyl][1,1' - Biphenyl]-2-Karbonsäure;
4'-[[5-Butyl-3-(1,1-Difluorobutyl)-1H-1,2,4-Triazol-1-yl]methyl] [1,1' - Biphenyl]-2-Karbonsäure;
4'-[[3-Butyl-5-(1,1-Difluorobutyl)-1H-1,2,4-Triazol-1-yl]methyl][1,1' - Biphenyl]-2-Karbonsäure;
4'-[[5-Butyl-3-(1,1-Difluoropentyl)-1H-1,2,4-Triazol-1-yl]methyl] [1,1' - Biphenyl]-2-Karbonsäure;
4'-[(3,5-Dipropyl-1H-1,2,4-Triazol-1-yl)methyl] [1,1' - Biphenyl]-2-Karbonsäure;
4'-[(3,5-Isopropyl-1H-1,2,4-Triazol-1-yl)methyl][1,1' - Biphenyl]-2-Karbonsäure;
4'-[(5-Propyl-3-Butyl-1H-1,2,4-Triazol-1-yl)methyl][1,1' - Biphenyl]-2-Karbonsäure;
4'-[[5-Propyl-3-(1,1-Difluoroethyl)-1H-1,2,4-Triazol-1-yl]methyl][1,1' - Biphenyl]-2-Karbonsäure;
4'-[[5-Propyl-3-(1,1-Difluoropropyl)-1H-1,2,4-Triazol-1-yl]methyl][1,1' - Biphenyl]-2-Karbonsäure;
4'-[[5-Propyl-3-(1,1-Difluorobutyl)-1H-1,2,4-Triazol-1-yl]methyl][1,1' - Biphenyl]-2-Karbonsäure;
4'-[[5-Propyl-3-(1,1-Difluoropentyl)-1H-1,2,4-Triazol-1-yl]methyl][1,1' - Biphenyl]-2-Karbonsäure;
4'-[[5-Ethyl-3-(1,1-Difluorobutyl)-1H-1,2,4-Triazol-1-yl]methyl][1,1' - Biphenyl]-2-Karbonsäure;
4'-[(3,5-Disecbutyl-1H-1,2,4-Triazol-1-yl)methyl] [1,1' - Biphenyl]-2-Karbonsäure;
4'-[[5-Secbutyl-3-(1,1-Difluorobutyl)-1H-1,2,4-Triazol-1-yl]methyl][1,1' - Biphenyl]-2-Karbonsäure;
4'-[(3,5-Diisobutyl-1H-1,2,4-Triazol-1-yl)methyl][1,1' - Biphenyl]-2-Karbonsäure;
4'-[[5-Isobutyl-3-(1,1-Difluorobutyl)-1H-1,2,4-Triazol-1-yl]methyl][1,1' - Biphenyl]-2-Karbonsäure;
4'-[(3,5-Ditertbutyl-1H-1,2,4-Triazol-1-yl)methyl][1,1' - Biphenyl]-2-Karbonsäure;
4'-[[5-Tertbutyl-3-(1,1-Difluorobutyl)-1H-1,2,4-Triazol-1-yl]methyl][1,1' - Biphenyl]-2-Karbonsäure;
4'-[(3,5-Dipentyl-1H-1,2,4-Triazol-1-yl)methyl][1,1' - Biphenyl]-2-Karbonsäure;
4'-[[5-Pentyl-3-(1,1-Difluorobutyl)-1H-1,2,4-Triazol-1-yl]methyl][1,1' - Biphenyl]-2-Karbonsäure;
4'-[(3,5-Diisopentyl-1H-1,2,4-Triazol-1-yl)methyl][1,1' - Biphenyl]-2-Karbonsäure;
4'-[[5-Isopentyl-3-(1,1-Difluorobutyl)-1H-1,2,4-Triazol-1-yl]methyl][1-1' - Biphenyl]-2-Karbonsäure;
4'-[[5-(1-Butenyl)-3-(1,1-Difluorobutyl)-1H-1,2,4-Triazol-1-yl]methyl][1,1' - Biphenyl]-2-Karbonsäure;
4'-[[5-(2-Butenyl)-3-(1,1-Difluorobutyl)-1H-1,2,4-Triazol-1-yl]methyl][1,1' - Biphenyl]-2-Karbonsäure;
4'-[[5-(3-Butenyl)-3-(1,1-Difluorobutyl)-1H-1,2,4-Triazol-1-yl]methyl][1,1' - Biphenyl]-2-Karbonsäure;
4'-[[5-(1-Butynyl)-3-(1,1-Difluorobutyl)-1H-1,2,4-Triazol-1-yl]methyl][1,1' - Biphenyl]-2-Karbonsäure;
4'-[[5-(2-Butynyl)-3-(1,1-Difluorobutyl)-1H-1,2,4-Triazol-1-yl]methyl][1,1' - Biphenyl]-2-Karbonsäure;
4'-[[5-(3-Butynyl)-3-(1,1-Difluorobutyl)-1H-1,2,4-Triazol-1-yl]methyl][1,1' - Biphenyl]-2-Karbonsäure;
4'-[(3,5-Diethoxy-1H-1,2,4-Triazol-1-yl)methyl][1,1' - Biphenyl]-2-Karbonsäure;
4'-[(3,5-Dipropoxy-1H-1,2,4-Triazol-1-yl)methyl][1,1' - Biphenyl]-2-Karbonsäure;
4'-[(3,5-Diisopropoxy-1H-1,2,4-Triazol-1-yl)methyl][1,1' - Biphenyl]-2-Karbonsäure;
4'-[(3,5-Dibutoxy-1H-1,2,4-Triazol-1-yl)methyl][1,1' - Biphenyl]-2-Karbonsäure;
4'-[[3,5-di (1-Butenyl)-1H-1,2,4-Triazol-1-yl]methyl][1,1' - Biphenyl]-2-Karbonsäure;
4'-[[3,5-di (2-Butenyl)-1H-1,2,4-Triazol-1-yl]methyl][1,1' - Biphenyl]-2-Karbonsäure;
4'-[[3,5-di (3-Butenyl)-1H-1,2,4-Triazol-1-yl]methyl][1,1' - Biphenyl]-2-Karbonsäure;
4'-[[3,5-di (1-Butynyl)-1H-1,2,4-Triazol-1-yl]methyl][1,1' - Biphenyl]-2-Karbonsäure;
4'-[[3,5-di (2-Butynyl)-1H-1,2,4-Triazol-1-yl]methyl] [1,1'- Biphenyl]-2-Karbonsäure;
4'-[[3,5-di (3-Butynyl)-1H-1,2,4-Triazol-1-yl]methyl] [1,1' - Biphenyl]-2-Karbonsäure;

4. Verfahren gemäß Anspruch 1, wobei die bereitete Verbindung ausgewählt ist aus den Verbindungen und deren pharmazeutisch akzeptablen Salzen der Gruppe von Verbindungen, bestehend aus
5-[4'-[(5-Butyl-3-Propyl-1H-1,2,4-Triazol-1-yl)methyl][1,1' - Biphenyl]-2-yl]-1H-Tetrazol;
5-[4'-[(3,5-Dibutyl-1H-1,2,4-Triazol-1-yl)methyl][1,1' - Biphenyl]-2-yl]-1H-Tetrazol;
5-[4'-[(5-Butyl-3-Isopentyl-1H-1,2,4-Triazol-1-yl)methyl][1,1' - Biphenyl]-2-yl]-1H-Tetrazol;
5-[4'-[[5-Butyl-3-(1,1-Dimethoxybutyl)-1H-1,2,4-Triazol-1-yl]methyl][1,1' - Biphenyl]-2-yl]-1H-Tetrazol;
5-[4'-[[5-Butyl-3-(1-Oxobutyl)-1H-1,2,4-Triazol-1-yl]methyl][1,1' - Biphenyl]-2-yl]-1H-Tetrazol;
5-[4'-[[5-Butyl-3-(1,1-Difluoroethyl)-1H-1,2,4-Triazol-1-yl]methyl][1,1' - Biphenyl]-2-yl]-1H-Tetrazol;
5-[4'-[[5-Butyl-3-(1,1-Difluoropropyl)-1H-1,2,4-Triazol-1-yl]methyl][1,1' - Biphenyl]-2-yl]-1H-Tetrazol;
5-[4'-[[5-Butyl-3-(1,1-Difluorobutyl)-1H-1,2,4-Triazol-1-yl]methyl][1,1' - Biphenyl]-2-yl]-1H-Tetrazol;
5-[4'-[[3-Butyl-5-(1,1-Difluorobutyl)-1H-1,2,4-Triazol-1-yl]methyl][1,1' - Biphenyl]-2-yl]-1H-Tetrazol;
5-[4'-[[5-Butyl-3-(1,1-Difluoropentyl)-1H-1,2,4-Triazol-1-yl]methyl][1,1' - Biphenyl]-2-yl]-1H-Tetrazol;
5-[4'-[(3,5-Dipropyl-1H-1,2,4-Triazol-1-yl)methyl][1,1' - Biphenyl]-2-yl]-1H-Tetrazol;
5-[4'-[(3-Butyl-5-Propyl-1H-1,2,4-Triazol-1-yl)methyl]-(1,1'-Biphenyl]-2-yl]-1H-Tetrazol;
5-[4'-[(5-Butyl-3-Isopentyl-1H-1,2,4-Triazol-1-yl)methyl]-(1,1'-Biphenyl]-2-yl]-1H-Tetrazol;
5-[4'-[(3-Phenyl-5-Butyl-1H-1,2,4-Triazol-1yl)methyl]-(1,1'-Biphenyl]-2-yl]-1H-Tetrazol;
5-[4'-[(5-Butyl-3-Phenylethyl-1H-1,2,4-Triazol-1-yl)methyl]-(1,1'-Biphenyl]-2-yl]-1H-Tetrazol;
5-[4'-[(5-Butyl-3-Ethyl-1H-1,2,4-Triazol-1-yl)methyl]-(1,1'-Biphenyl]-2-yl]-1H-Tetrazol;
5-[4'-[(3-Butyl-5-Ethyl-1H-1,2,4-Triazol-1-yl)methyl]-(1,1'-Biphenyl]-2-yl]-1H-Tetrazol;
Glutamin, 1,1-Dimethylethylester;
5-[4'-[[5-Butyl-3-(2-Cyklohexylethyl)-1 H-1,2,4-Triazol-1-yl)methyl][1,1'-Biphenyl]-2-yl]-1H-Tetrazol;
5-[4'-[[5-Butyl-3-(Phenylmethyl)-1H-1,2,4-Triazol-1-yl)methyl][1,1'-Biphenyl]-2-yl]-1H-Tetrazol;
5-[4'-[(5-Butyl-3-Cyclohexyl-1H-1,2,4-Triazol-1-yl)methyl][1,1'-Biphenyl]-2-yl]-1H-Tetrazol;
5-[4'-[(3-Cyclohexylmethyl-5-Butyl-1H-1,2,4-Triazol-1-yl)methyl][1,1'-Biphenyl]-2-yl]-1H-Tetrazol;
5-[4'-[(3-Butyl-5-Cyclohexyl-1H-1,2,4-Triazol-1-yl)methyl][1,1'-Biphenyl]-2-yl]-1H-Tetrazol;
5-[4'-[(3,5-Isopropyl-1H-1,2,4-Triazol-1-yl)methyl][1,1'-Biphenyl]-2-yl]-1H-Tetrazol;
5-[4'-[(5-Propyl-3-Isopentyl-1H-1,2,4-Triazol-1-yl)methyl][1,1'-Biphenyl]-2-yl]-1H-Tetrazol;
5-[4'-[[5-Propyl-3-(1,1-Dimethoxybutyl)-1H-1,2,4-Triazol-1-yl]methyl] [1,1 -Biphenyl]-2-yl]-1H-Tetrazol;
5-[4'-[[5-Propyl-3-(1-Oxobutyl)-1H-1,2,4-Triazol-1-yl]methyl][1,1'-Biphenyl]-2-yl]-1H-Tetrazol;
5-[4'-[[5-Propyl-3-(1,1-Difluoroethyl)-1H-1,2,4-Triazol-1-yl]methyl][1,1'-Biphenyl]-2-yl]-1H-Tetrazol;
5-[4'-[[5-Propyl-3-(1,1-Difluoropropyl)-1H-1,2,4-Triazol-1-yl]methyl][1,1'-Biphenyl]-2-yl]-1H-Tetrazol;
5-[4'-[[5-Propyl-3-(1,1-Difluorobutyl)-1H-1,2,4-Triazol-1-yl]methyl][1,1'-Biphenyl]-2-yl]-1H-Tetrazol;
5-[4'-[[5-Propyl-3-(1,1-Difluoropentyl)-1H-1,2,4-Triazol-1-yl]methyl][1,1'-Biphenyl]-2-yl]-1H-Tetrazol;
5-[4'[[5-Ethyl-3-(1,1-Difluorobutyl)-1H-1,2,4-Triazol-1-yl]methyl][1,1'-Biphenyl]-2-yl]-1H-Tetrazol;
5-[4'-[(5-Secbutyl-3-Propyl-1H-1,2,4-Triazol-1-yl)methyl][1,1'-Biphenyl]-2-yl]-1H-Tetrazol;
5-[4'-[(5-Secbutyl-3-Butyl-1H-1 2,4-Triazol-1-yl)methyl][1,1'-Biphenyl]-2-yl]-1H-Tetrazol;
5-[4'-[(3,5-Disecbutyl-1H-1,2,4-Triazol-1-yl)methyl][1,1'-Biphenyl]-2-yl]-1H-Tetrazol;
5-[4'-[(5-Secbutyl-3-Isopentyl-1H-1,2,4-Triazol-1-yl)methyl][1,1'-Biphenyl]-2-yl]-1H-Tetrazol;
5-[4'-[[5-Secbutyl-3-(1,1-Difluoroethyl)-1H-1,2,4-Triazol-1-yl]methyl][1,1'-Biphenyl]-2-yl]-1H-Tetrazol;
5-[4'-[[5-Secbutyl-3-(1,1-Difluoropropyl)-1H-1,2,4-Triazol-1-yl]methyl][1,1'-Biphenyl]-2-yl]-1H-Tetrazol;
5-[4'-[[5-Secbutyl-3-(1,1-Difluorobutyl)-1H-1,2,4-Triazol-1-yl]methyl][1,1'-Biphenyl]-2-yl]-1H-Tetrazol;
5-[4'-[[5-Secbutyl-3-(1,1-Difluoropentyl)-1H-1,2,4-Triazol-1-yl]methyl][1,1'-Biphenyl]-2-yl]-1H-Tetrazol;
5-[4'-[(5-Isobutyl-3-Propyl-1H-1,2,4-Triazol-1-yl)methyl][1,1'-Biphenyl]-2-yl]-1H-Tetrazol;
5-[4'-[(5-Isobutyl-3-Butyl-1H-1,2,4-Triazol-1-yl)methyl][1,1'-Biphenyl]-2-yl]-1H-Tetrazol;
5-[4'-[(3,5-Diisobutyl-1H-1,2,4-Triazol-1-yl)methyl][1,1'-Biphenyl]-2-yl]-1H-Tetrazol;
5-[4'-[(5-Isobutyl-3-Isopentyl-1H-1,2,4-Triazol-1-yl)methyl][1,1'-Biphenyl]-2-yl]-1H-Tetrazol;
5-[4'-[[5-Isobutyl-3-(1,1-Difluoroethyl)-1H-1,2,4-Triazol-1-yl]methyl][1,1'-Biphenyl]-2-yl]-1H-Tetrazol;
5-[4'-[[5-Isobutyl-3-(1,1-Difluoropropyl)-1H-1,2,4-Triazol-1-yl]methyl][1,1'-Biphenyl]-2-yl]-1H-Tetrazol;
5-[4'-[[5-Isobutyl-3-(1,1-Difluorobutyl)-1H-1,2,4-Triazol-1-yl]methyl][1,1'-Biphenyl]-2-yl]-1H-Tetrazol;
5-[4'-[[5-Isobutyl-3-(1,1-Difluoropentyl)-1H-1,2,4-Triazol-1-yl]methyl][1,1'-Biphenyl]-2-yl]-1H-Tetrazol;
5-[4'-[(5-Tertbutyl-3-Propyl-1H-1,2,4-Triazol-1-yl)methyl][1,1'-Biphenyl]-2-yl]-1H-Tetrazol;
5-[4'-[(5-Tertbutyl-3-Butyl-1H-1,2,4-Triazol-1-yl)methyl][1,1'-Biphenyl]-2-yl]-1H-Tetrazol;
5-[4'-[(3,5-Ditertbutyl-1H-1,2,4-Triazol-1-yl)methyl][1,1'-Biphenyl]-2-yl]-1H-Tetrazol;
5-[4'-[(5-Tertbutyl-3-Isopentyl-1H-1,2,4-Triazol-1-yl)methyl][1,1'-Biphenyl]-2-yl]-1H-Tetrazol;
5-[4'-[[5-Tertbutyl-3-(1,1-Difluoroethyl)-1H-1,2,4-Triazol-1-yl]methyl][1,1'-Biphenyl]-2-yl]-1H-Tetrazol;
5-[4'-[[5-Tertbutyl-3-(1,1-Difluoropropyl)-1H-1,2,4-Triazol-1-yl]methyl][1,1'-Biphenyl]-2-yl]-1H-Tetrazol;
5-[4'-[[5-Tertbutyl-3-(1,1-Difluorobutyl)-1H-1,2,4-Triazol-1-yl]methyl][1,1'-Biphenyl]-2-yl]-1H-Tetrazol;
5-[4'-[[5-Tertbutyl-3-(1,1-Difluoropentyl)-1H-1,2,4-Triazol-1-yl]methyl][1,1'-Biphenyl]-2-yl]-1H-Tetrazol;
5-[4'-[(5-Pentyl-3-Propyl-1H-1,2,4-Triazol-1-yl)methyl][1,1'-Biphenyl]-2-yl]-1H-Tetrazol;
5-[4'-[(5-Pentyl-3-Butyl-1H-1,2,4-Triazol-1-yl)methyl][1,1'-Biphenyl]-2-yl]-1H-Tetrazol;
5-[4'-[(3,5-Dipentyl-1H-1,2,4-Triazol-1-yl)methyl][1,1'-Biphenyl]-2-yl]-1H-Tetrazol;
5-[4'-[(5-Pentyl-3-Isopentyl-1H-1,2,4-Triazol-1-yl)methyl][1,1'-Biphenyl]-2-yl]-1H-Tetrazol;
5-[4'-[[5-Pentyl-3-(1,1-Difluoroethyl)-1H-1,2,4-Triazol-1-yl]methyl] [1,1'-Biphenyl]-2-yl]-1H-Tetrazol;
5-[4'-[[5-Pentyl-3-(1,1-Difluoropropyl)-1H-1,2,4-Triazol-1-yl]methyl][1,1'-Biphenyl]-2-yl]-1H-Tetrazol;
5-[4'-[[5-Pentyl-3-(1,1-Difluorobutyl)-1H-1,2,4-Triazol-1-yl]methyl][1,1'-Biphenyl]-2-yl]-1H-Tetrazol;
5-[4'-[[5-Pentyl-3-(1,1-Difluoropentyl)-1H-1,2,4-Triazol-1-yl]methyl][1,1'-Biphenyl]-2-yl]-1H-Tetrazol;
5-[4'-[(5-Isopentyl-3-Ethyl-1H-1,2,4-Triazol-1-yl)methyl][1,1'-Biphenyl]-2-yl]-1H-Tetrazol;
5-[4'-[(5-Isopentyl-3-Propyl-1H-1,2,4-Triazol-1-yl)methyl][1,1'-Biphenyl]-2-yl]-1H-Tetrazol;
5-[4'-[(5-Isopentyl-3-Butyl-1H-1,2,4-Triazol-1-yl)methyl][1,1'-Biphenyl]-2-yl]-1H-Tetrazol;
5-[4'-[(3,5-Diisopentyl-1H-1,2,4-Triazol-1-yl)methyl][1,1'-Biphenyl]-2-yl]-1H-Tetrazol;
5-[4'-[[5-Isopentyl-3-(1,1-Difluoroethyl)-1H-1,2,4-Triazol-1-yl]methyl][1,1'-Biphenyl]-2-yl]-1H-Tetrazol;
5-[4'-[[5-Isopentyl-3-(1,1-Difluoropropyl)-1H-1,2,4-Triazol-1-yl]methyl][1,1'-Biphenyl]-2-yl]-1H-Tetrazol;
5-[4'-[[5-Isopentyl-3-(1,1-Difluorobutyl)-1H-1,2,4-Triazol-1-yl]methyl][1,1'-Biphenyl]-2-yl]-1H-Tetrazol;
5-[4'-[[5-Isopentyl-3-(1,1-Difluoropentyl)-1H-1,2,4-Triazol-1-yl]methyl][1,1'-Biphenyl]-2-yl]-1H-Tetrazol;
5-[4'-[[5-(1-Butenyl)-3-Propyl-1H-1,2,4-Triazol-1-yl]methyl][1,1'-Biphenyl]-2-yl]-1H-Tetrazol;
5-[4'-[[5-(1-Butenyl)-3-Butyl-1H-1,2,4-Triazol-1-yl]methyl][1,1'-Biphenyl]-2-yl]-1H-Tetrazol;
5-[4'-[[5-(1-Butenyl)-3-Isopentyl-1H-1,2,4-Triazol-1-yl)methyl][1,1'-Biphenyl]-2-yl]-1H-Tetrazol;
5-[4'-[[5-(1-Butenyl)-3-(1,1-Dimethoxybutyl)-1H-1,2,4-Triazol-1-yl]methyl][1,1'-Biphenyl]-2-yl]-1H-Tetrazol;
5-[4'-[[5-(1-Butenyl)-3-(1,1-Difluorobutyl)-1H-1,2,4-Triazol-1-yl]methyl][1,1'-Biphenyl]-2-yl]-1H-Tetrazol;
5-[4'-[[5-(2-Butenyl)-3-(1,1-Difluorobutyl)-1H-1,2,4-Triazol-1-yl]methyl][1,1'-Biphenyl]-2-yl]-1H-Tetrazol;
5-[4'-[[5-(3-Butenyl)-3-(1,1-Difluorobutyl)-1H-1,2,4-Triazol-1-yl]methyl][1,1'-Biphenyl]-2-yl]-1H-Tetrazol;
5-[4'-[[5-(1-Butynyl)-3-(1,1-Difluorobutyl)-1H-1,2,4-Triazol-1-yl]methyl][1,1'-Biphenyl]-2-yl]-1H-Tetrazol;
5-[4'-[[5-(2-Butynyl)-3-(1,1-Difluorobutyl)-1H-1,2,4-Triazol-1-yl]methyl][1,1'-Biphenyl]-2-yl]-1H-Tetrazol;
5-[4'-[[5-(3-Butynyl)-3-(1,1-Difluorobutyl)-1H-1,2,4-Triazol-1-yl]methyl][1,1'-Biphenyl]-2-yl]-1H-Tetrazol;
5-[4'-[[3,5-di(1-Butenyl)-1H-1,2,4-Triazol-1-yl]methyl][1,1'-Biphenyl]-2-yl]-1H-Tetrazol;
5-[4'-[[3,5-di(2-Butenyl)-1H-1,2,4-Triazol-1-yl]methyl][1,1'-Biphenyl]-2-yl]-1H-Tetrazol;
5-[4'-[[3,5-di(3-Butenyl)-1H-1,2,4-Triazol-1-yl]methyl][1,1'-Biphenyl]-2-yl]-1H-Tetrazol;
5-[4'-[[3,5-di(1-Butynyl)-1H-1,2,4-Triazol-1-yl)methyl][1,1'-Biphenyl]-2-yl]-1H-Tetrazol;
5-[4'-[[3,5-di(2-Butynyl)-1H-1,2,4-Triazol-1-yl]methyl][1,1'-Biphenyl]-2-yl]-1H-Tetrazol;
5-[4'-[[3,5-di(3-Butynyl)-1H-1,2,4-Triazol-1-yl]methyl][1,1'-Biphenyl]-2-yl]-1H-Tetrazol;

5. Verfahren gemäß Anspruch 1, wobei die bereitete Verbindung ausgewählt ist aus Verbindungen und deren pharmazeutisch akzeptablen Salzen aus der Gruppe von Verbindungen, bestehend aus
4'-[(3,5-Dibutyl-1H-1,2,4-Triazol-1-yl)methyl][1,1'-bi-Phenyl]-2-Karbonsäure;
4'-[(3-Ethyl-5-Butyl-1H-1,2,4-Triazol-1-yl)methyl] [1,1'-Biphenyl]-2-Karbonsäure;
4'-[(3-Butyl-5-Ethyl-1H-1,2,4-Triazol-1-yl)methyl] [1,1'-Biphenyl]-2-Karbonsäure;
4'-[(5-Butyl-1H-1,2,4-Triazol-1-yl)methyl] [1,1'-Biphenyl]-2-Karbonsäure;
4'-[(3-Butyl-1H-1,2,4-Triazol-1-yl)methyl] [1,1'-Biphenyl]-2-Karbonsäure;

6. Verfahren zur Bereitung einer pharmazeutischen Zusammensetzung, die gekennzeichnet ist durch die Hinzumischung einer therapeutisch wirksamen Menge einer Angiotensin-II-antagonistischen Verbindung und eines pharmazeutisch akzeptablen Trägers oder Verdünnungsmittels, wobei die antagonistische Verbindung ausgewählt ist aus einer Familie von Verbindungen gemäß einem der Ansprüche 1 - 5 oder dem Amid der Verbindungen, worin R³ ist COOH, oder deren Salzen.

7. Benutzung einer Verbindung gemäß einem der Ansprüche 1 - 5 oder dem Amid der Verbindungen, worin R³ ist COOH, oder deren Salzen zur Herstellung eines Medikaments zur Behandlung einer Kreislauferkrankung.

8. Benutzung gemäß Anspruch 7, wobei die Erkrankung eine kardiovaskuläre Erkrankung ist.

9. Benutzung gemäß Anspruch 8, wobei die kardiovaskuläre Erkrankung Hypertonie ist.

10. Benutzung gemäß Anspruch 8, wobei die kardiovaskuläre Erkrankung kongestive Herzinsuffizienz ist.

11. Benutzung einer Verbindung gemäß einem der Ansprüche 1 - 5 oder des Amids der Verbindungen, wobei R³ ist COOH, oder deren Salzes zur Bereitung eines Medikaments zur Behandlung von Glaukoma.

12. Verfahren zur Bereitung einer Verbindung der Formel 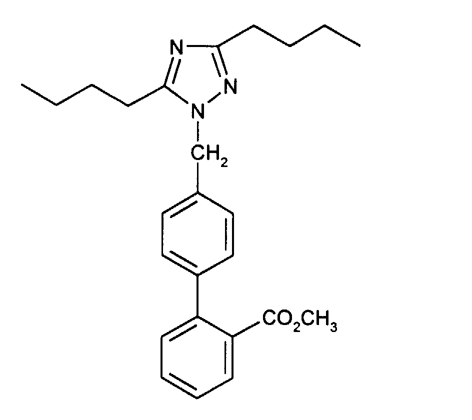 dadurch gekennzeichnet, daß 3,5-Dibutyl-1H-1,2,4-Triazol in Anwesenheit von Natriumhydrid zu 4-Bromo-Methyl-2'-Methoxycarbonyl-Biphenyl hinzugefügt wird.

13. Verfahren zur Bereitung einer Verbindung der Formel 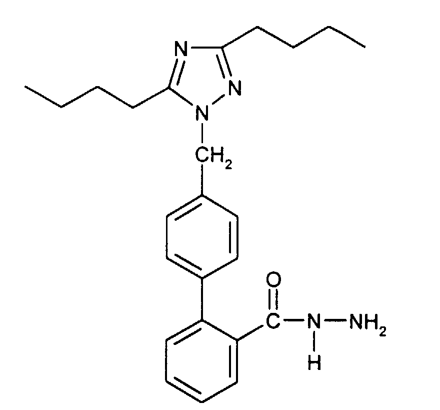 gekennzeichnet dadurch, daß die nach Anspruch 12 bereitete Verbindung mit wasserfreiem Hydrazin reagiert wird.

## Patentansprüche (Patentansprüche für folgende(n) Vertragsstaat(en): GR)

1. Verbindung der Formel I 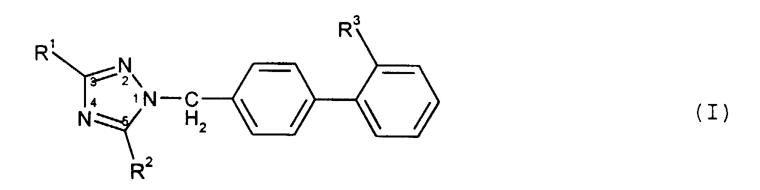 wobei R¹ und R² unabhängig voneinander ausgewählt werden aus Wasserstoff, C₁-C₂₀ Alkyl, Phenyl, durch C₁-C₂₀ Alkyl substituiertes Phenyl, C₃-C₁₀ Cycloalkyl, an C₁-C₂₀ Alkyl gebundenes oder durch dieses substituiertes C₃-C₁₀ Cycloalkyl, C₁-C₁₀ Alkoxy, durch Phenyl substituiertes C₁-C₁₀-Difluoroalkyl, C₂-C₂₀ Alkenyl, C₂-C₂₀ Alkynyl, C₁-C₂₀ Alkylcarbonyl; difluorosubstituiertes C₁-C₂₀ Alkyl; C₁-C₁₀-Hydroxyalkyl, durch eine oder mehrere Alkoxygruppen mit Alkylanteilen von 1 bis 10 Kohlenstoffatomen substituiertes C₁-C₂₀ Alkyl; wobei R³ eine Säurekomponente ist, ausgewählt aus CO₂H, CONHNHSO₂CF₃, 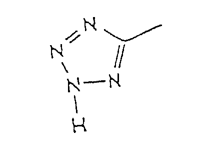 und den Salzen der Säurekomponenten; oder einem Tautomer hiervon oder einem pharmazeutisch akzeptablen Salz hiervon.

2. Verbindung von Anspruch 1, wobei R³ ausgewählt ist aus COOH oder Tetrazolyl oder einem Tautomer oder einem pharmazeutisch akzeptablem Salz hiervon.

3. Verbindung von Anspruch 2, ausgewählt aus Verbindungen und deren pharmazeutisch akzeptablen Salzen der Gruppe von Verbindungen, bestehend aus
4'-[(3,5-Dibutyl-1H-1,2,4-Triazol-1-yl)methyl] [1,1'- Biphenyl]-2-Karbonsäure, 2-[(Trifluoromethyl)sulfonyl]hydrazid;
4'-[(5-Butyl-3-Propyl-1H-1,2,4-Triazol-1-yl)methyl][1,1' - Biphenyl]-2-Karbonsäure;
4'-[(3,5-Dibutyl-1H-1,2,4-Triazol-1-yl)methyl][1,1' - Biphenyl]-2-Karbonsäure;
4'-[(5-Butyl-3-Isopentyl-1H-1,2,4-Triazol-1-yl)methyl] [1,1'- Biphenyl]-2-Karbonsäure;
4'-[[5-Butyl-3-(1,1-Dimethoxybutyl)-1H-1,2,4-Triazol-1-yl]methyl][1,1' - Biphenyl]-2-Karbonsäure;
4'-[[5-Butyl-3-(1-Oxobutyl)-1H-1,2,4-Triazol-1-yl]methyl] [1,1' - Biphenyl]-2-Karbonsäure;
4'-[[5-Butyl-3-(1-Oxopentyl)-1H-1,2,4-Triazol-1-yl]methyl] [1,1'- Biphenyl]-2-Karbonsäure;
4'-[[5-Butyl-3-(1,1-Difluoroethyl)-1H-1,2,4-Triazol-1-yl]methyl] [1,1'- Biphenyl]-2-Karbonsäure;
4'-[[5-Butyl-3-(1,1-Difluoropropyl)-1H-1,2,4-Triazol-1-yl]methyl][1,1' - Biphenyl]-2-Karbonsäure;
4'-[[5-Butyl-3-(1,1-Difluorobutyl)-1H-1,2,4-Triazol-1-yl]methyl] [1,1' - Biphenyl]-2-Karbonsäure;
4'-[[3-Butyl-5-(1,1-Difluorobutyl)-1H-1,2,4-Triazol-1-yl]methyl] [1,1' - Biphenyl]-2-Karbonsäure;
4'-[[5-Butyl-3-(1,1-Difluoropentyl)-1H-1,2,4-Triazol-1-yl]methyl] [1,1' - Biphenyl]-2-Karbonsäure;
4'-[(3,5-Dipropyl-1H-1,2,4-Triazol-1-yl)methyl] [1,1' - Biphenyl]-2-Karbonsäure;
4'-[(3,5-Isopropyl-1H-1,2,4-Triazol-1-yl)methyl][1,1' - Biphenyl]-2-Karbonsäure;
4'-[(5-Propyl-3-Butyl-1H-1,2,4-Triazol-1-yl)methyl][1,1' - Biphenyl]-2-Karbonsäure;
4'-[[5-Propyl-3-(1,1-Difluoroethyl)-1H-1,2,4-Triazol-1-yl]methyl] [1,1' - Biphenyl]-2-Karbonsäure;
4'-[[5-Propyl-3-(1,1-Difluoropropyl)-1H-1,2,4-Triazol-1-yl]methyl][1,1' - Biphenyl]-2-Karbonsäure;
4'-[[5-Propyl-3-(1,1-Difluorobutyl)-1H-1,2,4-Triazol-1-yl]methyl] [1,1' - Biphenyl]-2-Karbonsäure;
4'-[[5-Propyl-3-(1,1-Difluoropentyl)-1H-1,2,4-Triazol-1-yl]methyl] [1,1' - Biphenyl]-2-Karbonsäure;
4'-[[5-Ethyl-3-(1,1-Difluorobutyl)-1H-1,2,4-Triazol-1-yl]methyl][1,1' - Biphenyl]-2-Karbonsäure;
4'-[(3,5-Disecbutyl-1H-1,2,4-Triazol-1-yl)methyl][1,1' - Biphenyl]-2-Karbonsäure;
4'-[[5-Secbutyl-3-(1,1-Difluorobutyl)-1H-1,2,4-Triazol-1-yl]methyl] [1,1' - Biphenyl]-2-Karbonsäure;
4'-[(3,5-Diisobutyl-1H-1,2,4-Triazol-1-yl)methyl][1,1'- Biphenyl]-2-Karbonsäure;
4'-[[5-Isobutyl-3-(1,1-Difluorobutyl)-1H-1,2,4-Triazol-1-yl]methyl][1,1'- Biphenyl]-2-Karbonsäure;
4'-[(3,5-Ditertbutyl-1H-1,2,4-Triazol-1-yl)methyl][1,1' - Biphenyl]-2-Karbonsäure;
4'-[[5-Tertbutyl-3-(1,1-Difluorobutyl)-1H-1,2,4-Triazol-1-yl]methyl][1,1' - Biphenyl]-2-Karbonsäure;
4'-[(3,5-Dipentyl-1H-1,2,4-Triazol-1-yl)methyl] [1,1' - Biphenyl]-2-Karbonsäure;
4'-[[5-Pentyl-3-(1,1-Difluorobutyl)-1H-1,2,4-Triazol-1-yl]methyl] [1,1' - Biphenyl]-2-Karbonsäure;
4'-[(3,5-Diisopentyl-1H-1,2,4-Triazol-1-yl)methyl] [1,1' - Biphenyl]-2-Karbonsäure;
4'-[[5-Isopentyl-3-(1,1-Difluorobutyl)-1H-1,2,4-Triazol-1-yl]methyl] [1,1' - Biphenyl]-2-Karbonsäure;
4'-[[5-(1-Butenyl)-3-(1,1-Difluorobutyl)-1H-1,2,4-Triazol-1-yl]methyl][1,1' - Biphenyl]-2-Karbonsäure;
4'-[[5-(2-Butenyl)-3-(1,1-Difluorobutyl)-1H-1,2,4-Triazol-1-yl]methyl] [1,1' - Biphenyl]-2-Karbonsäure;
4'-[[5-(3-Butenyl)-3-(1,1-Difluorobutyl)-1H-1,2,4-Triazol-1-yl]methyl][1,1' - Biphenyl]-2-Karbonsäure;
4'-[[5-(1-Butynyl)-3-(1,1-Difluorobutyl)-1H-1,2,4-Triazol-1-yl]methyl][1,1' - Biphenyl]-2-Karbonsäure;
4'-[[5-(2-Butynyl)-3-(1,1-Difluorobutyl)-1H-1,2,4-Triazol-1-yl]methyl] [1,1' - Biphenyl]-2-Karbonsäure;
4'-[[5-(3-Butynyl)-3-(1,1-Difluorobutyl)-1H-1,2,4-Triazol-1-yl]methyl] [1,1' - Biphenyl]-2-Karbonsäure;
4'-[(3,5-Diethoxy-1H-1,2,4-Triazol-1-yl)methyl] [1,1' - Biphenyl]-2-Karbonsäure;
4'-[(3,5-Dipropoxy-1H-1,2,4-Triazol-1-yl)methyl] [1,1' - Biphenyl]-2-Karbonsäure;
4'-[(3,5-Diisopropoxy-1H-1,2,4-Triazol-1-yl)methyl] [1,1' - Biphenyl]-2-Karbonsäure;
4'-[(3,5-Dibutoxy-1H-1,2,4-Triazol-1-yl)methyl][1,1' - Biphenyl]-2-Karbonsäure;
4'-[[3,5-di (1-Butenyl)-1H-1,2,4-Triazol-1-yl]methyl][1,1' - Biphenyl]-2-Karbonsäure;
4'-[[3,5-di (2-Butenyl)-1H-1,2,4-Triazol-1-yl]methyl][1,1' - Biphenyl]-2-Karbonsäure;
4'-[[3,5-di (3-Butenyl)-1H-1,2,4-Triazol-1-yl]methyl][1,1' - Biphenyl]-2-Karbonsäure;
4'-[[3,5-di (1-Butynyl)-1H-1,2,4-Triazol-1-yl]methyl] [1,1' - Biphenyl]-2-Karbonsäure;
4'-[[3,5-di (2-Butynyl)-1H-1,2,4-Triazol-1-yl]methyl] [1,1' - Biphenyl]-2-Karbonsäure;
4'-[[3,5-di (3-Butynyl)-1H-1,2,4-Triazol-1-yl]methyl] [1,1' - Biphenyl]-2-Karbonsäure;

4. Verbindung von Anspruch 1, ausgewählt aus Verbindungen und deren pharmazeutisch akzeptablen Salzen der Gruppe von Verbindungen, bestehend aus
5-[4'-[(5-Butyl-3-Propyl-1H-1,2,4-Triazol-1-yl)methyl] [1,1' - Biphenyl]-2-yl]-1H-Tetrazol;
5-[4'-[(3,5-Dibutyl-1H-1,2,4-Triazol-1-yl)methyl] [1,1' - Biphenyl]-2-yl]-1H-Tetrazol;
5-[4'-[(5-Butyl-3-Isopentyl-1H-1,2,4-Triazol-1-yl)methyl][1,1' - Biphenyl]-2-yl]-1H-Tetrazol;
5-[4'-[[5-Butyl-3-(1,1-Dimethoxybutyl)-1H-1,2,4-Triazol-1-yl]methyl] [1,1' - Biphenyl]-2-yl]-1H-Tetrazol;
5-[4'-[[5-Butyl-3-(1-Oxobutyl)-1H-1,2,4-Triazol-1-yl]methyl] [1,1' - Biphenyl]-2-yl]-1H-Tetrazol;
5-[4'-[[5-Butyl-3-(1,1-Difluoroethyl)-1H-1,2,4-Triazol-1-yl]methyl][1,1' - Biphenyl]-2-yl]-1H-Tetrazol;
5-[4'-[[5-Butyl-3-(1,1-Difluoropropyl)-1H-1,2,4-Triazol-1-yl]methyl][1,1' - Biphenyl]-2-yl]-1H-Tetrazol;
5-[4'-[[5-Butyl-3-(1,1-Difluorobutyl)-1H-1,2,4-Triazol-1-yl]methyl][1,1' - Biphenyl]-2-yl]-1H-Tetrazol;
5-[4'-[[3-Butyl-5-(1,1-Difluorobutyl)-1H-1,2,4-Triazol-1-yl]methyl][1,1' - Biphenyl]-2-yl]-1H-Tetrazol;
5-[4'-[[5-Butyl-3-(1,1-Difluoropentyl)-1H-1,2,4-Triazol-1-yl]methyl][1,1' - Biphenyl]-2-yl]-1H-Tetrazol;
5-[4'-[(3,5-Dipropyl-1H-12,4-Triazol-1-yl)methyl][1,1' - Biphenyl]-2-yl]-1H-Tetrazol;
5-[4'-[(3-Butyl-5-Propyl-1H-1,2,4-Triazol-1-yl)methyl]-(1,1'-Biphenyl]-2-yl]-1H-Tetrazol;
5-[4'-[(5-Butyl-3-Isopentyl-1H-1,2,4-Triazol-1-yl)methyl]-(1,1'-Biphenyl]-2-yl]-1H-Tetrazol;
5-[4'-[(3-Phenyl-5-Butyl-1H-1,2,4-Triazol-1-yl)methyl]-(1,1'-Biphenyl]-2-yl]-1H-Tetrazol;
5-[4'-[(5-Butyl-3-Phenylethyl-1H-1,2,4-Triazol-1-yl)methyl]-(1,1'-Biphenyl]-2-yl]-1H-Tetrazol;
5-[4'-[(5-Butyl-3-Ethyl-1H-1,2,4-Triazol-1yl)methyl]-(1,1'-Biphenyl]-2-yl]-1H-Tetrazol;
5-[4'-[(3-Butyl-5-Ethyl-1H-1,2,4-Triazol-1-yl)methyl]-(1,1'-Biphenyl]-2-yl]-1H-Tetrazol;
Glutamin, 1,1-Dimethylethylester;
5-[4'-[[5-Butyl-3-(2-Cyklohexylethyl)-1H-1,2,4-Triazol-1-yl)methyl][1,1'-Biphenyl]-2-yl]-1H-Tetrazol;
5-[4'-[[5-Butyl-3-(Phenylmethyl)-1H-1,2,4-Triazol-1-yl)methyl][1,1'-Biphenyl]-2-yl]-1H-Tetrazol;
5-[4'-[(5-Butyl-3-Cyclohexyl-1H-1,2,4-Triazol-1-yl)methyl] [1,1'-Biphenyl]-2-yl]-1H-Tetrazol;
5-[4'-[(3-Cyclohexylmethyl-5-Butyl-1H-1,2,4-Triazol-1-yl)methyl][1,1'-Biphenyl]-2-yl]-1H-Tetrazol;
5-[4'-[(3-Butyl-5-Cyclohexyl-1H-1,2,4-Triazol-1-yl)methyl][1,1'-Biphenyl]-2-yl]-1H-Tetrazol;
5-[4'-[(3,5-Isopropyl-1H-1,2,4-Triazol-1-yl)methyl] [1,1'-Biphenyl]-2-yl]-1H-Tetrazol;
5-[4'-[(5-Propyl-3-Isopentyl-1H-1,2,4-Triazol-1-yl)methyl] [1,1'-Biphenyl]-2-yl]-1H-Tetrazol;
5-[4'-[[5-Propyl-3-(1,1-Dimethoxybutyl)-1H-1,2,4-Triazol-1-yl]methyl][1,1'-Biphenyl]-2-yl]-1H-Tetrazol;
5-[4'-[[5-Propyl-3-(1-Oxobutyl)-1H-1,2,4-Triazol-1-yl]methyl][1,1'-Biphenyl]-2-yl]-1H-Tetrazol;
5-[4'-[[5-Propyl-3-(1,1-Difluoroethyl)-1H-1,2,4-Triazol-1-yl]methyl][1,1'-Biphenyl]-2-yl]-1H-Tetrazol;
5-[4'-[[5-Propyl-3-(1,1-Difluoropropyl)-1H-1,2,4-Triazol-1-yl]methyl][1,1'-Biphenyl]-2-yl]-1H-Tetrazol;
5-[4'-[[5-Propyl-3-(1,1-Difluorobutyl)-1H-1,2,4-Triazol-1-yl]methyl][1,1'-Biphenyl]-2-yl]-1H-Tetrazol;
5-[4'-[[5-Propyl-3-(1,1-Difluoropentyl)-1H-1,2,4-Triazol-1-yl]methyl] [1,1'-Biphenyl]-2-yl]-1H-Tetrazol;
5-[4'-[[5-Ethyl-3-(1,1-Difluorobutyl)-1H-1,2,4-Triazol-1-yl]methyl][1,1'-Biphenyl]-2-yl]-1H-Tetrazol;
5-[4'-[(5-Secbutyl-3-Propyl-1H-1,2,4-Triazol-1-yl)methyl] [1,1' -Biphenyl]-2-yl]-1H-Tetrazol;
5-[4'-[(5-Secbutyl-3-Butyl-1H-1,2,4-Triazol-1-yl)methyl][1,1'- Biphenyl]-2-yl]-1H-Tetrazol;
5-[4'-[(3,5-Disecbutyl-1H-1,2,4-Triazol-1-yl)methyl] [1,1'-Biphenyl]-2-yl]-1H-Tetrazol;
5-[4'-[(5-Secbutyl-3-Isopentyl-1H-1,2,4-Triazol-1-yl)methyl][1,1'-Biphenyl]-2-yl]-1H-Tetrazol;
5-[4'-[[5-Secbutyl-3-(1,1-Difluoroethyl)-1H-1,2,4-Triazol-1-yl]methyl] [1,1'-Biphenyl]-2-yl]-1H-Tetrazol;
5-[4'-[[5-Secbutyl-3-(1,1-Difluoropropyl)-1H-1,2,4-Triazol-1-yl]methyl][1,1'-Biphenyl]-2-yl]-1H-Tetrazol;
5-[4'-[[5-Secbutyl-3-(1,1-Difluorobutyl)-1H-1,2,4-Triazol-1-yl]methyl][1,1' -Biphenyl]-2-yl]-1H-Tetrazol;
5-[4'-[[5-Secbutyl-3-(1,1-Difluoropentyl)-1H-1,2,4-Triazol-1-yl]methyl][1,1' -Biphenyl]-2-yl]-1H-Tetrazol;
5-[4'-[(5-Isobutyl-3-Propyl-1H-1,2,4-Triazol-1-yl)methyl][1,1'-Biphenyl]-2-yl]-1H-Tetrazol;
5-[4'-[(5-Isobutyl-3-Butyl-1H-1,2,4-Triazol-1-yl)methyl][1,1'-Biphenyl]-2-yl]-1H-Tetrazol;
5-[4'-((3,5-Diisobutyl-1H-1,2,4-Triazol-1-yl)methyl][1,1' -Biphenyl]-2-yl]-1H-Tetrazol;
5-[4'-((5-Isobutyl-3-Isopentyl-1H-1,2,4-Triazol-1-yl)methyl][1,1' -Biphenyl]-2-yl]-1H-Tetrazol;
5-[4'-[[5-Isobutyl-3-(1,1-Difluoromethyl)-1H-1,2,4-Triazol-1-yl]methyl][1,1'-Biphenyl]-2-yl]-1H-Tetrazol;
5-[4'-[[5-Isobutyl-3-(1,1-Difluoropropyl)-1H-1,2,4-Triazol-1-yl]methyl][1,1'-Biphenyl]-2-yl]-1H-Tetrazol;
5-[4'-[[5-Isobutyl-3-(1,1-Difluorobutyl)-1H-1,2,4-Triazol-1-yl]methyl][1,1' -Biphenyl]-2-yl]-1H-Tetrazol;
5-[4'-[[5-Isobutyl-3-(1,1-Difluoropentyl)-1H-1,2,4-Triazol-1-yl]methyl][1,1' -Biphenyl]-2-yl]-1H-Tetrazol;
5-[4'-[(5-Tertbutyl-3-Propyl-1H-1,2,4-Triazol-1-yl)methyl] [1,1'-Biphenyl]-2-yl]-1H-Tetrazol;
5-[4'-[(5-Tertbutyl-3-Butyl-1H-1,2,4-Triazol-1-yl)methyl][1,1'-Biphenyl]-2-yl]-1H-Tetrazol;
5-[4'-[(3'5-Ditertbutyl-1H-1,2,4-Triazol-1-yl)methyl][1,1' -Biphenyl]-2-yl]-1H-Tetrazol;
5-[4'-[(5-Tertbutyl-3-Isopentyl-1H-1,2,4-Triazol-1-yl)methyl][1,1'-Biphenyl]-2-yl]-1H-Tetrazol;
5-[4'-[[5-Tertbutyl-3-(1,1-Difluoroethyl)-1H-1,2,4-Triazol-1-yl]methyl][1,1'-Biphenyl]-2-yl]-1H-Tetrazol;
5-[4'-[[5-Tertbutyl-3-(1,1-Difluoropropyl)-1H-1,2,4-Triazol-1-yl]methyl][1,1'-Biphenyl]-2-yl]-1H-Tetrazol;
5-[4'-[[5-Tertbutyl-3-(1,1-Difluorobutyl)-1H-1,2,4-Triazol-1-yl]methyl] [1,1'-Biphenyl]-2-yl]-1H-Tetrazol;
5-[4'-[[5-Tertbutyl-3-(1,1-Difluoropentyl)-1H-1,2,4-Triazol-1-yl]methyl][1,1'-Biphenyl]-2-yl]-1H-Tetrazol;
5-[4'-[(5-Pentyl-3-Propyl-1 H-1,2,4-Triazol-1-yl)methyl] [1,1'-Biphenyl]-2-yl]-1H-Tetrazol;
5-[4'-[(5-Pentyl-3-Butyl-1H-1,2,4-Triazol-1-yl)methyl] [1,1'-Biphenyl]-2-yl]-1H-Tetrazol;
5-[4'-[(3,5-Dipentyl-1H-1,2,4-Triazol-1-yl)methyl][1,1' -Biphenyl]-2-yl]-1H-Tetrazol;
5-[4'-[(5-Pentyl-3-Isopentyl-1H-1,2,4-Triazol-1-yl)methyl][1,1 Biphenyl]-2-yl]-1H-Tetrazol;
5-[4'-[[5-Pentyl-3-(1,1-Difluoroethyl)-1H-1,2,4-Triazol-1-yl]methyl][1,1'-Biphenyl]-2-yl]-1H-Tetrazol;
5-[4'-[[5-Pentyl-3-(1,1-Difluoropropyl)-1H-1,2,4-Triazol-1-yl]methyl][1,1' -Biphenyl]-2-yl]-1H-Tetrazol;
5-[4'-[[5-Pentyl-3-(1,1-Difluorobutyl)-1H-1,2,4-Triazol-1-yl]methyl][1,1'-Biphenyl]-2-yl]-1H-Tetrazol;
5-[4'-[[5-Pentyl-3-(1,1-Difluoropentyl)-1H-1,2,4-Triazol-1-yl]methyl][1,1'-Biphenyl]-2-yl]-1H-Tetrazol;
5-[4'-[(5-Isopentyl-3-Ethyl-1H-1,2,4-Triazol-1-yl)methyl] [1,1'-Biphenyl]-2-yl]-1H-Tetrazol;
5-[4'-[(5-Isopentyl-3-Propyl-1H-1,2,4-Triazol-1-yl)methyl][1,1'-Biphenyl]-2-yl]-1H-Tetrazol;
5-[4'-[(5-Isopentyl-3-Butyl-1H-1,2,4-Triazol-1-yl)methyl][1,1'-Biphenyl]-2-yl]-1H-Tetrazol;
5-[4'-[(3,5-Diisopentyl-1H-1,2,4-Triazol-1-yl)methyl][1,1'-Biphenyl]-2-yl]-1H-Tetrazol;
5-[4'-[[5-Isopentyl-3-(1,1-Difluoroethyl)-1H-1,2,4-Triazol-1-yl]methyl][1,1'-Biphenyl]-2-yl]-1H-Tetrazol;
5-[4'-[[5-Isopentyl-3-(1,1-Difluoropropyl)-1H-1,2,4-Triazol-1-yl]methyl][1,1'-Biphenyl]-2-yl]-1H-Tetrazol;
5-[4'-[[5-Isopentyl-3-(1,1-Difluorobutyl)-1H-1,2,4-Triazol-1-yl]methyl][1,1'-Biphenyl]-2-yl]-1H-Tetrazol;
5-[4'-[[5-Isopentyl-3-(1,1-Difluoropentyl)-1H-1,2,4-Triazol-1-yl]methyl][1,1' -Biphenyl]-2-yl]-1H-Tetrazol;
5-[4'-[[5-(1-Butenyl)-3-Propyl-1H-1,2,4-Triazol-1-yl]methyl][1,1' -Biphenyl]-2-yl]-1H-Tetrazol;
5-[4'-[[5-(1 -Butenyl)-3-Butyl-1H-1,2,4-Triazol-1-yl]methyl][1,1'-Biphenyl]-2-yl]-1H-Tetrazol;
5-[4'-[[5-(1-Butenyl)-3-Isopentyl-1H-1,2,4-Triazol-1-yl)methyl] [1,1' -Biphenyl]-2-yl]-1H-Tetrazol;
5-[4'-[[5-(1-Butenyl)-3-(1,1-Dimethoxybutyl)-1H-1,2,4-Triazol-1-yl]methyl][1,1'-Biphenyl]-2-yl]-1H-Tetrazol;
5-[4'-[[5-(1-Butenyl)-3-(1,1-Difluorobutyl)-1H-1,2,4-Triazol-1-yl]methyl] [1,1'-Biphenyl]-2-yl]-1H-Tetrazol;
5-[4'-[[5-(2-Butenyl)-3-(1,1-Difluorobutyl)-1H-1,2,4-Triazol-1-yl]methyl] [1,1'-Biphenyl]-2-yl]-1H-Tetrazol;
5-[4'-[[5-(3-Butenyl)-3-(1,1-Difluorobutyl)-1H-1,2,4-Triazol-1-yl]methyl] [1,1'-Biphenyl]-2-yl]-1H-Tetrazol;
5-[4'-[[5-(1 -Butynyl)-3-(1,1-Difluorobutyl)-1H-1,2,4-Triazol-1-yl]methyl][1,1'-Biphenyl]-2-yl]-1H-Tetrazol;
5-[4'-[[5-(2-Butynyl)-3-(1,1-Difluorobutyl)-1H-1,2,4-Triazol-1-yl]methyl] [1,1'-Biphenyl]-2-yl]-1H-Tetrazol;
5-[4'-[[5-(3-Butynyl)-3-(1,1-Difluorobutyl)-1H-1,2,4-Triazol-1-yl]methyl][1,1'-Biphenyl]-2-yl]-1H-Tetrazol;
5-[4'-[[3,5-di(1-Butenyl)-1H-1,2,4-Triazol-1-yl]methyl][1,1-Biphenyl]-2-yl]-1H-Tetrazol;
5-[4'-[[3,5-di(2-Butenyl)-1H-1,2,4-Triazol-1-yl]methyl] [1,1'-Biphenyl]-2-yl]-1 H-Tetrazol;
5-[4'-[[3,5-di(3-Butenyl)-1H-1,2,4-Triazol-1-yl]methyl][1,1'-Biphenyl]-2-yl]-1H-Tetrazol;
5-[4'-[[3,5-di(1-Butynyl)-1H-1,2,4-Triazol-1-yl)methyl] [1,1'-Biphenyl]-2-yl]-1H-Tetrazol;
5-[4'-[[3,5-di(2-Butynyl)-1H-1,2,4-Triazol-1-yl]methyl] [1,1'-Biphenyl]-2-yl]-1H-Tetrazol;
5-[4'-[[3,5-di(3-Butynyl)-1H-1,2,4-Triazol-1-yl]methyl][1,1'-Biphenyl]-2-yl]-1H-Tetrazol;

5. Verbindung von Anspruch 1, ausgewählt aus Verbindungen und deren pharmazeutisch akzeptablen Salzen aus der Gruppe von Verbindungen, bestehend aus
4'-[(3,5-Dibutyl-1H-1,2,4-Triazol-1-yl)methyl] [1,1'-bi-Phenyl]-2-Karbonsäure;
4'-[(3-Ethyl-5-Butyl-1H-1,2,4-Triazol-1-yl)methyl] [1,1'-Biphenyl]-2-Karbonsäure;
4'-[(3-Butyl-5-Ethyl-1H-1,2,4-Triazol-1-yl)methyl] [1,1'-Biphenyl]-2-Karbonsäure;
4'-[(5-Butyl-1H-1,2,4-Triazol-1-yl)methyl] [1,1'-Biphenyl]-2-Karbonsäure;
4'-[(3-Butyl-1H-1,2,4-Triazol-1-yl)methyl] [1,1'-Biphenyl]-2-Karbonsäure;

6. Verfahren zur Herstellung einer pharmazeutischen Zusammensetzung, gekennzeichnet durch die Hinzumischung einer therapeutisch wirksamen Menge einer Angiotensin-II-antagonistischen Verbindung und eines pharmazeutisch akzeptablen Trägers oder Verdünnungsmittels, wobei die antagonistische Verbindung ausgewählt ist aus einer Familie von Verbindungen gemäß einem der Ansprüche 1 - 5 oder dem Amid der Verbindungen, worin R³ ist COOH, oder deren Salzen.

7. Benutzung einer Verbindung gemäß einem der Ansprüche 1 - 5 oder des Amids der Verbindungen, wobei R³ ist COOH, oder deren Salzen zur Herstellung eines Medikaments zur Behandlung einer Kreislauferkrankung.

8. Benutzung gemäß Anspruch 7, wobei die Erkrankung eine kardiovaskuläre Erkrankung ist.

9. Benutzung gemäß Anspruch 8, wobei die kardiovaskuläre Erkrankung Hypertonie ist.

10. Benutzung gemäß Anspruch 8, wobei die kardiovaskuläre Erkrankung kongestive Herzinsuffizienz ist.

11. Benutzung einer Verbindung gemäß einem der Ansprüche 1 - 5 oder des Amids der Verbindungen, wobei R³ ist COOH, oder deren Salzen zur Herstellung eines Medikaments zur Behandlung von Glaukoma.

12. Verbindung, die Methyl-4'-[(3,5-Dibutyl-1H-1,2,4-Triazol-1-yl)methyl] [1,1'-Biphenyl-2-Karboxylat oder 4'-[(3,5-Dibutyl-1 H-1,2,4-Triazol-1-yl)methyl][1,1'-Biphenyl]-2-Karbonsäure, Hydrazid ist.

## Revendications (Revendications pour l'(les) Etat(s) contractant(s) suivant(s): AT, BE, CH, LI, DE, DK, FR, GB, IT, LU, NL, SE)

1. Composé de formule I : 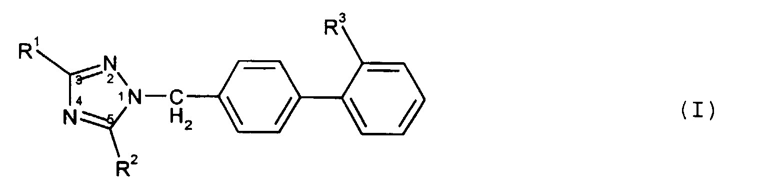 dans laquelle chacun de R¹ et R² est indépendamment choisi parmi l'hydrogène et les radicaux alkyle en C₁ à C₂₀, phényle, phényle substitué par un radical alkyle en C₁ à C₂₀, cycloalkyle en C₃ à C₁₀, cycloalkyle en C₃ à C₁₀ attaché à ou substitué par un radical alkyle en C₁ à C₂₀, alcoxy en C₁ à C₁₀, difluoroalkyle en C₁ à C₁₀ substitué par un radical phényle, alcényle en C₂ à C₂₀, alcynyle en C₂ à C₂₀, (alkyl en C₁ à C₂₀) carbonyle ; alkyle en C₁ à C₂₀ à substitution difluoro ; hydroxyalkyle en C₁ à C₁₀, alkyle en C₁ à C₂₀ substitué par un ou plusieurs groupes alcoxy dont les fragments alkyle ont de 1 à 10 atomes de carbone ;
dans laquelle R³ est un fragment acide choisi parmi CO₂H, CONHNHSO₂CF₃, 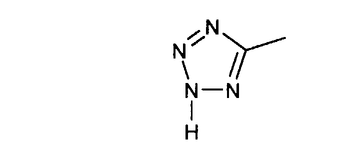 et les sels desdits fragments acides ;
ou un tautomère ou un sel acceptable en pharmacie d'un tel composé.

2. Composé selon la revendication 1, dans lequel R³ est choisi parmi COOH ou un radical tétrazolyle, ou un tautomère ou un sel acceptable en pharmacie d'un tel composé.

3. Composé selon la revendication 2, choisi parmi les composés, et les sels acceptables en pharmacie de tels composés, du groupe de composés constitué par les suivants :
2-[(trifluorométhyl)sulfonyl]hydrazide de l'acide 4'-[(3,5-dibutyl-1H-1,2,4-triazole-1-yl)méthyl]-[1,1'-biphényl]-2-carboxylique ;
acide 4'-[(5-butyl-3-propyl-1H-1,2,4-triazole-1-yl)méthyl]-[1,1'-biphényl]-2-carboxylique ;
acide 4'-[(3,5-dibutyl-1H-1,2,4-triazole-1-yl)méthyl]-[1,1'-biphényl]-2-carboxylique ;
acide 4'-[(5-butyl-3-isopentyl-1H-1,2,4-triazole-1-yl)méthyl]-[1,1'-biphényl]-2-carboxylique ;
acide 4'-[[5-butyl-3-(1,1-diméthoxybutyl)-1H-1,2,4-triazole-1-yl]méthyl]-[1,1'-biphényl]-2-carboxylique ;
acide 4'-[[5-butyl-3-(1-oxobutyl)-1H-1,2,4-triazole-1-yl]méthyl]-[1,1'-biphényl]-2-carboxylique ;
acide 4'-[[5-butyl-3-(1-oxopentyle)-1H-1,2,4-triazole-1-yl]méthyl]-[1,1'-biphényl]-2-carboxylique ;
acide 4'-[[5-butyl-3-(1,1-difluoroéthyl)-1H-1,2,4-triazole-1-yl]méthyl]-[1,1'-biphényl]-2-carboxylique ;
acide 4'-[[5-butyl-3-(1,1-difluoropropyl)-1H-1,2,4-triazole-1-yl]méthyl]-[1,1'-biphényl]-2-carboxylique ;
acide 4'-[[5-butyl-3-(1,1-difluorobutyl)-1H-1,2,4-triazole-1-yl]méthyl]-[1,1'-biphényl]-2-carboxylique ;
acide 4'-[[3-butyl-5-(1,1-difluorobutyl)-1H-1,2,4-triazole-1-yl]méthyl]-[1,1'-biphényl]-2-carboxylique ;
acide 4'-[[5-butyl-3-(1,1-difluoropentyl)-1H-1,2,4-triazole-1-yl]méthyl]-[1,1'-biphényl]-2-carboxylique ;
acide 4'-[(3,5-dipropyl-1H-1,2,4-triazole-1-yl)méthyl]-[1,1'-biphényl]-2-carboxylique ;
acide 4'-[(3,5-isopropyl-1H-1,2,4-triazole-1-yl)méthyl]-[1,1'-biphényl]-2-carboxylique ;
acide 4'-[(5-propyl-3-butyl-1H-1,2,4-triazole-1-yl)méthyl]-[1,1'-biphényl]-2-carboxylique ;
acide 4'-[[5-propyl-3-(1,1-difluoroéthyl)-1H-1,2,4-triazole-1-yl)méthyl]-[1,1'-biphényl]-2-carboxylique ;
acide 4'-[[5-propyl-3-(1,1-difluoropropyl)-1H-1,2,4-triazole-1-yl]méthyl]-[1,1'-biphényl]-2-carboxylique ;
acide 4'-[[5-propyl-3-(1,1-difluorobutyl)-1H-1,2,4-triazole-1-yl]méthyl]-[1,1'-biphényl]-2-carboxylique ;
acide 4'-[[5-propyl-3-(1,1-difluoropentyl)-1H-1,2,4-triazole-1-yl]méthyl]-[1,1'-biphényl]-2-carboxylique ;
acide 4'-[[5-éthyl-3-(1,1-difluoropentyl)-1H-1,2,4-triazole-1-yl]méthyl]-[1,1'-biphényl]-2-carboxylique ;
acide 4'-[(3,5-di-sec-butyl-1H-1,2,4-triazole-1-yl)méthyl] -[1,1'-biphényl]-2-carboxylique ;
acide 4'-[[5-sec-butyl-3-(1,1-difluorobutyl)-1H-1,2,4-triazole-1-yl]méthyl]-[1,1'-biphényl]-2-carboxylique ;
acide 4'-[(3,5-diisobutyl-1H-1,2,4-triazole-1-yl)méthyl]-[1,1'-biphényl]-2-carboxylique ;
acide 4'-[[5-isobutyl-3-(1,1-difluorobutyl)-1H-1,2,4-triazole-1-yl]méthyl]-[1,1'-biphényl]-2-carboxylique ;
acide 4'-[(3,5-di-tert-butyl-1H-1,2,4-triazole-1-yl)méthyl]-[1,1'-biphényl]-2-carboxylique ;
acide 4'-[[5-tert-butyl-3-(1,1-difluorobutyl)-1H-1,2,4-triazole-1-yl]méthyl]-[1,1'-biphényl]-2-carboxylique ;
acide 4'-[(3,5-dipentyl-1H-1,2,4-triazole-1-yl)méthyl]-[1,1'-biphényl]-2-carboxylique ;
acide 4'-[[5-pentyl-3-(1,1-difluorobutyl)-1H-1,2,4-triazole-1-yl]méthyl]-[1,1'-biphényl]-2-carboxylique ;
acide 4'-[(3,5-diisopentyl-1H-1,2,4-triazole-1-yl)méthyl]-[1,1'-biphényl]-2-carboxylique ;
acide 4'-[[5-isopentyl-3-(1,1-difluorobutyl)-1H-1,2,4-triazole-1-yl]méthyl]-[1,1'-biphényl]-2-carboxylique ;
acide 4'-[[5-(1-butényl)-3-(1,1-difluorobutyl)-1H-1,2,4-triazole-1-yl]méthyl]-[1,1'-biphényl]-2-carboxylique ;
acide 4'-[[5-(2-butényl)-3-(1,1-difluorobutyl)-1H-1,2,4-triazole-1-yl]méthyl]-[1,1'-biphényl]-2-carboxylique ;
acide 4'-[[5-(3-butényl)-3-(1,1-difluorobutyl)-1H-1,2,4-triazole-1-yl]méthyl]-[1,1'-biphényl]-2-carboxylique ;
acide 4'-[[5-(1-butynyl)-3-(1,1-difluorobutyl)-1H-1,2,4-triazole-1-yl]méthyl]-[1,1'-biphényl]-2-carboxylique ;
acide 4'-[[5-(2-butynyl)-3-(1,1-difluorobutyl)-1H-1,2,4-triazole-1-yl]méthyl]-[1,1'-biphényl]-2-carboxylique ;
acide 4'-[[5-(3-butynyl)-3-(1,1-difluorobutyl)-1H-1,2,4-triazole-1-yl]méthyl]-[1,1'-biphényl]-2-carboxylique ;
acide 4'-[(3,5-diéthoxy-1H-1,2,4-triazole-1-yl)méthyl]-[1,1'-biphényl]-2-carboxylique ;
acide 4'-[(3,5-dipropoxy-1H-1,2,4-triazole-1-yl)méthyl]-[1,1'-biphényl]-2-carboxylique ;
acide 4'-[(3,5-diisopropoxy-1H-1,2,4-triazole-1-yl)méthyl]-[1,1'-biphényl]-2-carboxylique ;
acide 4'-[(3,5-dibutoxy-1H-1,2,4-triazole-1-yl)méthyl]-[1,1'-biphényl]-2-carboxylique ;
acide 4'-[[3,5-di(1-butényl)-1H-1,2,4-triazole-1-yl]méthyl]-[1,1'-biphényl]-2-carboxylique ;
acide 4'-[[3,5-di(2-butényl)-1H-1,2,4-triazole-1-yl]méthyl]-[1,1'-biphényl]-2-carboxylique ;
acide 4'-[[3,5-di(3-butényl)-1H-1,2,4-triazole-1-yl]méthyl]-[1,1'-biphényl]-2-carboxylique ;
acide 4'-[[3,5-di(1-butynyl)-1H-1,2,4-triazole-1-yl]méthyl]-[1,1'-biphényl]-2-carboxylique ;
acide 4'-[[3,5-di(2-butynyl)-1H-1,2,4-triazole-1-yl]méthyl]-[1,1'-biphényl]-2-carboxylique ;
acide 4'-[[3,5-di(3-butynyl)-1H-1,2,4-triazole-1-yl]méthyl]-[1,1'-biphényl]-2-carboxylique.

4. Composé selon la revendication 1, choisi parmi les composés, et les sels acceptables en pharmacie de tels composés, du groupe de composés constitué par les suivants :
5-[4'-[(5-butyl-3-propyl-1H-1,2,4-triazole-1-yl)méthyl]-[1,1'-biphényl]-2-yl]-1H-tétrazole ;
5-[4'-[(3,5-dibutyl-1H-1,2,4-triazole-1-yl)méthyl]-[1,1'-biphényl]-2-yl]-1H-tétrazole ;
5-[4'-[(5-butyl-3-isopentyl-1H-1,2,4-triazole-1-yl)méthyl]-[1,1'-biphényl]-2-yl]-1H-tétrazole ;
5-[4'-[[5-butyl-3-(1,1-diméthoxybutyl)-1H-1,2,4-triazole-1-yl]méthyl]-[1,1'-biphényl]-2-yl]-1H-tétrazole ;
5-[4'-[[5-butyl-3-(1-oxobutyl)-1H-1,2,4-triazole-1-yl]méthyl]-[1,1'-biphényl]-2-yl]-1H-tétrazole ;
5-[4'-[[5-butyl-3-(1,1-difluoroéthyl)-1H-1,2,4-triazole-1-yl]méthyl]-[1,1'-biphényl]-2-yl]-1H-tétrazole ;
5-[4'-[[5-butyl-3-(1,1-difluoropropyl)-1H-1,2,4-triazole-1-yl]méthyl]-[1,1'-biphényl]-2-yl]-1H-tétrazole ;
5-[4'-[[5-butyl-3-(1,1-difluorobutyl)-1H-1,2,4-triazole-1-yl]méthyl]-[1,1'-biphényl]-2-yl]-1H-tétrazole ;
5- [4'-[[3-butyl-5-(1,1-difluorobutyl)-1H-1,2,4-triazole-1-yl]méthyl]-[1,1'-biphényl]-2-yl]-1H-tétrazole ;
5-[4'-[[5-butyl-3-(1,1-difluoropentyl)-1H-1,2,4-triazole-1-yl]méthyl]-[1,1'-biphényl]-2-yl]-1H-tétrazole ;
5-[4'-[(3,5-dipropyl-1H-1,2,4-triazole-1-yl)méthyl]-[1,1'-biphényl]-2-yl]-1H-tétrazole;
5-[4'-[(3-butyl-5-propyl-1H-1,2,4-triazole-1-yl)méthyl]-[1,1'-biphényl]-2-yl]-1H-tétrazole ;
5-[4'-[(5-butyl-3-isopentyl-1H-1,2,4-triazole-1-yl)méthyl]-[1,1'-biphényl]-2-yl]-1H-tétrazole ;
5-[4'-[(3-phényl-5-butyl-1H-1,2,4-triazole-1-yl)méthyl]-[1,1'-biphényl]-2-yl]-1H-tétrazole ;
5-[4'-[(5-butyl-3-phényléthyl-1H-1,2,4-triazole-1-yl)méthyl]-[1,1'-biphényl]-2-yl]-1H-tétrazole ;
5-[4'-[(5-butyl-3-éthyl-1H-1,2,4-triazole-1-yl)méthyl]-[1,1'-biphényl]-2-yl]-1H-tétrazole ;
5-[4'-[(3-butyl-5-éthyl-1H-1,2,4-triazole-1-yl)méthyl]-[1,1'-biphényl]-2-yl]-1H-tétrazole ;
ester 1,1-diméthyléthylique de glutamine ;
5-[4'-[[5-butyl-3-(2-cyclohexyléthyl)-1H-1,2,4-triazole-1-yl]méthyl]-[1,1'-biphényl]-2-yl]-1H-tétrazole ;
5-[4'-[[5-butyl-3-(phénylméthyl)-1H-1,2,4-triazole-1-yl]méthyl]-[1,1'-biphényl]-2-yl]-1H-tétrazole ;
5-[4'-[(5-butyl-3-cyclohexyl-1H-1,2,4-triazole-1-yl)méthyl]-[1,1'-biphényl]-2-yl]-1H-tétrazole ;
5-[4'-[(3-cyclohexylméthyl-5-butyl-1H-1,2,4-triazole-1-yl)méthyl]-[1,1'-biphényl]-2-yl]-1H-tétrazole ;
5-[4'-[(3-butyl-5-cyclohexyl-1H-1,2,4-triazole-1-yl)méthyl]-[1,1'-biphényl]-2-yl]-1H-tétrazole ;
5-[4'-[(3,5-isopropyl-1H-1,2,4-triazole-1-yl)méthyl]-[1,1'-biphényl]-2-yl]-1H-tétrazole ;
5-[4'-[(5-propyl-3-isopentyl-1H-1,2,4-triazole-1-yl)méthyl]-[1,1'-biphényl]-2-yl]-1H-tétrazole ;
5- [4'-[[5-propyl-3-(1,1-diméthoxybutyl)-1H-1,2,4-triazole-1-yl]méthyl]-[1,1'-biphényl]-2-yl]-1H-tétrazole ;
5- [4'-[[5-propyl-3-(1-oxobutyl)-1H-1,2,4-triazole-1-yl]méthyl]-[1,1'-biphényl]-2-yl]-1H-tétrazole ;
5-[4'-[[5-propyl-3-(1,1-difluoroéthyl)-1H-1,2,4-triazole-1-yl]méthyl]-[1,1'-biphényl]-2-yl]-1H-tétrazole ;
5-[4'-[[5-propyl-3-(1,1-difluoropropyl)-1H-1,2,4-triazole-1-yl]méthyl]-[1,1'-biphényl]-2-yl]-1H-tétrazole;
5-[4'-[[5-propyl-3-(1,1-difluorobutyl)-1H-1,2,4-triazole-1-yl]méthyl]-[1,1'-biphényl]-2-yl]-1H-tétrazole ;
5-[4'-[[5-propyl-3-(1,1-difluoropentyl)-1H-1,2,4-triazole-1-yl]méthyl]-[1,1'-biphényl]-2-yl]-1H-tétrazole;
5-[4'-[[5-éthyl-3-(1,1-difluorobutyl)-1H-1,2,4-triazole-1-yl]méthyl]-[1,1'-biphényl]-2-yl]-1H-tétrazole ;
5-[4'-[(5-sec-butyl-3-propyl-1H-1,2,4-triazole-1-yl)méthyl]-[1,1'-biphényl]-2-yl]-1H-tétrazole ;
5-[4'-[(5-sec-butyl-3-butyl-1H-1,2,4-triazole-1-yl)méthyl]-[1,1'-biphényl]-2-yl]-1H-tétrazole ;
5-[4'-[(3,5-di-sec-butyl-1H-1,2,4-triazole-1-yl)méthyl]-[1,1'-biphényl]-2-yl]-1H-tétrazole ;
5-[4'-[(5-sec-butyl-3-isopentyl-1H-1,2,4-triazole-1-yl)méthyl]-[1,1'-biphényl]-2-yl]-1H-tétrazole ;
5-[4'-[[5-sec-butyl-3-(1,1-difluoroéthyl)-1H-1,2,4-triazole-1-yl]méthyl]-[1,1'-biphényl]-2-yl]-1H-tétrazole ;
5-[4'-[[5-sec-butyl-3-(1,1-difluoropropyl)-1H-1,2,4-triazole-1-yl]méthyl]-[1,1'-biphényl]-2-yl]-1H-tétrazole ;
5-[4'-[[5-sec-butyl-3-(1,1-difluorobutyl)-1H-1,2,4-triazole-1-yl]méthyl]-[1,1'-biphényl]-2-yl]-1H-tétrazole ;
5-[4'-[[5-sec-butyl-3-(1,1-difluoropentyl)-1H-1,2,4-triazole-1-yl]méthyl]-[1,1'-biphényl]-2-yl]-1H-tétrazole ;
5-[4'-[(5-isobutyl-3-propyl-1H-1,2,4-triazole-1-yl)méthyl]-[1,1'-biphényl]-2-yl]-1H-tétrazole ;
5-[4'-[(5-isobutyl-3-butyl-1H-1,2,4-triazole-1-yl)méthyl]-[1,1'-biphényl]-2-yl]-1H-tétrazole ;
5-[4'-[(3,5-diisobutyl-1H-1,2,4-triazole-1-yl)méthyl]-[1,1'-biphényl]-2-yl]-1H-tétrazole ;
5-[4'-[(5-isobutyl-3-isopentyl-1H-1,2,4-triazole-1-yl)méthyl]-[1,1'-biphényl]-2-yl]-1H-tétrazole ;
5-[4'-[[5-isobutyl-3-(1,1-difluoroéthyl)-1H-1,2,4-triazole-1-yl]méthyl]-[1,1'-biphényl]-2-yl]-1H-tétrazole ;
5-[4'-[[5-isobutyl-3-(1,1-difluoropropyl)-1H-1,2,4-triazole-1-yl]méthyl]-[1,1'-biphényl]-2-yl]-1H-tétrazole ;
5-[4'-[[5-isobutyl-3-(1,1-difluorobutyl)-1H-1,2,4-triazole-1-yl]méthyl]-[1,1'-biphényl]-2-yl]-1H-tétrazole ;
5-[4'-[[5-isobutyl-3-(1,1-difluoropentyl)-1H-1,2,4-triazole-1-yl]méthyl]-[1,1'-biphényl]-2-yl]-1H-tétrazole ;
5-[4'-[(5-tert-butyl-3-propyl-1H-1,2,4-triazole-1-yl)méthyl]-[1,1'-biphényl]-2-yl]-1H-tétrazole ;
5-[4'-[(5-tert-butyl-3-butyl-1H-1,2,4-triazole-1-yl)méthyl]-[1,1'-biphényl]-2-yl]-1H-tétrazole ;
5-[4'-[(3,5-di-tert-butyl-1H-1,2,4-triazole-1-yl)méthyl]-[1,1'-biphényl]-2-yl]-1H-tétrazole ;
5-[4'-[(5-tert-butyl-3-isopentyl-1H-1,2,4-triazole-1-yl)méthyl]-[1,1'-biphényl]-2-yl]-1H-tétrazole ;
5-[4'-[[5-tert-butyl-3-(1,1-difluoroéthyl)-1H-1,2,4-triazole-1-yl]méthyl]-[1,1'-biphényl]-2-yl]-1H-tétrazole ;
5-[4'-[[5-tert-butyl-3-(1,1-difluoropropyl)-1H-1,2,4-triazole-1-yl]méthyl]-[1,1'-biphényl]-2-yl]-1H-tétrazole ;
5-[4'-[[5-tert-butyl-3-(1,1-difluorobutyl)-1H-1,2,4-triazole-1-yl]méthyl]-[1,1'-biphényl]-2-yl]-1H-tétrazole ;
5-[4'-[[5-tert-butyl-3-(1,1-difluoropentyl)-1H-1,2,4-triazole-1-yl]méthyl]-[1,1'-biphényl]-2-yl]-1H-tétrazole ;
5-[4'-[(5-pentyl-3-propyl-1H-1,2,4-triazole-1-yl)méthyl]-[1,1'-biphényl]-2-yl]-1H-tétrazole ;
5-[4'-[(5-pentyl-3-butyl-1H-1,2,4-triazole-1-yl)méthyl]-[1,1'-biphényl]-2-yl]-1H-tétrazole ;
5-[4'-[(3,5-dipentyl-1H-1,2,4-triazole-1-yl)méthyl]-[1,1'-biphényl]-2-yl]-1H-tétrazole ;
5-[4'-[(5-pentyl-3-isopentyl-1H-1,2,4-triazole-1-yl)méthyl]-[1,1'-biphényl]-2-yl]-1H-tétrazole ;
5-[4'-[[5-pentyl-3-(1,1-difluoroéthyl)-1H-1,2,4-triazole-1-yl]méthyl]-[1,1'-biphényl]-2-yl]-1H-tétrazole ;
5-[4'-[[5-pentyl-3-(1,1-difluoropropyl)-1H-1,2,4-triazole-1-yl]méthyl]-[1,1'-biphényl]-2-yl]-1H-tétrazole ;
5-[4'-[[5-pentyl-3-(1,1-difluorobutyl)-1H-1,2,4-triazole-1-yl]méthyl]-[1,1'-biphényl]-2-yl]-1H-tétrazole ;
5-[4'-[[5-pentyl-3-(1,1-difluoropentyl)-1H-1,2,4-triazole-1-yl]méthyl]-[1,1'-biphényl]-2-yl]-1H-tétrazole ;
5-[4'-[(5-isopentyl-3-éthyl-1H-1,2,4-triazole-1-yl)méthyl]-[1,1'-biphényl]-2-yl]-1H-tétrazole ;
5-[4'-[(5-isopentyl-3-propyl-1H-1,2,4-triazole-1-yl)méthyl]-[1,1'-biphényl]-2-yl]-1H-tétrazole ;
5-[4'-[(5-isopentyl-3-butyl-1H-1,2,4-triazole-1-yl)méthyl]-[1,1'-biphényl]-2-yl]-1H-tétrazole ;
5-[4'-[(3,5-diisopentyl-1H-1,2,4-triazole-1-yl)méthyl]-[1,1'-biphényl]-2-yl]-1H-tétrazole ;
5-[4'-[[5-isopentyl-3-(1,1-difluoroéthyl)-1H-1,2,4-triazole-1-yl]méthyl]-[1,1'-biphényl]-2-yl]-1H-tétrazole ;
5-[4'-[[5-isopentyl-3-(1,1-difluoropropyl)-1H-1,2,4-triazole-1-yl]méthyl]-[1,1'-biphényl]-2-yl]-1H-tétrazole ;
5-[4'-[[5-isopentyl-3-(1,1-difluorobutyl)-1H-1,2,4-triazole-1-yl]méthyl]-[1,1'-biphényl]-2-yl]-1H-tétrazole ;
5-[4'-[[5-isopentyl-3-(1,1-difluoropentyl)-1H-1,2,4-triazole-1-yl]méthyl]-[1,1'-biphényl]-2-yl]-1H-tétrazole ;
5-[4'-[[5-(1-butényl)-3-propyl-1H-1,2,4-triazole-1-yl]méthyl]-[1,1'-biphényl]-2-yl]-1H-tétrazole;
5-[4'-[[5-(1-butényl)-3-butyl-1H-1,2,4-triazole-1-yl]méthyl]-[1,1'-biphényl]-2-yl]-1H-tétrazole ;
5-[4'-[[5-(1-butényl)-3-isopentyl-1H-1,2,4-triazole-1-yl]méthyl]-[1,1'-biphényl]-2-yl]-1H-tétrazole ;
5-[4'-[[5-(1-butényl)-3-(1,1-diméthoxybutyl)-1H-1,2,4-triazole-1-yl]méthyl]-[1,1'-biphényl]-2-yl]-1H-tétrazole ;
5-[4'-[[5-(1-butényl)-3-(1,1-difluorobutyl)-1H-1,2,4-triazole-1-yl]méthyl]-[1,1'-biphényl]-2-yl]-1H-tétrazole ;
5-[4'-[[5-(2-butényl)-3-(1,1-difluorobutyl)-1H-1,2,4-triazole-1-yl]méthyl]-[1,1'-biphényl]-2-yl]-1H-tétrazole ;
5-[4'-[[5-(3-butényl)-3-(1,1-difluorobutyl)-1H-1,2,4-triazole-1-yl]méthyl]-[1,1'-biphényl]-2-yl]-1H-tétrazole ;
5-[4'-[[5-(1-butynyl)-3-(1,1-difluorobutyl)-1H-1,2,4-triazole-1-yl]méthyl]-[1,1'-biphényl]-2-yl]-1H-tétrazole ;
5-[4'-[[5-(2-butynyl)-3-(1,1-difluorobutyl)-1H-1,2,4-triazole-1-yl]méthyl]-[1,1'-biphényl]-2-yl]-1H-tétrazole ;
5-[4'-[[5-(3-butynyl)-3-(1,1-difluorobutyl)-1H-1,2,4-triazole-1-yl]méthyl]-[1,1'-biphényl]-2-yl]-1H-tétrazole ;
5-[4'-[[3,5-di(1-butényl)-1H-1,2,4-triazole-1-yl]méthyl]-[1,1'-biphényl]-2-yl]-1H-tétrazole ;
5-[4'-[[3,5-di (2-butényl)-1H-1,2,4-triazole-1-yl]méthyl]-[1,1'-biphényl]-2-yl]-1H-tétrazole ;
5-[4'-[[3,5-di (3-butényl)-1H-1,2,4-triazole-1-yl]méthyl]-[1,1'-biphényl]-2-yl]-1H-tétrazole ;
5-[4'-[[3,5-di(1-butynyl)-1H-1,2,4-triazole-1-yl]méthyl]-[1,1'-biphényl]-2-yl]-1H-tétrazole ;
5- [4'-[[3,5-di (2-butynyl)-1H-1,2,4-triazole-1-yl]méthyl]-[1,1'-biphényl]-2-yl]-1H-tétrazole; et
5-[4'-[[3,5-di (3-butynyl)-1H-1,2,4-triazole-1-yl]méthyl]-[1,1'-biphényl]-2-yl]-1H-tétrazole.

5. Composé selon la revendication 1, choisi parmi les composés, et les sels acceptables en pharmacie de tels composés, du groupe de composés constitué par les suivants :
acide 4'-[(3,5-dibutyl-1H-1,2,4-triazole-1-yl)méthyl]-[1,1'-biphényl]-2-carboxylique ;
acide 4'-[(3-éthyl-5-butyl-1H-1,2,4-triazole-1-yl)méthyl]-[1,1'-biphényl]-2-carboxylique ;
acide 4'-[(3-butyl-5-éthyl-1H-1,2,4-triazole-1-yl)méthyl]-[1,1'-biphényl]-2-carboxylique ;
acide 4'-[(5-butyl-1H-1,2,4-triazole-1-yl)méthyl]-[1,1'-biphényl]-2-carboxylique;
acide 4'-[(3-butyl-1H-1,2,4-triazole-1-yl)méthyl]-[1,1'-biphényl]-2-carboxylique.

6. Composition pharmaceutique comprenant une quantité, efficace du point de vue thérapeutique, d'un composé antagoniste de l'angiotensine II et un véhicule ou diluant acceptable en pharmacie, ledit composé antagoniste étant choisi parmi une famille de composés selon l'une quelconque des revendications 1 à 5 ou les amides de composés dans lesquels R³ = COOH, ou les sels de tels composés.

7. Utilisation d'un composé selon l'une quelconque des revendications 1 à 5 ou d'amides de composés dans lesquels R³ = COOH ou de sels de tels composés, pour préparer un médicament destiné au traitement d'un trouble circulatoire.

8. Utilisation selon la revendication 7, dans laquelle ledit trouble est un trouble cardio-vasculaire.

9. Utilisation selon la revendication 8, dans laquelle ledit trouble cardio-vasculaire est l'hypertension.

10. Utilisation selon la revendication 8, dans laquelle ledit trouble cardio-vasculaire est l'insuffisance cardiaque congestive.

11. Utilisation d'un composé selon l'une quelconque des revendications 1 à 5, ou des amides de composés dans lesquels R³ = COOH, ou des sels de tels composés, pour préparer un médicament destiné à traiter un glaucome.

12. Composé, qui est le 4'-[(3,5-dibutyl-1H-1,2,4-triazole-1-yl)méthyl]-[1,1'-biphényl]-2-carboxylate de méthyle ou l'hydrazide de l'acide 4'-[(3,5-dibutyl-1H-1,2,4-triazole-1-yl)méthyl]-[1,1'-biphényl]-2-carboxylique.

## Revendications (Revendications pour l'(les) Etat(s) contractant(s) suivant(s): ES)

1. Procédé pour préparer un composé de formule I : 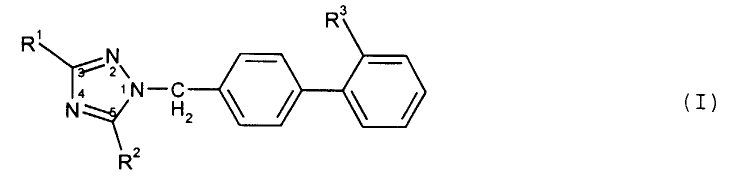 dans laquelle chacun de R¹ et R² est indépendamment choisi parmi l'hydrogène et les radicaux alkyle en C₁ à C₂₀, phényle, phényle substitué par un radical alkyle en C₁ à C₂₀, cycloalkyle en C₃ à C₁₀, cycloalkyle en C₃ à C₁₀ attaché à ou substitué par un radical alkyle en C₁ à C₂₀, alcoxy en C₁ à C₁₀, difluoroalkyle en C₁ à C₁₀ substitué par un radical phényle, alcényle en C₂ à C₂₀, alcynyle en C₂ à C₂₀, (alkyl en C₁ à C₂₀)carbonyle ; alkyle en C₁ à C₂₀ à substitution difluoro ; hydroxyalkyle en C₁ à C₁₀, alkyle en C₁ à C₂₀ substitué par un ou plusieurs groupes alcoxy dont les fragments alkyle ont de 1 à 10 atomes de carbone ;
dans laquelle R³ est un fragment acide choisi parmi CO₂H, CONHNHSO₂CF₃, 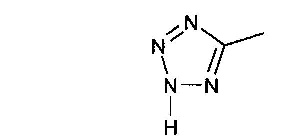 et les sels desdits fragments acides ;
ou un tautomère ou un sel acceptable en pharmacie d'un tel composé,
caractérisé en ce que
A) un composé de formule 7 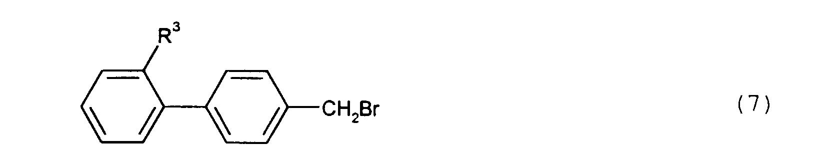 dans laquelle R³ est tel que défini ci-dessus, sous une forme protégée, est mis à réagir avec un composé 1 : 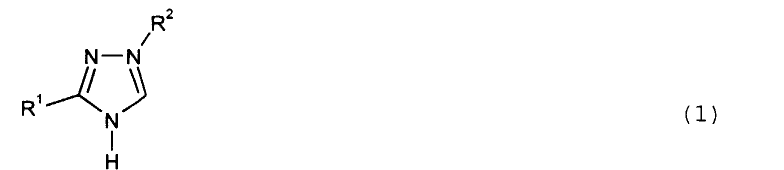 dans lequel R¹ et R² sont définis comme indiqué ci-dessus, dans des conditions basiques, pour donner les régioisomères couplés respectifs qui peuvent être convertis par un traitement acide ou basique pour donner les composés dans lesquels R³ est un tétrazole ou un acide carboxylique ou un de leurs dérivés ;
ou bien
B) un composé de formule 15 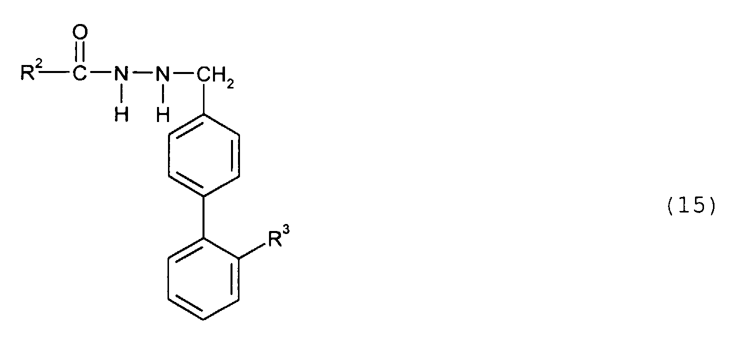 dans laquelle R² et R³ sont définis comme indiqué ci-dessus, est mis à réagir avec un imidate 2 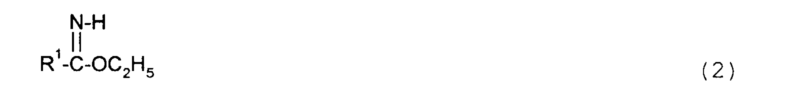 dans lequel R¹ est tel que défini ci-dessus, dans des conditions appropriées pour donner, lors d'une cyclisation thermique, les produits finales correspondants,
ou bien
C) une imidazone convenablement substituée 17 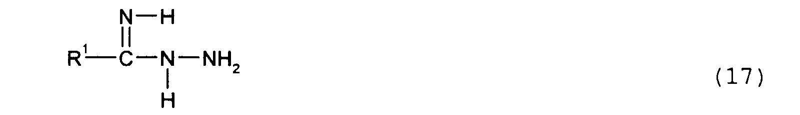 dans laquelle R¹ est tel que défini ci-dessus,
est mise à réagir dans des conditions appropriées avec un réactif d'alkylation 7 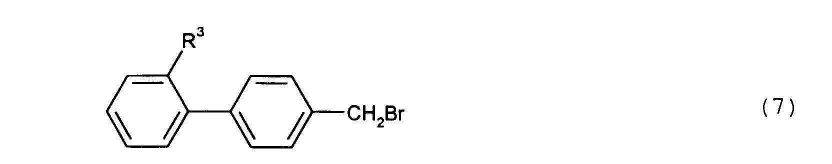 dans lequel R³ est tel que défini ci-dessus,
pour donner un intermédiaire qui est ensuite cyclisé en présence de chaleur et d'un ortho-ester approprié pour donner les composés finals souhaités,
qui peuvent éventuellement être transformés d'une manière connue en leurs amides, esters, sels ou sels acceptables en pharmacie de ceux-ci respectifs.

2. Procédé selon la revendication 1, dans lequel R³ du composé préparé est choisi parmi COOH ou un radical tétrazolyle, ou un tautomère ou un sel acceptable en pharmacie d'un tel composé.

3. Procédé selon la revendication 2, dans lequel le composé préparé est choisi parmi les composés, et les sels acceptables en pharmacie de tels composés, du groupe de composés constitué par les suivants :
2-[(trifluorométhyl)sulfonyl]hydrazide de l'acide 4'-[(3,5-dibutyl-1H-1,2,4-triazole-1-yl)méthyl]-[1,1'-biphényl]-2-carboxylique ;
acide 4'-[(5-butyl-3-propyl-1H-1,2,4-triazole-1-yl)méthyl]-[1,1'-biphényl]-2-carboxylique ;
acide 4'-[(3,5-dibutyl-1H-1,2,4-triazole-1-yl)méthyl]-[1,1'-biphényl]-2-carboxylique ;
acide 4'-[(5-butyl-3-isopentyl-1H-1,2,4-triazole-1-yl)méthyl]-[1,1'-biphényl]-2-carboxylique ;
acide 4'-[[5-butyl-3-(1,1-diméthoxybutyl)-1H-1,2,4-triazole-1-yl]méthyl]-[1,1'-biphényl]-2-carboxylique ;
acide 4'-[[5-butyl-3-(1-oxobutyl)-1H-1,2,4-triazole-1-yl]méthyl]-[1,1'-biphényl]-2-carboxylique ;
acide 4'-[[5-butyl-3-(1-oxopentyle)-1H-1,2,4-triazole-1-yl]méthyl]-[1,1'-biphényl]-2-carboxylique ;
acide 4'-[[5-butyl-3-(1,1-difluoroéthyl)-1H-1,2,4-triazole-1-yl]méthyl]-[1,1'-biphényl]-2-carboxylique ;
acide 4'-[[5-butyl-3-(1,1-difluoropropyl)-1H-1,2,4-triazole-1-yl]méthyl]-[1,1'-biphényl]-2-carboxylique ;
acide 4'-[[5-butyl-3-(1,1-difluorobutyl)-1H-1,2,4-triazole-1-yl]méthyl]-[1,1'-biphényl]-2-carboxylique ;
acide 4'-[[3-butyl-5-(1,1-difluorobutyl)-1H-1,2,4-triazole-1-yl]méthyl]-[1,1'-biphényl]-2-carboxylique ;
acide 4'-[[5-butyl-3-(1,1-difluoropentyl)-1H-1,2,4-triazole-1-yl]méthyl]-[1,1'-biphényl]-2-carboxylique ;
acide 4'-[(3,5-dipropyl-1H-1,2,4-triazole-1-yl)méthyl]-[1,1'-biphényl]-2-carboxylique ;
acide 4'-[(3,5-isopropyl-1H-1,2,4-triazole-1-yl)méthyl]- -biphényl]-2-carboxylique ;
acide 4'-[(5-propyl-3-butyl-1H-1,2,4-triazole-1-yl)méthyl]-[1,1'-biphényl]-2-carboxylique ;
acide 4'-[[5-propyl-3-(1,1-difluoroéthyl)-1H-1,2,4-triazole-l-yl)méthyl]-[1,1'-biphényl]-2-carboxylique ;
acide 4'-[[5-propyl-3-(1,1-difluoropropyl)-1H-1,2,4-triazole-1-yl]méthyl]-[1,1'-biphényl]-2-carboxylique ;
acide 4'-[[5-propyl-3-(1,1-difluorobutyl)-1H-1,2,4-triazole-1-yl]méthyl]-[1,1'-biphényl]-2-carboxylique ;
acide 4'-[[5-propyl-3-(1,1-difluoropentyl)-1H-1,2,4-triazole-1-yl]méthyl]-[1,1'-biphényl]-2-carboxylique ;
acide 4'-[[5-éthyl-3-(1,1-difluoropentyl)-1H-1,2,4-triazole-1-yl]méthyl]-[1,1'-biphényl]-2-carboxylique ;
acide 4'-[(3,5-di-sec-butyl-1H-1,2,4-triazole-1-yl)méthyl]-[1,1'-biphényl]-2-carboxylique ;
acide 4'-[[5-sec-butyl-3-(1,1-difluorobutyl)-1H-1,2,4-triazole-1-yl]méthyl]-[1,1'-biphényl]-2-carboxylique ;
acide 4'-[(3,5-diisobutyl-1H-1,2,4-triazole-1-yl)méthyl]-[1,1'-biphényl]-2-carboxylique ;
acide 4'-[[5-isobutyl-3-(1,1-difluorobutyl)-1H-1,2,4-triazole-1-yl]méthyl]-[1,1'-biphényl]-2-carboxylique ;
acide 4'-[(3,5-di-tert-butyl-1H-1,2,4-triazole-1-yl)méthyl]-[1,1'-biphényl]-2-carboxylique ;
acide 4'-[[5-tert-butyl-3-(1,1-difluorobutyl)-1H-1,2,4-triazole-1-yl]méthyl]-[1,1'-biphényl]-2-carboxylique ;
acide 4'-[(3,5-dipentyl-1H-1,2,4-triazole-1-yl)méthyl]-[1,1'-biphényl]-2-carboxylique ;
acide 4'-[[5-pentyl-3-(1,1-difluorobutyl)-1H-1,2,4-triazole-1-yl]méthyl]-[1,1'-biphényl]-2-carboxylique ;
acide 4'-[(3,5-diisopentyl-1H-1,2,4-triazole-1-yl)méthyl]-[1,1'-biphényl]-2-carboxylique ;
acide 4'-[[5-isopentyl-3-(1,1-difluorobutyl)-1H-1,2,4-triazole-1-yl]méthyl]-[1,1'-biphényl]-2-carboxylique ;
acide 4'-[[5-(1-butényl)-3-(1,1-difluorobutyl)-1H-1,2,4-triazole-1-yl]méthyl]-[1,1'-biphényl]-2-carboxylique ;
acide 4'-[[5-(2-butényl)-3-(1,1-difluorobutyl)-1H-1,2,4-triazole-1-yl]méthyl]-[1,1'-biphényl]-2-carboxylique ;
acide 4'-[[5-(3-butényl)-3-(1,1-difluorobutyl)-1H-1,2,4-triazole-1-yl]méthyl]-[1,1'-biphényl]-2-carboxylique ;
acide 4'-[[5-(1-butynyl)-3-(1,1-difluorobutyl)-1H-1,2,4-triazole-1-yl]méthyl]-[1,1'-biphényl]-2-carboxylique ;
acide 4'-[[5-(2-butynyl)-3-(1,1-difluorobutyl)-1H-1,2,4-triazole-1-yl]méthyl]-[1,1'-biphényl]-2-carboxylique ;
acide 4'-[[5-(3-butynyl)-3-(1,1-difluorobutyl)-1H-1,2,4-triazole-1-yl]méthyl]-[1,1'-biphényl]-2-carboxylique ;
acide 4'-[(3,5-diéthoxy-1H-1,2,4-triazole-1-yl)méthyl]-[1,1'-biphényl]-2-carboxylique ;
acide 4'-[(3,5-dipropoxy-1H-1,2,4-triazole-1-yl)méthyl]-[1,1'-biphényl]-2-carboxylique ;
acide 4'-[(3,5-diisopropoxy-1H-1,2,4-triazole-1-yl)méthyl]-[1,1'-biphényl]-2-carboxylique ;
acide 4'-[(3,5-dibutoxy-1H-1,2,4-triazole-1-yl)méthyl]-[1,1'-biphényl]-2-carboxylique ;
acide 4'-[[3,5-di(1-butényl)-1H-1,2,4-triazole-1-yl]méthyl]-[1,1'-biphényl]-2-carboxylique ;
acide 4'-[[3,5-di(2-butényl)-1H-1,2,4-triazole-1-yl]méthyl]-[1,1'-biphényl]-2-carboxylique ;
acide 4'-[[3,5-di(3-butényl)-1H-1,2,4-triazole-1-yl]méthyl]-[1,1'-biphényl]-2-carboxylique ;
acide 4'-[[3,5-di(1-butynyl)-1H-1,2,4-triazole-1-yl]méthyl]-[1,1'-biphényl]-2-carboxylique ;
acide 4'-[[3,5-di(2-butynyl)-1H-1,2,4-triazole-1-yl]méthyl]-[1,1'-biphényl]-2-carboxylique ;
acide 4'-[[3,5-di(3-butynyl)-1H-1,2,4-triazole-1-yl]méthyl]-[1,1'-biphényl]-2-carboxylique.

4. Procédé selon la revendication 1, dans lequel le composé préparé est choisi parmi les composés, et les sels acceptables en pharmacie de tels composés, du groupe de composés constitué par les suivants :
5-[4'-[(5-butyl-3-propyl-1H-1,2,4-triazole-1-yl)méthyl]-[1,1'-biphényl]-2-yl]-1H-tétrazole ;
5-[4'-[(3,5-dibutyl-1H-1,2,4-triazole-1-yl)méthyl]-[1,1'-biphényl]-2-yl]-1H-tétrazole ;
5-[4'-[(5-butyl-3-isopentyl-1H-1,2,4-triazole-1-yl)méthyl]-[1,1'-biphényl]-2-yl]-1H-tétrazole ;
5-[4'-[[5-butyl-3-(1,1-diméthoxybutyl)-1H-1,2,4-triazole-1-yl]méthyl]-[1,1'-biphényl]-2-yl]-1H-tétrazole ;
5-[4'-[[5-butyl-3-(1-oxobutyl)-1H-1,2,4-triazole-1-yl]méthyl]-[1,1'-biphényl]-2-yl]-1H-tétrazole ;
5-[4'-[[5-butyl-3-(1,1-difluoroéthyl)-1H-1,2,4-triazole-1-yl]méthyl]-[1,1'-biphényl]-2-yl]-1H-tétrazole ;
5-[4'-[[5-butyl-3-(1,1-difluoropropyl)-1H-1,2,4-triazole-1-yl]méthyl]-[1,1'-biphényl]-2-yl]-1H-tétrazole ;
5-[4'-[[5-butyl-3-(1,1-difluorobutyl)-1H-1,2,4-triazole-1-yl]méthyl]-[1,1'-biphényl]-2-yl]-1H-tétrazole ;
5-[4'-[[3-butyl-5-(1,1-difluorobutyl)-1H-1,2,4-triazole-1-yl]méthyl]-[1,1'-biphényl]-2-yl]-1H-tétrazole ;
5-[4'-[[5-butyl-3-(1,1-difluoropentyl)-1H-1,2,4-triazole-1-yl]méthyl]-[1,1'-biphényl]-2-yl]-1H-tétrazole ;
5-[4'-[(3,5-dipropyl-1H-1,2,4-triazole-1-yl)méthyl]-[1,1'-biphényl]-2-yl]-1H-tétrazole ;
5-[4'-[(3-butyl-5-propyl-1H-1,2,4-triazole-1-yl)méthyl]-[1,1'-biphényl]-2-yl]-1H-tétrazole ;
5-[4'-[(5-butyl-3-isopentyl-1H-1,2,4-triazole-1-yl)méthyl]-[1,1'-biphényl]-2-yl]-1H-tétrazole ;
5-[4'-[(3-phényl-5-butyl-1H-1,2,4-triazole-1-yl)méthyl]-[1,1'-biphényl]-2-yl]-1H-tétrazole ;
5-[4'-[(5-butyl-3-phényléthyl-1H-1,2,4-triazole-1-yl)méthyl]-[1,1'-biphényl]-2-yl]-1H-tétrazole ;
5-[4'-[(5-butyl-3-éthyl-1H-1,2,4-triazole-1-yl)méthyl]-[1,1'-biphényl]-2-yl]-1H-tétrazole ;
5-[4'-[(3-butyl-5-éthyl-1H-1,2,4-triazole-1-yl)méthyl]-[1,1'-biphényl]-2-yl]-1H-tétrazole ;
ester 1,1-diméthyléthylique de glutamine ;
5-[4'-[[5-butyl-3-(2-cyclohexyléthyl)-1H-1,2,4-triazole-1-yl]méthyl]-[1,1'-biphényl]-2-yl]-1H-tétrazole ;
5-[4'-[[5-butyl-3-(phénylméthyl)-1H-1,2,4-triazole-1-yl]méthyl]-[1,1'-biphényl]-2-yl]-1H-tétrazole ;
5-[4'-[(5-butyl-3-cyclohexyl-1H-1,2,4-triazole-1-yl)méthyl]-[1,1'-biphényl]-2-yl]-1H-tétrazole ;
5-[4'-[(3-cyclohexylméthyl-5-butyl-1H-1,2,4-triazole-1-yl)méthyl]-[1,1'-biphényl]-2-yl]-1H-tétrazole ;
5-[4'-[(3-butyl-5-cyclohexyl-1H-1,2,4-triazole-1-yl)méthyl]-[1,1'-biphényl]-2-yl]-1H-tétrazole ;
5-[4'-[(3,5-isopropyl-1H-1,2,4-triazole-1-yl)méthyl]-[1,1'-biphényl]-2-yl]-1H-tétrazole ;
5-[4'-[(5-propyl-3-isopentyl-1H-1,2,4-triazole-1-yl)méthyl]-[1,1'-biphényl]-2-yl]-1H-tétrazole ;
5-[4'-[[5-propyl-3-(1,1-diméthoxybutyl)-1H-1,2,4-triazole-1-yl]méthyl]-[1,1'-biphényl]-2-yl]-1H-tétrazole ;
5-[4'-[[5-propyl-3-(1-oxobutyl)-1H-1,2,4-triazole-1-yl]méthyl]-[1,1'-biphényl]-2-yl]-1H-tétrazole ;
5-[4'-[[5-propyl-3-(1,1-difluoroéthyl)-1H-1,2,4-triazole-1-yl]méthyl]-[1,1'-biphényl]-2-yl]-1H-tétrazole ;
5-[4'-[[5-propyl-3-(1,1-difluoropropyl)-1H-1,2,4-triazole-1-yl]méthyl]-[1,1'-biphényl]-2-yl]-1H-tétrazole ;
5-[4'-[[5-propyl-3-(1,1-difluorobutyl)-1H-1,2,4-triazole-1-yl]méthyl]-[1,1'-biphényl]-2-yl]-1H-tétrazole;
5-[4'-[[5-propyl-3-(1,1-difluoropentyl)-1H-1,2,4-triazole-1-yl]méthyl]-[1,1'-biphényl]-2-yl]-1H-tétrazole ;
5-[4'-[[5-éthyl-3-(1,1-difluorobutyl)-1H-1,2,4-triazole-1-yl]méthyl]-[1,1'-biphényl]-2-yl]-1H-tétrazole ;
5-[4'-[(5-sec-butyl-3-propyl-1H-1,2,4-triazole-1-yl)méthyl]-[1,1'-biphényl]-2-yl]-1H-tétrazole ;
5-[4'-[(5-sec-butyl-3-butyl-1H-1,2,4-triazole-1-yl)méthyl]-[1,1'-biphényl]-2-yl]-1H-tétrazole ;
5-[4'-[(3,5-di-sec-butyl-1H-1,2,4-triazole-1-yl)méthyl]-[1,1'-biphényl]-2-yl]-1H-tétrazole;
5-[4'-[(5-sec-butyl-3-isopentyl-1H-1,2,4-triazole-1-yl)méthyl]-[1,1'-biphényl]-2-yl]-1H-tétrazole ;
5-[4'-[[5-sec-butyl-3-(1,1-difluoroéthyl)-1H-1,2,4-triazole-1-yl]méthyl]-[1,1'-biphényl]-2-yl]-1H-tétrazole ;
5-[4'-[[5-sec-butyl-3-(1,1-difluoropropyl)-1H-1,2,4-triazole-1-yl]méthyl]-[1,1'-biphényl]-2-yl]-1H-tétrazole ;
5-[4'-[[5-sec-butyl-3-(1,1-difluorobutyl)-1H-1,2,4-triazole-1-yl]méthyl]-[1,1'-biphényl]-2-yl]-1H-tétrazole ;
5-[4'-[[5-sec-butyl-3-(1,1-difluoropentyl)-1H-1,2,4-triazole-1-yl]méthyl]-[1,1'-biphényl]-2-yl]-1H-tétrazole ;
5-[4'-[(5-isobutyl-3-propyl-1H-1,2,4-triazole-1-yl)méthyl]-[1,1'-biphényl]-2-yl]-1H-tétrazole ;
5-[4'-[(5-isobutyl-3-butyl-1H-1,2,4-triazole-1-yl)méthyl]-[1,1'-biphényl]-2-yl]-1H-tétrazole ;
5-[4'-[(3,5-diisobutyl-1H-1,2,4-triazole-1-yl)méthyl]-[1,1'-biphényl]-2-yl]-1H-tétrazole ;
5-[4'-[(5-isobutyl-3-isopentyl-1H-1,2,4-triazole-1-yl)méthyl]-[1,1'-biphényl]-2-yl]-1H-tétrazole ;
5-[4'-[[5-isobutyl-3-(1,1-difluoroéthyl)-1H-1,2,4-triazole-1-yl]méthyl]-[1,1'-biphényl]-2-yl]-1H-tétrazole ;
5-[4'-[[5-isobutyl-3-(1,1-difluoropropyl)-1H-1,2,4-triazole-1-yl]méthyl]-[1,1'-biphényl]-2-yl]-1H-tétrazole ;
5-[4'-[[5-isobutyl-3-(1,1-difluorobutyl)-1H-1,2,4-triazole-1-yl]méthyl]-[1,1'-biphényl]-2-yl]-1H-tétrazole ;
5-[4'-[[5-isobutyl-3-(1,1-difluoropentyl)-1H-1,2,4-triazole-1-yl]méthyl]-[1,1'-biphényl]-2-yl]-1H-tétrazole
5-[4'-[(5-tert-butyl-3-propyl-1H-1,2,4-triazole-1-yl)méthyl]-[1,1'-biphényl]-2-yl]-1H-tétrazole ;
5-[4'-[(5-tert-butyl-3-butyl-1H-1,2,4-triazole-1-yl)méthyl]-[1,1'-biphényl]-2-yl]-1H-tétrazole ;
5-[4'-[(3,5-di-tert-butyl-1H-1,2,4-triazole-1-yl)méthyl]-[1,1'-biphényl]-2-yl]-1H-tétrazole ;
5-[4'-[(5-tert-butyl-3-isopentyl-1H-1,2,4-triazole-1-yl)méthyl]-[1,1'-biphényl]-2-yl]-1H-tétrazole ;
5-[4'-[[5-tert-butyl-3-(1,1-difluoroéthyl)-1H-1,2,4-triazole-1-yl]méthyl]-[1,1'-biphényl]-2-yl]-1H-tétrazole ;
5-[4'-[[5-tert-butyl-3-(1,1-difluoropropyl)-1H-1,2,4-triazole-1-yl]méthyl]-[1,1'-biphényl]-2-yl]-1H-tétrazole ;
5-[4'-[[5-tert-butyl-3-(1,1-difluorobutyl)-1H-1,2,4-triazole-1-yl]méthyl]-[1,1'-biphényl]-2-yl]-1H-tétrazole ;
5-[4'-[[5-tert-butyl-3-(1,1-difluoropentyl)-1H-1,2,4-triazole-1-yl]méthyl]-[1,1'-biphényl]-2-yl]-1H-tétrazole ;
5-[4'-[(5-pentyl-3-propyl-1H-1,2,4-triazole-1-yl)méthyl]-[1,1'-biphényl]-2-yl]-1H-tétrazole ;
5-[4'-[(5-pentyl-3-butyl-1H-1,2,4-triazole-1-yl)méthyl]-[1,1'-biphényl]-2-yl]-1H-tétrazole ;
5-[4'-[(3,5-dipentyl-1H-1,2,4-triazole-1-yl)méthyl]-[1,1'-biphényl]-2-yl]-1H-tétrazole ;
5-[4'-[(5-pentyl-3-isopentyl-1H-1,2,4-triazole-1-yl)méthyl]-[1,1'-biphényl]-2-yl]-1H-tétrazole ;
5-[4'-[[5-pentyl-3-(1,1-difluoroéthyl)-1H-1,2,4-triazole-1-yl]méthyl]-[1,1'-biphényl]-2-yl]-1H-tétrazole ;
5-[4'-[[5-pentyl-3-(1,1-difluoropropyl)-1H-1,2,4-triazole-1-yl]méthyl]-[1,1'-biphényl]-2-yl]-1H-tétrazole ;
5-[4'-[[5-pentyl-3-(1,1-difluorobutyl)-1H-1,2,4-triazole-1-yl]méthyl]-[1,1'-biphényl]-2-yl]-1H-tétrazole ;
5-[4'-[[5-pentyl-3-(1,1-difluoropentyl)-1H-1,2,4-triazole-1-yl]méthyl]-[1,1'-biphényl]-2-yl]-1H-tétrazole ;
5-[4'-[(5-isopentyl-3-éthyl-1H-1,2,4-triazole-1-yl)méthyl]-[1,1'-biphényl]-2-yl]-1H-tétrazole ;
5-[4'-[(5-isopentyl-3-propyl-1H-1,2,4-triazole-1-yl)méthyl]-[1,1'-biphényl]-2-yl]-1H-tétrazole ;
5-[4'-[(5-isopentyl-3-butyl-1H-1,2,4-triazole-1-yl)méthyl]-[1,1'-biphényl]-2-yl]-1H-tétrazole ;
5-[4'-[(3,5-diisopentyl-1H-1,2,4-triazole-1-yl)méthyl]-[1,1'-biphényl]-2-yl]-1H-tétrazole ;
5-[4'-[[5-isopentyl-3-(1,1-difluoroéthyl)-1H-1,2,4-triazole-1-yl]méthyl]-[1,1'-biphényl]-2-yl]-1H-tétrazole ;
5-[4'-[[5-isopentyl-3-(1,1-difluoropropyl)-1H-1,2,4-triazole-1-yl]méthyl]-[1,1'-biphényl]-2-yl]-1H-tétrazole ;
5-[4'-[[5-isopentyl-3-(1,1-difluorobutyl)-1H-1,2,4-triazole-1-yl]méthyl]-[1,1'-biphényl]-2-yl]-1H-tétrazole ;
5-[4'-[[5-isopentyl-3-(1,1-difluoropentyl)-1H-1,2,4-triazole-1-yl]méthyl]-[1,1'-biphényl]-2-yl]-1H-tétrazole ;
5-[4'-[[5-(1-butényl)-3-propyl-1H-1,2,4-triazole-1-yl]méthyl]-[1,1'-biphényl]-2-yl]-1H-tétrazole ;
5-[4'-[[5-(1-butényl)-3-butyl-1H-1,2,4-triazole-1-yl]méthyl]-[1,1'-biphényl]-2-yl]-1H-tétrazole ;
5-[4'-[[5-(1-butényl)-3-isopentyl-1H-1,2,4-triazole-1-yl]méthyl]-[1,1'-biphényl]-2-yl]-1H-tétrazole ;
5-[4'-[[5-(1-butényl)-3-(1,1-diméthoxybutyl)-1H-1,2,4-triazole-1-yl]méthyl]-[1,1'-biphényl]-2-yl]-1H-tétrazole ;
5-[4'-[[5-(1-butényl)-3-(1,1-difluorobutyl)-1H-1,2,4-triazole-1-yl]méthyl]-[1,1'-biphényl]-2-yl]-1H-tétrazole ;
5-[4'-[[5-(2-butényl)-3-(1,1-difluorobutyl)-1H-1,2,4-triazole-1-yl]méthyl]-[1,1'-biphényl]-2-yl]-1H-tétrazole ;
5-[4'-[[5-(3-butényl)-3-(1,1-difluorobutyl)-1H-1,2,4-triazole-1-yl]méthyl]-[1,1'-biphényl]-2-yl]-1H-tétrazole ;
5-[4'-[[5-(1-butynyl)-3-(1,1-difluorobutyl)-1H-1,2,4-triazole-1-yl]méthyl]-[1,1'-biphényl]-2-yl]-1H-tétrazole ;
5-[4'-[[5-(2-butynyl)-3-(1,1-difluorobutyl)-1H-1,2,4-triazole-1-yl]méthyl]-[1,1'-biphényl]-2-yl]-1H-tétrazole ;
5-[4'-[[5-(3-butynyl)-3-(1,1-difluorobutyl)-1H-1,2,4-triazole-1-yl]méthyl]-[1,1'-biphényl]-2-yl]-1H-tétrazole ;
5-[4'-[[3,5-di(1-butényl)-1H-1,2,4-triazole-1-yl]méthyl]-[1,1'-biphényl]-2-yl]-1H-tétrazole ;
5-[4'-[[3,5-di (2-butényl)-1H-1,2,4-triazole-1-yl]méthyl]-[1,1'-biphényl]-2-yl]-1H-tétrazole ;
5-[4'-[[3,5-di (3-butényl)-1H-1,2,4-triazole-1-yl]méthyl]-[1,1'-biphényl]-2-yl]-1H-tétrazole ;
5-[4'-[[3,5-di(1-butynyl)-1H-1,2,4-triazole-1-yl]méthyl]-[1,1'-biphényl]-2-yl]-1H-tétrazole ;
5-[4'-[[3,5-di (2-butynyl)-1H-1,2,4-triazole-1-yl]méthyl]-[1,1'-biphényl]-2-yl]-1H-tétrazole ; et
5-[4'-[[3,5-di (3-butynyl)-1H-1,2,4-triazole-1-yl]méthyl]-[1,1'-biphényl]-2-yl]-1H-tétrazole.

5. Procédé selon la revendication 1, dans lequel le composé préparé est choisi parmi les composés, et les sels acceptables en pharmacie de tels composés, du groupe de composés constitué par les suivants :
acide 4'-[(3,5-dibutyl-1H-1,2,4-triazole-1-yl)méthyl]-[1,1'-biphényl]-2-carboxylique ;
acide 4'-[(3-éthyl-5-butyl-1H-1,2,4-triazole-1-yl)méthyl]-[1,1'-biphényl]-2-carboxylique ;
acide 4'-[(3-butyl-5-éthyl-1H-1,2,4-triazole-1-yl)méthyl]-[1,1'-biphényl]-2-carboxylique ;
acide 4'-[(5-butyl-1H-1,2,4-triazole-1-yl)méthyl]-[1,1'-biphényl]-2-carboxylique ;
acide 4'-[(3-butyl-1H-1,2,4-triazole-1-yl)méthyl]-[1,1'-biphényl]-2-carboxylique.

6. Procédé pour préparer une composition pharmaceutique, caractérisé par le mélange d'une quantité, efficace du point de vue thérapeutique, d'un composé antagoniste de l'angiotensine II et d'un véhicule ou diluant acceptable en pharmacie, ledit composé antagoniste étant choisi parmi une famille de composés selon l'une quelconque des revendications 1 à 5 ou les amides de composés dans lesquels R³ = COOH, ou les sels de tels composés.

7. Utilisation d'un composé selon l'une quelconque des revendications 1 à 5 ou d'amides de composés dans lesquels R³ = COOH ou de sels de tels composés, pour préparer un médicament destiné au traitement d'un trouble circulatoire.

8. Utilisation selon la revendication 7, dans laquelle ledit trouble est un trouble cardio-vasculaire.

9. Utilisation selon la revendication 8, dans laquelle ledit trouble cardio-vasculaire est l'hypertension.

10. Utilisation selon la revendication 8, dans laquelle ledit trouble cardio-vasculaire est l'insuffisance cardiaque congestive.

11. Utilisation d'un composé selon l'une quelconque des revendications 1 à 5, ou des amides de composés dans lesquels R³ = COOH, ou des sels de tels composés, pour préparer un médicament destiné à traiter un glaucome.

12. Procédé pour préparer un composé de formule 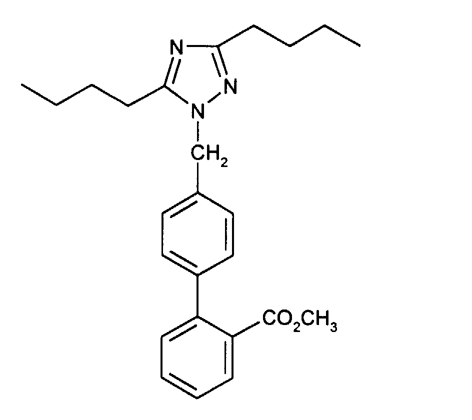 caractérisé en ce que du 3,5-dibutyl-1H-1,2,4-triazole est ajouté à du 4-bromométhyl-2'-méthoxycarbonyl-biphényle en présence d'hydrure de sodium.

13. Procédé pour préparer un composé de formule : 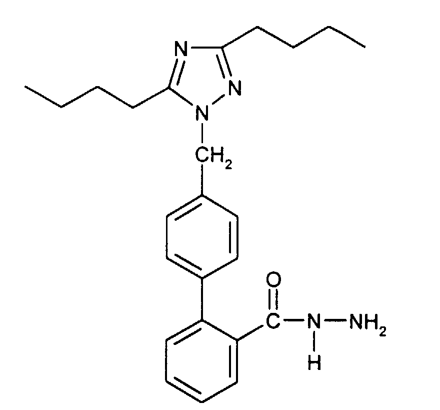 caractérisé en ce que le composé tel que préparé conformément à la revendication 12 est mis à réagir avec de l'hydrazine anhydre.

## Revendications (Revendications pour l'(les) Etat(s) contractant(s) suivant(s): GR)

1. Composé de formule I : 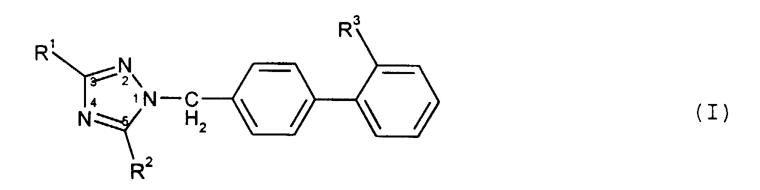 dans laquelle chacun de R¹ et R² est indépendamment choisi parmi l'hydrogène et les radicaux alkyle en C₁ à C₂₀, phényle, phényle substitué par un radical alkyle en C₁ à C₂₀, cycloalkyle en C₃ à C₁₀, cycloalkyle en C₃ à C₁₀ attaché à ou substitué par un radical alkyle en C₁ à C₂₀, alcoxy en C₁ à C₁₀, difluoroalkyle en C₁ à C₁₀ substitué par un radical phényle, alcényle en C₂ à C₂₀, alcynyle en C₂ à C₂₀, (alkyl en C₁ à C₂₀)carbonyle ; alkyle en C₁ à C₂₀ à substitution difluoro ; hydroxyalkyle en C₁ à C₁₀, alkyle en C₁ à C₂₀ substitué par un ou plusieurs groupes alcoxy dont les fragments alkyle ont de 1 à 10 atomes de carbone ;
dans laquelle R³ est un fragment acide choisi parmi CO₂H, CONHNHSO₂CF₃, 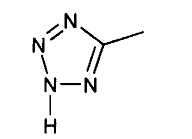 et les sels desdits fragments acides ;
ou un tautomère ou un sel acceptable en pharmacie d'un tel composé.

2. Composé selon la revendication 1, dans lequel R³ est choisi parmi COOH ou un radical tétrazolyle, ou un tautomère ou un sel acceptable en pharmacie d'un tel composé.

3. Composé selon la revendication 2, choisi parmi les composés, et les sels acceptables en pharmacie de tels composés, du groupe de composés constitué par les suivants :
2-[(trifluorométhyl)sulfonyl]hydrazide de l'acide 4'-[(3,5-dibutyl-1H-1,2,4-triazole-1-yl)méthyl]-[1,1'-biphényl]-2-carboxylique ;
acide 4'-[(5-butyl-3-propyl-1H-1,2,4-triazole-1-yl)méthyl]-[1,1'-biphényl]-2-carboxylique ;
acide 4'-[(3,5-dibutyl-1H-1,2,4-triazole-1-yl)méthyl]-[1,1'-biphényl]-2-carboxylique ;
acide 4'-[(5-butyl-3-isopentyl-1H-1,2,4-triazole-1-yl)méthyl]-[1,1'-biphényl]-2-carboxylique ;
acide 4'-[[5-butyl-3-(1,1-diméthoxybutyl)-1H-1,2,4-triazole-1-yl]méthyl]-[1,1'-biphényl]-2-carboxylique ;
acide 4'-[[5-butyl-3-(1-oxobutyl)-1H-1,2,4-triazole-1-yl]méthyl]-[1,1'-biphényl]-2-carboxylique ;
acide 4'-[[5-butyl-3-(1-oxopentyle)-1H-1,2,4-triazole-1-yl]méthyl]-[1,1'-biphényl]-2-carboxylique ;
acide 4'-[[5-butyl-3-(1,1-difluoroéthyl)-1H-1,2,4-triazole-1-yl]méthyl]-[1,1'-biphényl]-2-carboxylique ;
acide 4'-[[5-butyl-3-(1,1-difluoropropyl)-1H-1,2,4-triazole-1-yl]méthyl]-[1,1'-biphényl]-2-carboxylique ;
acide 4'-[[5-butyl-3-(1,1-difluorobutyl)-1H-1,2,4-triazole-1-yl]méthyl]-[1,1'-biphényl]-2-carboxylique ;
acide 4'-[[3-butyl-5-(1,1-difluorobutyl)-1H-1,2,4-triazole-1-yl]méthyl]-[1,1'-biphényl]-2-carboxylique ;
acide 4'-[[5-butyl-3-(1,1-difluoropentyl)-1H-1,2,4-triazole-1-yl]méthyl]-[1,1'-biphényl]-2-carboxylique ;
acide 4'-[(3,5-dipropyl-1H-1,2,4-triazole-1-yl)méthyl]-[1,1'-biphényl]-2-carboxylique ;
acide 4'-[(3,5-isopropyl-1H-1,2,4-triazole-1-yl)méthyl]-[1,1'-biphényl]-2-carboxylique ;
acide 4'-[(5-propyl-3-butyl-1H-1,2,4-triazole-1-yl)méthyl]-[1,1'-biphényl]-2-carboxylique ;
acide 4'-[[5-propyl-3-(1,1-difluoroéthyl)-1H-1,2,4-triazole-1-yl)méthyl]-[1,1'-biphényl]-2-carboxylique;
acide 4'-[[5-propyl-3-(1,1-difluoropropyl)-1H-1,2,4-triazole-1-yl]méthyl]-[1,1'-biphényl]-2-carboxylique;
acide 4'-[[5-propyl-3-(1,1-difluorobutyl)-1H-1,2,4-triazole-1-yl]méthyl]-[1,1'-biphényl]-2-carboxylique ;
acide 4'-[[5-propyl-3-(1,1-difluoropentyl)-1H-1,2,4-triazole-1-yl]méthyl]-[1,1'-biphényl]-2-carboxylique ;
acide 4'-[[5-éthyl-3-(1,1-difluoropentyl)-1H-1,2,4-triazole-1-yl]méthyl]-[1,1'-biphényl]-2-carboxylique ;
acide 4'-[(3,5-di-sec-butyl-1H-1,2,4-triazole-1-yl)méthyl]-[1,1'-biphényl]-2-carboxylique ;
acide 4'-[[5-sec-butyl-3-(1,1-difluorobutyl)-1H-1,2,4-triazole-1-yl]méthyl]-[1,1'-biphényl]-2-carboxylique ;
acide 4'-[(3,5-diisobutyl-1H-1,2,4-triazole-1-yl)méthyl]-[1,1'-biphényl]-2-carboxylique ;
acide 4'-[[5-isobutyl-3-(1,1-difluorobutyl)-1H-1,2,4-triazole-1-yl]méthyl]-[1,1'-biphényl]-2-carboxylique ;
acide 4'-[(3,5-di-tert-butyl-1H-1,2,4-triazole-1-yl)méthyl]-[1,1'-biphényl]-2-carboxylique ;
acide 4'-[[5-tert-butyl-3-(1,1-difluorobutyl)-1H-1,2,4-triazole-1-yl]méthyl]-[1,1'-biphényl]-2-carboxylique ;
acide 4'-[(3,5-dipentyl-1H-1,2,4-triazole-1-yl)méthyl]-[1,1'-biphényl]-2-carboxylique ;
acide 4'-[[5-pentyl-3-(1,1-difluorobutyl)-1H-1,2,4-triazole-1-yl]méthyl]-[1,1'-biphényl]-2-carboxylique ;
acide 4'-[(3,5-diisopentyl-1H-1,2,4-triazole-1-yl)méthyl]-[1,1'-biphényl]-2-carboxylique ;
acide 4'-[[5-isopentyl-3-(1,1-difluorobutyl)-1H-1,2,4-triazole-1-yl]méthyl]-[1,1'-biphényl]-2-carboxylique ;
acide 4'-[[5-(1-butényl)-3-(1,1-difluorobutyl)-1H-1,2,4-triazole-1-yl]méthyl]-[1,1'-biphényl]-2-carboxylique ;
acide 4'-[[5-(2-butényl)-3-(1,1-difluorobutyl)-1H-1,2,4-triazole-1-yl]méthyl]-[1,1'-biphényl]-2-carboxylique ;
acide 4'-[[5-(3-butényl)-3-(1,1-difluorobutyl)-1H-1,2,4-triazole-1-yl]méthyl]-[1,1'-biphényl]-2-carboxylique ;
acide 4'-[[5-(1-butynyl)-3-(1,1-difluorobutyl)-1H-1,2,4-triazole-1-yl]méthyl]-[1,1'-biphényl]-2-carboxylique ;
acide 4'-[[5-(2-butynyl)-3-(1,1-difluorobutyl)-1H-1,2,4-triazole-l-yl]méthyl]-[1,1'-biphényl]-2-carboxylique ;
acide 4'-[[5-(3-butynyl)-3-(1,1-difluorobutyl)-1H-1,2,4-triazole-1-yl]méthyl]-[1,1'-biphényl]-2-carboxylique ;
acide 4'-[(3,5-diéthoxy-1H-1,2,4-triazole-1-yl)méthyl]-[1,1'-biphényl]-2-carboxylique ;
acide 4'-[(3,5-dipropoxy-1H-1,2,4-triazole-1-yl)méthyl]-[1,1'-biphényl]-2-carboxylique ;
acide 4'-[(3,5-diisopropoxy-1H-1,2,4-triazole-1-yl)méthyl]-[1,1'-biphényl]-2-carboxylique ;
acide 4'-[(3,5-dibutoxy-1H-1,2,4-triazole-1-yl)méthyl]-[1,1'-biphényl]-2-carboxylique ;
acide 4'-[[3,5-di(l-butényl)-1H-1,2,4-triazole-1-yl]méthyl]-[1,1'-biphényl]-2-carboxylique ;
acide 4'-[[3,5-di(2-butényl)-1H-1,2,4-triazole-1-yl]méthyl]-[1,1'-biphényl]-2-carboxylique ;
acide 4'-[[3,5-di(3-butényl)-1H-1,2,4-triazole-1-yl]méthyl]-[1,1'-biphényl]-2-carboxylique ;
acide 4'-[[3,5-di(1-butynyl)-1H-1,2,4-triazole-1-yl]méthyl]-[1,1'-biphényl]-2-carboxylique ;
acide 4'-[[3,5-di(2-butynyl)-1H-1,2,4-triazole-1-yl]méthyl]-[1,1'-biphényl]-2-carboxylique ;
acide 4'-[[3,5-di(3-butynyl)-1H-1,2,4-triazole-1-yl]méthyl]-[1,1'-biphényl]-2-carboxylique.

4. Composé selon la revendication 1, choisi parmi les composés, et les sels acceptables en pharmacie de tels composés, du groupe de composés constitué par les suivants :
5-[4'-[(5-butyl-3-propyl-1H-1,2,4-triazole-1-yl)méthyl]-[1,1'-biphényl]-2-yl]-1H-tétrazole ;
5-[4'-[(3,5-dibutyl-1H-1,2,4-triazole-1-yl)méthyl]-[1,1'-biphényl]-2-yl]-1H-tétrazole ;
5-[4'-[(5-butyl-3-isopentyl-1H-1,2,4-triazole-1-yl)méthyl]-[1,1'-biphényl]-2-yl]-1H-tétrazole ;
5-[4'-[[5-butyl-3-(1,1-diméthoxybutyl)-1H-1,2,4-triazole-1-yl]méthyl]-[1,1'-biphényl]-2-yl]-1H-tétrazole ;
5-[4'-[[5-butyl-3-(1-oxobutyl)-1H-1,2,4-triazole-1-yl]méthyl]-[1,1'-biphényl]-2-yl]-1H-tétrazole ;
5-[4'-[[5-butyl-3-(1,1-difluoroéthyl)-1H-1,2,4-triazole-1-yl]méthyl]-[1,1'-biphényl]-2-yl]-1H-tétrazole ;
5-[4'-[[5-butyl-3-(1,1-difluoropropyl)-1H-1,2,4-triazole-1-yl]méthyl]-[1,1'-biphényl]-2-yl]-1H-tétrazole ;
5-[4'-[[5-butyl-3-(1,1-difluorobutyl)-1H-1,2,4-triazole-1-yl]méthyl]-[1,1'-biphényl]-2-yl]-1H-tétrazole ;
5-[4'-[[3-butyl-5-(1,1-difluorobutyl)-1H-1,2,4-triazole-1-yl]méthyl]-[1,1'-biphényl]-2-yl]-1H-tétrazole ;
5-[4'-[[5-butyl-3-(1,1-difluoropentyl)-1H-1,2,4-triazole-1-yl]méthyl]-[1,1'-biphényl]-2-yl]-1H-tétrazole ;
5-[4'-[(3,5-dipropyl-1H-1,2,4-triazole-1-yl)méthyl]-[1,1'-biphényl]-2-yl]-1H-tétrazole;
5-[4'-[(3-butyl-5-propyl-1H-1,2,4-triazole-1-yl)méthyl]-[1,1'-biphényl]-2-yl]-1H-tétrazole ;
5-[4'-[(5-butyl-3-isopentyl-1H-1,2,4-triazole-1-yl)méthyl]-[1,1'-biphényl]-2-yl]-1H-tétrazole ;
5-[4'-[(3-phényl-5-butyl-1H-1,2,4-triazole-1-yl)méthyl]-[1,1'-biphényl]-2-yl]-1H-tétrazole ;
5-[4'-[(5-butyl-3-phényléthyl-1H-1,2,4-triazole-1-yl)méthyl]-[1,1'-biphényl]-2-yl]-1H-tétrazole ;
5-[4'-[(5-butyl-3-éthyl-1H-1,2,4-triazole-1-yl)méthyl]-[1,1'-biphényl]-2-yl]-1H-tétrazole ;
5-[4'-[(3-butyl-5-éthyl-1H-1,2,4-triazole-1-yl)méthyl]-[1,1'-biphényl]-2-yl]-1H-tétrazole ;
ester 1,1-diméthyléthylique de glutamine ;
5-[4'-[[5-butyl-3-(2-cyclohexyléthyl)-1H-1,2,4-triazole-1-yl]méthyl]-[1,1'-biphényl]-2-yl]-1H-tétrazole ;
5-[4'-[[5-butyl-3-(phénylméthyl)-1H-1,2,4-triazole-1-yl]méthyl]-[1,1'-biphényl]-2-yl]-1H-tétrazole ;
5-[4'-[(5-butyl-3-cyclohexyl-1H-1,2,4-triazole-1-yl)méthyl]-[1,1'-biphényl]-2-yl]-1H-tétrazole ;
5-[4'-[(3-cyclohexylméthyl-5-butyl-1H-1,2,4-triazole-1-yl)méthyl]-[1,1'-biphényl]-2-yl]-1H-tétrazole ;
5-[4'-[(3-butyl-5-cyclohexyl-1H-1,2,4-triazole-1-yl)méthyl]-[1,1'-biphényl]-2-yl]-1H-tétrazole ;
5-[4'-[(3,5-isopropyl-1H-1,2,4-triazole-1-yl)méthyl]-[1,1'-biphényl]-2-yl]-1H-tétrazole ;
5-[4'-[(5-propyl-3-isopentyl-1H-1,2,4-triazole-1-yl)méthyl]-[1,1'-biphényl]-2-yl]-1H-tétrazole ;
5-[4'-[[5-propyl-3-(1,1-diméthoxybutyl)-1H-1,2,4-triazole-1-yl]méthyl]-[1,1'-biphényl]-2-yl]-1H-tétrazole ;
5-[4'-[[5-propyl-3-(1-oxobutyl)-1H-1,2,4-triazole-1-yl]méthyl]-[1,1'-biphényl]-2-yl]-1H-tétrazole ;
5-[4'-[[5-propyl-3-(1,1-difluoroéthyl)-1H-1,2,4-triazole-1-yl]méthyl]-[1,1'-biphényl]-2-yl]-1H-tétrazole ;
5-[4'-[[5-propyl-3-(1,1-difluoropropyl)-1H-1,2,4-triazole-1-yl]méthyl]-[1,1'-biphényl]-2-yl]-1H-tétrazole ;
5-[4'-[[5-propyl-3-(1,1-difluorobutyl)-1H-1,2,4-triazole-1-yl]méthyl]-[1,1'-biphényl]-2-yl]-1H-tétrazole ;
5-[4'-[[5-propyl-3-(1,1-difluoropentyl)-1H-1,2,4-triazole-1-yl]méthyl]-[1,1'-biphényl]-2-yl]-1H-tétrazole ;
5-[4'-[[5-éthyl-3-(1,1-difluorobutyl)-1H-1,2,4-triazole-1-yl]méthyl]-[1,1'-biphényl]-2-yl]-1H-tétrazole ;
5-[4'-[(5-sec-butyl-3-propyl-1H-1,2,4-triazole-1-yl)méthyl]-[1,1'-biphényl]-2-yl]-1H-tétrazole ;
5-[4'-[(5-sec-butyl-3-butyl-1H-1,2,4-triazole-1-yl)méthyl]-[1,1'-biphényl]-2-yl]-1H-tétrazole ;
5-[4'-[(3,5-di-sec-butyl-1H-1,2,4-triazole-1-yl)méthyl]-[1,1'-biphényl]-2-yl]-1H-tétrazole ;
5-[4'-[(5-sec-butyl-3-isopentyl-1H-1,2,4-triazole-1-yl)méthyl]-[1,1'-biphényl]-2-yl]-1H-tétrazole ;
5-[4'-[[5-sec-butyl-3-(1,1-difluoroéthyl)-1H-1,2,4-triazole-1-yl]méthyl]-[1,1'-biphényl]-2-yl]-1H-tétrazole ;
5-[4'-[[5-sec-butyl-3-(1,1-difluoropropyl)-1H-1,2,4-triazole-1-yl]méthyl]-[1,1'-biphényl]-2-yl]-1H-tétrazole ;
5-[4'-[[5-sec-butyl-3-(1,1-difluorobutyl)-1H-1,2,4-triazole-1-yl]méthyl]-[1,1'-biphényl]-2-yl]-1H-tétrazole ;
5-[4'-[[5-sec-butyl-3-(1,1-difluoropentyl)-1H-1,2,4-triazole-1-yl]méthyl]-[1,1'-biphényl]-2-yl]-1H-tétrazole ;
5-[4'-[(5-isobutyl-3-propyl-1H-1,2,4-triazole-1-yl)méthyl]-[1,1'-biphényl]-2-yl]-1H-tétrazole ;
5-[4'-[(5-isobutyl-3-butyl-1H-1,2,4-triazole-1-yl)méthyl]-[1,1'-biphényl]-2-yl]-1H-tétrazole ;
5-[4'-[(3,5-diisobutyl-1H-1,2,4-triazole-1-yl)méthyl]-[1,1'-biphényl]-2-yl]-1H-tétrazole ;
5-[4'-[(5-isobutyl-3-isopentyl-1H-1,2,4-triazole-1-yl)méthyl]-[1,1'-biphényl]-2-yl]-1H-tétrazole ;
5-[4'-[[5-isobutyl-3-(1,1-difluoroéthyl)-1H-1,2,4-triazole-1-yl]méthyl]-[1,1'-biphényl]-2-yl]-1H-tétrazole ;
5-[4'-[[5-isobutyl-3-(1,1-difluoropropyl)-1H-1,2,4-triazole-1-yl]méthyl]-[1,1'-biphényl]-2-yl]-1H-tétrazole ;
5-[4'-[[5-isobutyl-3-(1,1-difluorobutyl)-1H-1,2,4-triazole-1-yl]méthyl]-[1,1'-biphényl]-2-yl]-1H-tétrazole
5-[4'-[[5-isobutyl-3-(1,1-difluoropentyl)-1H-1,2,4-triazole-1-yl]méthyl]-[1,1'-biphényl]-2-yl]-1H-tétrazole
5-[4'-[(5-tert-butyl-3-propyl-1H-1,2,4-triazole-1-yl)méthyl]-[1,1'-biphényl]-2-yl]-1H-tétrazole ;
5-[4'-[(5-tert-butyl-3-butyl-1H-1,2,4-triazole-1-yl)méthyl]-[1,1'-biphényl]-2-yl]-1H-tétrazole ;
5-[4'-[(3,5-di-tert-butyl-1H-1,2,4-triazole-1-yl)méthyl]-[1,1'-biphényl]-2-yl]-1H-tétrazole ;
5-[4'-[(5-tert-butyl-3-isopentyl-1H-1,2,4-triazole-1-yl)méthyl]-[1,1'-biphényl]-2-yl]-1H-tétrazole ;
5-[4'-[[5-tert-butyl-3-(1,1-difluoroéthyl)-1H-1,2,4-triazole-1-yl]méthyl]-[1,1'-biphényl]-2-yl]-1H-tétrazole ;
5-[4'-[[5-tert-butyl-3-(1,1-difluoropropyl)-1H-1,2,4-triazole-1-yl]méthyl]-[1,1'-biphényl]-2-yl]-1H-tétrazole ;
5-[4'-[[5-tert-butyl-3-(1,1-dif1uorobutyl)-1H-1,2,4-triazole-1-yl]méthyl]-[1,1'-biphényl]-2-yl]-1H-tétrazole ;
5-[4'-[[5-tert-butyl-3-(1,1-difluoropentyl)-1H-1,2,4-triazole-1-yl]méthyl]-[1,1'-biphényl]-2-yl]-1H-tétrazole ;
5-[4'-[(5-pentyl-3-propyl-1H-1,2,4-triazole-1-yl)méthyl]-[1,1'-biphényl]-2-yl]-1H-tétrazole ;
5-[4'-[(5-pentyl-3-butyl-1H-1,2,4-triazole-1-yl)méthyl]-[1,1'-biphényl]-2-yl]-1H-tétrazole ;
5-[4'-[(3,5-dipentyl-1H-1,2,4-triazole-1-yl)méthyl]-[1,1'-biphényl]-2-yl]-1H-tétrazole;
5-[4'-[(5-pentyl-3-isopentyl-1H-1,2,4-triazole-1-yl)méthyl]-[1,1'-biphényl]-2-yl]-1H-tétrazole ;
5-[4'-[[5-pentyl-3-(1,1-difluoroéthyl)-1H-1,2,4-triazole-1-yl]méthyl]-[1,1'-biphényl]-2-yl]-1H-tétrazole ;
5-[4'-[[5-pentyl-3-(1,1-difluoropropyl)-1H-1,2,4-triazole-1-yl]méthyl]-[1,1'-biphényl]-2-yl]-1H-tétrazole ;
5-[4'-[[5-pentyl-3-(1,1-difluorobutyl)-1H-1,2,4-triazole-1-yl]méthyl]-[1,1'-biphényl]-2-yl]-1H-tétrazole ;
5-[4'-[[5-pentyl-3-(1,1-difluoropentyl)-1H-1,2,4-triazole-1-yl]méthyl]-[1,1'-biphényl]-2-yl]-1H-tétrazole ;
5-[4'-[(5-isopentyl-3-éthyl-1H-1,2,4-triazole-1-yl)méthyl]-[1,1'-biphényl]-2-yl]-1H-tétrazole ;
5-[4'-[(5-isopentyl-3-propyl-1H-1,2,4-triazole-1-yl)méthyl]-[1,1'-biphényl]-2-yl]-1H-tétrazole ;
5-[4'-[(5-isopentyl-3-butyl-1H-1,2,4-triazole-1-yl)méthyl]-[1,1'-biphényl]-2-yl]-1H-tétrazole ;
5-[4'-[(3,5-diisopentyl-1H-1,2,4-triazole-1-yl)méthyl]-[1,1'-biphényl]-2-yl]-1H-tétrazole ;
5-[4'-[[5-isopentyl-3-(1,1-difluoroéthyl)-1H-1,2,4-triazole-1-yl]méthyl]-[1,1'-biphényl]-2-yl]-1H-tétrazole ;
5-[4'-[[5-isopentyl-3-(1,1-difluoropropyl)-1H-1,2,4-triazole-1-yl]méthyl]-[1,1'-biphényl]-2-yl]-1H-tétrazole;
5-[4'-[[5-isopentyl-3-(1,1-difluorobutyl)-1H-1,2,4-triazole-1-yl]méthyl]-[1,1'-biphényl]-2-yl]-1H-tétrazole;
5-[4'-[[5-isopentyl-3-(1,1-difluoropentyl)-1H-1,2,4-triazole-1-yl]méthyl]-[1,1'-biphényl]-2-yl]-1H-tétrazole;
5-[4'-[[5-(1-butényl)-3-propyl-1H-1,2,4-triazole-1-yl]méthyl]-[1,1'-biphényl]-2-yl]-1H-tétrazole ;
5-[4'-[[5-(1-butényl)-3-butyl-1H-1,2,4-triazole-1-yl]méthyl]-[1,1'-biphényl]-2-yl]-1H-tétrazole ;
5-[4'-[[5-(1-butényl)-3-isopentyl-1H-1,2,4-triazole-1-yl]méthyl]-[1,1'-biphényl]-2-yl]-1H-tétrazole ;
5-[4'-[[5-(1-butényl)-3-(1,1-diméthoxybutyl)-1H-1,2,4-triazole-1-yl]méthyl]-[1,1'-biphényl]-2-yl]-1H-tétrazole ;
5-[4'-[[5-(1-butényl)-3-(1,1-difluorobutyl)-1H-1,2,4-triazole-1-yl]méthyl]-[1,1'-biphényl]-2-yl]-1H-tétrazole ;
5-[4'-[[5-(2-butényl)-3-(1,1-difluorobutyl)-1H-1,2,4-triazole-1-yl]méthyl]-[1,1'-biphényl]-2-yl]-1H-tétrazole ;
5-[4'-[[5-(3-butényl)-3-(1,1-difluorobutyl)-1H-1,2,4-triazole-1-yl]méthyl]-[1,1'-biphényl]-2-yl]-1H-tétrazole ;
5-[4'-[[5-(1-butynyl)-3-(1,1-difluorobutyl)-1H-1,2,4-triazole-1-yl]méthyl]-[1,1'-biphényl]-2-yl]-1H-tétrazole ;
5-[4'-[[5-(2-butynyl)-3-(1,1-difluorobutyl)-1H-1,2,4-triazole-1-yl]méthyl]-[1,1'-biphényl]-2-yl]-1H-tétrazole ;
5-[4'-[[5-(3-butynyl)-3-(1,1-difluorobutyl)-1H-1,2,4-triazole-1-yl]méthyl]-[1,1'-biphényl]-2-yl]-1H-tétrazole ;
5-[4'-[[3,5-di(l-butényl)-1H-1,2,4-triazole-1-yl]méthyl]-[1,1'-biphényl]-2-yl]-1H-tétrazole ;
5-[4'-[[3,5-di(2-butényl)-1H-1,2,4-triazole-1-yl]méthyl]-[1,1'-biphényl]-2-yl]-1H-tétrazole ;
5-[4'-[[3,5-di(3-butényl)-1H-1,2,4-triazole-1-yl]méthyl]-[1,1'-biphényl]-2-yl]-1H-tétrazole ;
5-[4'-[[3,5-di(1-butynyl)-1H-1,2,4-triazole-1-yl]méthyl]-[1,1'-biphényl]-2-yl]-1H-tétrazole ;
5-[4'-[[3,5-di(2-butynyl)-1H-1,2,4-triazole-1-yl]méthyl]-[1,1'-biphényl]-2-yl]-1H-tétrazole; et
5-[4'-[[3,5-di(3-butynyl)-1H-1,2,4-triazole-1-yl]méthyl]-[1,1'-biphényl]-2-yl]-1H-tétrazole.

5. Composé selon la revendication 1, choisi parmi les composés, et les sels acceptables en pharmacie de tels composés, du groupe de composés constitué par les suivants :
acide 4'-[(3,5-dibutyl-1H-1,2,4-triazole-1-yl)méthyl]-[1,1'-biphényl]-2-carboxylique ;
acide 4'-[(3-éthyl-5-butyl-1H-1,2,4-triazole-1-yl)méthyl]-[1,1'-biphényl]-2-carboxylique ;
acide 4'-[(3-butyl-5-éthyl-1H-1,2,4-triazole-1-yl)méthyl]-[1,1'-biphényl]-2-carboxylique ;
acide 4'-[(5-butyl-1H-1,2,4-triazole-1-yl)méthyl]-[1,1'-biphényl]-2-carboxylique;
acide 4'-[(3-butyl-1H-1,2,4-triazole-1-yl)méthyl]-[1,1'-biphényl]-2-carboxylique.

6. Procédé pour préparer une composition pharmaceutique, caractérisé par le mélange d'une quantité, efficace du point de vue thérapeutique, d'un composé antagoniste de l'angiotensine II et d'un véhicule ou diluant acceptable en pharmacie, ledit composé antagoniste étant choisi parmi une famille de composés selon l'une quelconque des revendications 1 à 5 ou les amides de composés dans lesquels R³ = COOH, ou les sels de tels composés.

7. Utilisation d'un composé selon l'une quelconque des revendications 1 à 5 ou d'amides de composés dans lesquels R³ = COOH ou de sels de tels composés, pour préparer un médicament destiné au traitement d'un trouble circulatoire.

8. Utilisation selon la revendication 7, dans laquelle ledit trouble est un trouble cardio-vasculaire.

9. Utilisation selon la revendication 8, dans laquelle ledit trouble cardio-vasculaire est l'hypertension.

10. Utilisation selon la revendication 8, dans laquelle ledit trouble cardio-vasculaire est l'insuffisance cardiaque congestive.

11. Utilisation d'un composé selon l'une quelconque des revendications 1 à 5, ou des amides de composés dans lesquels R³ = COOH, ou des sels de tels composés, pour préparer un médicament destiné à traiter un glaucome.

12. Composé, qui est le 4'-[(3,5-dibutyl-1H-1,2,4-triazole-1-yl)méthyl]-[1,1'-biphényl]-2-carboxylate de méthyle ou l'hydrazide de l'acide 4'-[(3,5-dibutyl-1H-1,2,4-triazole-1-yl)méthyl]-[1,1'-biphényl]-2-carboxylique.
